# EUROPEAN PATENT APPLICATION

(11) **EP 1 985 297 A1**
(43) Date of publication of application: **29.10.2008**
(21) Application number: 07707492.0
(22) Date of filing: 26.01.2007
(51) Int. Cl.: A61K 31/538, A61K 31/5415, A61P 13/02, A61P 13/04, A61P 19/02, A61P 19/06, A61P 43/00, A61P 9/10, C07D 265/34, C07D 265/36, C07D 279/16

(54) **CARBOXYLIC ACID COMPOUND AND USE THEREOF**

(30) Priority: 27.01.2006 JP 2006019800; 10.02.2006 US 772218 P
(71) Applicant: Japan Tobacco, Inc., Tokyo 105-8422 (JP)
(72) Inventor: INOUE, Teruhiko, Takatsuki-shi Osaka 569-1125 (JP); KIGUCHI, Toshihiro, Takatsuki-shi Osaka 569-1125 (JP); HIRATA, Kazuyuki, Takatsuki-shi Osaka 569-1125 (JP); SHINAGAWA, Yuko, Takatsuki-shi Osaka 569-1125 (JP); OGAWA, Naoki, Takatsuki-shi Osaka 569-1125 (JP); DEAI, Katsuya, Takatsuki-shi Osaka 569-1125 (JP)
(74) Representative: Vossius & Partner
(86) International application number: PCT/JP2007/051260
(87) International publication number: WO 2007/086504

(57) **Abstract**

Provision of a superior URAT1 activity inhibitor effective for the treatment and the like of a pathology involving uric acid, such as hyperuricemia, gouty tophus, acute gouty arthritis, chronic gouty arthritis, gouty kidney, urinary lithiasis, renal dysfunction, coronary heart disease, ischemic cardiac diseases and the like.

A URAT1 activity inhibitor containing a compound represented by the following formula [1] or a pharmaceutically acceptable salt thereof, or a solvate thereof as an active ingredient: 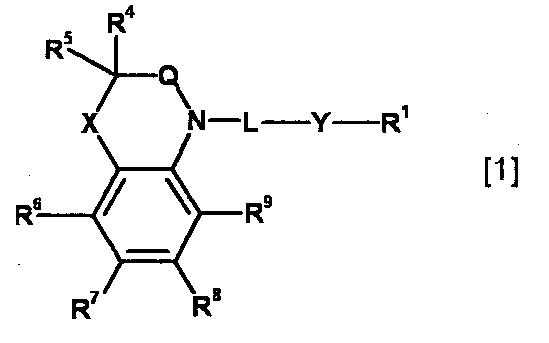 wherein each symbol is as defined in the specification.

## Description

### [Technical Field]

The present invention relates to a carboxylic acid compound and use thereof.

### [Background Art]

Uric acid is a substance having a molecular weight of 168 and a dissociation constant (pKa value) of 5.75, which is present in the form of uric acid or a conjugate base (urate) thereof in the body fluid depending on the pH of the body fluid. In human, since the function of urate oxidase (uricase) in the liver is deficient due to mutation, uric acid is the final metabolite in a purine form. To be specific, dietarily or endogenously produced purine form becomes inosine from adenosine, then hypoxanthine, and then xanthine, or becomes guanine from guanosine, and then xanthine, and this xanthine is subject to oxidation by xanthine oxidase or xanthine dehydrogenase to become uric acid. Uric acid is mainly excreted from the kidney.

When hyperuricemia becomes severe and the blood uric acid level exceeds the upper limit of solubility, sodium urate crystal is formed in the cartilage tissues and joints and then sediments called gouty tophus (tophi) are produced. This gouty tophus causes acute gouty arthritis, which progresses into chronic gouty arthritis. Besides these, it has been clarified that nephropathy (gouty kidney) and urolithiasis occur as complications of sodium urate crystal deposition due to hyperuricemia, and that hyperuricemia itself induces renal function disorder (see non-patent reference 1).

Many hyperuricemia patients have complications such as hyperlipidemia, diabetes, hypertension, obesity and the like. While these complications by themselves are each a risk factor for coronary arterial diseases and death rates, hyperuricemia patients have long been known to show significantly high complication rate of coronary artery diseases and short survival, as compared to patient groups having normal blood uric acid level. Fang et al. conducted a large-scale investigation on the death rates of coronary artery disease in 5926 cases ranging from 25 to 74 years old, whose blood uric acid level was measured during the period of 22 years from 1971 to 1992, and clarified that increased blood uric acid level alone can be a risk for ischemic heart diseases (see non-patent reference 2). It has also been reported that a treatment aiming at decreasing the blood uric acid level itself, along with the treatment of complications, is useful for preventing the onset of and decreasing the death rates of coronary artery disease, and a treatment to decrease the blood uric acid level should be positively employed also for asymptomatic hyperuricemia (see non-patent reference 3). Recently, it has become determinative that hyperuricemia associated with hypertension is an independent risk factor for cardiovascular diseases (see non-patent reference 4). According to non-patent reference 5, moreover, it has also been described that 1) nephropathy (also referred to as gouty kidney) and urolithiasis, which are complications associated with sodium urate crystal deposition, highly frequently complicated with gout and hyperuricemia, 2) control of blood uric acid level is important from the aspect of prevention of a recurrence of cerebral or cardiovascular incidents, 3) patients with hyperuricemia or gout show frequent complication of hyperlipidemia, 4) obesity should not be ignored as etiology or exacerbation factor of hyperuricemia, 5) uricosuric agents are basically used for decreased uric acid excretion hyperuricemia, 6) hyperuricemia associated with hypertension is highly likely an independent risk factor of cardiovascular incidents, and the like. Therefore, it is almost certain that decreasing the blood uric acid level is not the only effective measure for the prophylaxis or treatment of the above-mentioned diseases, but so is combined use of a pharmaceutical agent that decreases the blood uric acid level with therapeutic or prophylactic agents for these above-mentioned diseases.

While uric acid is mainly excreted from the kidney, uric acid in blood is once filtered off almost completely by renal glomerulus, after which uric acid is mostly reabsorbed by proximal renal tubule. Therefore, only a small amount of uric acid is excreted into urine. The reabsorption of uric acid in proximal renal tubule has been clarified to be a transport via a transporter by an experiment using membrane vesicle prepared from renal cortex (see non-patent reference 6), and its substrate selectivity, inhibitors thereof and the like have also been elucidated (see non-patent references 7 and 8).

In recent years, a gene encoding a human kidney uric acid transporter (SLC22A12) has been identified (see non-patent reference 9). The transporter (urate transporter 1, URAT1) encoded by this gene is a 12-spanning transmembrane molecule belonging to the organic anion transporter (OAT) family, and Northern blot using the full-length cDNA thereof as a probe has revealed that it specifically expresses in adult and embryo kidneys. It has been also confirmed by immunostaining of human renal tissue section conducted using a polyclonal antibody specific to C-terminal peptide thereof that it is localized on the lumen of the proximal renal tubule in the cortex. In an experiment using a Xenopus laevis oocyte expression system, uric acid uptake via URAT1 increased in a time-dependent manner, and the uric acid uptake showed saturation at high uric acid concentration, which is characteristic of carrier transport. Moreover, it has been clarified that the uptake is based on the exchange with organic anion such as lactic acid, pyrazine carboxylic acid, nicotinic acid and the like, and that the uptake is inhibited by uricosuric agents such as probenecid, benzbromarone and the like, and URAT1 has been demonstrated to be the transporter being elucidated by experiments using the above-mentioned membrane vesicle (see non-patent reference 10). In other words, URAT1 has been clarified to be a main transporter responsible for reabsorption of uric acid in the kidney.

Furthermore, gene mutations of URAT1 have been identified by gene analysis of idiopathic renal hypouricemia patients, and when these mutant URAT1 were expressed in Xenopus laevis oocyte, the uric acid transport activity had been lost. These facts also clarify that URAT1 is involved in the control of blood uric acid level (see the aforementioned non-patent reference 9).

With regard to the relationship between URAT1 and diseases, moreover, many reports have documented that probenecid and benzbromarone that inhibit the uric acid transport activity of URAT1 are therapeutic drugs for hyperuricemia, and useful as drugs for the prophylaxis or treatment of pathology exhibiting high blood uric acid level, such as hyperuricemia, gouty tophus, gout arthritis, gouty kidney, urolithiasis and renal function disorder (see non-patent reference 11).
Furthermore, since some of the drugs of nucleic acid metabolic antagonists, hypotensive diuretics, antituberculosis, anti-inflammatory analgesic drugs, hyperlipidemic drugs, therapeutic drugs for asthma, immunosuppressants and the like increase blood uric acid level, thus creating the problems of progress into or exacerbation of pathology caused by increase in the above-mentioned blood uric acid level.

Therefore, a substance having an inhibitory action on URAT1 activity would be useful as a drug for the prophylaxis or treatment of pathology suggesting the involvement of uric acid, such as pathology suggesting the involvement of high blood uric acid level, specifically, hyperuricemia, gouty tophus, gout arthritis, gouty kidney, urolithiasis, renal function disorder and the like, and further as a drug for the prophylaxis or treatment of hyperlipidemia, diabetes, obesity or cardiovascular diseases (e.g., hypertension, coronary arterial disease, vascular endothelial disorder, ischemic heart disease etc.) because it decreases the blood uric acid level. A concurrent use of these other prophylactic or therapeutic drugs with the substance having an inhibitory action on URAT1 activity would be useful for more effective prophylaxis or treatment of these diseases.

A substance having an inhibitory action on URAT1 activity can be said to be useful because it can prevent increase in the blood uric acid level when concurrently used together with a pharmaceutical agent that increases the blood uric acid level, such as nucleic acid metabolic antagonist, hypotensive diuretic, anti-tuberculosis, anti-inflammatory analgesic drugs, hyperlipidemic drugs, therapeutic drugs for asthma, immunosuppressants and the like.

As the background art, the following compounds are known. For example, patent reference 1 describes, as a compound having an aldose reductase inhibitory activity and a platelet coagulation suppressive action, a compound represented by the following formula:

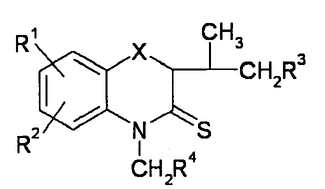

wherein each symbol is as defined in the reference.

Patent reference 2 describes, as a compound having a serine protease inhibitory action, a compound represented by the following formula:

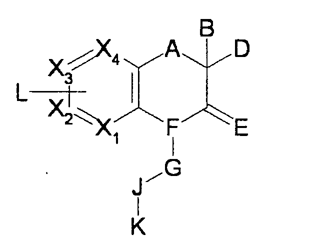

wherein each symbol is as defined in the reference.

Patent reference 3 describes, as a compound inhibiting matrix metal protease, a compound represented by the following formula:

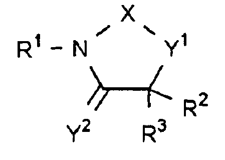

wherein each symbol is as defined in the reference.

Patent reference 4 describes, as a compound inhibiting peptide deformylase (PDF), a compound represented by the following formula:

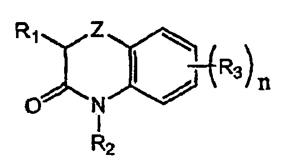

wherein each symbol is as defined in the reference.

Patent reference 5 describes, as a compound having a potassium channel activating action, a compound represented by the following formula:

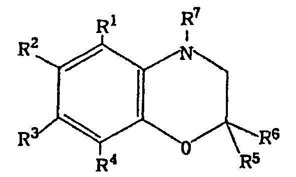

wherein each symbol is as defined in the reference, and

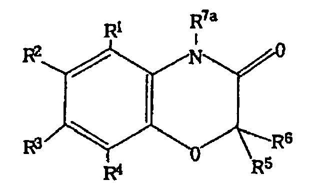

wherein each symbol is as defined in the reference.

Non-patent reference 12 describes, as a potassium channel opener, a compound represented by the following formula:

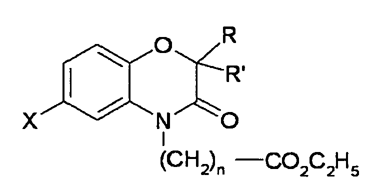

wherein each symbol is as defined in the reference.

Patent reference 6 describes a compound represented by the following formula and the like, which acts on a retinoic acid receptor and a retinoid X receptor:

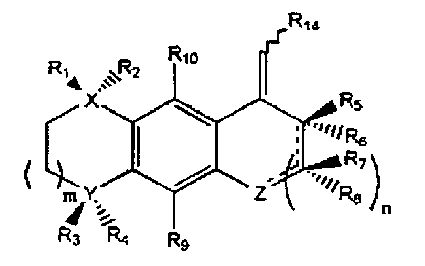

wherein R₁₄ is the formula:

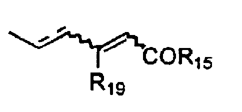

and the like; and other symbols are as defined in the reference.

Patent reference 7 describes, as a compound useful as an intermediate for a dye, a compound represented by the following formula:

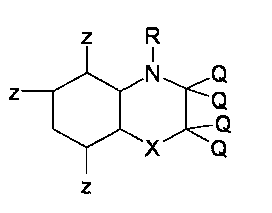

wherein each symbol is as defined in the reference.

Patent reference 8 describes a uric acid excretion agent comprising a compound represented by the following formula:

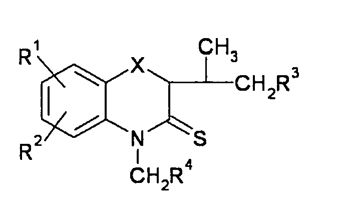

wherein each symbol is as defined in the reference.

For example, patent reference 9 describes, as a compound showing a uric acid excretion promoting action, the following compound, or benzbromarone.

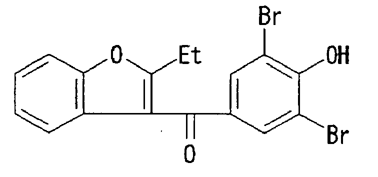

However, this compound has a different structure from that of the compound of the present invention, and no description suggestive of the compound of the present invention can be found.

[patent reference 1] JP-A-63-107970
[patent reference 2] National Publication of Translated Version No. 2002-509925
[patent reference 3] National Publication of Translated Version No. 2002-542238
[patent reference 4] WO2005/011611
[patent reference 5] EP0432893
[patent reference 6] WO96/20914
[patent reference 7] USP2374181
[patent reference 8] JP-A-3-135917
[patent reference 9] USP265559
[non-patent reference 1] Johnson RJ, Kivilighn SD, Kim YG et al. Reappraisal of the pathogenesis and consequences of hyperuricemia in hypertension, cardiovascular disease, and renal disease. Am. J. Kidney Dis. 33,225-234(1999)
[non-patent reference 2] Fang J., Alderman MH. JAMA 283(18)2404-2410(2000)
[non-patent reference 3] Tohru Nakamura, Hyperuricemia and Gout, 9(1) 61-65 (2001)
[non-patent reference 4] Ichiro Hisatome, Nikkei Medical, January 2004, 100-101
[non-patent reference 5] Guideline for the management of hyperuricemia and gout (1st Edit.), Gout and Nucleic acid metabolism, vol. 26, suppl. 1 (2002), Japanese Society of Gout and Nucleic Acid Metabolism
[non-patent reference 6] Sica DA and Schoolwerth AC. The Kidney, 6th edition, pp. 680-700, Saunders, Philadelphia (2000)
[non-patent reference 7] Roch-Ramel F., Werner D., and Guisan B. Am. J. Physiol. 266, F797-F805 (1994)
[non-patent reference 8] Roch-Ramel F., Guisan B., and Diezi J. J. Pharmacol. Exp. Ther. 280, 839-845(1997)
[non-patent reference 9] Enomoto A. et al., Nature 417, 447-452 (2002)
[non-patent reference 10] Naohiko Anzai et. al., Biochemical 76(2) 101-110 (2004)
[non-patent reference 11] Hisashi Yamanaka, Diagnosis and Treatment, vol. 92, No. 1, 125-128 (2004)
[non-patent reference 12] Goiseppe C. et al., Bioorganic&Medicinal Chemistry,10,2663-2669(2002)

### [Disclosure of the Invention]

### [Problems to be Solved by the Invention]

At present, as an agent for the prophylaxis or treatment of hyperuricemia, a uricosuric agent, benzbromarone, having an inhibitory action on URAT1 activity is used. However, the inhibitory action on URAT1 activity of benzbromarone is not sufficient. Therefore, there is a strong demand for the development of an agent for the prophylaxis or treatment of hyperuricemia, which has more potent inhibitory action on URAT1 activity. The present invention aims at providing a novel agent for the prophylaxis or treatment of hyperuricemia, which has more potent activity as compared to the conventional URAT1 activity inhibitors, namely, a URAT1 activity inhibitor and the like.

### [Means of Solving the Problems]

The present inventors have conducted intensive studies in an attempt to develop a new agent for the prophylaxis or treatment of hyperuricemia, which takes the place of conventional agents for the prophylaxis or treatment of hyperuricemia, and found a carboxylic acid compound having a superior inhibitory action on URAT1 activity, which resulted in the completion of the present invention.

Accordingly, the present invention provides the following.
<1>
A URAT1 activity inhibitor comprising a compound represented by the following formula [1] or a pharmaceutically acceptable salt thereof, or a solvate thereof

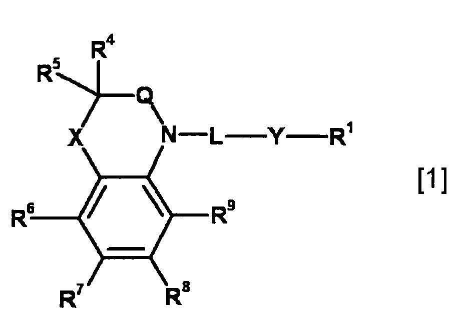

wherein
Q is
1) -CH₂- or
2) -(C=Z)-:
Z is
1) an oxygen atom, or
2) a sulfur atom:
   X is

1) an oxygen atom,
2) a sulfur atom,
3) -S(=O)- or
4) -S(=O)₂-:
L is
1) a single bond, or
2) -(C=O)-:
Y is
1) a single bond,
2) a C₁₋₄ alkylene group optionally substituted by the same or different one or more substituents selected from the following group A,
3) a C₂₋₄ alkenylene group optionally substituted by the same or different one or more substituents selected from the following group A,
4) a C₂₋₄ alkynylene group optionally substituted by the same or different one or more substituents selected from the following group A, or
5) a C₁₋₄ alkylene-C₆₋₁₄ arylene group optionally substituted by the same or different one or more substituents selected from the following group A:
R¹ is
1) -COOH, or
2) -COOR wherein R is
   (a) a C₁₋₆ alkyl group optionally substituted by the same or different one or more substituents selected from the following group A,
   (b) an allyl group optionally substituted by the same or different one or more substituents selected from the following group A,
   (c) a phenyl group optionally substituted by the same or different one or more substituents selected from the following group A, or
   (d) a benzyl group optionally substituted by the same or different one or more substituents selected from the following group A;
R⁴ and R⁵ are the same or different and each is
1) a hydrogen atom,
2) a C₁₋₆ alkyl group optionally substituted by the same or different one or more substituents selected from the following group A,
3) a C₂₋₆ alkenyl group optionally substituted by the same or different one or more substituents selected from the following group A,
4) a C₆₋₁₄ aryl group optionally substituted by the same or different one or more substituents selected from the following group A,
5) a C₇₋₁₂ aralkyl group optionally substituted by the same or different one or more substituents selected from the following group A, or
6) a halogen atom, or
7) R⁴ and R⁵ form, together with the carbon atom bonded thereto, a saturated carbocycle having 3 to 6 carbon atoms (said carbocycle is optionally substituted by the same or different one or more substituents selected from the following group A); R⁶, R⁷, R⁸ and R⁹ are the same or different and each is

1) a hydrogen atom,
2) a halogen atom,
3) a C₁₋₆ alkyl group optionally substituted by the same or different one or more substituents selected from the following group A,
4) a C₁₋₆ alkoxy group optionally substituted by the same or different one or more substituents selected from the following group A,
5) a C₃₋₈ cycloalkyl group optionally substituted by the same or different one or more substituents selected from the following group A,
6) a C₆₋₁₄ aryl group optionally substituted by the same or different one or more substituents selected from the following group A,
7) a nitro group,
8) a cyano group,
9) -COOH, or
10) -COOR wherein R is as defined above, or
11) R⁶ and R⁷, R⁷ and R⁸ or R⁸ and R⁹ form, together with the carbon atom bonded thereto, a saturated or unsaturated carbocycle having 3 to 14 carbon atoms (said carbocycle is optionally substituted by the same or different one or more substituents selected from the following group A), provided that when
Q is -(C=Z)-,
Z is a sulfur atom,
L is a single bond, and
one of R⁴ and R⁵ is a hydrogen atom, and the other is an isopropyl group or a sec-butyl group,
Y is
1) a single bond,
2) a C₂₋₄ alkylene group optionally substituted by the same or different one or more substituents selected from the following group A,
3) a C₂₋₄ alkenylene group optionally substituted by the same or different one or more substituents selected from the following group A,
4) a C₂₋₄ alkynylene group optionally substituted by the same or different one or more substituents selected from the following group A, or
5) a C₁₋₄ alkylene-C₆₋₁₄ arylene group optionally substituted by the same or different one or more substituents selected from the following group A:
[group A]
1) a halogen atom,
2) -OR¹⁰ wherein R¹⁰ is
   (a) a hydrogen atom,
   (b) a C₁₋₆ alkyl group optionally substituted by the same or different one or more substituents selected from the following group B, or
   (c) -COR¹¹ wherein R¹¹ is
      a) a hydrogen atom,
      b) a hydroxyl group,
      c) a C₁₋₆ alkyl group optionally substituted by the same or different one or more substituents selected from the following group B,
      d) a C₁₋₆ alkoxy group (said C₁₋₆ alkoxy group is optionally substituted by the same or different one or more substituents selected from the following (i) and (ii),
         (i) a substituent selected from the following group B,
         (ii) a C₁₋₆ alkyl group optionally substituted by the same or different one or more substituents selected from the following group B),
      e) a saturated or unsaturated carbocyclic group having 3 to 14 carbon atoms (said carbocyclic group is optionally substituted by the same or different one or more substituents selected from the following (i) and (ii),
         (i) a substituent selected from the following group B,
         (ii) a C₁₋₆ alkyl group optionally substituted by the same or different one or more substituents selected from the following group B),
      f) a cycloalkylalkoxy group (said cycloalkylalkoxy group is optionally substituted by the same or different one or more substituents selected from the following (i) and (ii),
         (i) a substituent selected from the following group B,
         (ii) a C₁₋₆ alkyl group optionally substituted by the same or different one or more substituents selected from the following group B),
      g) a C₇₋₁₂ aralkyl group (said C₇₋₁₂ aralkyl group is optionally substituted by the same or different one or more substituents selected from the following (i) and (ii),
         (i) a substituent selected from the following group B,
         (ii) a C₁₋₆ alkyl group optionally substituted by the same or different one or more substituents selected from the following group B), or
      h) an aralkoxy group (said aralkoxy group is optionally substituted by the same or different one or more substituents selected from the following (i) and (ii),
         (i) a substituent selected from the following group B,
         (ii) a C₁₋₆ alkyl group optionally substituted by the same or different one or more substituents selected from the following group B),
3) a C₁₋₆ alkyl group optionally substituted by the same or different one or more substituents selected from the following group B,
4) a cycloalkylalkoxy group (said cycloalkylalkoxy group is optionally substituted by the same or different one or more substituents selected from the following (a) and (b),
   (a) a substituent selected from the following group B,
   (b) a C₁₋₆ alkyl group optionally substituted by the same or different one or more substituents selected from the following group B),
5) a C₇₋₁₂ aralkyl group (said C₇₋₁₂ aralkyl group is optionally substituted by the same or different one or more substituents selected from the following (a) and (b),
   (a) a substituent selected from the following group B,
   (b) a C₁₋₆ alkyl group optionally substituted by the same or different one or more substituents selected from the following group B),
6) an aralkoxy group (said aralkoxy group is optionally substituted by the same or different one or more substituents selected from the following (a) and (b),
   (a) a substituent selected from the following group B,
   (b) a C₁₋₆ alkyl group optionally substituted by the same or different one or more substituents selected from the following group B),
7) -COR¹¹ wherein R¹¹ is as defined above,
8) -NR¹²R¹³ wherein R¹² and R¹³ are the same or different and each is
   (a) a hydrogen atom, or
   (b) a C₁₋₆ alkyl group optionally substituted by the same or different one or more substituents selected from the following group B, or
   (c) R¹² and R¹³ optionally form, together with the nitrogen atom bonded thereto, monocyclic nitrogen-containing saturated heterocycle (said heterocycle is optionally substituted by the same or different one or more substituents selected from the following (i) and (ii),
      (i) a substituent selected from the following group B,
      (ii) a C₁₋₆ alkyl group optionally substituted by the same or different one or more substituents selected from the following group B),
9) -CONR¹²R¹³ wherein R¹² and R¹³ is as defined above,
10) -NR¹⁴COR¹¹ wherein R¹¹ is as defined above, R¹⁴ is
   (a) a hydrogen atom, or
   (b) a C₁₋₆ alkyl group optionally substituted by the same or different one or more substituents selected from the following group B,
11) -NR¹⁴S(=O)₂R¹¹ wherein R¹¹ and R¹⁴ are as defined above,
12) -NR¹⁴CONR¹²R¹³ wherein R¹², R¹³ and R¹⁴ are as defined above,
13) -SR¹⁰ wherein R¹⁰ is as defined above,
14) -S(=O)R¹¹ wherein R¹¹ is as defined above,
15) -S(=O)₂R¹¹ wherein R¹¹ is as defined above,
16) -S(=O)₂NR¹²R¹³ wherein R¹² and R¹³ are as defined above,
17) a saturated or unsaturated carbocyclic group having 3 to 14 carbon atoms (said carbocyclic group is optionally substituted by the same or different one or more substituents selected from the following (a) and (b),
   (a) a substituent selected from the following group B,
   (b) a C₁₋₆ alkyl group optionally substituted by the same or different one or more substituents selected from the following group B),
18) a saturated or unsaturated heterocyclic group containing at least one hetero atom selected from a nitrogen atom, an oxygen atom and a sulfur atom (said heterocyclic group is optionally substituted by the same or different one or more substituents selected from the following (a) and (b),
   (a) a substituent selected from the following group B,
   (b) a C₁₋₆ alkyl group optionally substituted by the same or different one or more substituents selected from the following group B,
19) an aryloxy group (said aryloxy group is optionally substituted by the same or different one or more substituents selected from the following (a) and (b),
   (a) a substituent selected from the following group B,
   (b) a C₁₋₆ alkyl group optionally substituted by the same or different one or more substituents selected from the following group B,
20) a cyano group, and
21) a nitro group
[group B]
1) a halogen atom,
2) a hydroxyl group,
3) a C₁₋₆ alkoxy group,
4) -NR¹⁵R¹⁶ wherein R¹⁵ and R¹⁶ are the same or different and each is
   (a) a hydrogen atom, or
   (b) a C₁₋₆ alkyl group, or
   (c) R¹⁵ and R¹⁶ optionally form, together with the nitrogen atom bonded thereto, monocyclic nitrogen-containing saturated heterocycle,
5) -CONR¹⁵R¹⁶ wherein R¹⁵ and R¹⁶ are as defined above,
6) -COR¹⁷ wherein R¹⁷ is
   (a) a hydrogen atom,
   (b) a hydroxyl group,
   (c) a C₁₋₆ alkyl group, or
   (d) a C₁₋₆ alkoxy group,
7) -NR¹⁸COR¹⁷ wherein R¹⁷ is as defined above, R¹⁸ is
   (a) a hydrogen atom, or
   (b) a C₁₋₆ alkyl group,
8) -NR¹⁸CONR¹⁵R¹⁶ wherein R¹⁵, R¹⁶ and R¹⁸ are as defined above,
9) -NR¹⁸S(=O)₂R¹⁹ wherein R¹⁸ is as defined above, R¹⁹ is a C₁₋₆ alkyl group, and
10) -S(=O)₂R¹⁹ wherein R¹⁹ is as defined above.

<2> A compound represented by the following formula [1'] or a pharmaceutically acceptable salt thereof, or a solvate thereof:

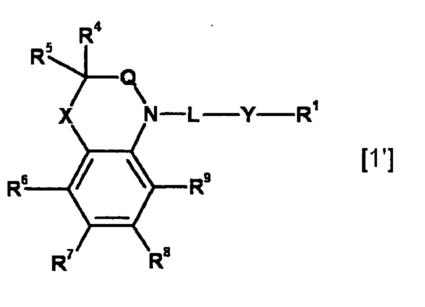

wherein
Q is
1) -CH₂- or
2) -(C=Z)-:
Z is
1) an oxygen atom, or
2) a sulfur atom:
X is
1) an oxygen atom,
2) a sulfur atom,
3) -S(=O)- or
4) -S(=O)₂-:
L is
1) a single bond, or
2) -(C=O)-:
Y is
1) a single bond,
2) a C₁₋₄ alkylene group optionally substituted by the same or different one or more substituents selected from the following group A,
3) a C₂₋₄ alkenylene group optionally substituted by the same or different one or more substituents selected from the following group A, or
4) a C₁₋₄ alkylene-C₆₋₁₄ arylene group optionally substituted by the same or different one or more substituents selected from the following group A:
R¹ is
1) -COOH, or
2) -COOR wherein R is
   (a) a C₁₋₆ alkyl group optionally substituted by the same or different one or more substituents selected from the following group A,
   (b) an allyl group optionally substituted by the same or different one or more substituents selected from the following group A,
   (c) a phenyl group optionally substituted by the same or different one or more substituents selected from the following group A, or
   (d) a benzyl group optionally substituted by the same or different one or more substituents selected from the following group A;
R⁴ and R⁵ are the same or different and each is
1) a hydrogen atom,
2) a C₁₋₆ alkyl group optionally substituted by the same or different one or more substituents selected from the following group A,
3) a C₂₋₆ alkenyl group optionally substituted by the same or different one or more substituents selected from the following group A,
4) a C₆₋₁₄ aryl group optionally substituted by the same or different one or more substituents selected from the following group A,
5) a C₇₋₁₂ aralkyl group optionally substituted by the same or different one or more substituents selected from the following group A, or
6) a halogen atom, or
7) R⁴ and R⁵ form, together with the carbon atom bonded thereto, a saturated carbocycle having 3 to 6 carbon atoms (said carbocycle is optionally substituted by the same or different one or more substituents selected from the following group A); R⁶, R⁷, R⁸ and R⁹ are the same or different and each is
   1) a hydrogen atom,
   2) a halogen atom,
   3) a C₁₋₆ alkyl group optionally substituted by the same or different one or more substituents selected from the following group A,
   4) a C₁₋₆ alkoxy group optionally substituted by the same or different one or more substituents selected from the following group A,
   5) a C₃₋₈ cycloalkyl group optionally substituted by the same or different one or more substituents selected from the following group A,
   6) a C₆₋₁₄ aryl group optionally substituted by the same or different one or more substituents selected from the following group A,
   7) a nitro group,
8) a cyano group,
9) -COOH, or
10) -COOR wherein R is as defined above, or
11) R⁶ and R⁷, R⁷ and R⁸ or R⁸ and R⁹ form, together with the carbon atom bonded thereto, a saturated or unsaturated carbocycle having 3 to 14 carbon atoms (said carbocycle is optionally substituted by the same or different one or more substituents selected from the following group A), provided that when
   (i) Q is -(C=Z)-, and
L is a single bond,
Y is
1) a single bond,
2) a C₂₋₄ alkylene group optionally substituted by the same or different one or more substituents selected from the following group A,
3) a C₂₋₄ alkenylene group optionally substituted by the same or different one or more substituents selected from the following group A, or
4) a C₁₋₄ alkylene-C₆₋₁₄ arylene group optionally substituted by the same or different one or more substituents selected from the following group A, and
R⁵ is
1) a C₂₋₆ alkyl group optionally substituted by the same or different one or more substituents selected from the following group A,
2) a C₂₋₆ alkenyl group optionally substituted by the same or different one or more substituents selected from the following group A,
3) a C₆₋₁₄ aryl group optionally substituted by the same or different one or more substituents selected from the following group A,
4) a C₇₋₁₂ aralkyl group optionally substituted by the same or different one or more substituents selected from the following group A, or
5) a halogen atom, or
6) R⁴ and R⁵ form, together with the carbon atom bonded thereto, a saturated carbocycle having 3 to 6 carbon atoms (said carbocycle is optionally substituted by the same or different one or more substituents selected from the following group A); (ii) the following compounds are excluded:
   3-[2-(4-methoxybenzyl)-3-oxo-2,3-dihydrobenzo[1,4]thiazin-4-yl]propionic acid,
   (E)-4-(2,2-dimethyl-6-nitro-2,3-dihydrobenzo[1,4]oxazin-4-yl)-4-oxo-2-butenoic acid,
   (6-methoxy-8-methyl-2,3-dihydrobenzo[1,4]oxazin-4-yl)acetic acid,
   (2,2-dimethyl-6-nitro-2,3-dihydrobenzo[1,4]oxazin-4-yl)acetic acid, and
   (2E,4E)-3-methyl-6-oxo-6-(6,6,9,9-tetramethyl-2,3,6,7,8,9-hexahydronaphtho[2,3-b][1,4]oxazin-4-yl)2,4-hexadienoic acid,
[group A]
1) a halogen atom,
2) -OR¹⁰ wherein R¹⁰ is
   (a) a hydrogen atom,
   (b) a C₁₋₆ alkyl group optionally substituted by the same or different one or more substituents selected from the following group B, or
   (c) -COR¹¹ wherein R¹¹ is
      a) a hydrogen atom,
      b) a hydroxyl group,
      c) a C₁₋₆ alkyl group optionally substituted by the same or different one or more substituents selected from the following group B,
      d) a C₁₋₆ alkoxy group (said C₁₋₆ alkoxy group is optionally substituted by the same or different one or more substituents selected from the following (i) and (ii),
         (i) a substituent selected from the following group B,
         (ii) a C₁₋₆ alkyl group optionally substituted by the same or different one or more substituents selected from the following group B,
      e) a saturated or unsaturated carbocyclic group having 3 to 14 carbon atoms (said carbocyclic group is optionally substituted by the same or different one or more substituents selected from the following (i) and (ii),
         (i) a substituent selected from the following group B,
         (ii) a C₁₋₆ alkyl group optionally substituted by the same or different one or more substituents selected from the following group B,
      f) a cycloalkylalkoxy group (said cycloalkylalkoxy group is optionally substituted by the same or different one or more substituents selected from the following (i) and (ii),
         (i) a substituent selected from the following group B,
         (ii) a C₁₋₆ alkyl group optionally substituted by the same or different one or more substituents selected from the following group B,
      g) a C₇₋₁₂ aralkyl group (said C₇₋₁₂ aralkyl group is optionally substituted by the same or different one or more substituents selected from the following (i) and (ii),
         (i) a substituent selected from the following group B,
         (ii) a C₁₋₆ alkyl group optionally substituted by the same or different one or more substituents selected from the following group B, or
      h) an aralkoxy group (said aralkoxy group is optionally substituted by the same or different one or more substituents selected from the following (i) and (ii),
         (i) a substituent selected from the following group B,
         (ii) a C₁₋₆ alkyl group optionally substituted by the same or different one or more substituents selected from the following group B),
3) a C₁₋₆ alkyl group optionally substituted by the same or different one or more substituents selected from the following group B,
4) a cycloalkylalkoxy group (said cycloalkylalkoxy group is optionally substituted by the same or different one or more substituents selected from the following (a) and (b),
   (a) a substituent selected from the following group B,
   (b) a C₁₋₆ alkyl group optionally substituted by the same or different one or more substituents selected from the following group B,
5) a C₇₋₁₂ aralkyl group (said C₇₋₁₂ aralkyl group is optionally substituted by the same or different one or more substituents selected from the following (a) and (b),
   (a) a substituent selected from the following group B,
   (b) a C₁₋₆ alkyl group optionally substituted by the same or different one or more substituents selected from the following group B,
6) an aralkoxy group (said aralkoxy group is optionally substituted by the same or different one or more substituents selected from the following (a) and (b),
   (a) a substituent selected from the following group B,
   (b) a C₁₋₆ alkyl group optionally substituted by the same or different one or more substituents selected from the following group B,
7) -COR¹¹ wherein R¹¹ is as defined above,
8) -NR¹²R¹³ wherein R¹² and R¹³ are the same or different and each is
   (a) a hydrogen atom, or
   (b) a C₁₋₆ alkyl group optionally substituted by the same or different one or more substituents selected from the following group B, or
   (c) R¹² and R¹³ optionally form, together with the nitrogen atom bonded thereto, monocyclic nitrogen-containing saturated heterocycle (said heterocycle is optionally substituted by the same or different one or more substituents selected from the following (i) and (ii),
      (i) a substituent selected from the following group B,
      (ii) a C₁₋₆ alkyl group optionally substituted by the same or different one or more substituents selected from the following group B),
9) -CONR¹²R¹³ wherein R¹² and R¹³ are as defined above,
10) -NR¹⁴COR¹¹ wherein R¹¹ is as defined above, R¹⁴ is
   (a) a hydrogen atom, or
   (b) a C₁₋₆ alkyl group optionally substituted by the same or different one or more substituents selected from the following group B,
11) -NR¹⁴S(=O)₂R¹¹ wherein R¹¹ and R¹⁴ are as defined above,
12) -NR¹⁴CONR¹²R¹³ wherein R¹², R¹³ and R¹⁴ are as defined above, 13) -SR¹⁰ wherein R¹⁰ is as defined above,
14) -S(=O)R¹¹ wherein R¹¹ is as defined above,
15) -S(=O)₂R¹¹ wherein R¹¹ is as defined above,
16) -S(=O)₂NR¹²R¹³ wherein R¹² and R¹³ are as defined above,
17) a saturated or unsaturated carbocyclic group having 3 to 14 carbon atoms (said carbocyclic group is optionally substituted by the same or different one or more substituents selected from the following (a) and (b),
   (a) a substituent selected from the following group B,
   (b) a C₁₋₆ alkyl group optionally substituted by the same or different one or more substituents selected from the following group B,
18) a saturated or unsaturated heterocyclic group containing at least one hetero atom selected from a nitrogen atom, an oxygen atom and a sulfur atom (said heterocyclic group is optionally substituted by the same or different one or more substituents selected from the following (a) and (b),
   (a) a substituent selected from the following group B,
   (b) a C₁₋₆ alkyl group optionally substituted by the same or different one or more substituents selected from the following group B,
19) an aryloxy group (said aryloxy group is optionally substituted by the same or different one or more substituents selected from the following (a) and (b),
   (a) a substituent selected from the following group B,
   (b) a C₁₋₆ alkyl group optionally substituted by the same or different one or more substituents selected from the following group B,
20) a cyano group, and
21) a nitro group,
[group B]
1) a halogen atom,
2) a hydroxyl group,
3) a C₁₋₆ alkoxy group,
4) -NR¹⁵R¹⁶ wherein R¹⁵ and R¹⁶ are the same or different and each is
   (a) a hydrogen atom, or
   (b) a C₁₋₆ alkyl group, or
   (c) R¹⁵ and R¹⁶ optionally form, together with the nitrogen atom bonded thereto, monocyclic nitrogen-containing saturated heterocycle,
5) -CONR¹⁵R¹⁶ wherein R¹⁵ and R¹⁶ are as defined above,
6) -COR¹⁷ wherein R¹⁷ is
   (a) a hydrogen atom,
   (b) a hydroxyl group,
   (c) a C₁₋₆ alkyl group, or
   (d) a C₁₋₆ alkoxy group,
7) -NR¹⁸COR¹⁷ wherein R¹⁷ is as defined above, R¹⁸ is
   (a) a hydrogen atom, or
   (b) a C₁₋₆ alkyl group,
8) -NR¹⁸CONR¹⁵R¹⁶ wherein R¹⁵, R¹⁶ and R¹⁸ are as defined above,
9) -NR¹⁸S(=O)₂R¹⁹ wherein R¹⁸ is as defined above, R¹⁹ is a C₁₋₆ alkyl group, and
10) -S(=O)₂R¹⁹ wherein R¹⁹ is as defined above.

<3>
The URAT1 activity inhibitor of the above-mentioned <1>,
wherein the compound represented by the formula [1], or a pharmaceutically acceptable salt thereof, or a solvate thereof, which does not substantially inhibit CYP.
<4>
The URAT1 activity inhibitor of the above-mentioned <3>,
wherein the CYP is CYP2C9.
<5>
The compound of the above-mentioned <2>, or a pharmaceutically acceptable salt thereof, or a solvate thereof, which does not substantially inhibit CYP.
<6>
The compound of the above-mentioned <5>, or a pharmaceutically acceptable salt thereof, or a solvate thereof, wherein the CYP is CYP2C9.

### [Effect of the Invention]

Since the carboxylic acid compound of the present invention effectively inhibits the activity of URAT1, it is effective as an agent for the treatment or prophylaxis of a pathology involving uric acid, such as hyperuricemia, gouty tophus, acute gouty arthritis, chronic gouty arthritis, gouty kidney, urinary lithiasis, renal dysfunction, coronary heart disease, ischemic cardiac diseases and the like. Unlike the above-mentioned conventional agents for the treatment or prophylaxis of a pathology involving uric acid, since the agent does not substantially inhibit CYP, it has an extremely low possibility of causing pharmacokinetic drug interaction, and therefore, is expected to provide an effect of reducing side effects.

### [Best Mode for Embodying the Invention]

Each group used in the present specification is defined in the following.

The "C₁₋₆ alkyl group" is a straight chain or branched chain alkyl group having 1 to 6 carbon atoms and, for example, methyl group, ethyl group, n-propyl group, isopropyl group, n-butyl group, isobutyl group, sec-butyl group, tert-butyl group, n-pentyl group, isopentyl group, neopentyl group, tert-pentyl group, n-hexyl group and the like can be mentioned. Preferred is a C₁₋₄ alkyl group and particularly preferred are methyl group, ethyl group, n-propyl group, isopropyl group, n-butyl group, isobutyl group, sec-butyl group and tert-butyl group.

The "C₁₋₄ alkyl group" is a straight chain or branched chain alkyl group having 1 to 4 carbon atoms and, for example, methyl group, ethyl group, n-propyl group, isopropyl group, n-butyl group, isobutyl group, sec-butyl group, tert-butyl group and the like can be mentioned. Preferred are methyl group, ethyl group, n-propyl group, isopropyl group, isobutyl group, sec-butyl group and tert-butyl group.

The "C₁₋₂ alkyl group" is an alkyl group having 1 to 2 carbon atoms and, for example, methyl group and ethyl group can be mentioned.

The "C₂₋₆ alkyl group" is a straight chain or branched chain alkyl group having 2 to 6 carbon atoms and, for example, ethyl group, n-propyl group, isopropyl group, n-butyl group, isobutyl group, sec-butyl group, tert-butyl group, n-pentyl group, isopentyl group, neopentyl group, tert-pentyl group, n-hexyl group and the like can be mentioned.

The "C₃₋₄ alkyl group" is a straight chain or branched chain alkyl group having 3 to 4 carbon atoms and, for example, n-propyl group, isopropyl group, n-butyl group, isobutyl group, sec-butyl group, tert-butyl group and the like can be mentioned.

The "C₂₋₆ alkenyl group" is a straight chain or branched chain alkenyl group having 2 to 6 carbon atoms and, for example, vinyl group, n-propenyl group, isopropenyl group, n-butenyl group, isobutenyl group, sec-butenyl group, n-pentenyl group, isopentenyl group, 1-methylpropenyl group, n-hexenyl group, isohexenyl group, 1,1-dimethylbutenyl group, 2,2-dimethylbutenyl group, 3,3-dimethylbutenyl group, 3,3-dimethylpropenyl group, 2-ethylbutenyl group and the like can be mentioned. Preferred is a straight chain or branched chain alkenyl group having 2 to 4 carbon atoms and particularly preferred are vinyl group, n-propenyl group and isopropenyl group.

The "allyl group" is 2-propenyl group (or n-propenyl group; -CH₂CH=CH₂).

The "halogen atom" is fluorine atom, chlorine atom, bromine atom or iodine atom.

The "C₁₋₆ alkoxy group" is an alkoxy group wherein the alkyl moiety is the "C₁₋₆ alkyl group" defined above and, for example, methoxy group, ethoxy group, n-propoxy group, isopropoxy group, n-butoxy group, isobutoxy group, sec-butoxy group, tert-butoxy group, n-pentyloxy group, n-hexyloxy group and the like can be mentioned. Preferred is an alkoxy group wherein the alkyl moiety is the "C₁₋₄ alkyl group" defined above and particularly preferred are methoxy group and ethoxy group.

The "C₁₋₄ alkoxy group" is an alkoxy group wherein the alkyl moiety is the "C₁₋₄ alkyl group" defined above and, for example, methoxy group, ethoxy group, n-propoxy group, isopropoxy group, n-butoxy group, isobutoxy group, sec-butoxy group, tert-butoxy group and the like can be mentioned. Particularly preferred are methoxy group and ethoxy group.

The "saturated or unsaturated carbocyclic group having 3 to 14 carbon atoms" is a saturated or unsaturated cyclic hydrocarbon group having 3 to 14 carbon atoms, which is specifically a C₆₋₁₄ aryl group, a C₃₋₈ cycloalkyl group, a C₃₋₈ cycloalkenyl group, a group derived from a fused carbocycle, wherein two or more of rings constituting them are condensed, and the like.

The "C₆₋₁₄ aryl group" is an aromatic hydrocarbon group having 6 to 14 carbon atoms and, for example, phenyl group, naphthyl group, biphenyl group, anthryl group, azulenyl group, phenanthryl group, pentalenyl group and the like can be mentioned. Preferred is phenyl group.

The "C₃₋₈ cycloalkyl group" is a cycloalkyl group having 3 to 8 carbon atoms and, for example, cyclopropyl group, cyclobutyl group, cyclopentyl group, cyclohexyl group, cycloheptyl group, cyclooctyl group and the like can be mentioned. Preferred is a cycloalkyl group having 3 to 6 carbon atoms, which is specifically cyclopropyl group, cyclobutyl group, cyclopentyl group and cyclohexyl group. Particularly preferred are cyclopropyl group and cyclohexyl group.

The "C₃₋₈ cycloalkenyl group" is a cycloalkenyl group having 3 to 8 carbon atoms, and contains at least one, preferably 1 or 2 double bonds. For example, cyclopropenyl group, cyclobutenyl group, cyclopentenyl group, cyclopentadienyl group, cyclohexenyl group, cyclohexadienyl group (2,4-cyclohexadien-1-yl group, 2,5-cyclohexadien-1-yl group etc.), cycloheptenyl group, cyclooctenyl group and the like can be mentioned. Preferred is a cycloalkenyl group having 3 to 6 carbon atoms, and particularly preferred is cyclohexenyl group.

As the group derived from a fused carbocycle, wherein two or more rings constituting these "C₆₋₁₄ aryl group", "C₃₋₈ cycloalkyl group" and "C₃₋₈ cycloalkenyl group" are condensed, for example, indenyl group, indanyl group, fluorenyl group, 1,4-dihydronaphthyl group, 1,2,3,4-tetrahydronaphthyl group (1,2,3,4-tetrahydro-2-naphthyl group, 5,6,7,8-tetrahydro-2-naphthyl group etc.), perhydronaphthyl group and the like can be mentioned.

The "saturated or unsaturated carbocycle having 3 to 14 carbon atoms" is a ring constituting the "saturated or unsaturated carbocyclic group having 3 to 14 carbon atoms" defined above.

The "saturated carbocycle having 3 to 6 carbon atoms" is a saturated carbocycle having 3 to 6 carbon atoms from among a ring constituting the "saturated or unsaturated carbocyclic group having 3 to 14 carbon atoms" defined above. Specifically, a cycloalkane having 3 to 6 carbon atoms, for example, cyclopropane, cyclobutane, cyclopentane, cyclohexane and the like can be mentioned.

The "aralkyl group" is an arylalkyl group wherein the aryl moiety is the "C₆₋₁₄ aryl group" defined above and the alkyl moiety is the "C₁₋₆ alkyl group" defined above and, for example, benzyl group, phenethyl group, 3-phenylpropyl group, 4-phenylbutyl group, 6-phenylhexyl group and the like can be mentioned. Preferred is an aralkyl group having 7 to 12 carbon atoms (C₇₋₁₂ aralkyl group) and particularly preferred is benzyl group.

The "aralkoxy group" is an arylalkoxy group wherein the aryl moiety is the "C₆₋₁₄ aryl group" defined above and the alkoxy moiety is the "C₁₋₆ alkoxy group" defined above and, for example, benzyloxy group, 3-phenylpropyloxy group, 4-phenylbutyloxy group, 6-phenylhexyloxy group and the like can be mentioned. Preferred is an aralkoxy group having 7 to 14 carbon atoms and particularly preferred is benzyloxy group.

The "cycloalkylalkoxy group" is a cycloalkylalkoxy group wherein the cycloalkyl moiety is the "C₃₋₈ cycloalkyl group" defined above and the alkoxy moiety is the "C₁₋₆ alkoxy group" defined above and, for example, cyclopropylmethoxy group, cyclobutylmethoxy group, cyclopentylmethoxy group, cyclohexylmethoxy group and the like can be mentioned. Preferred is a cycloalkylalkoxy group having 4 to 8 carbon atoms and particularly preferred are cyclopropylmethoxy group and cyclohexylmethoxy group.

The "aryloxy group" is an aryloxy group wherein the aryl moiety is the "C₆₋₁₄ aryl group" defined above and, for example, phenoxy group, naphthyloxy group, biphenyloxy group and the like can be mentioned. Preferred is phenoxy group.

The "saturated or unsaturated heterocyclic group containing at least one heteroatom selected from a nitrogen atom, an oxygen atom and a sulfur atom" is a saturated or unsaturated (including partially unsaturated and completely unsaturated) monocyclic 5-membered or 6-membered heterocyclic group, containing, besides carbon atoms, at least one, preferably 1 to 4, heteroatoms selected from a nitrogen atom, an oxygen atom and a sulfur atom; a fused ring group wherein two or more of these heterocycles are condensed; or a fused ring group wherein one of the heterocycles and a carbocycle selected from benzene, cyclopentane and cyclohexane are condensed.

As the "saturated monocyclic 5-membered or 6-membered heterocyclic group", for example, pyrrolidinyl group, tetrahydrofuryl group, tetrahydrothienyl group, imidazolidinyl group, pyrazolidinyl group, 1,3-dioxolanyl group, 1,3-oxathiolanyl group, oxazolidinyl group, thiazolidinyl group, piperidinyl group, piperazinyl group, tetrahydropyranyl group, tetrahydrothiopyranyl group, dioxanyl group, morpholinyl group, thiomorpholinyl group, 2-oxopyrrolidinyl group, 2-oxopiperidinyl group, 4-oxopiperidinyl group, 2,6-dioxopiperidinyl group and the like can be mentioned.

As the "unsaturated monocyclic 5-membered or 6-membered heterocyclic group", for example, pyrrolyl group, furyl group, thienyl group, imidazolyl group, 1,2-dihydro-2-oxoimidazolyl group, pyrazolyl group, oxazolyl group, isoxazolyl group, thiazolyl group, isothiazolyl group, triazolyl group (e.g., 1,2,4-triazolyl group, 1,2,3-triazolyl group etc.), tetrazolyl group, 1,3,4-oxadiazolyl group, 1,2,4-oxadiazolyl group, 1,3,4-thiadiazolyl group, 1,2,4-thiadiazolyl group, furazanyl group, pyridyl group, pyrimidinyl group, 3,4-dihydro-4-oxopyrimidinyl group, pyridazinyl group, pyrazinyl group, 1,3,5-triazinyl group, imidazolinyl group, pyrazolinyl group, oxazolinyl group (e.g., 2-oxazolinyl group, 3-oxazolinyl group, 4-oxazolinyl group etc.), isoxazolinyl group, thiazolinyl group, isothiazolinyl group, pyranyl group, 2-oxopyranyl group, 2-oxo-2,5-dihydrofuranyl group, 1,1-dioxo-1H-isothiazolyl group and the like can be mentioned.

As the "fused heterocyclic group", for example, indolyl group (e.g., 4-indolyl group, 7-indolyl group etc.), isoindolyl group, 1,3-dihydro-1,3-dioxoisoindolyl group, benzofuranyl group (e.g., 4-benzofuranyl group, 7-benzofuranyl group etc.), indazolyl group, isobenzofuranyl group, benzothiophenyl group (e.g., 4-benzothiophenyl group, 7-benzothiophenyl group etc.), benzoxazolyl group (e.g., 4-benzoxazolyl group, 7-benzoxazolyl group etc.), benzimidazolyl group (e.g., 4-benzimidazolyl group, 7-benzimidazolyl group etc.), benzothiazolyl group (e.g., 4-benzothiazolyl group, 7-benzothiazolyl group etc.), indolizinyl group, quinolyl group, isoquinolyl group, 1,2-dihydro-2-oxoquinolyl group, quinazolinyl group, quinoxalinyl group, cinnolinyl group, phthalazinyl group, quinolizinyl group, purinyl group, pteridinyl group, indolinyl group, isoindolinyl group, 5,6,7,8-tetrahydroquinolyl group, 1,2,3,4-tetrahydroquinolyl group, 2-oxo-1,2,3,4-tetrahydroquinolyl group, benzo[1,3]dioxolyl group, 3,4-methylenedioxypyridyl group, 4,5-ethylenedioxypyrimidinyl group, chromenyl group, chromanyl group, isochromanyl group and the like can be mentioned.

The "monocyclic nitrogen-containing saturated heterocycle" formed together with the nitrogen atom bonded thereto is a saturated 5-membered or 6-membered monocyclic heterocycle containing at least one nitrogen atom, such as piperidine, morpholine, piperazine, pyrrolidine and the like.

The "C₁₋₄ alkylene group" is a straight chain or branched chain alkylene group having 1 to 4 carbon atoms, preferably a straight chain or branched chain alkylene group having 2 to 4 carbon atoms (C₂₋₄ alkylene group), more preferably a straight chain or branched chain alkylene group having 2 to 3 carbon atoms (C₂₋₃ alkylene group). As the C₁₋₄ alkylene group, for example, methylene group, ethylene group, trimethylene group, tetramethylene group,

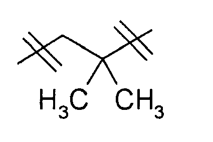

and the like can be mentioned. Preferred are methylene group, ethylene group, trimethylene group, tetramethylene group,

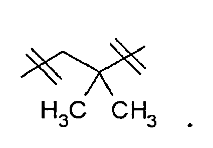

The "C₂₋₄ alkenylene group" is a straight chain or branched chain alkenylene group having 2 to 4 carbon atoms and, for example, vinylene group, 1-propenylene group, 2-propenylene group, 1-butenylene group, 2-butenylene group, 3-butenylene group, buta-1,3-dienylene group and the like can be mentioned. Preferred is vinylene group.

The "C₂₋₄ alkynylene group" is a straight chain or branched chain alkynylene group having 2 to 4 carbon atoms and, for example, ethynylene group, 1-propynylene group, 2-propynylene group, 1-butynylene group, 2-butynylene group, 3-butynylene group and the like can be mentioned.

The "C₁₋₄ alkylene-C₆₋₁₄ arylene group" is that wherein its alkylene moiety is the "C₁₋₄ alkylene group" defined above and its arylene moiety is arylene group induced from the "C₆₋₁₄ aryl group" defined above and, for example,

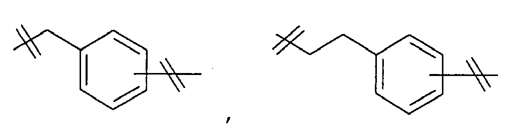

and the like, can be mentioned.

Being "optionally substituted by one or more, the same or different substituents" means being unsubstituted or being substituted by at least one to the acceptable maximum number of substituents. In the case of a methyl group, for example, it means being optionally substituted by 1 to 3 substituents, and in the case of an ethyl group, it means being optionally substituted by 1 to 5 substituents. When substituted by 2 or more substituents, the substituents may be the same or different and the position of the substituents may be any, without any particular limitation. Preferred is being "optionally substituted by the same or different, 1 to 3 substituents".

Preferable examples of each group in the formula [1] are as follows.

Z is preferably an oxygen atom.
X is preferably
1) an oxygen atom, or
2) a sulfur atom,
more preferably an oxygen atom.

Y is preferably,
1) a single bond,
2) a C₁₋₄ alkylene group (preferably methylene group, ethylene group, trimethylene group, tetramethylene group) optionally substituted by 1 to 3 C₁₋₆ alkyl groups (preferably C₁₋₄ alkyl group, more preferably methyl group),
3) a C₂₋₄ alkenylene group (preferably vinylene group), or
4) a C₁₋₄ alkylene-C₆₋₁₄ arylene group (preferably methylene-phenylene group), more preferably a C₂₋₄ alkylene group (preferably ethylene group).

R¹ is preferably,
1) -COOH,
2) a -COO-C₁₋₆ alkyl group (preferably -COO-C₁₋₄ alkyl group (said C₁₋₄ alkyl group is preferably methyl group, ethyl group or tert-butyl group)), more preferably -COOH.

R⁴ and R⁵ are the same or different and each is preferably
1) a hydrogen atom,
2) a C₁₋₆ alkyl group (preferably methyl group, ethyl group, n-propyl group, isopropyl group, n-butyl group, sec-butyl group or isobutyl group),
3) a phenyl group, or
4) a benzyl group.

R⁴ and R⁵ are more preferably the same or different and each is
1) a hydrogen atom,
2) a C₂₋₆ alkyl group (preferably ethyl group, n-propyl group, isopropyl group, n-butyl group, sec-butyl group or isobutyl group), or
3) a phenyl group.

R⁴ and R⁵ are particularly preferably the same or different and each is
1) a hydrogen atom, or
2) a C₂₋₆ alkyl group (preferably ethyl group, n-propyl group, isopropyl group, n-butyl group, sec-butyl group or isobutyl group).

R⁶, R⁷, R⁸ and R⁹ are preferably the same or different and each is
1) a hydrogen atom,
2) a halogen atom (preferably chlorine atom or bromine atom),
3) a trifluoromethyl group,
4) a C₁₋₆ alkyl group (preferably C₁₋₄ alkyl group, more preferably methyl group, ethyl group, isopropyl group or tert-butyl group),
5) a C₁₋₆ alkoxy group (preferably C₁₋₄ alkoxy group, more preferably methoxy group),
6) a C₃₋₈ cycloalkyl group (preferably C₃₋₆ cycloalkyl group, more preferably cyclohexyl group), or
7) a nitro group, or
8) R⁶ and R⁷ form, together with the carbon atom bonded thereto, a saturated or unsaturated carbocycle having 3 to 14 carbon atoms (preferably aromatic hydrocarbon, more preferably benzene ring).
   Of these, at least one of R⁶, R⁷, R⁸ and R⁹ is preferably a C₁₋₆ alkyl group, a trifluoromethyl group, a C₁₋₆ alkoxy group, or a C₃₋₈ cycloalkyl group.

Each of R⁶, R⁷, R⁸ and R⁹ is preferably the following.
R⁶ is preferably
1) a hydrogen atom,
2) a halogen atom (preferably chlorine atom or bromine atom),
3) a trifluoromethyl group,
4) a C₁₋₆ alkyl group (preferably C₁₋₄ alkyl group, more preferably methyl group, ethyl group, isopropyl group or tert-butyl group), or
5) a C₃₋₈ cycloalkyl group (preferably C₃₋₆ cycloalkyl group, more preferably cyclohexyl group).

R⁷ is preferably
1) a hydrogen atom,
2) a halogen atom (preferably chlorine atom),
3) a C₁₋₆ alkyl group (preferably C₁₋₄ alkyl group, more preferably methyl group), or
4) a nitro group.

R⁸ is preferably
1) a hydrogen atom,
2) a halogen atom (preferably chlorine atom),
3) a trifluoromethyl group,
4) a C₁₋₆ alkyl group (preferably C₁₋₄ alkyl group, more preferably methyl group, ethyl group or tert-butyl group),
5) a C₁₋₆ alkoxy group (preferably C₁₋₄ alkoxy group, more preferably methoxy group), or
6) a nitro group.

R⁹ is preferably a hydrogen atom.

R⁶, R⁷, R⁸ and R⁹ are more preferably the same or different and each is
1) a hydrogen atom,
2) a halogen atom (preferably chlorine atom),
3) a C₁₋₆ alkyl group (preferably C₁₋₄ alkyl group, more preferably methyl group, ethyl group, isopropyl group), or
4) a C₁₋₆ alkoxy group (preferably C₁₋₄ alkoxy group, more preferably methoxy group).
   Of these, at least one of R⁶, R⁷, R⁸ and R⁹ is preferably a C₁₋₆ alkyl group or a C₁₋₆ alkoxy group.

Each of R⁶, R⁷, R⁸ and R⁹ is more preferably the following.
R⁶ is more preferably
1) a hydrogen atom,
2) a halogen atom (preferably chlorine atom), or
3) a C₁₋₆ alkyl group (preferably C₁₋₄ alkyl group, more preferably methyl group, isopropyl group).

R⁷ is more preferably
1) a hydrogen atom, or
2) a C₁₋₆ alkyl group (preferably C₁₋₄ alkyl group, more preferably methyl group).

R⁸ is more preferably
1) a hydrogen atom,
2) a halogen atom (preferably chlorine atom),
4) a C₁₋₆ alkyl group (preferably C₁₋₄ alkyl group, more preferably methyl group, ethyl group), or
5) a C₁₋₆ alkoxy group (preferably C₁₋₄ alkoxy group, more preferably methoxy group).

R⁹ is more preferably a hydrogen atom.

It is preferable that one of R⁶ and R⁸ be substituted, and it is more preferable that both of R⁶ and R⁸ be substituted.

Of the compounds represented by the formula [1] (hereinafter compound [1]), a compound represented by the following formula [1']:

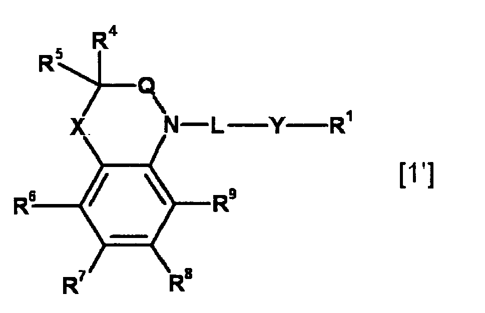

wherein
Q is
1) -CH₂- or
2) -(C=Z)-:
Z is
1) an oxygen atom, or
2) a sulfur atom:
X is
1) an oxygen atom,
2) a sulfur atom,
3) -S(=O)- or
4) -S(=O)₂-:
L is
1) a single bond, or 2) -(C=O)-:
Y is
1) a single bond,
2) a C₁₋₄ alkylene group optionally substituted by the same or different one or more substituents selected from the aforementioned group A,
3) a C₂₋₄ alkenylene group optionally substituted by the same or different one or more substituents selected from the aforementioned group A, or
4) a C₁₋₄ alkylene-C₆₋₁₄ arylene group optionally substituted by the same or different one or more substituents selected from the aforementioned group A:
R¹ is
1) -COOH, or
2) -COOR wherein R is
   (a) a C₁₋₆ alkyl group optionally substituted by the same or different one or more substituents selected from the aforementioned group A,
   (b) an allyl group optionally substituted by the same or different one or more substituents selected from the aforementioned group A,
   (c) a phenyl group optionally substituted by the same or different one or more substituents selected from the aforementioned group A, or
   (d) a benzyl group optionally substituted by the same or different one or more substituents selected from the aforementioned group A;
R⁴ and R⁵ are the same or different and each is
1) a hydrogen atom,
2) a C₁₋₆ alkyl group optionally substituted by the same or different one or more substituents selected from the aforementioned group A,
3) a C₂₋₆ alkenyl group optionally substituted by the same or different one or more substituents selected from the aforementioned group A,
4) a C₆₋₁₄ aryl group optionally substituted by the same or different one or more substituents selected from the aforementioned group A,
5) a C₇₋₁₂ aralkyl group optionally substituted by the same or different one or more substituents selected from the aforementioned group A, or
6) a halogen atom, or
7) R⁴ and R⁵, form, together with the carbon atom bonded thereto, a saturated carbocycle having 3 to 6 carbon atoms (said carbocycle is optionally substituted by the same or different one or more substituents selected from the aforementioned group A);
R⁶, R⁷, R⁸ and R⁹ are the same or different and each is
1) a hydrogen atom,
2) a halogen atom,
3) a C₁₋₆ alkyl group optionally substituted by the same or different one or more substituents selected from the aforementioned group A,
4) a C₁₋₆ alkoxy group optionally substituted by the same or different one or more substituents selected from the aforementioned group A,
5) a C₃₋₈ cycloalkyl group optionally substituted by the same or different one or more substituents selected from the aforementioned group A,
6) a C₆₋₁₄ aryl group optionally substituted by the same or different one or more substituents selected from the aforementioned group A,
7) a nitro group,
8) a cyano group,
9) -COOH, or
10) -COOR wherein R is as defined above, or
11) R⁶ and R⁷, R⁷ and R⁸ or R⁸ and R⁹ form, together with the carbon atom bonded thereto, a saturated or unsaturated carbocycle having 3 to 14 carbon atoms (said carbocycle is optionally substituted by the same or different one or more substituents selected from the aforementioned group A), provided that when
   (i) Q is -(C=Z)- and
L is a single bond,
Y is
1) a single bond,
2) a C₂₋₄ alkylene group optionally substituted by the same or different one or more substituents selected from the aforementioned group A,
3) a C₂₋₄ alkenylene group optionally substituted by the same or different one or more substituents selected from the aforementioned group A, or
4) a C₁₋₄ alkylene-C₆₋₁₄ arylene group optionally substituted by the same or different one or more substituents selected from the aforementioned group A, and
R⁵ is
1) a C₂₋₆ alkyl group optionally substituted by the same or different one or more substituents selected from the aforementioned group A,
2) a C₂₋₆ alkenyl group optionally substituted by the same or different one or more substituents selected from the aforementioned group A,
3) a C₆₋₁₄ aryl group optionally substituted by the same or different one or more substituents selected from the aforementioned group A,
4) a C₇₋₁₂ aralkyl group optionally substituted by the same or different one or more substituents selected from the aforementioned group A, or
5) a halogen atom, or
6) R⁴ and R⁵ form, together with the carbon atom bonded thereto, a saturated carbocycle having 3 to 6 carbon atoms (said carbocycle is optionally substituted by the same or different one or more substituents selected from the following group A); and
   (ii) the following compounds are excluded,
3-[2-(4-methoxybenzyl)-3-oxo-2,3-dihydrobenzo[1,4]thiazin-4-yl]propionic acid,
(E)-4-(2,2-dimethyl-6-nitro-2,3-dihydrobenzo[1,4]oxazin-4-yl)-4-oxo-2-butenoic acid,
(6-methoxy-8-methyl-2,3-dihydrobenzo[1,4]oxazin-4-yl)acetic acid,
(2,2-dimethyl-6-nitro-2,3-dihydrobenzo[1,4]oxazin-4-yl)acetic acid, and
(2E,4E)-3-methyl-6-oxo-6-(6,6,9,9-tetramethyl-2,3,6,7,8,9-hexahydronaphtho[2,3-b][1,4]oxazin-4-yl)2,4-hexadienoic acid (hereinafter also referred to as compound [1']) is preferable.

Of these, a compound wherein
X is
1) an oxygen atom, or
2) a sulfur atom:
L is
1) a single bond, or
2) -(C=O)-:
Y is
1) a single bond,
2) a C₁₋₄ alkylene group optionally substituted by the same or different one or more substituents selected from the aforementioned group A,
3) a C₂₋₄ alkenylene group optionally substituted by the same or different one or more substituents selected from the aforementioned group A, or
4) a C₁₋₄ alkylene-C₆₋₁₄ arylene group optionally substituted by the same or different one or more substituents selected from the aforementioned group A:
R¹ is
1) -COOH, or
2) -COOR wherein R is
   (a) a C₁₋₆ alkyl group optionally substituted by the same or different one or more substituents selected from the aforementioned group A,
   (b) an allyl group optionally substituted by the same or different one or more substituents selected from the aforementioned group A,
   (c) a phenyl group optionally substituted by the same or different one or more substituents selected from the aforementioned group A, or
   (d) a benzyl group optionally substituted by the same or different one or more substituents selected from the aforementioned group A;
R⁴ and R⁵ are the same or different and each is
1) a hydrogen atom,
2) a C₁₋₆ alkyl group,
3) a phenyl group, or
4) a benzyl group:
R⁶, R⁷, R⁸ and R⁹ are the same or different and each is
1) a hydrogen atom,
2) a halogen atom,
3) a trifluoromethyl group,
4) a C₁₋₆ alkyl group,
5) a C₁₋₆ alkoxy group,
6) a C₃₋₈ cycloalkyl group, or
7) a nitro group, or
8) R⁶ and R⁷, R⁷ and R⁸ or R⁸ and R⁹ form, together with the carbon atom bonded thereto, a saturated or unsaturated carbocycle having 3 to 14 carbon atoms (said carbocycle is optionally substituted by the same or different one or more substituents selected from the aforementioned group A), provided that the following compounds are excluded,
(E)-4-(2,2-dimethyl-6-nitro-2,3-dihydrobenzo[1,4]oxazin-4-yl)-4-oxo-2-butenoic acid,
(6-methoxy-8-methyl-2,3-dihydrobenzo[1,4]oxazin-4-yl)acetic acid,
(2,2-dimethyl-6-nitro-2,3-dihydrobenzo[1,4]oxazin-4-yl)acetic acid, and
(2E,4E)-3-methyl-6-oxo-6-(6,6,9,9-tetramethyl-2,3,6,7,8,9-hexahydronaphtho[2,3-b][1,4]oxazin-4-yl)2,4-hexadienoic acid is preferable.

Especially, a compound wherein
X is an oxygen atom;
L is -(C=O)-;
Y is
1) a single bond,
2) a C₁₋₄ alkylene group, or
3) a C₂₋₄ alkenylene group:
R¹ is
1) -COOH, or
2) a -COO-C₁₋₆ alkyl group:
R⁴ and R⁵ are the same or different and each is
1) a hydrogen atom,
2) a C₂₋₆ alkyl group,
3) a phenyl group, or
4) a benzyl group:
R⁶ and R⁸ are the same or different and each is
1) a halogen atom,
2) a trifluoromethyl group,
3) a C₁₋₆ alkyl group, or
4) a C₃₋₈ cycloalkyl group:
R⁷ and R⁹ are each a hydrogen atom;
is more preferable.

Compound [1'] is divided into a compound represented by the formula [A-1] (hereinafter also referred to as compound [A-1]) and a compound represented by the formula [B-1] (hereinafter also referred to as compound [B-1]) based on Q. Preferable compounds of both are shown below.

### Compound [A-1]

Compound [A-1] is compound [1'] wherein Q is -(C=Z)-, i.e., a compound represented by the following formula [A-1]:

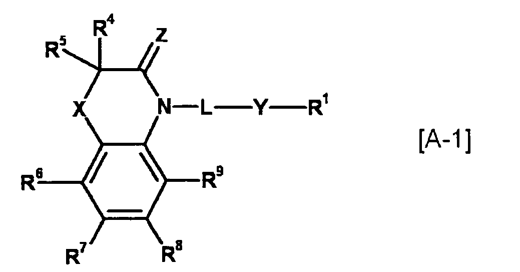

wherein
Z is
1) an oxygen atom, or
2) a sulfur atom:
X is
1) an oxygen atom,
2) a sulfur atom,
3) -S(=O)- or
4) -S(=O)₂-:
L is
1) a single bond, or
2) -(C=O)-:
Y is
1) a single bond,
2) a C₁₋₄ alkylene group optionally substituted by the same or different one or more substituents selected from the aforementioned group A,
3) a C₂₋₄ alkenylene group optionally substituted by the same or different one or more substituents selected from the aforementioned group A, or
4) a C₁₋₄ alkylene-C₆₋₁₄ arylene group optionally substituted by the same or different one or more substituents selected from the aforementioned group A:
R¹ is
1) -COOH, or
2) -COOR wherein R is
   (a) a C₁₋₆ alkyl group optionally substituted by the same or different one or more substituents selected from the aforementioned group A,
   (b) an allyl group optionally substituted by the same or different one or more substituents selected from the aforementioned group A,
   (c) a phenyl group optionally substituted by the same or different one or more substituents selected from the aforementioned group A, or
   (d) a benzyl group optionally substituted by the same or different one or more substituents selected from the aforementioned group A;
R⁴ and R⁵ are the same or different and each is
1) a hydrogen atom,
2) a C₁₋₆ alkyl group optionally substituted by the same or different one or more substituents selected from the aforementioned group A,
3) a C₂₋₆ alkenyl group optionally substituted by the same or different one or more substituents selected from the aforementioned group A,
4) a C₆₋₁₄ aryl group optionally substituted by the same or different one or more substituents selected from the aforementioned group A,
5) a C₇₋₁₂ aralkyl group optionally substituted by the same or different one or more substituents selected from the aforementioned group A, or
6) a halogen atom, or
7) R⁴ and R⁵ form, together with the carbon atom bonded thereto, a saturated carbocycle having 3 to 6 carbon atoms (said carbocycle is optionally substituted by the same or different one or more substituents selected from the aforementioned group A);
R⁶, R⁷, R⁸ and R⁹ are the same or different and each is
1) a hydrogen atom,
2) a halogen atom,
3) a C₁₋₆ alkyl group optionally substituted by the same or different one or more substituents selected from the aforementioned group A,
4) a C₁₋₆ alkoxy group optionally substituted by the same or different one or more substituents selected from the aforementioned group A,
5) a C₃₋₈ cycloalkyl group optionally substituted by the same or different one or more substituents selected from the aforementioned group A,
6) a C₆₋₁₄ aryl group optionally substituted by the same or different one or more substituents selected from the aforementioned group A,
7) a nitro group,
8) a cyano group,
9) -COOH, or
10) -COOR wherein R is as defined above, or
11) R⁶ and R⁷, R⁷ and R⁸ or R⁸ and R⁹ form, together with the carbon atom bonded thereto, a saturated or unsaturated carbocycle having 3 to 14 carbon atoms (said carbocycle is optionally substituted by the same or different one or more substituents selected from the aforementioned group A), provided that when
   (i) L is a single bond,
Y is
1) a single bond,
2) a C₂₋₄ alkylene group optionally substituted by the same or different one or more substituents selected from the aforementioned group A,
3) a C₂₋₄ alkenylene group optionally substituted by the same or different one or more substituents selected from the aforementioned group A, or
4) a C₁₋₄ alkylene-C₆₋₁₄ arylene group optionally substituted by the same or different one or more substituents selected from the aforementioned group A, and
R⁵ is
1) a C₂₋₆ alkyl group optionally substituted by the same or different one or more substituents selected from the aforementioned group A,
2) a C₂₋₆ alkenyl group optionally substituted by the same or different one or more substituents selected from the aforementioned group A,
3) a C₆₋₁₄ aryl group optionally substituted by the same or different one or more substituents selected from the aforementioned group A,
4) a C₇₋₁₂ aralkyl group optionally substituted by the same or different one or more substituents selected from the aforementioned group A, or
5) a halogen atom, or
6) R⁴ and R⁵ form, together with the carbon atom bonded thereto, a saturated carbocycle having 3 to 6 carbon atoms (said carbocycle is optionally substituted by the same or different one or more substituents selected from the aforementioned group A);
   (ii) the following compound is excluded,
   3-[2-(4-methoxybenzyl)-3-oxo-2,3-dihydrobenzo[1,4]thiazin-4-yl]propionic acid.
   Of those, a compound represented by the following formula [A-2]:

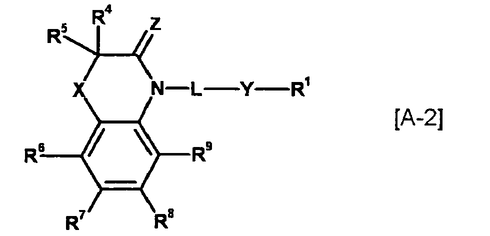

wherein
Z is
1) an oxygen atom, or
2) a sulfur atom:
X is
1) an oxygen atom, or
2) a sulfur atom:
L is
1) a single bond, or
2) -(C=O)-:
Y is
1) a single bond,
2) a C₁₋₄ alkylene group optionally substituted by the same or different one or more substituents selected from the aforementioned group A,
3) a C₂₋₄ alkenylene group optionally substituted by the same or different one or more substituents selected from the aforementioned group A, or
4) a C₁₋₄ alkylene-C₆₋₁₄ arylene group optionally substituted by the same or different one or more substituents selected from the aforementioned group A:
R¹ is
1) -COOH, or
2) -COOR wherein R is
   (a) a C₁₋₆ alkyl group optionally substituted by the same or different one or more substituents selected from the aforementioned group A,
   (b) an allyl group optionally substituted by the same or different one or more substituents selected from the aforementioned group A,
   (c) a phenyl group optionally substituted by the same or different one or more substituents selected from the aforementioned group A, or
   (d) a benzyl group optionally substituted by the same or different one or more substituents selected from the aforementioned group A;
R⁴ and R⁵ are the same or different and each is
1) a hydrogen atom,
2) a C₁₋₆ alkyl group,
3) a phenyl group, or
4) a benzyl group:
R⁶, R⁷, R⁸ and R⁹ are the same or different and each is
1) a hydrogen atom,
2) a halogen atom,
3) a trifluoromethyl group,
4) a C₁₋₆ alkyl group,
5) a C₁₋₆ alkoxy group,
6) a C₃₋₈ cycloalkyl group, or
7) a nitro group, or
8) R⁶ and R⁷ form, together with the carbon atom bonded thereto, a saturated or unsaturated carbocycle having 3 to 14 carbon atoms,
provided that when L is a single bond,
Y is
1) a single bond,
2) a C₂₋₄ alkylene group optionally substituted by the same or different one or more substituents selected from the aforementioned group A,
3) a C₂₋₄ alkenylene group optionally substituted by the same or different one or more substituents selected from the aforementioned group A, or
4) a C₁₋₄ alkylene-C₆₋₁₄ arylene group optionally substituted by the same or different one or more substituents selected from the aforementioned group A, and
R⁵ is
1) a C₂₋₆ alkyl group,
2) a phenyl group, or
3) a benzyl group (hereinafter also referred to as compound [A-2]) is preferable.
   Of compound [A-2], a compound represented by the following formula [A-3]:

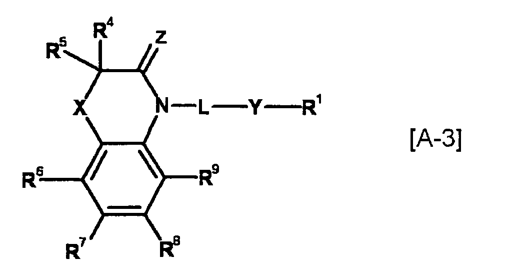

wherein
Z is an oxygen atom;
X is an oxygen atom;
L is a single bond;
Y is a C₂₋₄ alkylene group;
R¹ is
1) -COOH, or
2) -COOR wherein R is
   (a) a C₁₋₆ alkyl group optionally substituted by the same or different one or more substituents selected from the aforementioned group A,
   (b) an allyl group optionally substituted by the same or different one or more substituents selected from the aforementioned group A,
   (c) a phenyl group optionally substituted by the same or different one or more substituents selected from the aforementioned group A, or
   (d) a benzyl group optionally substituted by the same or different one or more substituents selected from the aforementioned group A;
R⁴ is
1) a hydrogen atom,
2) a C₂₋₆ alkyl group, or
3) a phenyl group:
R⁵ is
1) a C₂₋₆ alkyl group, or
2) a phenyl group:
R⁶ and R⁸ are the same or different and each is
1) a halogen atom,
2) a trifluoromethyl group,
3) a C₁₋₆ alkyl group,
4) a C₁₋₆ alkoxy group, or
5) a C₃₋₈ cycloalkyl group,
R⁷ and R⁹ are the same or different and each is
1) a hydrogen atom,
2) a halogen atom,
3) a trifluoromethyl group,
4) a C₁₋₆ alkyl group,
5) a C₁₋₆ alkoxy group,
6) a C₃₋₈ cycloalkyl group, or
7) a nitro group, or
R⁶ and R⁷ form, together with the carbon atom bonded thereto, a saturated or unsaturated carbocycle having 3 to 14 carbon atoms (preferably aromatic hydrocarbon, more preferably benzene ring)
(hereinafter also referred to as compound [A-3]) is more preferable.
Furthermore, of compounds [A-3], a compound represented by the following formula [A-4]:

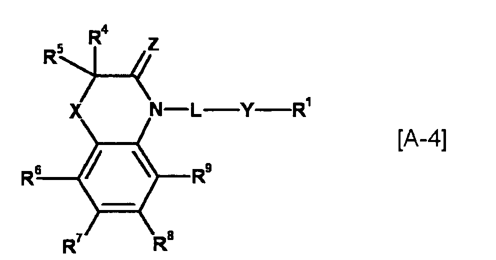

wherein
Z is an oxygen atom;
X is an oxygen atom;
L is a single bond;
Y is a C₂₋₃ alkylene group;
R¹ is
1) -COOH, or
2) -COOR wherein R is a C₁₋₆ alkyl group optionally substituted by the same or different one or more substituents selected from the aforementioned group A:
R⁴ is
1) a hydrogen atom,
2) a C₂₋₆ alkyl group, or
3) a phenyl group:
R⁵ is
1) a C₂₋₆ alkyl group, or
2) a phenyl group:
R⁶ and R⁸ are the same or different and each is
1) a halogen atom,
2) a trifluoromethyl group,
3) a C₁₋₆ alkyl group,
4) a C₁₋₆ alkoxy group, or
5) a C₃₋₈ cycloalkyl group,
R⁷ and R⁹ are the same or different and each is
1) a hydrogen atom,
2) a halogen atom,
3) a trifluoromethyl group,
4) a C₁₋₆ alkyl group,
5) a C₁₋₆ alkoxy group,
6) a C₃₋₈ cycloalkyl group, or
7) a nitro group
(hereinafter also referred to as compound [A-4]) is more preferable.
Of compounds [A-4], particularly, a compound represented by the following formula [A-5]:

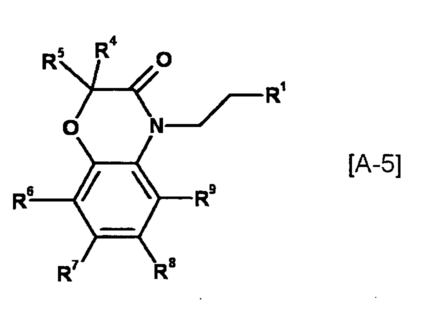

wherein
R¹ is
1) -COOH, or
2) a -COO-C₁₋₆ alkyl group:
R⁴ is
1) a hydrogen atom, or
2) a C₂₋₆ alkyl group, :
R⁵ is
1) a C₂₋₆ alkyl group, or
2) a phenyl group:
R⁶ and R⁸ are the same or different and each is
1) a halogen atom,
2) a trifluoromethyl group,
3) a C₁₋₆ alkyl group,
4) a C₁₋₆ alkoxy group, or
5) a C₃₋₈ cycloalkyl group:
R⁷ and R⁹ are the same or different and each is
1) a hydrogen atom,
2) a halogen atom,
3) a trifluoromethyl group,
4) a C₁₋₆ alkyl group,
5) a C₁₋₆ alkoxy group,
6) a C₃₋₈ cycloalkyl group, or
7) a nitro group
(hereinafter also referred to as compound [A-5]) is preferable.

Of compounds [A-5], a compound represented by the following formula [A-6]:

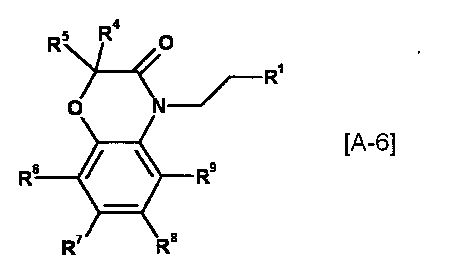

wherein
R¹ is
1) -COOH, or
2) a -COO-C₁₋₆ alkyl group:
R⁴ is
1) a hydrogen atom, or
2) a C₂₋₆ alkyl group, :
R⁵ is
1) a C₂₋₆ alkyl group, or
2) a phenyl group:
R⁶ and R⁸ are the same or different and each is
1) a halogen atom,
2) a trifluoromethyl group,
3) a C₁₋₆ alkyl group,
4) a C₁₋₆ alkoxy group, or
5) a C₃₋₈ cycloalkyl group:
R⁷ and R⁹ are the same or different and each is
1) a hydrogen atom,
2) a halogen atom,
3) a trifluoromethyl group,
4) a C₁₋₆ alkyl group,
5) a C₁₋₆ alkoxy group,
6) a C₃₋₈ cycloalkyl group, or
7) a nitro group,
provided that at least one of R⁶, R⁷, R⁸ and R⁹ is a C₁₋₆ alkyl group, a trifluoromethyl group, a C₁₋₆ alkoxy group, or a C₃₋₈ cycloalkyl group
(hereinafter also referred to as compound [A-6]), or a compound represented by the following formula [C-1]:

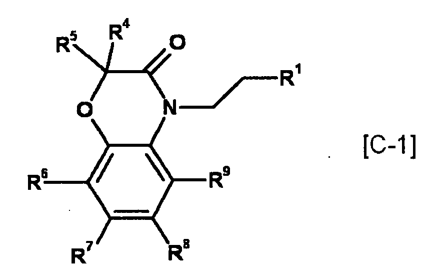

wherein
R¹ is
1) -COOH, or
2) a -COO-C₁₋₆ alkyl group:
R⁴ is
1) a hydrogen atom, or
2) a C₂₋₆ alkyl group, :
R⁵ is
1) a C₂₋₆ alkyl group, or
2) a phenyl group:
R⁶ and R⁸ are each a halogen atom (preferably chlorine atom);
R⁷ and R⁹ are each a hydrogen atom
(hereinafter also referred to as compound [C-1]) is more preferable.

Moreover, of compounds [C-1], a compound represented by the following formula [C-2]:

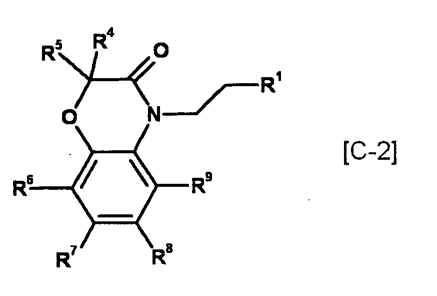

wherein
R¹ is -COOH;
R⁴ is a hydrogen atom;
R⁵ is
1) an isopropyl group or
2) a sec-butyl group:
R⁶ and R⁸ are each a chlorine atom;
R⁷ and R⁹ are each a hydrogen atom
(hereinafter also referred to as compound [C-2]) is more preferable.

Furthermore, of compounds [A-6], a compound represented by the following formula [A-7]:

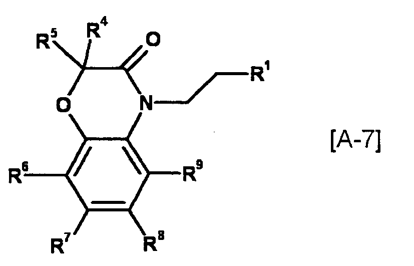

wherein
R¹ is
1) -COOH, or
2) a -COO-C₁₋₂ alkyl group:
R⁴ is
1) a hydrogen atom, or
2) a C₃₋₄ alkyl group:
R⁵ is a C₃₋₄ alkyl group;
R⁶ and R⁸ are the same or different and each is
1) a halogen atom,
2) a trifluoromethyl group,
3) a C₁₋₆ alkyl group, or
4) a C₅₋₆ cycloalkyl group:
R⁷ and R⁹ are the same or different and each is
1) a hydrogen atom,
2) a halogen atom,
3) a trifluoromethyl group,
4) a C₁₋₆ alkyl group,
5) a C₁₋₆ alkoxy group, or
6) a C₅₋₆ cycloalkyl group,
provided that at least one of R⁶, R⁷, R⁸ and R⁹ is a C₁₋₆ alkyl group, a trifluoromethyl group, a C₁₋₆ alkoxy group, or a C₅₋₆ cycloalkyl group
(hereinafter also referred to as compound [A-7]) is more preferable.

Especially, of compounds [A-7], a compound represented by the following formula [A-8]:

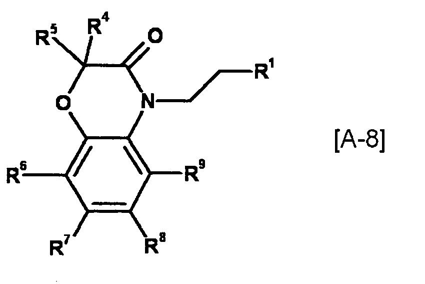

wherein
R¹ is
1) -COOH, or
2) a -COO-C₁₋₂ alkyl group:
R⁴ is a hydrogen atom;
R⁵ is a C₃₋₄ alkyl group;
R⁶ and R⁸ are the same or different and each is
1) a halogen atom,
2) a trifluoromethyl group,
3) a C₁₋₆ alkyl group, or
4) a C₅₋₆ cycloalkyl group:
R⁷ is
1) a hydrogen atom,
2) a halogen atom,
3) a trifluoromethyl group, or
4) a C₁₋₆ alkyl group:
R⁹ is a hydrogen atom,
provided that at least one of R⁶, R⁷ and R⁸ is a C₁₋₆ alkyl group, a trifluoromethyl group, or a C₅₋₆ cycloalkyl group
(hereinafter also referred to as compound [A-8]) is particularly preferable.

More especially, of compounds [A-8], a compound represented by the following formula [A-9]:

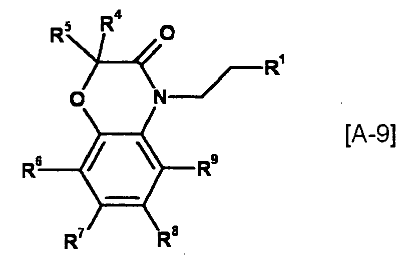

wherein
R¹ is -COOH;
R⁴ is a hydrogen atom;
R⁵ is a C₃₋₄ alkyl group;
R⁶ is
1) a chlorine atom, or
2) a C₁₋₆ alkyl group:
R⁷ is
1) a hydrogen atom, or
2) a C₁₋₆ alkyl group:
R⁸ is
1) a chlorine atom, or
2) a C₁₋₆ alkyl group:
R⁹ is a hydrogen atom
provided that at least one of R⁶, R⁷ and R⁸ is a C₁₋₆ alkyl group (hereinafter also referred to as compound [A-9]) is still more preferable.

As compound [A-1], compound selected from the following group or a pharmaceutically acceptable salt thereof, or a solvate thereof is also preferable:
(8-isopropyl-3-oxo-2,3-dihydrobenzo[1,4]oxazin-4-yl)acetic acid (Example 10),
3-(2,8-diisopropyl-3-oxo-2,3-dihydrobenzo[1,4]oxazin-4-yl)propionic acid (Example 14),
(8-isopropyl-3-thioxo-2,3-dihydrobenzo[1,4]oxazin-4-yl)acetic acid (Example 15),
3-(2,8-diisopropyl-3-thioxo-2,3-dihydrobenzo[1,4]oxazin-4-yl)propionic acid (Example 16),
4-(2,8-diisopropyl-3-oxo-2,3-dihydrobenzo[1,4]oxazin-4-yl)butyric acid (Example 18),
4-(2,8-diisopropyl-3-thioxo-2,3-dihydrobenzo[1,4]oxazin-4-yl)butyric acid (Example 20),
(8-isopropyl-3-oxo-2-phenyl-2,3-dihydrobenzo[1,4]oxazin-4-yl)acetic acid (Example 26),
3-(8-isopropyl-2,2-dimethyl-3-oxo-2,3-dihydrobenzo[1,4]oxazin-4-yl)propionic acid (Example 33),
(8-isopropyl-2,2-dimethyl-3-oxo-2,3-dihydrobenzo[1,4]oxazin-4-yl)acetic acid (Example 34),
3-(8-isopropyl-3-oxo-2,3-dihydrobenzo[1,4]oxazin-4-yl)propionic acid (Example 35),
3-(8-isopropyl-3-oxo-2-phenyl-2,3-dihydrobenzo[1,4]oxazin-4-yl)propionic acid (Example 38),
(2-ethyl-8-isopropyl-3-oxo-2,3-dihydrobenzo[1,4]oxazin-4-yl)acetic acid (Example 39),
3-(2-ethyl-8-isopropyl-3-oxo-2,3-dihydrobenzo[1,4]oxazin-4-yl)propionic acid (Example 40),
2-[(2,8-diisopropyl-3-oxo-2,3-dihydrobenzo[1,4]oxazin-4-yl)methyl]benzoic acid (Example 41),
3-(2,8-diisopropyl-3-oxo-2,3-dihydrobenzo[1,4]oxazin-4-yl)-2,2-dimethylpropionic acid (Example 42),
3-(2-isopropyl-7-methyl-3-oxo-2,3-dihydrobenzo[1,4]oxazin-4-yl)propionic acid (Example 43),
3-(2-isopropyl-8-methyl-3-oxo-2,3-dihydrobenzo[1,4]oxazin-4-yl)propionic acid (Example 44),
3-(8-tert-butyl-2-isopropyl-3-oxo-2,3-dihydrobenzo[1,4]oxazin-4-yl)propionic acid (Example 46),
3-(8-methyl-3-oxo-2-phenyl-2,3-dihydrobenzo[1,4]oxazin-4-yl)propionic acid (Example 47),
3-(7-methyl-3-oxo-2-phenyl-2,3-dihydrobenzo[1,4]oxazin-4-yl)propionic acid (Example 48),
3-(8-tert-butyl-3-oxo-2-phenyl-2,3-dihydrobenzo[1,4]oxazin-4-yl)propionic acid (Example 49),
3-(8-cyclohexyl-2-isopropyl-3-oxo-2,3-dihydrobenzo[1,4]oxazin-4-yl)propionic acid (Example 50),
3-(8-ethyl-2-isopropyl-3-oxo-2,3-dihydrobenzo[1,4]oxazin-4-yl)propionic acid (Example 53),
3-(2-isopropyl-6-methyl-3-oxo-2,3-dihydrobenzo[1,4]oxazin-4-yl)propionic acid (Example 58),
3-(8-ethyl-3-oxo-2-phenyl-2,3-dihydrobenzo[1,4]oxazin-4-yl)propionic acid (Example 59),
3-(2-isopropyl-6,8-dimethyl-3-oxo-2,3-dihydrobenzo[1,4]oxazin-4-yl)propionic acid (Example 61),
3-(6,8-dimethyl-3-oxo-2-phenyl-2,3-dihydrobenzo[1,4]oxazin-4-yl)propionic acid (Example 72),
3-((S)-2-isopropyl-6,8-dimethyl-3-oxo-2,3-dihydrobenzo[1,4]oxazin-4-yl)propionic acid (Example 73),
3-((R)-2-isopropyl-6,8-dimethyl-3-oxo-2,3-dihydrobenzo[1,4]oxazin-4-yl)propionic acid (Example 74),
3-(6-methyl-3-oxo-2-phenyl-2,3-dihydrobenzo[1,4]oxazin-4-yl)propionic acid (Example 75),
3-(6-chloro-2-isopropyl-8-methyl-3-oxo-2,3-dihydrobenzo[1,4]oxazin-4-yl)propionic acid (Example 81),
3-(6,8-dimethyl-3-oxo-2,3-dihydrobenzo[1,4]oxazin-4-yl)propionic acid (Example 82),
3-(2,6,8-trimethyl-3-oxo-2,3-dihydrobenzo[1,4]oxazin-4-yl)propionic acid (Example 86),
3-(2-ethyl-6,8-dimethyl-3-oxo-2,3-dihydrobenzo[1,4]oxazin-4-yl)propionic acid (Example 87),
3-(6-tert-butyl-2-isopropyl-3-oxo-2,3-dihydrobenzo[1,4]oxazin-4-yl)propionic acid (Example 88),
3-(2,2,6,8-tetramethyl-3-oxo-2,3-dihydrobenzo[1,4]oxazin-4-yl)propionic acid (Example 89),
3-(2-isobutyl-6,8-dimethyl-3-oxo-2,3-dihydrobenzo[1,4]oxazin-4-yl)propionic acid (Example 90),
3-((R)-2-isobutyl-6,8-dimethyl-3-oxo-2,3-dihydrobenzo[1,4]oxazin-4-yl)propionic acid (Example 91),
3-(2-benzyl-6,8-dimethyl-3-oxo-2,3-dihydrobenzo[1,4]oxazin-4-yl)propionic acid (Example 92),
3-(2-butyl-6,8-dimethyl-3-oxo-2,3-dihydrobenzo[1,4]oxazin-4-yl)propionic acid (Example 93),
3-(6,8-dichloro-2-isopropyl-7-methyl-3-oxo-2,3-dihydrobenzo[1,4]oxazin-4-yl)propionic acid (Example 94),
3-(2-isopropyl-3-oxo-6-trifluoromethyl-2,3-dihydrobenzo[1,4]oxazin-4-yl)propionic acid (Example 97),
3-(8-chloro-2-isopropyl-6-methyl-3-oxo-2,3-dihydrobenzo[1,4]oxazin-4-yl)propionic acid (Example 98),
3-(2-isopropyl-3-oxo-2,3-dihydronaphtho[1,2-b][1,4]oxazin-4-yl)propionic acid (Example 99),
3-(6-ethyl-2-isopropyl-8-methyl-3-oxo-2,3-dihydrobenzo[1,4]oxazin-4-yl)propionic acid (Example 101),
3-(6,8-dimethyl-3-oxo-2-propyl-2,3-dihydrobenzo[1,4]oxazin-4-yl)propionic acid (Example 102),
3-[2-isopropyl-3-oxo-6,8-bis(trifluoromethyl)-2,3-dihydrobenzo[1,4]oxazin-4-yl]propionic acid (Example 103),
3-(2-isopropyl-6-methoxy-8-methyl-3-oxo-2,3-dihydrobenzo[1,4]oxazin-4-yl)propionic acid (Example 104),
3-[(R)-2-((S)-sec-butyl)-6,8-dimethyl-3-oxo-2,3-dihydrobenzo[1,4]oxazin-4-yl]propionic acid (Example 105),
3-((R)-8-chloro-2-isopropyl-6-methyl-3-oxo-2,3-dihydrobenzo[1,4]oxazin-4-yl)propionic acid (Example 106),
3-((R)-6-chloro-2-isopropyl-8-methyl-3-oxo-2,3-dihydrobenzo[1,4]oxazin-4-yl)propionic acid (Example 107),
3-((S)-8-chloro-2-isopropyl-6-methyl-3-oxo-2,3-dihydrobenzo[1,4]oxazin-4-yl)propionic acid (Example 109),
3-((S)-6-chloro-2-isopropyl-8-methyl-3-oxo-2,3-dihydrobenzo[1,4]oxazin-4-yl)propionic acid (Example 110), (3-oxo-2,3-dihydrobenzo[1,4]oxazin-4-yl)acetic acid methyl ester (Example 1),
(3-oxo-2,3-dihydrobenzo[1,4]thiazin-4-yl)acetic acid methyl ester (Example 2),
(2,2-dimethyl-3-oxo-2,3-dihydrobenzo[1,4]oxazin-4-yl)acetic acid (Example 5),
(2-isopropyl-3-thioxo-2,3-dihydrobenzo[1,4]oxazin-4-yl)acetic acid (Example 13),
(3-thioxo-2,3-dihydrobenzo[1,4]oxazin-4-yl)acetic acid (Example 17),
3-(2-isopropyl-3-oxo-2,3-dihydrobenzo[1,4]thiazin-4-yl)propionic acid (Example 22),
(2-isopropyl-3-oxo-2,3-dihydrobenzo[1,4]thiazin-4-yl)acetic acid (Example 30),
3-(2-isopropyl-3-oxo-2,3-dihydrobenzo[1,4]oxazin-4-yl)propionic acid (Example 32),
3-(8-bromo-2-isopropyl-3-oxo-2,3-dihydrobenzo[1,4]oxazin-4-yl)propionic acid (Example 51),
3-(7-chloro-2-isopropyl-3-oxo-2,3-dihydrobenzo[1,4]oxazin-4-yl)propionic acid (Example 62),
3-(6-chloro-2-isopropyl-3-oxo-2,3-dihydrobenzo[1,4]oxazin-4-yl)propionic acid (Example 63),
3-(8-bromo-3-oxo-2-phenyl-2,3-dihydrobenzo[1,4]oxazin-4-yl)propionic acid (Example 66),
3-(7-chloro-3-oxo-2-phenyl-2,3-dihydrobenzo[1,4]oxazin-4-yl)propionic acid (Example 76),
3-(6-chloro-3-oxo-2-phenyl-2,3-dihydrobenzo[1,4]oxazin-4-yl)propionic acid (Example 77),
3-(6,8-dichloro-2-isopropyl-3-oxo-2,3-dihydrobenzo[1,4]oxazin-4-yl)propionic acid (Example 80),
3-(2-isopropyl-7-nitro-3-oxo-2,3-dihydrobenzo[1,4]oxazin-4-yl)propionic acid (Example 95),
3-(2-isopropyl-6-nitro-3-oxo-2,3-dihydrobenzo[1,4]oxazin-4-yl)propionic acid (Example 96),
3-(8-chloro-2-isopropyl-3-oxo-2,3-dihydrobenzo[1,4]oxazin-4-yl)propionic acid (Example 100),
3-((R)-6,8-dichloro-2-isopropyl-3-oxo-2,3-dihydrobenzo[1,4]oxazin-4-yl)propionic acid (Example 108), and
3-((S)-6,8-dichloro-2-isopropyl-3-oxo-2,3-dihydrobenzo[1,4]oxazin-4-yl)propionic acid (Example 111).

Of these, a compound selected from the following group or a pharmaceutically acceptable salt thereof, or a solvate thereof is more preferable:
(8-isopropyl-3-oxo-2,3-dihydrobenzo[1,4]oxazin-4-yl)acetic acid (Example 10),
3-(2,8-diisopropyl-3-oxo-2,3-dihydrobenzo[1,4]oxazin-4-yl)propionic acid (Example 14),
(8-isopropyl-3-thioxo-2,3-dihydrobenzo[1,4]oxazin-4-yl)acetic acid (Example 15),
3-(2,8-diisopropyl-3-thioxo-2,3-dihydrobenzo[1,4]oxazin-4-yl)propionic acid (Example 16),
4-(2,8-diisopropyl-3-oxo-2,3-dihydrobenzo[1,4]oxazin-4-yl)butyric acid (Example 18),
4-(2,8-diisopropyl-3-thioxo-2,3-dihydrobenzo[1,4]oxazin-4-yl)butyric acid (Example 20),
(8-isopropyl-3-oxo-2-phenyl-2,3-dihydrobenzo[1,4]oxazin-4-yl)acetic acid (Example 26),
3-(8-isopropyl-2,2-dimethyl-3-oxo-2,3-dihydrobenzo[1,4]oxazin-4-yl)propionic acid (Example 33),
(8-isopropyl-2,2-dimethyl-3-oxo-2,3-dihydrobenzo[1,4]oxazin-4-yl)acetic acid (Example 34),
3-(8-isopropyl-3-oxo-2,3-dihydrobenzo[1,4]oxazin-4-yl)propionic acid (Example 35),
3-(8-isopropyl-3-oxo-2-phenyl-2,3-dihydrobenzo[1,4]oxazin-4-yl)propionic acid (Example 38),
(2-ethyl-8-isopropyl-3-oxo-2,3-dihydrobenzo[1,4]oxazin-4-yl)acetic acid (Example 39),
3-(2-ethyl-8-isopropyl-3-oxo-2,3-dihydrobenzo[1,4]oxazin-4-yl)propionic acid (Example 40),
2-[(2,8-diisopropyl-3-oxo-2,3-dihydrobenzo[1,4]oxazin-4-yl)methyl]benzoic acid (Example 41),
3-(2,8-diisopropyl-3-oxo-2,3-dihydrobenzo[1,4]oxazin-4-yl)-2,2-dimethylpropionic acid (Example 42),
3-(2-isopropyl-7-methyl-3-oxo-2,3-dihydrobenzo[1,4]oxazin-4-yl)propionic acid (Example 43),
3-(2-isopropyl-8-methyl-3-oxo-2,3-dihydrobenzo[1,4]oxazin-4-yl)propionic acid (Example 44),
3-(8-tert-butyl-2-isopropyl-3-oxo-2,3-dihydrobenzo[1,4]oxazin-4-yl)propionic acid (Example 46),
3-(8-methyl-3-oxo-2-phenyl-2,3-dihydrobenzo[1,4]oxazin-4-yl)propionic acid (Example 47),
3-(7-methyl-3-oxo-2-phenyl-2,3-dihydrobenzo[1,4]oxazin-4-yl)propionic acid (Example 48),
3-(8-tert-butyl-3-oxo-2-phenyl-2,3-dihydrobenzo[1,4]oxazin-4-yl)propionic acid (Example 49),
3-(8-cyclohexyl-2-isopropyl-3-oxo-2,3-dihydrobenzo[1,4]oxazin-4-yl)propionic acid (Example 50),
3-(8-ethyl-2-isopropyl-3-oxo-2,3-dihydrobenzo[1,4]oxazin-4-yl)propionic acid (Example 53),
3-(2-isopropyl-6-methyl-3-oxo-2,3-dihydrobenzo[1,4]oxazin-4-yl)propionic acid (Example 58),
3-(8-ethyl-3-oxo-2-phenyl-2,3-dihydrobenzo[1,4]oxazin-4-yl)propionic acid (Example 59),
3-(2-isopropyl-6,8-dimethyl-3-oxo-2,3-dihydrobenzo[1,4]oxazin-4-yl)propionic acid (Example 61),
3-(6,8-dimethyl-3-oxo-2-phenyl-2,3-dihydrobenzo[1,4]oxazin-4-yl)propionic acid (Example 72),
3-((S)-2-isopropyl-6,8-dimethyl-3-oxo-2,3-dihydrobenzo[1,4]oxazin-4-yl)propionic acid (Example 73),
3-((R)-2-isopropyl-6,8-dimethyl-3-oxo-2,3-dihydrobenzo[1,4]oxazin-4-yl)propionic acid (Example 74),
3-(6-methyl-3-oxo-2-phenyl-2,3-dihydrobenzo[1,4]oxazin-4-yl)propionic acid (Example 75),
3-(6-chloro-2-isopropyl-8-methyl-3-oxo-2,3-dihydrobenzo[1,4]oxazin-4-yl)propionic acid (Example 81),
3-(6,8-dimethyl-3-oxo-2,3-dihydrobenzo[1,4]oxazin-4-yl)propionic acid (Example 82),
3-(2,6,8-trimethyl-3-oxo-2,3-dihydrobenzo[1,4]oxazin-4-yl)propionic acid (Example 86),
3-(2-ethyl-6,8-dimethyl-3-oxo-2,3-dihydrobenzo[1,4]oxazin-4-yl)propionic acid (Example 87),
3-(6-tert-butyl-2-isopropyl-3-oxo-2,3-dihydrobenzo[1,4]oxazin-4-yl)propionic acid (Example 88),
3-(2,2,6,8-tetramethyl-3-oxo-2,3-dihydrobenzo[1,4]oxazin-4-yl)propionic acid (Example 89),
3-(2-isobutyl-6,8-dimethyl-3-oxo-2,3-dihydrobenzo[1,4]oxazin-4-yl)propionic acid (Example 90),
3-((R)-2-isobutyl-6,8-dimethyl-3-oxo-2,3-dihydrobenzo[1,4]oxazin-4-yl)propionic acid (Example 91),
3-(2-benzyl-6,8-dimethyl-3-oxo-2,3-dihydrobenzo[1,4]oxazin-4-yl)propionic acid (Example 92),
3-(2-butyl-6,8-dimethyl-3-oxo-2,3-dihydrobenzo[1,4]oxazin-4-yl)propionic acid (Example 93),
3-(6,8-dichloro-2-isopropyl-7-methyl-3-oxo-2,3-dihydrobenzo[1,4]oxazin-4-yl)propionic acid (Example 94),
3-(2-isopropyl-3-oxo-6-trifluoromethyl-2,3-dihydrobenzo[1,4]oxazin-4-yl)propionic acid (Example 97),
3-(8-chloro-2-isopropyl-6-methyl-3-oxo-2,3-dihydrobenzo[1,4]oxazin-4-yl)propionic acid (Example 98),
3-(2-isopropyl-3-oxo-2,3-dihydronaphtho[1,2-b][1,4]oxazin-4-yl)propionic acid (Example 99),
3-(6-ethyl-2-isopropyl-8-methyl-3-oxo-2,3-dihydrobenzo[1,4]oxazin-4-yl)propionic acid (Example 101),
3-(6,8-dimethyl-3-oxo-2-propyl-2,3-dihydrobenzo[1,4]oxazin-4-yl)propionic acid (Example 102),
3-[2-isopropyl-3-oxo-6,8-bis(trifluoromethyl)-2,3-dihydrobenzo[1,4]oxazin-4-yl]propionic acid (Example 103),
3-(2-isopropyl-6-methoxy-8-methyl-3-oxo-2,3-dihydrobenzo[1,4]oxazin-4-yl)propionic acid (Example 104),
3-[(R)-2-((S)-sec-butyl)-6,8-dimethyl-3-oxo-2,3-dihydrobenzo[1,4]oxazin-4-yl]propionic acid (Example 105),
3-((R)-8-chloro-2-isopropyl-6-methyl-3-oxo-2,3-dihydrobenzo[1,4]oxazin-4-yl)propionic acid (Example 106),
3-((R)-6-chloro-2-isopropyl-8-methyl-3-oxo-2,3-dihydrobenzo[1,4]oxazin-4-yl)propionic acid (Example 107),
3-((S)-8-chloro-2-isopropyl-6-methyl-3-oxo-2,3-dihydrobenzo[1,4]oxazin-4-yl)propionic acid (Example 109), and
3-((S)-6-chloro-2-isopropyl-8-methyl-3-oxo-2,3-dihydrobenzo[1,4]oxazin-4-yl)propionic acid (Example 110).

Of these, a compound selected from the following group or a pharmaceutically acceptable salt thereof, or a solvate thereof is particularly preferable:
3-(2-isopropyl-6,8-dimethyl-3-oxo-2,3-dihydrobenzo[1,4]oxazin-4-yl)propionic acid (Example 61),
3-((S)-2-isopropyl-6,8-dimethyl-3-oxo-2,3-dihydrobenzo[1,4]oxazin-4-yl)propionic acid (Example 73),
3-((R)-2-isopropyl-6,8-dimethyl-3-oxo-2,3-dihydrobenzo[1,4]oxazin-4-yl)propionic acid (Example 74),
3-(6-chloro-2-isopropyl-8-methyl-3-oxo-2,3-dihydrobenzo[1,4]oxazin-4-yl)propionic acid (Example 81),
3-(2-isobutyl-6,8-dimethyl-3-oxo-2,3-dihydrobenzo[1,4]oxazin-4-yl)propionic acid (Example 90),
3-((R)-2-isobutyl-6,8-dimethyl-3-oxo-2,3-dihydrobenzo[1,4]oxazin-4-yl)propionic acid (Example 91),
3-(8-chloro-2-isopropyl-6-methyl-3-oxo-2,3-dihydrobenzo[1,4]oxazin-4-yl)propionic acid (Example 98),
3-(6-ethyl-2-isopropyl-8-methyl-3-oxo-2,3-dihydrobenzo[1,4]oxazin-4-yl)propionic acid (Example 101),
3-(6,8-dimethyl-3-oxo-2-propyl-2,3-dihydrobenzo[1,4]oxazin-4-yl)propionic acid (Example 102),
3-[(R)-2-((S)-sec-butyl)-6,8-dimethyl-3-oxo-2,3-dihydrobenzo[1,4]oxazin-4-yl]propionic acid (Example 105),
3-((R)-8-chloro-2-isopropyl-6-methyl-3-oxo-2,3-dihydrobenzo[1,4]oxazin-4-yl)propionic acid (Example 106), and
3-((R)-6-chloro-2-isopropyl-8-methyl-3-oxo-2,3-dihydrobenzo[1,4]oxazin-4-yl)propionic acid (Example.107).

### Compound [B-1]

Compound [B-1] is a compound [1'] wherein Q is -CH₂-, i.e., a compound represented by the following formula [B-1]:

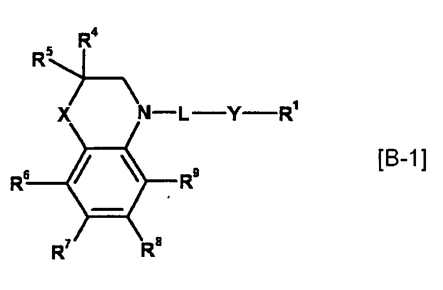

wherein
X is
1) an oxygen atom,
2) a sulfur atom,
3) -S(=O)- or
4) -S(=O)₂-:
L is
1) a single bond, or
2) -(C=O)-:
Y is
1) a single bond,
2) a C₁₋₄ alkylene group optionally substituted by the same or different one or more substituents selected from the aforementioned group A,
3) a C₂₋₄ alkenylene group optionally substituted by the same or different one or more substituents selected from the aforementioned group A, or
4) a C₁₋₄ alkylene-C₆₋₁₄ arylene group optionally substituted by the same or different one or more substituents selected from the aforementioned group A:
R¹ is
1) -COOH, or
2) -COOR wherein R is
   (a) a C₁₋₆ alkyl group optionally substituted by the same or different one or more substituents selected from the aforementioned group A,
   (b) an allyl group optionally substituted by the same or different one or more substituents selected from the aforementioned group A,
   (c) a phenyl group optionally substituted by the same or different one or more substituents selected from the aforementioned group A, or
   (d) a benzyl group optionally substituted by the same or different one or more substituents selected from the aforementioned group A;
R⁴ and R⁵ are the same or different and each is
1) a hydrogen atom,
2) a C₁₋₆ alkyl group optionally substituted by the same or different one or more substituents selected from the aforementioned group A,
3) a C₂₋₆ alkenyl group optionally substituted by the same or different one or more substituents selected from the aforementioned group A,
4) a C₆₋₁₄ aryl group optionally substituted by the same or different one or more substituents selected from the aforementioned group A,
5) a C₇₋₁₂ aralkyl group optionally substituted by the same or different one or more substituents selected from the aforementioned group A, or
6) a halogen atom, or
7) R⁴ and R⁵ form, together with the carbon atom bonded thereto, a saturated carbocycle having 3 to 6 carbon atoms (said carbocycle is optionally substituted by the same or different one or more substituents selected from the aforementioned group A);
R⁶, R⁷, R⁸ and R⁹ are the same or different and each is
1) a hydrogen atom,
2) a halogen atom,
3) a C₁₋₆ alkyl group optionally substituted by the same or different one or more substituents selected from the aforementioned group A,
4) a C₁₋₆ alkoxy group optionally substituted by the same or different one or more substituents selected from the aforementioned group A,
5) a C₃₋₈ cycloalkyl group optionally substituted by the same or different one or more substituents selected from the aforementioned group A,
6) a C₆₋₁₄ aryl group optionally substituted by the same or different one or more substituents selected from the aforementioned group A,
7) a nitro group,
8) a cyano group,
9) -COOH, or
10) -COOR wherein R is as defined above, or
11) R⁶ and R⁷, R⁷ and R⁸ or R⁸ and R⁹ form, together with the carbon atom bonded thereto, a saturated or unsaturated carbocycle having 3 to 14 carbon atoms (said carbocycle is optionally substituted by the same or different one or more substituents selected from the aforementioned group A), provided that the following compounds are excluded,
   (E)-4-(2,2-dimethyl-6-nitro-2,3-dihydrobenzo[1,4]oxazin-4-yl)-4-oxo-2-butenoic acid,
   (6-methoxy-8-methyl-2,3-dihydrobenzo[1,4]oxazin-4-yl)acetic acid,
   (2,2-dimethyl-6-nitro-2,3-dihydrobenzo[1,4]oxazin-4-yl)acetic acid, and
   (2E,4E)-3-methyl-6-oxo-6-(6,6,9,9-tetramethyl-2,3,6,7,8,9-hexahydronaphtho[2,3-b][1,4]oxazin-4-yl)2,4-hexadienoic acid.

Of these, a compound wherein
X is
1) an oxygen atom, or
2) a sulfur atom:
L is
1) a single bond, or
2) -(C=O)-:
Y is
1) a single bond,
2) a C₁₋₄ alkylene group optionally substituted by the same or different one or more substituents selected from the aforementioned group A,
3) a C₂₋₄ alkenylene group optionally substituted by the same or different one or more substituents selected from the aforementioned group A, or
4) a C₁₋₄ alkylene-C₆₋₁₄ arylene group optionally substituted by the same or different one or more substituents selected from the aforementioned group A:
R¹ is
1) -COOH, or
2) -COOR wherein R is
   (a) a C₁₋₆ alkyl group optionally substituted by the same or different one or more substituents selected from the aforementioned group A,
   (b) an allyl group optionally substituted by the same or different one or more substituents selected from the aforementioned group A,
   (c) a phenyl group optionally substituted by the same or different one or more substituents selected from the aforementioned group A, or
   (d) a benzyl group optionally substituted by the same or different one or more substituents selected from the aforementioned group A;
R⁴ and R⁵ are the same or different and each is
1) a hydrogen atom,
2) a C₁₋₆ alkyl group,
3) a phenyl group, or
4) a benzyl group:
R⁶, R⁷, R⁸ and R⁹ are the same or different and each is
1) a hydrogen atom,
2) a halogen atom,
3) a trifluoromethyl group,
4) a C₁₋₆ alkyl group,
5) a C₁₋₆ alkoxy group,
6) a C₃₋₈ cycloalkyl group, or
7) a nitro group, or
8) R⁶ and R⁷, R⁷ and R⁸ or R⁸ and R⁹ form, together with the carbon atom bonded thereto, a saturated or unsaturated carbocycle having 3 to 14 carbon atoms (said carbocycle is optionally substituted by the same or different one or more substituents selected from the aforementioned group A), provided that the following compounds are excluded,
   (E)-4-(2,2-dimethyl-6-nitro-2,3-dihydrobenzo[1,4]oxazin-4-yl)-4-oxo-2-butenoic acid,
   (6-methoxy-8-methyl-2,3-dihydrobenzo[1,4]oxazin-4-yl)acetic acid,
   (2,2-dimethyl-6-nitro-2,3-dihydrobenzo[1,4]oxazin-4-yl)acetic acid, and
   (2E,4E)-3-methyl-6-oxo-6-(6,6,9,9-tetramethyl-2,3,6,7,8,9-hexahydronaphtho[2,3-b][1,4]oxazin-4-yl)2,4-hexadienoic acid is preferable.

Especially, a compound wherein
X is an oxygen atom;
L is -(C=O)-;
Y is
1) a single bond,
2) a C₁₋₄ alkylene group, or
3) a C₂₋₄ alkenylene group:
R¹ is
1) -COOH, or
2) a -COO-C₁₋₆ alkyl group:
R⁴ and R⁵ are the same or different and each is
1) a hydrogen atom,
2) a C₂₋₆ alkyl group,
3) a phenyl group, or
4) a benzyl group:
R⁶ and R⁸ are the same or different and each is
1) a halogen atom,
2) a trifluoromethyl group,
3) a C₁₋₆ alkyl group, or
4) a C₃₋₈ cycloalkyl group:
R⁷ and R⁹ are each a hydrogen atom
is more preferable.

Particularly, a compound wherein
X is an oxygen atom;
L is -(C=O)-;
Y is
1) a single bond, or
2) a vinylene group:
R¹ is -COOH;
R⁴ is a hydrogen atom;
R⁵ is a phenyl group;
R⁶ and R⁸ are the same or different and each is
1) a chlorine atom,
2) a trifluoromethyl group, or
3) a C₁₋₆ alkyl group:
R⁷ and R⁹ are each a hydrogen atom
is more preferable.

As compound [B-1], a compound selected from the following group or a pharmaceutically acceptable salt thereof, or a solvate thereof is also preferable:
4-(2,8-diisopropyl-2,3-dihydrobenzo[1,4]oxazin-4-yl)-4-oxobutyric acid (Example 21),
4-(2,3-dihydrobenzo[1,4]oxazin-4-yl)-4-oxobutyric acid (Example 23),
5-(2,3-dihydrobenzo[1,4]oxazin-4-yl)-5-oxovaleric acid (Example 24),
(Z)-4-(2,3-dihydrobenzo[1,4]oxazin-4-yl)-4-oxo-2-butenoic acid (Example 25),
6-(2,3-dihydrobenzo[1,4]oxazin-4-yl)-6-oxohexanoic acid (Example 27),
(E)-4-(2,3-dihydrobenzo[1,4]oxazin-4-yl)-4-oxo-2-butenoic acid (Example 28),
(2,8-diisopropyl-2,3-dihydrobenzo[1,4]oxazin-4-yl)oxoacetic acid (Example 29),
3-(2,8-diisopropyl-2,3-dihydrobenzo[1,4]oxazin-4-yl)-3-oxopropionic acid (Example 31),
(E)-4-(2,8-diisopropyl-2,3-dihydrobenzo[1,4]oxazin-4-yl)-4-oxo-2-butenoic acid (Example 36),
(Z)-4-(2,8-diisopropyl-2,3-dihydrobenzo[1,4]oxazin-4-yl)-4-oxo-2-butenoic acid (Example 37),
(E)-4-(8-isopropyl-2-phenyl-2,3-dihydrobenzo[1,4]oxazin-4-yl)-4-oxo-2-butenoic acid (Example 45),
(E)-4-(8-tert-butyl-2-isopropyl-2,3-dihydrobenzo[1,4]oxazin-4-yl)-4-oxo-2-butenoic acid (Example 52),
(E)-4-(2-isopropyl-8-methyl-2,3-dihydrobenzo[1,4]oxazin-4-yl)-4-oxo-2-butenoic acid (Example 54),
(E)-4-(8-methyl-2-phenyl-2,3-dihydrobenzo[1,4]oxazin-4-yl)-4-oxo-2-butenoic acid (Example 55),
(E)-4-(2-isopropyl-7-methyl-2,3-dihydrobenzo[1,4]oxazin-4-yl)-4-oxo-2-butenoic acid (Example 56),
(E)-4-(7-methyl-2-phenyl-2,3-dihydrobenzo[1,4]oxazin-4-yl)-4-oxo-2-butenoic acid (Example 57),
(E)-4-(8-ethyl-2-isopropyl-2,3-dihydrobenzo[1,4]oxazin-4-yl)-4-oxo-2-butenoic acid (Example 60),
(E)-4-(2-isopropyl-6-methyl-2,3-dihydrobenzo[1,4]oxazin-4-yl)-4-oxo-2-butenoic acid (Example 64),
(E)-4-(6-methyl-2-phenyl-2,3-dihydrobenzo[1,4]oxazin-4-yl)-4-oxo-2-butenoic acid (Example 65),
(E)-4-(2-ethyl-8-isopropyl-2,3-dihydrobenzo[1,4]oxazin-4-yl)-4-oxo-2-butenoic acid (Example 67),
(E)-4-(7-chloro-2-isopropyl-2,3-dihydrobenzo[1,4]oxazin-4-yl)-4-oxo-2-butenoic acid (Example 68),
(E)-4-(6-chloro-2-isopropyl-2,3-dihydrobenzo[1,4]oxazin-4-yl)-4-oxo-2-butenoic acid (Example 69),
(E)-4-(7-chloro-2-phenyl-2,3-dihydrobenzo[1,4]oxazin-4-yl)-4-oxo-2-butenoic acid (Example 70),
(E)-4-(6-chloro-2-phenyl-2,3-dihydrobenzo[1,4]oxazin-4-yl)-4-oxo-2-butenoic acid (Example 71),
(E)-4-(8-bromo-2-isopropyl-2,3-dihydrobenzo[1,4]oxazin-4-yl)-4-oxo-2-butenoic acid (Example 78),
(E)-4-(8-cyclohexyl-2-isopropyl-2,3-dihydrobenzo[1,4]oxazin-4-yl)-4-oxo-2-butenoic acid (Example 79),
(E)-4-(6-chloro-2-isopropyl-8-methyl-2,3-dihydrobenzo[1,4]oxazin-4-yl)-4-oxo-2-butenoic acid (Example 83),
(E)-4-(6-chloro-8-methyl-2-phenyl-2,3-dihydrobenzo[1,4]oxazin-4-yl)-4-oxo-2-butenoic acid (Example 84), and
(E)-4-(6,8-dimethyl-2,3-dihydrobenzo[1,4]oxazin-4-yl)-4-oxo-2-butenoic acid (Example 85).

The "pharmaceutically acceptable salt thereof" may be any as long as it forms nontoxic salts with compounds [1] and [1'] (hereinafter they are also to be collectively referred to as the compound of the present invention) and, for example, salts with inorganic acids such as hydrochloric acid, sulfuric acid, phosphoric acid, hydrobromic acid and the like; salts with organic acids such as oxalic acid, malonic acid, citric acid, fumaric acid, lactic acid, malic acid, succinic acid, tartaric acid, acetic acid, trifluoroacetic acid, gluconic acid, ascorbic acid, methanesulfonic acid, benzenesulfonic acid and the like; salts with inorganic bases such as sodium hydroxide, potassium hydroxide, calcium hydroxide, magnesium hydroxide, ammonium hydroxide and the like; salts with organic bases such as methylamine, diethylamine, triethylamine, triethanolamine, ethylenediamine, tris(hydroxymethyl)methylamine, guanidine, choline, cinchonine and the like; salts with amino acids such as lysine, arginine, alanine and the like, and the like can be mentioned.

The "solvate" is a compound of the present invention or a pharmaceutically acceptable salt thereof, wherein the molecule of the solvent is coordinated and includes a hydrate (also referred to as a water-containing product). The solvate is preferably a pharmaceutically acceptable solvate and, for example, monohydrate, 1/2 hydrate or dihydrate of a compound represented by the formula [1], monohydrate, monomethanolate, monoethanolate, monoacetonitrile of sodium salt, 2/3 ethanolate of dihydrochloride and the like can be mentioned. These solvates can be prepared according to a method known per se.

In addition, the compound of the present invention has various isomers. For example, E form and Z form can be present as geometric isomers, when an asymmetric carbon atom is present, enantiomer and diastereomer are present as stereoisomers based thereon, and tautomers can also be present. Therefore, the present invention encompasses all of these isomers and mixtures thereof. In the present invention, a prodrug or a conjugate of the compound of the present invention can also be a useful pharmaceutical agent.

As used herein, the "prodrug" is a derivative having a chemically or metabolically decomposable group, which shows a pharmaceutical activity upon decomposition by hydrolysis or solvolysis, or under physiological conditions. A prodrug is used for, for example, improving absorption by oral administration or targeting the object site. Inasmuch as what the chemically or metabolically decomposable group is, and how to introduced the group into a compound have been sufficiently established in the field of pharmaceutical agents, such known techniques can be employed in the present invention. As the moiety to be modified for producing a prodrug, for example, highly reactive functional groups such as a hydroxyl group, a carboxyl group, an amino group, a thiol group and the like in the compound of the present invention can be mentioned.

For example, a derivative wherein a substituent such as -CO-C₁₋₆ alkyl group, -CO₂-C₁₋₆ alkyl group, -CONH-C₁₋₆ alkyl group, -CO-C₂₋₆ alkenyl group, -CO₂-C₂₋₆ alkenyl group, -CONH-C₂₋₆ alkenyl group, -CO-aryl group, -CO₂-aryl group, -CONH-aryl group, -CO-heterocyclic group, -CO₂-heterocyclic group, -CONH-heterocyclic group (the C₁₋₆ alkyl group, C₂₋₆ alkenyl group, aryl group and heterocyclic group are each optionally substituted by halogen atom, C₁₋₆ alkyl group, hydroxyl group, C₁₋₆ alkoxy group, carboxy group, amino group, amino acid residue, -PO₃H₂, -SO₃H, -CO-polyethylene glycol residue, -CO₂-polyethylene glycol residue, -CO-polyethylene glycol monoalkyl ether residue, -CO₂-polyethylene glycol monoalkyl ether residue and the like) and the like has been introduced into a hydroxyl group can be mentioned.

In addition, a derivative wherein a substituent such as -CO-C₁₋₆ alkyl group, -CO₂-C₁₋₆ alkyl group, -CO-C₂₋₆ alkenyl group, -CO₂-C₂₋₆ alkenyl group, -CO-aryl group, -CO₂-aryl group, -CO-heterocyclic group, -CO₂-heterocyclic group (the C₁₋₆ alkyl group, C₂₋₆ alkenyl group, aryl group and heterocyclic group are each optionally substituted by halogen atom, C₁₋₆ alkyl group, hydroxyl group, C₁₋₆ alkoxy group, carboxy group, amino group, amino acid residue, -PO₃H₂, -SO₃H, -CO-polyethylene glycol residue, -CO₂-polyethylene glycol residue, -CO-polyethylene glycol monoalkyl ether residue, -CO₂-polyethylene glycol monoalkyl ether residue, -PO₃H₂ and the like) and the like has been introduced into an amino group can be mentioned.

Furthermore, a derivative wherein a substituent such as C₁₋₆ alkoxy group, aryloxy group (the C₁₋₆ alkoxy group and aryloxy group are each optionally substituted by halogen atom, C₁₋₆ alkyl group, hydroxyl group, C₁₋₆ alkoxy group, carboxy group, amino group, amino acid residue, -PO₃H₂, -SO₃H, polyethylene glycol residue, polyethylene glycol monoalkyl ether residue and the like) and the like has been introduced into a carboxyl group can be mentioned.

The "conjugate" is a substance wherein a hydrophilic molecule (sulfuric acid, glucuronic acid, glycine, glutathione, taurine and the like) is added to a foreign substance such as a drug and the like, in a conjugation reaction which is one type of metabolism.

As a specific prodrug or conjugate of the compound of the present invention, for example, compounds of the formulas [1], [1'], [A-1], [B-1] and the like, wherein R¹ is a substituent shown below can be mentioned:

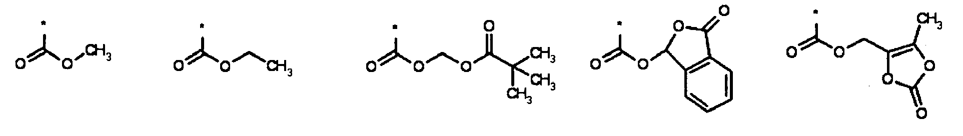

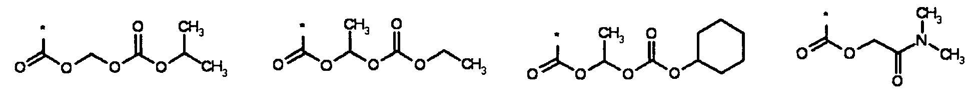

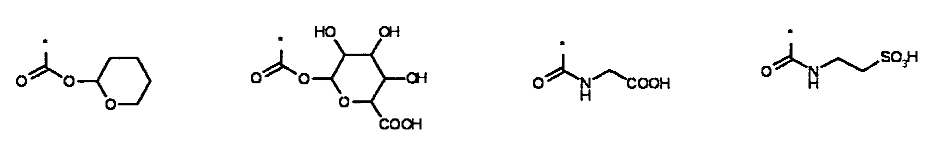

More specific prodrug or conjugate of the compound of the present invention, for example, compounds shown below can be mentioned.

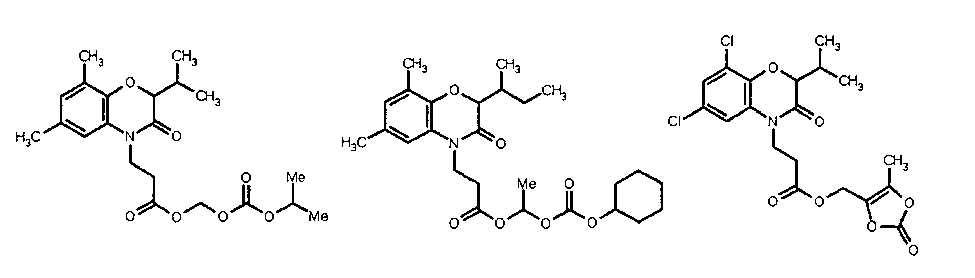

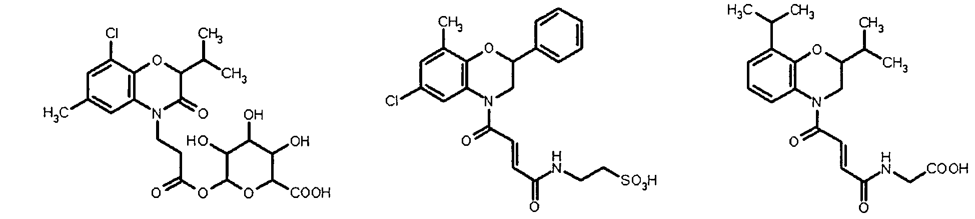

The "pharmaceutical composition" includes, besides what is called a "composition" comprising an active ingredient as a pharmaceutical agent and a pharmaceutically acceptable carrier and the like, a combination agent with other pharmaceutical agents, and the like. It is needless to say that the pharmaceutical composition of the present invention can be concurrently used with any other pharmaceutical agent within the range acceptable in clinical situations. Therefore, the present pharmaceutical composition can also be considered a pharmaceutical composition to be combined with other pharmaceutical agents.

Moreover, the pharmaceutical composition of the present invention can be administered to not only human but also other mammals (mouse, rat, hamster, rabbit, cat, dog, bovine, horse, sheep, monkey etc.). Therefore, the pharmaceutical composition of the present invention is also useful as a pharmaceutical product for animals, not to mention human.

To not "substantially inhibit CYP" means that the function of the drug metabolizing enzyme, cytochrome P450 (CYP), preferably CYP3A4, CYP2C9, CYP2D6, more preferably CYP2C9, is not substantially inhibited and that, for example, the concentration of a substance necessary for inhibiting CYP by 50% is not less than 1 µM, preferably not less than 10 µM, more preferably not less than 30 µM, particularly preferably not less than 50 µM, based on the conditions of the below-mentioned Experimental Example 2, preferably under the conditions of Experimental Example 2.

To "inhibit URAT1 activity" means to specifically inhibit the function of URAT1 as a uric acid transporter to eliminate or attenuate the activity and means, for example, to specifically inhibit the function of URAT1 based on the conditions of the below-mentioned Experimental Example 1 and preferably means that the concentration necessary for inhibiting URAT1 by 50% is less than 3000 nM, more preferably less than 300 nM, under the conditions of the below-mentioned Experimental Example 1. The URAT1 activity inhibitor does not include biological substrates of URAT1, such as uric acid and the like.

To "decrease the blood uric acid level" means to decrease uric acid (including urate) in blood (including in serum and plasma), preferably to decrease high blood uric acid level, more preferably serum uric acid level, to less than 8 mg/dL (preferably less than 7 mg/dl, more preferably less than 6 mg/dL, as serum uric acid level).

The "pathology showing involvement of uric acid" means pathology in which uric acid (including urate) in the body such as blood (including in serum and plasma) or urine is involved. Specifically, pathology caused by high blood (including in serum and plasma) uric acid level or urine uric acid level such as hyperuricemia, gouty tophus, acute gouty arthritis, chronic gouty arthritis, gouty kidney, urolithiasis, renal function disorder, coronary artery disease, ischemic heart disease and the like can be mentioned.

For example, the "high blood uric acid level" means a serum uric acid level of not less than 6 mg/dL, preferably not less than 7 mg/dL, more preferably not less than 8 mg/dL. The "pathology caused by high blood uric acid level" is pathology caused by high blood uric acid level, or to which high blood uric acid level contributes. For example, according to "Guideline for the management of hyperuricemia and gout (1st Edit.)" (Gout and Nucleic Acid Metabolism, vol. 26, suppl. 1 (2002), Japanese Society of Gout and Nucleic Acid Metabolism), 7 mg/dL, which is a dissolution concentration of uric acid in plasma, is the normal upper limit, irrespective of sex•age, and any value exceeding this level is defined to be hyperuricemia, and concludes that the serum uric acid level should be desirably controlled to not more than 6 mg/dL for the treatment of hyperuricemia or gout and for the prevention of the onset of gout arthritis.

As the "pharmaceutically acceptable carrier", various organic or inorganic carrier substances conventionally used as preparation materials are employed, which are added as excipient, lubricant, binder, disintegrant, solvent, dissolution aids, suspending agent, isotonicity agent, buffer, soothing agent and the like. Where necessary, preparation additives such as preservative, antioxidant, sweetening agent, coloring agent and the like can also be used. As preferable examples of the above-mentioned excipient, lactose, sucrose, D-mannitol, starch, crystalline cellulose, light silicic anhydride and the like can be mentioned. As preferable examples of the above-mentioned lubricant, magnesium stearate, calcium stearate, talc, colloidal silica and the like can be mentioned. As preferable examples of the above-mentioned binder, polymer compound such as crystalline cellulose, sucrose, D-mannitol, dextrin, hydroxypropylcellulose, hydroxypropylmethylcellulose, polyvinylpyrrolidone and the like can be mentioned. As preferable examples of the above-mentioned disintegrant, starch, carboxymethylcellulose, carboxymethylcellulose calcium, croscarmellose sodium, sodium carboxymethyl starch and the like can be mentioned. As preferable examples of the above-mentioned solvent, water for injection, alcohol, propylene glycol, macrogol, sesame oil, corn oil, propylene glycol esters of fatty acids and the like can be mentioned. As preferable examples of the above-mentioned dissolution aids, polyethylene glycol, propylene glycol, D-mannitol, benzyl benzoate, ethanol, trisaminomethane, cholesterol, triethanolamine, sodium carbonate, sodium citrate and the like can be mentioned. As preferable examples of the above-mentioned suspending agent, surfactants such as stearyl triethanolamine, sodium lauryl sulfate, lauryl aminopropionic acid, lecithin, benzalkonium chloride, benzethonium chloride, glyceryl monostearate and the like; polyvinyl alcohol, polyvinylpyrrolidone, carboxymethylcellulose sodium, methylcellulose, hydroxymethylcellulose and the like can be mentioned. As preferable examples of the above-mentioned isotonicity agent, sodium chloride, glycerol, D-mannitol and the like can be mentioned. As preferable examples of the above-mentioned buffer, buffers such as phosphate, acetate, carbonate, citrate and the like, and the like can be mentioned. As preferable examples of the above-mentioned soothing agent, benzyl alcohol and the like can be mentioned. As preferable examples of the above-mentioned preservative, paraoxybenzoates, chlorobutanol, benzyl alcohol, phenethyl alcohol, dehydroacetic acid, sorbic acid and the like can be mentioned. As preferable examples of the above-mentioned antioxidant, sulfite, ascorbic acid and the like can be mentioned. As preferable examples of the above-mentioned sweetening agent, aspartam, saccharin sodium, stevia and the like can be mentioned. As preferable examples of the above-mentioned coloring agent, foodcolors such as Food Color Yellow No. 5, Food Color Red No. 2 and Food Color Blue No. 2 and the like, food lake colors, iron oxide and the like can be mentioned.

The compound of the present invention can be contained as an active ingredient of a pharmaceutical composition, a URAT1 activity inhibitor, an agent for lowering a blood uric acid level, or an agent for the prophylaxis or treatment of pathology showing involvement of uric acid, along with a pharmaceutically acceptable carrier.

When the compound of the present invention is used as the pharmaceutical composition, the URAT1 activity inhibitor, the agent for lowering a blood uric acid level, or the drug for the prophylaxis or treatment of pathology showing involvement of uric acid, it can be systemically or topical, and orally or parenterally administered. While the dose varies depending on the age, body weight, condition, treatment effect and the like, for example, it can be generally administered within the range of 0.1 mg to 1 g per administration to an adult once to several times a day. The compound of the present invention can also be used as a drug for the treatment or prophylaxis of the aforementioned diseases in, not to mention human, animals other than human, particularly mammals. The same applies when the compound of the present invention is used as a drug for the prophylaxis or treatment of hyperlipidemia, diabetes, obesity or cardiovascular disease, such as hypertension, coronary artery disease, vascular endothelial disorder, ischemic heart disease and the like.

To produce a preparation of the compound of the present invention such as a solid composition or liquid composition for oral administration, or an injection for parenteral administration and the like, the compound can be admixed with additives such as a suitable diluent, a dispersing agent, an adsorbent, a solubilizer and the like. In addition, the pharmaceutical composition of the present invention may have known form such as tablet, pill, powder, granule, suppository, injection, eye drop, liquid, capsule, troche, aerosol, elixir, suspension, emulsion, syrup and the like.

When the pharmaceutical composition, the URAT1 activity inhibitor, the agent for lowering a blood uric acid level, or the agent for the prophylaxis or treatment of pathology showing involvement of uric acid of the present invention is, for example, a solid preparation such as tablet, pill, powder, granule etc., the additive includes, for example, lactose, mannitol, glucose, hydroxypropylcellulose, microcrystalline cellulose, starch, polyvinylpyrrolidone, magnesium aluminometasilicate, silicic anhydride powder and the like. When a tablet or pill is to be prepared, a film coating of gastrosoluble or enteric substance such as sucrose, gelatin, hydroxypropylcellulose, hydroxymethylcellulose phthalate etc. may be applied as necessary, or a multi-layer tablet having two or more layers may be produced.

As the pharmaceutical composition, the URAT1 activity inhibitor, the agent for lowering a blood uric acid level, or the agent for the prophylaxis or treatment of pathology showing involvement of uric acid of the present invention, capsules obtained by dissolving the compound of the present invention in a solvent, adding an additive to give a liquid, semi-solid or solid content and packing the content therein can also be mentioned. As the aforementioned solvent, for example, purified water, ethanol or vegetable oil and the like can be mentioned. Of these, ethanol or a mixture of purified water and ethanol is preferably used. As the additive, additives generally used for the production of capsules can be used without any particularly limitation. As the aforementioned additive, for example, propylene glycol esters of fatty acids; low molecular weight polyethylene glycol such as polyethylene glycol 200-600 etc., low molecular weight polyethylene glycol (such as polyethylene glycol 200-600 etc.) esters of fatty acids, middle chain fatty acid triglyceride; alcohol/polyhydric alcohols such as stearyl alcohol, cetanol, polyethylene glycol etc., and esters thereof; fats and oils such as sesame oil, soy bean oil, peanut oil, corn oil, hydrogenated oil, paraffin oil, white beeswax etc.; organic carboxylic acid or fatty acid such as triethyl citrate, triacetine, stearic acid, palmitic acid, myristic acid etc. and derivatives thereof and the like can be mentioned, which are suitable for the production of liquid or semi-solid contents. For capsules of the present invention, propylene glycol esters of fatty acids are preferably used as additive. As propylene glycol esters of fatty acids, for example, propylene glycol monocaprylate (Capmul PG-8 (trade name), Sefol218 (trade name), Capryol90 (trade name)), propylene glycol monolaurate (Lauroglycol FCC (trade name)), propylene glycol monooleate (Myverol P-O6 (trade name)), propylene glycol myristate, propylene glycol monostearate, propylene glycol ricinoleate (Propymuls (trade name)), propylene glycol dicaprylate/dicaprate (Captex (trademark) 200 (trade name)), propylene glycol dilaurate, propylene glycol distearate, and propylene glycol dioctanoate (Captex (trademark) 800 (trade name)) and the like can be mentioned. While the material constituting the capsule of the present invention is not particularly limited, natural polysacchalides such as agar, alginate, starch, xanthan, dextran etc.; protein such as gelatin, casein etc.; chemically processed products such as hydroxystarch, pullulan, hydroxypropylcellulose, hydroxypropylmethylcellulose, polyvinyl alcohol and derivatives thereof, polyacryl derivative, polyvinylpyrrolidone and derivatives thereof, polyethylene glycol and the like can be mentioned.

When the pharmaceutical composition, the URAT1 activity inhibitor, the agent for lowering a blood uric acid level, or the agent for the prophylaxis or treatment of pathology showing involvement of uric acid of the present invention is, for example, a liquid preparation for oral administration of a pharmaceutically acceptable suspending agent, a solubilizer, a suspension, a syrup, an elixir etc., as the diluent to be used, for example, purified water, ethanol, vegetable oil, emulsifier and the like can be mentioned. This liquid preparation may contain, besides the diluent, an auxiliary agent such as an infiltration, a suspension, a sweetening agent, a flavor, an aromatic, a preservative and the like.

When the pharmaceutical composition, the URAT1 activity inhibitor, the agent for lowering a blood uric acid level, or the agent for the prophylaxis or treatment of pathology showing involvement of uric acid of the present invention is a parenteral preparation such as injection etc., sterile aqueous or non-aqueous solution, solubilizer, suspension, emulsifier and the like are used as additives. As the aqueous solution, solubilizer and suspension, for example, distilled water for injection; saline, cyclodextrin and derivatives thereof; organic amines such as triethanolamine, diethanolamine, monoethanolamine, triethylamine and the like, inorganic alkali solution and the like can be mentioned. When a water-soluble solution is used, for example, propylene glycol, polyethylene glycol or vegetable oil such as olive oil, alcohols such as ethanol, and the like may be further added. As the solubilizer, for example, surfactants such as polyoxyethylene hydrogenated castor oil, sucrose esters of fatty acids and the like (for forming mixed micelle), lecithin or hydrogenated lecithin (for forming liposome) and the like can also be used. In addition, the parenteral preparation of the present invention can be produced as an emulsion preparation containing water-insoluble solubilizer such as vegetable oil etc., lecithin, polyoxyethylene hydrogenated castor oil, polyoxyethylenepolyoxypropylene glycol and the like.

The compound, pharmaceutical composition, URAT1 activity inhibitor, agent for lowering a blood uric acid level, or agent for the prophylaxis or treatment of pathology showing involvement of uric acid of the present invention can be used in combination with other pharmaceutical composition or pharmaceutical agent (hereinafter to be also referred to as a concomitant drug).

The "use in combination" means use of multiple pharmaceutical agents in combination as active ingredients, and use as a combination drug, use as a kit, use in a combination characterized by independent administration of each by the same or different administration routes and the like can be mentioned.

The administration time of the compound, the pharmaceutical composition, the URAT1 activity inhibitor, the agent for lowering a blood uric acid level, or the agent for the prophylaxis or treatment of pathology showing involvement of uric acid of the present invention and a concomitant drug is not limited, and these may be administered simultaneously to the subject of administration or administered in a staggered manner. The dose of the concomitant drug can be determined according to the dose employed clinically, and appropriately determined depending on the subject of administration, the age and body weight of the subject of administration, condition, administration time, dosage form, administration method, combination and the like. The administration mode of the concomitant drug is not particularly limited, and it is only necessary to combine the compound, a pharmaceutical composition, a URAT1 activity inhibitor, an agent for lowering a blood uric acid level, or an agent for the prophylaxis or treatment of pathology showing involvement of uric acid of the present invention and a concomitant drug at the time of administration.

As the concomitant drug,
(1) an agent for the prophylaxis and/or treatment of hyperuricemia,
(2) an agent for the prophylaxis and/or treatment of gout arthritis,
(3) an agent for the prophylaxis and/or treatment of gouty kidney,
(4) an agent for the prophylaxis and/or treatment of urolithiasis,
(5) an agent for the prophylaxis and/or treatment of hypertension or hypertensive complications,
(6) an agent for the prophylaxis and/or treatment of hyperlipidemia or hyperlipidemic complications,
(7) an agent for the prophylaxis and/or treatment of diabetes or diabetic complications,
(8) an agent for the prophylaxis and/or treatment of obesity or obesity complications,
(9) an agent for the prophylaxis and/or treatment of primary disease causing decreased uric acid excretion secondary hyperuricemia,
(10) an agent for the prophylaxis and/or treatment of kidney failure, cardiovascular disorder, cerebrovascular disorder caused by hyperuricemia, and
(11) a nucleic acid metabolic antagonist can be mentioned. One to three of these agents and the compound of the present invention can be used in combination.

As the "agent for the prophylaxis and/or treatment of hyperuricemia", for example, uric acid production suppressants such as xanthine oxidase inhibitor etc., uricosuric agents and the like can be mentioned, and allopurinol, probenecid, bucolome, febuxostat, benzbromarone, oxipurinol and the like can be specifically mentioned.

As the "agent for the prophylaxis and/or treatment of gout arthritis", for example, NSAIDs such as indomethacin, naproxen, fenbufen, pranoprofen, oxaprozin and the like, colchicine, corticosteroid and the like can be mentioned.

As the "agent for the prophylaxis and/or treatment of gouty kidney", for example, uric acid production suppressants such as xanthine oxidase inhibitor etc., uricosuric agents, alkalinizing urine agents such as citric acid preparation, sodium bicarbonate etc., and the like can be mentioned, and allopurinol, probenecid, bucolome, febuxostat, benzbromarone, oxipurinol and the like can be specifically mentioned.

As the "agent for the prophylaxis and/or treatment of urolithiasis", for example, alkalinizing urine agents such as citric acid preparation, sodium bicarbonate etc., and the like can be mentioned.

As the "agent for the prophylaxis and/or treatment of hypertension or hypertensive complications", for example, loop diuretics, angiotensin-convertase inhibitors, angiotensin II receptor antagonists, Ca antagonists, β blockers, α,β blockers, α blockers and the like can be mentioned. More specifically, for example, sustained-release furosemide preparation, captopril, sustained-release captopril preparation, enalapril maleate, alacepril, delapril hydrochloride, cilazapril, lisinopril, benazepril hydrochloride, imidapril hydrochloride, temocapril hydrochloride, quinapril hydrochloride, trandolapril, perindopril erbumine, losartan potassium, candesartan cilexetil, nicardipine hydrochloride, sustained-release nicardipine hydrochloride preparation, nilvadipine, nifedipine, sustained-release nifedipine preparation, benidipine hydrochloride, diltiazem hydrochloride, sustained-release diltiazem hydrochloride preparation, nisoldipine, nitrendipine, manidipine hydrochloride, barnidipine hydrochloride, efonidipine hydrochloride, amlodipine besylate, felodipine, cilnidipine, aranidipine, propranolol hydrochloride, sustained-release propranolol hydrochloride preparation, pindolol, sustained-release pindolol preparation, indenolol hydrochloride, carteolol hydrochloride, sustained-release carteolol hydrochloride preparation, bunitrolol hydrochloride, sustained-release bunitrolol hydrochloride preparation, atenolol, acebutolol hydrochloride, metoprolol tartrate, sustained-release metoprolol tartrate preparation, nipradilol, penbutolol sulfate, tilisolol hydrochloride, carvedilol, bisoprolol fumarate, betaxolol hydrochloride, celiprolol hydrochloride, bopindolol malonate, bevantolol hydrochloride, labetalol hydrochloride, arotinolol hydrochloride, amosulalol hydrochloride, prazosin hydrochloride, terazosin hydrochloride, doxazosin mesilate, bunazosin hydrochloride, sustained-release bunazosin hydrochloride preparation, urapidil, phentolamine mesylate and the like can be mentioned.

As the "agent for the prophylaxis and/or treatment of hyperlipidemia or hyperlipidemic complications", for example, statin pharmaceutical agents, anion exchange resins, probucol, nicotinic acid preparations, fibrate pharmaceutical agents, eicosapentaenoic acid preparations and the like can be mentioned. More specifically, for example, lovastatin, simvastatin, pravastatin, fluvastatin, atorvastatin, cerivastatin, colestimide, colestyramine, niceritrol, nicomol, fenofibrate, bezafibrate, clinofibrate, clofibrate, ethyl icosapentate and the like can be mentioned.

As the "agent for the prophylaxis and/or treatment of diabetes or diabetic complications", for example, insulin preparations, sulfonylureas, insulin secretagogues, sulfonamides, biguanides, α glucosidase inhibitors, insulin sensitizers, angiotensin-convertase inhibitors, aldose reductase inhibitors, antiarrhythmic drugs and the like can be mentioned. More specifically, for example, insulin, chlorpropamide, glibenclamide, glipizide, tolbutamide, glyclopyramide, acetohexamide, glimepiride, tolazamide, gliclazide, nateglinide, glybuzole, metformin hydrochloride, buformin hydrochloride, voglibose, acarbose, pioglitazone hydrochloride, mexiletine and the like can be mentioned.

As the "agent for the prophylaxis and/or treatment of obesity or obesity complications", for example, mazindol, acarbose, voglibose, orlistat and the like can be mentioned.

As the "agent for the prophylaxis and/or treatment of primary disease causing decreased uric acid excretion secondary hyperuricemia", for example, agents for the prophylaxis or treatment of chronic renal disease, polycyctic kidney, toxemia of pregnancy, lead nephropathy, hyperlactacidemia, Down's syndrome, sarcoidosis, glycogenosis I type (via hyperlactacidemia), dehydrating etc., and the like can be mentioned.

As the "agent for the prophylaxis and/or treatment of kidney failure, cardiovascular disorder, cerebrovascular disorder caused by hyperuricemia", for example, loop diuretics (e.g., furosemide), citric acid preparations, sodium bicarbonate, cation exchange resins, aluminum hydroxide, alfacalcidol, β-blockers (e.g., propranolol hydrochloride), ACE inhibitors (e.g., captopril), cardiac stimulants (e.g., digoxin), angina pectoris therapeutic agents (e.g., isosorbide nitrate), Ca antagonists (e.g., diltiazem hydrochloride), uric acid production suppressants (e.g., allopurinol), amino acid preparations, hyperammonemia improvers, therapeutic agents for antiarrhythmic (e.g., mexiletine), therapeutic agents for anemia (e.g., mepitiostane, erythropoietin), other "agents for the prophylaxis and/or treatment of hypertension or hypertensive complications", "agents for the prophylaxis and/or treatment of hyperlipidemia or hyperlipidemic complications", "agents for the prophylaxis and/or treatment of diabetes or diabetic complications", "agents for the prophylaxis and/or treatment of obesity or obesity complications" and the like can be mentioned.

As the "nucleic acid metabolic antagonist", for example, azathiopurine, mizoribine, mycophenolic acid and the like can be mentioned.

Furthermore, the compound, a pharmaceutical composition, a URAT1 activity inhibitor, an agent for lowering a blood uric acid level, and an agent for the prophylaxis or treatment of pathology showing involvement of uric acid of the present invention can be used in combination with a pharmaceutical agent that increases the blood uric acid level, thereby to suppress increase in the blood uric acid level.

As the "pharmaceutical agent that increases the blood uric acid level", nucleic acid metabolic antagonists, hypotensive diuretics, anti-tuberculosis, anti-inflammatory analgesic drugs, hyperlipidemic drugs, therapeutic drugs for asthma, immunosuppressants, salicylic acid, pyrazinamide, ethambutol, nicotinic acid, ethanol, cyclosporine and the like can be mentioned.

The production methods of the compound of the present invention are specifically explained below. It is needless to say that the present invention is not limited to the production methods below. For production of the compound of the present invention, the order of the reaction can be appropriately changed. The reaction only needs to be carried out from a step or a position that seems to be reasonable.

In addition, a step for appropriately changing substituents (change or further modification of substituents) may be inserted between respective steps. When a reactive functional group is involved, appropriate protection or deprotection may be conducted. To promote progress of the reaction, moreover, reagents other than those exemplified can be appropriately used. The starting material compounds, for which a production method is not described, are commercially available or can be easily produced by combining known synthetic reactions.

The compound obtained in each step can be isolated and purified by conventional methods such as crystallization, recrystallization, column chromatography, preparative HPLC and the like. In some cases, it is possible to proceed to the next step without isolation and purification.
In the following production methods, the "room temperature" means 1-40°C.

### Production method 1

Compound [A-1] wherein Z is an oxygen atom, and L is a single bond can be produced by the following steps.

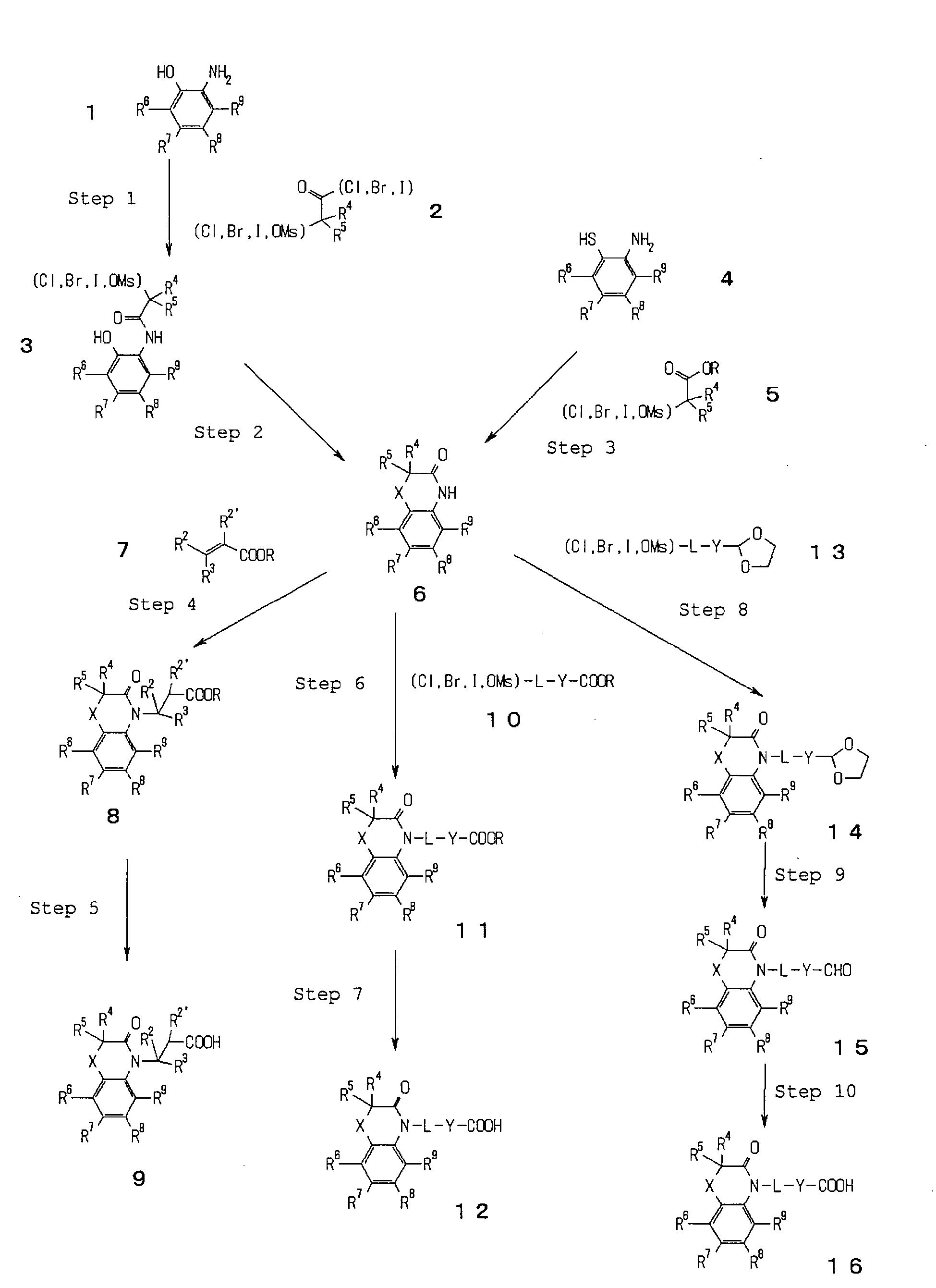

wherein R², R^{2'} and R³ are the same or different and each is a hydrogen atom, or a C₁₋₆ alkyl group optionally substituted by the same or different one or more substituents selected from the aforementioned group A, -OMs is -OS(=O)₂-CH₃, and other each symbols are as defined above.

### Step 1

Compound 3 can be obtained by amidating compound 1 with acid halide 2 in a solvent in the presence of a base.

As the solvent to be used for the reaction, for example, ethers such as diethyl ether, tetrahydrofuran, dioxane, 1,2-dimethoxyethane, diglyme and the like; hydrocarbons such as benzene, toluene, hexane, xylene and the like; halogenated hydrocarbons such as methylene chloride, chloroform, carbon tetrachloride, 1,2-dichloroethane and the like; esters such as ethyl acetate, methyl acetate, butyl acetate and the like; polar solvents such as acetone, N,N-dimethylformamide, dimethyl sulfoxide, water and the like; and the like can be mentioned. These can be used alone or in a mixture of two or more kinds thereof. The preferable solvents for this reaction include methylene chloride, chloroform, toluene, ethyl acetate, water and tetrahydrofuran.

As the base to be used for the reaction, for example, organic bases such as triethylamine, pyridine, 4-dimethylaminopyridine, N-methylmorpholine and the like; alkali metal hydroxides such as lithium hydroxide, sodium hydroxide, potassium hydroxide and the like; alkali metal hydrides such as sodium hydride, potassium hydride and the like; alkali metal carbonates such as sodium carbonate, potassium carbonate and the like; alkali metal hydrogencarbonates such as sodium hydrogencarbonate, potassium hydrogencarbonate and the like; and the like can be mentioned, with preference given to triethylamine, pyridine, sodium hydroxide and sodium hydrogencarbonate.

The reaction temperature is about 0°C to 80°C, preferably about 0°C to room temperature.
The reaction time is about 10 min to 48 hr, preferably about 30 min to 24 hr.

### Step 2

Compound 6 can be obtained by subjecting compound 3 to cyclization in a solvent in the presence of a base and, where necessary in the presence of sodium iodide, potassium iodide and the like.

As the solvent to be used for the reaction, for example, ethers such as diethyl ether, tetrahydrofuran, dioxane, 1,2-dimethoxyethane, diglyme and the like; hydrocarbons such as benzene, toluene, hexane, xylene and the like; halogenated hydrocarbons such as methylene chloride, chloroform, carbon tetrachloride, 1,2-dichloroethane and the like; esters such as ethyl acetate, methyl acetate, butyl acetate and the like; polar solvents such as acetone, N,N-dimethylformamide, dimethylsulfoxide and the like; and the like can be mentioned. These can be used alone or in a mixture of two or more kinds thereof. The preferable solvents for this reaction include acetone and N,N-dimethylformamide.

As the base to be used for the reaction, for example, organic bases such as triethylamine, pyridine, 4-dimethylaminopyridine, N-methylmorpholine and the like; alkali metal hydroxides such as lithium hydroxide, sodium hydroxide, potassium hydroxide and the like; alkali metal carbonates such as sodium carbonate, potassium carbonate and the like; alkali metal hydrogencarbonates such as sodium hydrogencarbonate, potassium hydrogencarbonate and the like; alkali metal carboxylates such as sodium acetate, potassium acetate and the like; alkali metal hydrides such as sodium hydride, potassium hydride and the like; alkali metal alkoxides such as sodium ethoxide, sodium methoxide, potassium tert-butoxide and the like; alkyllithiums such as n-butyllithium, sec-butyllithium and the like; alkali metal amides such as lithium diisopropylamide, sodium amide, lithium bistrimethylsilylamide and the like; and the like can be mentioned, with preference given to potassium carbonate.

The reaction temperature is about 0°C to 150°C, preferably room temperature to 100°C.
The reaction time is about 10 min to 7 days, preferably about 1 hr to 4 days.

### Step 3

Compound 6 can be obtained by reacting compound 4 with compound 5 in a solvent in the presence of a base and, where necessary, in the presence of sodium iodide, potassium iodide and the like.

As the solvent to be used for the reaction, for example, ethers such as diethyl ether, tetrahydrofuran, dioxane, 1,2-dimethoxyethane, diglyme and the like; hydrocarbons such as benzene, toluene, hexane, xylene and the like; halogenated hydrocarbons such as methylene chloride, chloroform, carbon tetrachloride, 1,2-dichloroethane and the like; esters such as ethyl acetate, methyl acetate, butyl acetate and the like; polar solvents such as acetone, N,N-dimethylformamide, dimethylsulfoxide and the like; and the like can be mentioned. These can be used alone or in a mixture of two or more kinds thereof. The preferable solvents for this reaction include acetone, dimethyl sulfoxide or N,N-dimethylformamide.

As the base to be used for the reaction, for example, organic bases such as triethylamine, pyridine, 4-dimethylaminopyridine, N-methylmorpholine and the like; alkali metal hydroxides such as lithium hydroxide, sodium hydroxide, potassium hydroxide and the like; alkali metal carbonates such as sodium carbonate, potassium carbonate and the like; alkali metal hydrogencarbonates such as sodium hydrogencarbonate, potassium hydrogen carbonate and the like; alkali metal carboxylates such as sodium acetate, potassium acetate and the like; alkali metal hydrides such as sodium hydride, potassium hydride and the like; alkali metal alkoxides such as sodium ethoxide, sodium methoxide, potassium tert-butoxide and the like; alkyllithium such as n-butyllithium, sec-butyllithium and the like; alkali metal amides such as lithium diisopropylamide, sodium amide, lithium bistrimethylsilylamide and the like; and the like can be mentioned, with preference given to sodium hydrogencarbonate and potassium carbonate.

The reaction temperature is about 0°C to 150°C, preferably room temperature to 100°C.
The reaction time is about 10 min to 48 hr, preferably about 30 min to 24 hr.

### Step 4

Compound 8 can be obtained by reacting compound 6 with compound 7 in a solvent in the presence of a base.

As the solvent to be used for the reaction, for example, ethers such as diethyl ether, tetrahydrofuran, dioxane, 1,2-dimethoxyethane, diglyme and the like; hydrocarbons such as benzene, toluene, hexane, xylene and the like; halogenated hydrocarbons such as methylene chloride, chloroform, carbon tetrachloride, 1,2-dichloroethane and the like; esters such as ethyl acetate, methyl acetate, butyl acetate and the like; polar solvents such as acetone, N,N-dimethylformamide, dimethylsulfoxide and the like; and the like can be mentioned. These can be used alone or in a mixture of two or more kinds thereof. The preferable solvents for this reaction include acetone, dimethyl sulfoxide or N,N-dimethylformamide.

As the base to be used for the reaction, for example, organic bases such as triethylamine, pyridine, 4-dimethylaminopyridine, N-methylmorpholine and the like; alkali metal hydroxides such as lithium hydroxide, sodium hydroxide, potassium hydroxide and the like; alkali metal carbonates such as sodium carbonate, potassium carbonate and the like; alkali metal hydrogencarbonates such as sodium hydrogencarbonate, potassium hydrogen carbonate and the like; alkali metal carboxylates such as sodium acetate, potassium acetate and the like; alkali metal hydrides such as sodium hydride, potassium hydride and the like; alkali metal alkoxides such as sodium ethoxide, sodium methoxide, potassium tert-butoxide and the like; alkyllithiums such as n-butyllithium, sec-butyllithium and the like; alkali metal amides such as lithium diisopropylamide, sodium amide, lithium bistrimethylsilylamide and the like and the like can be mentioned, with preference given to sodium hydride and potassium carbonate.

The reaction temperature is about 0°C to 150°C, preferably room temperature to 100°C.
The reaction time is about 10 min to 48 hr, preferably about 1 hr to 24 hr.

### Step 5

Compound 9 can be obtained by hydrolyzing compound 8 in a solvent in the presence of a base or acid.

As the solvent to be used for the reaction, for example, ether solvents such as diethyl ether, tetrahydrofuran, dioxane, 1,2-dimethoxyethane, diglyme and the like; alcohol solvents such as methanol, ethanol, isopropyl alcohol, tert-butanol and the like; hydrocarbon solvents such as benzene, toluene, hexane, xylene and the like; halogenated hydrocarbon solvents such as methylene chloride, chloroform, carbon tetrachloride, 1,2-dichloroethane and the like; ester solvents such as ethyl acetate, methyl acetate, butyl acetate and the like; polar solvents such as acetone, N,N-dimethylformamide, dimethyl sulfoxide and the like; and the like can be mentioned, which may be used alone or two or more kinds thereof can be used in a mixture. A preferable solvent for this reaction is tetrahydrofuran, dioxane, methanol, ethanol, tert-butanol, ethyl acetate or toluene.

As the base to be used for the reaction, for example, alkali metal hydroxides such as lithium hydroxide, sodium hydroxide, potassium hydroxide and the like; alkali metal carbonates such as sodium carbonate, potassium carbonate and the like; alkali metal hydrogencarbonates such as sodium hydrogencarbonate, potassium hydrogen carbonate and the like; alkali metal carboxylates such as sodium acetate, potassium acetate and the like; alkali metal alkoxides such as sodium ethoxide, sodium methoxide, potassium tert-butoxide and the like and the like can be mentioned, with preference given to lithium hydroxide and sodium hydroxide.

As the acid to be used for the reaction, for example, organic acids such as formic acid, acetic acid, trifluoroacetic acid, p-toluenesulfonic acid and the like; hydrochloric acid, hydrobromic acid and the like can be mentioned, with preference given to trifluoroacetic acid and hydrochloric acid.

The reaction temperature is about 0°C to 150°C, preferably room temperature to 100°C.
The reaction time is about 10 min to 5 days, preferably about 30 min to 3 days.

### Step 6

Compound 11 can be obtained by reacting compound 6 with compound 10 in a solvent in the presence of a base and, where necessary, in the presence of sodium iodide, potassium iodide and the like.

As the solvent to be used for the reaction, for example, ethers such as diethyl ether, tetrahydrofuran, dioxane, 1,2-dimethoxyethane, diglyme and the like; hydrocarbons such as benzene, toluene, hexane, xylene and the like; halogenated hydrocarbons such as methylene chloride, chloroform, carbon tetrachloride, 1,2-dichloroethane and the like; esters such as ethyl acetate, methyl acetate, butyl acetate and the like; polar solvents such as acetone, N,N-dimethylformamide, dimethylsulfoxide and the like; and the like can be mentioned. These can be used alone or in a mixture of two or more kinds thereof. The preferable solvents for this reaction include tetrahydrofuran or N,N-dimethylformamide.

As the base to be used for the reaction, for example, organic bases such as triethylamine, pyridine, 4-dimethylaminopyridine, N-methylmorpholine and the like; alkali metal hydroxides such as lithium hydroxide, sodium hydroxide, potassium hydroxide and the like; alkali metal carbonates such as sodium carbonate, potassium carbonate and the like; alkali metal hydrogencarbonates such as sodium hydrogencarbonate, potassium hydrogen carbonate and the like; alkali metal carboxylates such as sodium acetate, potassium acetate and the like; alkali metal hydrides such as sodium hydride, potassium hydride and the like; alkali metal alkoxides such as sodium ethoxide, sodium methoxide, potassium tert-butoxide and the like; alkyllithiums such as n-butyllithium, sec-butyllithium and the like; alkali metal amides such as lithium diisopropylamide, sodium amide, lithium bistrimethylsilylamide and the like, and the like can be mentioned, with preference given to potassium carbonate and sodium hydride.

The reaction temperature is about 0°C to 180°C, preferably room temperature to 150°C.
The reaction time is about 10 min to 48 hr, preferably about 30 min to 24 hr.

### Step 7

Compound 12 can be obtained by hydrolyzing compound 11 in a solvent in the presence of a base or an acid.

As the solvent to be used for the reaction, for example, ether solvents such as diethyl ether, tetrahydrofuran, dioxane, 1,2-dimethoxyethane, diglyme and the like; alcohol solvents such as methanol, ethanol, isopropyl alcohol, tert-butanol and the like; hydrocarbon solvents such as benzene, toluene, hexane, xylene and the like; halogenated hydrocarbon solvents such as methylene chloride, chloroform, carbon tetrachloride, 1,2-dichloroethane and the like; ester solvents such as ethyl acetate, methyl acetate, butyl acetate and the like; polar solvents such as acetone, N,N-dimethylformamide, dimethylsulfoxide and the like, and the like can be mentioned. These can be used alone or in a mixture of two or more kinds thereof. The preferable solvents for this reaction include tetrahydrofuran, dioxane, methanol, ethanol, tert-butanol, ethyl acetate and toluene.

As the base to be used for the reaction, for example, alkali metal hydroxides such as lithium hydroxide, sodium hydroxide, potassium hydroxide and the like; alkali metal carbonates such as sodium carbonate, potassium carbonate and the like; alkali metal hydrogencarbonates such as sodium hydrogencarbonate, potassium hydrogen carbonate and the like; alkali metal carboxylates such as sodium acetate, potassium acetate and the like; alkali metal alkoxides such as sodium ethoxide, sodium methoxide, potassium tert-butoxide and the like, and the like can be mentioned, with preference given to lithium hydroxide and sodium hydroxide.

As the acid to be used for the reaction, for example, organic acids such as formic acid, acetic acid, trifluoroacetic acid, p-toluenesulfonic acid and the like; hydrochloric acid, hydrobromic acid and the like can be mentioned, with preference given to trifluoroacetic acid and hydrochloric acid.

The reaction temperature is about 0°C to 150°C, preferably room temperature to 100°C.
The reaction time is about 10 min to 5 days, preferably about 30 min to 3 days.

### Step 8

Compound 14 can be obtained by reacting compound 6 with compound 13 in a solvent in the presence of a base and, where necessary, in the presence of sodium iodide, potassium iodide and the like.

As the solvent to be used for the reaction, for example, ethers such as diethyl ether, tetrahydrofuran, dioxane, 1,2-dimethoxyethane, diglyme and the like; hydrocarbons such as benzene, toluene, hexane, xylene and the like; halogenated hydrocarbons such as methylene chloride, chloroform, carbon tetrachloride, 1,2-dichloroethane and the like; esters such as ethyl acetate, methyl acetate, butyl acetate and the like; polar solvents such as acetone, N,N-dimethylformamide, dimethylsulfoxide and the like; and the like can be mentioned. These can be used alone or in a mixture of two or more kinds thereof. The preferable solvents for this reaction include tetrahydrofuran or N,N-dimethylformamide.

As the base to be used for the reaction, for example, organic bases such as triethylamine, pyridine, 4-dimethylaminopyridine, N-methylmorpholine and the like; alkali metal hydroxides such as lithium hydroxide, sodium hydroxide, potassium hydroxide and the like; alkali metal carbonate such as sodium carbonate, potassium carbonate and the like; alkali metal hydrogencarbonates such as sodium hydrogencarbonate, potassium hydrogen carbonate and the like; alkali metal carboxylates such as sodium acetate, potassium acetate and the like; alkali metal hydrides such as sodium hydride, potassium hydride and the like; alkali metal alkoxides such as sodium ethoxide, sodium methoxide, potassium tert-butoxide and the like; alkyllithiums such as n-butyllithium, sec-butyllithium and the like; alkali metal amides such as lithium diisopropylamide, sodium amide, lithium bistrimethylsilylamide and the like, and the like can be mentioned, with preference given to potassium carbonate and sodium hydride.

The reaction temperature is about 0°C to 150°C, preferably room temperature to 100°C.
The reaction time is about 10 min to 5 days, preferably about 30 min to 3 days.

### Step 9

Compound 15 can be obtained by reacting compound 14 in a solvent in the presence of an acid.

As the solvent to be used for the reaction, for example, ether solvents such as diethyl ether, tetrahydrofuran, dioxane, 1,2-dimethoxyethane, diglyme and the like; alcohol solvents such as methanol, ethanol, isopropyl alcohol, tert-butanol and the like; hydrocarbon solvents such as benzene, toluene, hexane, xylene and the like; halogenated hydrocarbon solvents such as methylene chloride, chloroform, carbon tetrachloride, 1,2-dichloroethane and the like; ester solvents such as ethyl acetate, methyl acetate, butyl acetate and the like; polar solvents such as acetone, N,N-dimethylformamide, dimethyl sulfoxide, water and the like, and the like can be mentioned. These can be used alone or in a mixture of two or more kinds thereof. The preferable solvents for this reaction include water, acetone and tetrahydrofuran.

As the acid to be used for the reaction, for example, organic acids such as formic acid, acetic acid, trifluoroacetic acid, p-toluenesulfonic acid, PPTS (pyridinium p-toluenesulfonate) and the like; hydrochloric acid, hydrobromic acid and the like can be mentioned, with preference given to PPTS (pyridinium p-toluenesulfonate) and hydrochloric acid.

The reaction temperature is about 0°C to 150°C, preferably room temperature to 100°C.
The reaction time is about 10 min to 48 hr, preferably about 1 hr to 24 hr.

### Step 10

Compound 16 can be obtained by reacting compound 15 using an oxidant in a solvent.

As the solvent to be used for the reaction, for example, ether solvents such as diethyl ether, tetrahydrofuran, dioxane, 1,2-dimethoxyethane, diglyme and the like; alcohol solvents such as methanol, ethanol, isopropyl alcohol, tert-butanol and the like; hydrocarbon solvents such as benzene, toluene, hexane, xylene and the like; halogenated hydrocarbon solvents such as methylene chloride, chloroform, carbon tetrachloride, 1,2-dichloroethane and the like; ester solvents such as ethyl acetate, methyl acetate, butyl acetate and the like; polar solvents such as acetone, N,N-dimethylformamide, dimethyl sulfoxide, water and the like, and the like can be mentioned. These can be used alone or in a mixture of two or more kinds thereof. The preferable solvents for this reaction include tert-butanol, water, acetone and tetrahydrofuran.

As the oxidant to be used for the reaction, for example, sodium chlorite, potassium permanganate, sodium periodate, potassium dichromate, chrome oxide, aqueous hydrogen peroxide and the like can be mentioned. The preferable oxidants for this reaction include potassium permanganate and sodium chlorite.

The reaction temperature is about 0°C to 150°C, preferably room temperature to 100°C.
The reaction time is about 10 min to 48 hr, preferably about 30 min to 24 hr.

Alternatively, compound 6 can be obtained in one step according to Synthesis, 10, 851-852 (1984), instead of Step 1 and Step 2.

When the carbon atom to which R⁴ and R⁵ are bonded is an asymmetric carbon, optically active compound [A-1] can be obtained by using optically active compound 2 or 5. To further increase optical purity, repeat recrystallization, or conversion to a salt with an optically active amine (e.g., (R)-1-phenylethylamine etc.), repeat recrystallization of the salt and acid-treatment thereof is performed to give optically active compound [A-1] having higher optical purity.

In addition, when the carbon atom to which R⁴ and R⁵ are bonded is an asymmetric carbon, racemic carboxylic acid compound [A-1] can be converted to an ester by condensing with an optically active alcohol (e.g., (S)-methyl lactate etc.). Since this ester is a diastereomer mixture, optically active carboxylic acid compound [A-1] can be obtained by separation and purification, and hydrolysis. To further increase optical purity, repeat recrystallization, or conversion to a salt with an optically active amine (e.g., (R)-1-phenylethylamine etc.), repeat recrystallization of the salt and acid-treatment thereof is performed to give optically active compound [A-1] having higher optical purity.

### Production method 2

Compound [A-1] wherein Z is a sulfur atom, and L is a single bond can be produced by the following steps.

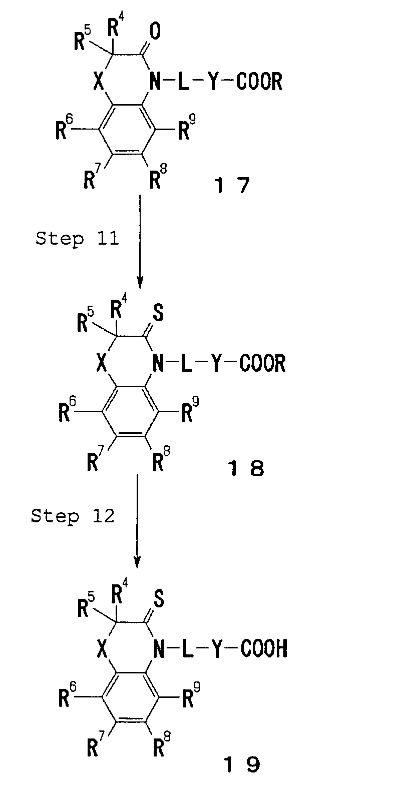

wherein each symbol is as defined above.

### Step 11

Compound 18 can be obtained by reacting compound 17 with a Lawesson reagent, diphosphorus pentasulfide and the like in a solvent.

As the solvent to be used for the reaction, for example, ethers such as diethyl ether, tetrahydrofuran, dioxane, 1,2-dimethoxyethane, diglyme and the like; hydrocarbons such as benzene, toluene, hexane, xylene and the like; halogenated hydrocarbons such as methylene chloride, chloroform, carbon tetrachloride, 1,2-dichloroethane and the like; alcohols such as methanol, ethanol, isopropyl alcohol, tert-butanol and the like; esters such as ethyl acetate, methyl acetate, butyl acetate and the like; polar solvents such as acetone, N,N-dimethylformamide, dimethylsulfoxide and the like; and the like can be mentioned. These can be used alone or in a mixture of two or more kinds thereof. The preferable solvents for this reaction include tetrahydrofuran and toluene.

The reaction temperature is about 0°C to 150°C, preferably room temperature to 120°C.
The reaction time is about 10 min to 48 hr, preferably about 30 min to 24 hr.

### Step 12

Compound 19 can be obtained by hydrolyzing compound 18 in a solvent in the presence of a base or an acid.

As the solvent to be used for the reaction, for example, ether solvents such as diethyl ether, tetrahydrofuran, dioxane, 1,2-dimethoxyethane, diglyme and the like; alcohol solvents such as methanol, ethanol, isopropyl alcohol, tert-butanol and the like; hydrocarbon solvents such as benzene, toluene, hexane, xylene and the like; halogenated hydrocarbon solvents such as methylene chloride, chloroform, carbon tetrachloride, 1,2-dichloroethane and the like; ester solvents such as ethyl acetate, methyl acetate, butyl acetate and the like; polar solvents such as acetone, N,N-dimethylformamide, dimethylsulfoxide and the like, and the like can be mentioned. These can be used alone or in a mixture of two or more kinds thereof. The preferable solvents for this reaction include tetrahydrofuran, dioxane, methanol, ethanol, tert-butanol, ethyl acetate and toluene.

As the base to be used for the reaction, for example, alkali metal hydroxides such as lithium hydroxide, sodium hydroxide, potassium hydroxide and the like; alkali metal carbonates such as sodium carbonate, potassium carbonate and the like; alkali metal hydrogencarbonates such as sodium hydrogencarbonate, potassium hydrogen carbonate and the like; alkali metal carboxylates such as sodium acetate, potassium acetate and the like; alkali metal alkoxides such as sodium ethoxide, sodium methoxide, potassium tert-butoxide and the like, and the like can be mentioned, with preference given to lithium hydroxide and sodium hydroxide.

As the acid to be used for the reaction, for example, organic acids such as formic acid, acetic acid, trifluoroacetic acid, p-toluenesulfonic acid and the like; hydrochloric acid, hydrobromic acid and the like can be mentioned, with preference given to trifluoroacetic acid and hydrochloric acid.

The reaction temperature is about 0°C to 150°C, preferably room temperature to 100°C.
The reaction time is about 10 min to 48 hr, preferably about 1 hr to 24 hr.

When the carbon atom to which R⁴ and R⁵ are bonded is an asymmetric carbon, optically active compound 19 can be obtained by using optically active compound 17. To further increase optical purity, repeat recrystallization, or conversion to a salt with an optically active amine (e.g., (R)-1-phenylethylamine etc.), repeat recrystallization of the salt and acid-treatment thereof is performed to give optically active compound 19 having higher optical purity.

In addition, when the carbon atom to which R⁴ and R⁵ are bonded is an asymmetric carbon, racemic carboxylic acid compound 19 can be converted to an ester by condensing with an optically active alcohol (e.g., (S)-methyl lactate etc.). Since this ester is a diastereomer mixture, optically active carboxylic acid compound 19 can be obtained by separation and purification, and hydrolysis. To further increase optical purity, repeat recrystallization, or conversion to a salt with an optically active amine (e.g., (R)-1-phenylethylamine etc.), repeat recrystallization of the salt and acid-treatment thereof is performed to give optically active compound 19 having higher optical purity.

### Production method 3

Compound [B-1] wherein L is -(C=O)- can be produced by the following steps.

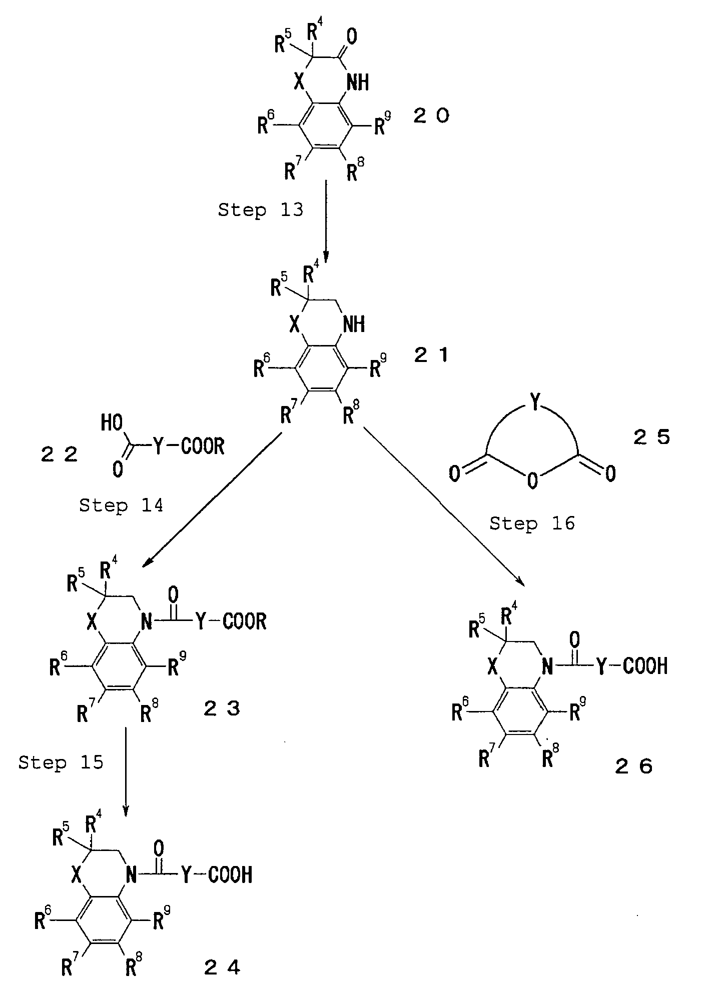

wherein each symbol is as defined above.

### Step 13

Compound 21 can be obtained by reducing compound 20 with a reducing agent in a solvent.

As the solvent to be used for the reaction, for example, ether solvents such as diethyl ether, tetrahydrofuran, dioxane, 1,2-dimethoxyethane, diglyme and the like; alcohol solvents such as methanol, ethanol, isopropyl alcohol, tert-butanol and the like; hydrocarbon solvents such as benzene, toluene, hexane, xylene and the like and the like can be mentioned, with preference given to toluene, diethyl ether and tetrahydrofuran.

As the reducing agent to be used for the reaction, for example, lithium aluminum hydride, sodium borohydride, diborane, diisobutylaluminum hydride, borane-tetrahydrofuran complex, sodium bis(2-methoxyethoxy)aluminum hydride and the like can be mentioned, with preference given to sodium bis(2-methoxyethoxy)aluminum hydride, lithium aluminum hydride and borane-tetrahydrofuran complex.

The reaction temperature is about 0°C to 150°C, preferably room temperature to 130°C.
The reaction time is about 10 min to 48 hr, preferably about 30 min to 24 hr.

### Step 14

Compound 23 can be obtained by reacting carboxylic acid compound 22 with oxalyl chloride, oxalyl bromide, thionyl chloride, thionyl bromide and the like in a solvent to give an acid halide, and amidating compound 21 with the acid halide in a solvent and, where necessary, in the presence of a base.

As the solvent to be used for the reaction to obtain an acid chloride, for example, ether solvents such as diethyl ether, tetrahydrofuran, dioxane, 1,2-dimethoxyethane, diglyme and the like; hydrocarbon solvents such as benzene, toluene, hexane, xylene and the like; halogenated hydrocarbon solvents such as methylene chloride, chloroform, carbon tetrachloride, 1,2-dichloroethane and the like; ester solvents such as ethyl acetate, methyl acetate, butyl acetate and the like and the like can be mentioned. These can be used alone or in a mixture of two or more kinds thereof. The preferable solvents for this reaction include 1,2-dimethoxyethane, ethyl acetate, and methylene chloride, chloroform, toluene, 1,2-dimethoxyethane and ethyl acetate each containing a catalytic amount of N,N-dimethylformamide.

The reaction temperature is about -20°C to 120°C, preferably about 0°C to 80°C.
The reaction time is about 10 min to 48 hr, preferably about 30 min to 24 hr.

As the solvent to be used for the amidation reaction, for example, ether solvents such as diethyl ether, tetrahydrofuran, dioxane, 1,2-dimethoxyethane, diglyme and the like; hydrocarbon solvents such as benzene, toluene, hexane, xylene and the like; halogenated hydrocarbon solvents such as methylene chloride, chloroform, carbon tetrachloride, 1,2-dichloroethane and the like; ester solvents such as ethyl acetate, methyl acetate, butyl acetate and the like; polar solvents such as acetone, N,N-dimethylformamide, dimethylsulfoxide and the like, and the like can be mentioned. These can be used alone or in a mixture of two or more kinds thereof. The preferable solvents for this reaction include methylene chloride, chloroform, toluene, ethyl acetate, N,N-dimethylformamide and tetrahydrofuran.

As the base to be used for the reaction, for example, organic bases such as triethylamine, pyridine, 4-dimethylaminopyridine, N-methylmorpholine and the like; alkali metal hydroxides such as lithium hydroxide, sodium hydroxide, potassium hydroxide and the like; alkali metal hydrides such as sodium hydride, potassium hydride and the like; alkali metal carbonates such as sodium carbonate, potassium carbonate and the like; alkali metal hydrogencarbonates such as sodium hydrogencarbonate, potassium hydrogen carbonate and the like, and the like can be mentioned. This reaction is preferably carried out in the absence of a base, or in the presence of triethylamine, pyridine, sodium hydroxide, sodium hydride or sodium hydrogencarbonate.

The reaction temperature is about 0°C to 120°C, preferably about 0°C to 95°C.
The reaction time is about 10 min to 7 days, preferably about 30 min to 5 days.

The compound 23 can also be obtained by subjecting compound 21 and carboxylic acid compound 22 to condensation using, for example, water-soluble carbodiimide (WSC HCl: 1-ethyl-3-(3'-dimethylaminopropyl)carbodiimide hydrochloride), dicyclohexylcarbodiimide (DCC), diphenylphosphoryl azide (DPPA), carbonyldiimidazole (CDI), 1-hydroxy-1H-benzotriazole (HOBT), 4-dimethylaminopyridine (DMAP) and the like; or by converting carboxylic acid compound 22 to a mixed acid anhydride with a chloroformate such as ethyl chloroformate, isopropyl chloroformate, isobutyl chloroformate and the like in the presence of a base such as triethylamine and the like, and reacting compound 21 with the mixed acid anhydride in the presence of a base.

### Step 15

Compound 24 can be obtained by hydrolyzing compound 23 in a solvent inn the presence of a base or an acid.

As the solvent to be used for the reaction, for example, ether solvents such as diethyl ether, tetrahydrofuran, dioxane, 1,2-dimethoxyethane, diglyme and the like; alcohol solvents such as methanol, ethanol, isopropyl alcohol, tert-butanol and the like; hydrocarbon solvents such as benzene, toluene, hexane, xylene and the like; halogenated hydrocarbon solvents such as methylene chloride, chloroform, carbon tetrachloride, 1,2-dichloroethane and the like; ester solvents such as ethyl acetate, methyl acetate, butyl acetate and the like; polar solvents such as acetone, N,N-dimethylformamide, dimethylsulfoxide and the like, and the like can be mentioned. These can be used alone or in a mixture of two or more kinds thereof. The preferable solvents for this reaction include tetrahydrofuran, dioxane, methanol, ethanol, tert-butanol, ethyl acetate and toluene.

As the base to be used for the reaction, for example, alkali metal hydroxides such as lithium hydroxide, sodium hydroxide, potassium hydroxide and the like; alkali metal carbonates such as sodium carbonate, potassium carbonate and the like; alkali metal hydrogencarbonates such as sodium hydrogencarbonate, potassium hydrogen carbonate and the like; alkali metal carboxylates such as sodium acetate, potassium acetate and the like; alkali metal alkoxides such as sodium ethoxide, sodium methoxide, potassium tert-butoxide and the like, and the like can be mentioned, with preference given to lithium hydroxide and sodium hydroxide.

As the acid to be used for the reaction, for example, organic acids such as formic acid, acetic acid, trifluoroacetic acid, p-toluenesulfonic acid and the like; hydrochloric acid, hydrobromic acid and the like can be mentioned, with preference given to trifluoroacetic acid and hydrochloric acid.

The reaction temperature is about 0°C to 150°C, preferably room temperature to 100°C.
The reaction time is about 10 min to 48 hr, preferably about 30 min to 24 hr.

### Step 16

Compound 26 can be obtained by reacting compound 21 with acid anhydride 25 in a solvent.

As the solvent to be used for the reaction, for example, ethers such as diethyl ether, tetrahydrofuran, dioxane, 1,2-dimethoxyethane, diglyme and the like; hydrocarbons such as benzene, toluene, hexane, xylene and the like; halogenated hydrocarbons such as methylene chloride, chloroform, carbon tetrachloride, 1,2-dichloroethane and the like; esters such as ethyl acetate, methyl acetate, butyl acetate and the like; polar solvents such as acetone, N,N-dimethylformamide, dimethylsulfoxide and the like; and the like can be mentioned. These can be used alone or in a mixture of two or more kinds thereof. The preferable solvents for this reaction include tetrahydrofuran, dioxane, ethyl acetate or toluene.

The reaction temperature is about 0°C to 150°C, preferably room temperature to 130°C.
The reaction time is about 10 min to 48 hr, preferably about 30 min to 24 hr.

When the carbon atom to which R⁴ and R⁵ are bonded is an asymmetric carbon, optically active compound [B-1] can be obtained by using optically active compound 20. To further increase optical purity, repeat recrystallization, or conversion to a salt with an optically active amine (e.g., (R)-1-phenylethylamine etc.), repeat recrystallization of the salt and acid-treatment thereof is performed to give optically active compound [B-1] having higher optical purity.

In addition, when the carbon atom to which R⁴ and R⁵ are bonded is an asymmetric carbon, racemic carboxylic acid compound [B-1] can be converted to an ester by condensing with an optically active alcohol (e.g., (S)-methyl lactate etc.). Since this ester is a diastereomer mixture, optically active carboxylic acid compound [B-1] can be obtained by separation and purification, and hydrolysis. To further increase optical purity, repeat recrystallization, or conversion to a salt with an optically active amine (e.g., (R)-1-phenylethylamine etc.), repeat recrystallization of the salt and acid-treatment thereof is performed to give optically active compound [B-1] having higher optical purity.

### [Examples]

The production of the compound of the present invention is explained in detail in the following by referring to Examples, which are not to be construed as limitative.
In the Examples, room temperature means 1 to 40°C.

### Example 1

### Production of (3-oxo-2,3-dihydrobenzo[1,4]oxazin-4-yl) acetic acid methyl ester

2H-1,4-Benzoxazin-3(4H)-one (3.00 g) was dissolved in N,N-dimethylformamide (30 mL), 60% sodium hydride (0.964 g) was added, and the mixture was stirred at room temperature for 10 min. Methyl bromoacetate (2.22 mL) was added, and the mixture was stirred at room temperature for 2 hr. Water and a 10% aqueous citric acid solution were added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=2:1) to give the title compound (2.08 g) as white crystals.

### Example 2

### Production of (3-oxo-2,3-dihydrobenzo[1,4]thiazin-4-yl)acetic acid methyl ester

4H-Benzo[1,4]thiazin-3-one (3.00 g) was dissolved in N,N-dimethylformamide (30 mL), 60% sodium hydride (0.872 g) was added under ice-cooling, and the mixture was stirred at room temperature for 30 min. Methyl bromoacetate (2.01 mL) was added, and the mixture was stirred at room temperature for 2.5 hr. Water and a 10% aqueous citric acid solution were added to the reaction mixture, and the precipitated solid was collected by filtration. The obtained gray solid was recrystallized from n-hexane-ethyl acetate to give the title compound (2.75 g) as white crystals.

### Example 3

### Production of (3-oxo-2,3-dihydrobenzo[1,4]oxazin-4-yl)acetic acid

(3-Oxo-2,3-dihydrobenzo[1,4]oxazin-4-yl)acetic acid methyl ester obtained in Example 1 (300 mg) was dissolved in methanol (5 mL), and a 2N aqueous sodium hydroxide solution (1.36 mL) was added. The mixture was stirred at room temperature for 3.5 hr, and the solvent was evaporated. The mixture was acidified with water and 1N hydrochloric acid, and the mixture was extracted with ethyl acetate. The obtained ethyl acetate layer was washed successively with water and saturated brine, and dried over anhydrous sodium sulfate. The solvent was evaporated to give the title compound (270 mg) as white crystal.

### Example 4

### Production of (3-oxo-2,3-dihydrobenzo[1,4]thiazin-4-yl)acetic acid

(3-Oxo-2,3-dihydrobenzo[1,4]thiazin-4-yl)acetic acid methyl ester obtained in Example 2 (300 mg) was dissolved in methanol (5 mL), and a 2N aqueous sodium hydroxide solution (1.26 mL) was added. The mixture was stirred at room temperature for 2.5 hr, and the solvent was evaporated. The mixture was acidified with water and 1N hydrochloric acid, and the precipitated solid was collected by filtration to give the title compound (133 mg) as a cream-colored solid.

### Example 5

### Production of (2,2-dimethyl-3-oxo-2,3-dihydrobenzo[1,4]oxazin-4-yl)acetic acid

### step 1

### Production of 2-bromo-N-(2-hydroxyphenyl)-2-methylpropionamide

2-Aminophenol (6.00 g) was dissolved by adding ethyl acetate (62 mL) and water (62 mL), sodium hydrogencarbonate (6.93 g) and 2-bromo-2-methylpropionylbromide (7.44 mL) were added under ice-cooling, and the mixture was stirred at room temperature for 2 hr. The reaction mixture was partitioned, and the obtained ethyl acetate layer was washed successively with water and saturated brine, and dried over anhydrous sodium sulfate. The solvent was evaporated to give the title compound (14.1 g) as an ocher solid.

### step 2

### Production of 2,2-dimethyl-4H-benzo[1,4]oxazin-3-one

2-Bromo-N-(2-hydroxyphenyl)-2-methylpropionamide (14.1 g) was dissolved in N,N-dimethylformamide (70 mL), potassium carbonate (10.2 g) was added, and the mixture was stirred with heating at 60°C for 2 hr. To the reaction mixture was added 1N hydrochloric acid, and the precipitated solid was collected by filtration. The obtained bright yellow solid was crystallized form diisopropyl ether to give the title compound (2.97 g) as an ocher solid.

### step 3

### Production of (2,2-dimethyl-3-oxo-2,3-dihydrobenzo[1,4]oxazin-4-yl)acetic acid methyl ester

2,2-Dimethyl-4H-benzo[1,4]oxazin-3-one (2.94 g) was dissolved in N,N-dimethylformamide (30 mL), 60% sodium hydride (0.796 g) was added under ice-cooling, and the mixture was stirred at room temperature for 1 hr. Methyl bromoacetate (1.83 mL) was added under ice-cooling, and the mixture was stirred at room temperature for 1 hr. Water and 1N hydrochloric acid were added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=3:1) to give the title compound (4.15 g) as a colorless oil.

### step 4

### Production of (2,2-dimethyl-3-oxo-2,3-dihydrobenzo[1,4]oxazin-4-yl)acetic acid

(2,2-Dimethyl-3-oxo-2,3-dihydrobenzo[1,4]oxazin-4-yl)acetic acid methyl ester (300 mg) was dissolved in methanol (5 mL), and a 4N aqueous sodium hydroxide solution (0.603 mL) was added. After stirring the mixture overnight at room temperature, the solvent was evaporated. The mixture was acidified with water and 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (297 mg) as white crystals.

### Example 6

### Production of (2-isopropyl-3-oxo-2,3-dihydrobenzo[1,4]oxazin-4-yl)acetic acid

### step 1

### Production of 2-bromo-3-methylbutyryl chloride

2-Bromo-3-methylbutyric acid (25 g) was dissolved in chloroform (200 mL), oxalyl chloride (14.5 mL) and N,N-dimethylformamide (three drops) were added. After stirring the mixture overnight at room temperature, the mixture was concentrated, and the residue was azeotroped with chloroform and toluene to give the title compound as a yellow oil.

### step 2

### Production of 2-bromo-N-(2-hydroxyphenyl)-3-methylbutylamide

2-Aminophenol (6.00 g) was dissolved by adding ethyl acetate (62 mL) and water (62 mL), sodium hydrogencarbonate (6.93 g) and 2-bromo-3-methylbutyryl chloride (12.1 g) were added under ice-cooling, and the mixture was stirred at room temperature for 3 hr. The reaction mixture was partitioned, and the obtained ethyl acetate layer was washed successively with water and saturated brine, and dried over anhydrous sodium sulfate. The solvent was evaporated to give the title compound (15.2 g) as an ocher solid.

### step 3

### Production of 2-isopropyl-4H-benzo[1,4]oxazin-3-one

2-Bromo-N-(2-hydroxyphenyl)-3-methylbutylamide (15.2 g) was dissolved in N,N-dimethylformamide (75 mL), potassium carbonate (10.2 g) was added, and the mixture was stirred overnight at room temperature. To the reaction mixture was added 1N hydrochloric acid, and the precipitated solid was collected by filtration. The obtained ocher solid was crystallized from n-hexane-ethyl acetate to give the title compound (7.97 g) as a cream-colored solid.

### step 4

### Production of (2-isopropyl-3-oxo-2,3-dihydrobenzo[1,4]oxazin-4-yl)acetic acid methyl ester

2-Isopropyl-4H-benzo[1,4]oxazin-3-one (2.50 g) was dissolved in N,N-dimethylformamide (20 mL), 60% sodium hydride (0.628 g) was added under ice-cooling, and the mixture was stirred at room temperature for 1 hr. Methyl bromoacetate (1.45 mL) was added, and the mixture was stirred at room temperature for 1 hr. Water and 1N hydrochloric acid were added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=3:1) to give the title compound (3.47 g) as a colorless oil.

### step 5

### Production of (2-isopropyl-3-oxo-2,3-dihydrobenzo[1,4]oxazin-4-yl)acetic acid

(2-Isopropyl-3-oxo-2,3-dihydrobenzo[1,4]oxazin-4-yl)acetic acid methyl ester (300 mg) was dissolved in methanol (5 mL) and a 4N aqueous sodium hydroxide solution (0.57 mL) was added. The mixture was stirred at room temperature for 1 hr, and the solvent was evaporated. The mixture was acidified with water and 1N hydrochloric acid, and the precipitated solid was collected by filtration to give the title compound (206 mg) as white crystals.

### Example 7

### Production of ((S)-2,8-diisopropyl-3-thioxo-2,3-dihydrobenzo[1,4]oxazin-4-yl)acetic acid

### step 1

### Production of (S)-2-[2-((S)-2,8-diisopropyl-3-thioxo-2,3-dihydrobenzo[1,4]oxazin-4-yl)acetoxy]propionic acid methyl ester

(2,8-Diisopropyl-3-thioxo-2,3-dihydrobenzo[1,4]oxazin-4-yl)acetic acid (4.9349 g) and (S)-methyl lactate (1.693 g) were dissolved in chloroform (50 mL), and 4-dimethylaminopyridine (DMAP) (2.19 g) and water-soluble carbodiimide (WSC·HCl: 1-ethyl-3-(3'-dimethylaminopropyl)carbodiimide hydrochloride) (3.43 g) were added under ice-cooling. After stirring the mixture overnight at room temperature, the reaction mixture was purified by silica gel chromatography (n-hexane-ethyl acetate=8:1) to give the title compound (1.6876 g) as a yellow oil.

### step 2

### Production of ((S)-2,8-diisopropyl-3-thioxo-2,3-dihydrobenzo[1,4]oxazin-4-yl)acetic acid

(S)-2-[2-((S)-2,8-Diisopropyl-3-thioxo-2,3-dihydrobenzo[1,4]oxazin-4-yl)acetoxy]propionic acid methyl ester (0.5025 g) was dissolved in methanol (3.2 mL) and 1,4-dioxane (3.2 mL), and a 2N aqueous sodium hydroxide solution (3.2 mL) was added. After stirring the mixture at room temperature for 0.5 hr, the mixture was acidified with water and 1N hydrochloric acid under ice-cooling, and the mixture was extracted with ethyl acetate. The obtained ethyl acetate layer was washed successively with water and saturated brine, and dried over anhydrous sodium sulfate. The solvent was evaporated, n-hexane (1.5 mL) was added to the obtained residue, and the residue was recrystallized. The precipitated solid was removed by filtration, and the filtrate was concentrated. n-Hexane (1.0 mL) was added to the obtained residue, and the residue was recrystallized. The precipitated solid was collected by filtration to give the title compound (0.0117 g) as an amorphous solid.

### Example 8

### Production of ((R)-2,8-diisopropyl-3-thioxo-2,3-dihydrobenzo[1,4]oxazin-4-yl)acetic acid

### step 1

### Production of (S)-2-[2-((R)-2,8-diisopropyl-3-thioxo-2,3-dihydrobenzo[1,4]oxazin-4-yl)acetoxy]propionic acid methyl ester

(2,8-Diisopropyl-3-thioxo-2,3-dihydrobenzo[1,4]oxazin-4-yl)acetic acid (4.9349 g) and (S)-methyl lactate (1.693 g) were dissolved in chloroform (50 mL), and 4-dimethylaminopyridine (DMAP) (2.19 g) and water-soluble carbodiimide (WSC·HCl: 1-ethyl-3-(3'-dimethylaminopropyl)carbodiimide hydrochloride) (3.43 g) were added under ice-cooling. After stirring the mixture overnight at room temperature, the reaction mixture was purified by silica gel chromatography (n-hexane-ethyl acetate=8:1) to give the title compound (1.7564 g) as a yellow oil.

### step 2

### Production of ((R)-2,8-diisopropyl-3-thioxo-2,3-dihydrobenzo[1,4]oxazin-4-yl)acetic acid

(S)-2-[2-((R)-2,8-Diisopropyl-3-thioxo-2,3-dihydrobenzo[1,4]oxazin-4-yl)acetoxy]propionic acid methyl ester (0.5085 g) was dissolved in methanol (3.2 mL) and 1,4-dioxane (3.2 mL), and a 2N aqueous sodium hydroxide solution (3.2 mL) was added. After stirring the mixture at room temperature for 0.5 hr, the mixture was acidified with water and 1N hydrochloric acid under ice-cooling, and the mixture was extracted with ethyl acetate. The obtained ethyl acetate layer was washed successively with water and saturated brine, and dried over anhydrous sodium sulfate. The solvent was evaporated, n-hexane (1.5 mL) was added to the obtained residue, and the residue was recrystallized. The precipitated solid was removed by filtration, and the filtrate was concentrated. n-Hexane (1.0 mL) was added to the obtained residue, and the residue was recrystallized. The precipitated solid was collected by filtration to give the title compound (0.2604 g) as yellow crystals.

### Example 9

### Production of (2,8-diisopropyl-3-oxo-2,3-dihydrobenzo[1,4]oxazin-4-yl)acetic acid

### step 1

### Production of 4-bromo-2-isopropylphenol

2-Isopropylphenol (25 g) was dissolved in acetic acid (250 mL) and 48% hydrobromic acid (125.mL), and dimethyl sulfoxide (125 mL) was added dropwise at room temperature. After stirring the mixture at room temperature for 2 hr, the reaction mixture was neutralized with sodium carbonate (257 g). Water was added, and the mixture was extracted with diethyl ether. The obtained diethyl ether layer was washed successively with water and saturated brine, and dried over anhydrous sodium sulfate. The solvent was evaporated to give the title compound (40.2 g) as a pale-yellow solid.

### step 2

### Production of 4-bromo-2-isopropyl-6-nitrophenol

To a mixture of 4-bromo-2-isopropylphenol (40.2 g) and sodium nitrite (41.5 g), n-hexane (300 mL) and diisopropyl ether (130 mL) and water (200 mL) were added, and 4.5N sulfuric acid (430 mL) was added dropwise at room temperature. After stirring the mixture at room temperature for 1 hr, the reaction mixture was washed successively with saturated aqueous sodium hydrogencarbonate solution, water and saturated brine, and dried over anhydrous sodium sulfate. The solvent was evaporated, and the obtained residue was purified by silica gel chromatography (n-hexane-ethyl acetate=20:1) to give the title compound (41.1 g) as a yellow oil.

### step 3

### Production of 2-amino-6-isopropylphenol hydrobromide

4-Bromo-2-isopropyl-6-nitrophenol (41.1 g) was dissolved in methanol (300 mL). To this solution was added 7.5% palladium-carbon (8 g), and the mixture was stirred overnight at room temperature under hydrogen atmosphere (2 kgf/cm²). The reaction mixture was filtered through celite, and the filtrate was concentrated under reduced pressure. The obtained solid was crystallized from ethyl acetate to give the title compound (27.4 g) as a beige solid.

### step 4

### Production of 2-bromo-N-(2-hydroxy-3-isopropylphenyl)-3-methylbutylamide

2-Amino-6-isopropylphenol hydrobromide (6.14 g) was dissolved by adding ethyl acetate (70 mL) and water (80 mL), and sodium hydrogencarbonate (6.68 g) and 2-bromo-3-methylbutyryl chloride (5.80 g) obtained in step 1 of Example 6 were added at room temperature, and the mixture was stirred at room temperature for 2 hr. The reaction mixture was partitioned, and the obtained ethyl acetate layer was washed successively with saturated aqueous sodium hydrogencarbonate solution, water and saturated brine, and dried over anhydrous sodium sulfate. The solvent was evaporated to give the title compound (9.0 g) as a pale-pink solid.

### step 5

### Production of 2,8-diisopropyl-4H-benzo[1,4]oxazin-3-one

2-Bromo-N-(2-hydroxy-3-isopropylphenyl)-3-methylbutylamide (9.0 g) was dissolved in N,N-dimethylformamide (45 mL), potassium carbonate (4.94 g) was added, and the mixture was stirred overnight at room temperature. To the reaction mixture was added 1N hydrochloric acid, water was added, and the precipitated solid was collected by filtration. The obtained solid was crystallized from n-hexane-diisopropyl ether to give the title compound (4.5 g) as a white solid.

### step 6

### Production of (2,8-diisopropyl-3-oxo-2,3-dihydrobenzo[1,4]oxazin-4-yl)acetic acid methyl ester

2,8-Diisopropyl-4H-benzo[1,4]oxazin-3-one (800 mg) was dissolved in N,N-dimethylformamide (8 mL), and 60% sodium hydride (164 mg) was added. After stirring the mixture at room temperature for 1 hr, methyl bromoacetate (0.379 mL) was added. After stirring the mixture overnight at room temperature, water and 1N hydrochloric acid were added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=4:1) to give the title compound (1.11 g) as a colorless oil.

### step 7

### Production of (2,8-diisopropyl-3-oxo-2,3-dihydrobenzo[1,4]oxazin-4-yl)acetic acid

(2,8-Diisopropyl-3-oxo-2,3-dihydrobenzo[1,4]oxazin-4-yl)acetic acid methyl ester (300 mg) was dissolved in methanol (5 mL), a 4N aqueous sodium hydroxide solution (1 mL) was added. After stirring the mixture overnight at room temperature, the solvent was evaporated. The mixture was acidified with water and 1N hydrochloric acid, and extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (241 mg) as white crystals.

### Example 10

### Production of (8-isopropyl-3-oxo-2,3-dihydrobenzo[1,4]oxazin-4-yl)acetic acid

### step 1

### Production of 2-bromo-N-(2-hydroxy-3-isopropylphenyl)acetamide

2-Amino-6-isopropylphenol hydrobromide (4.61 g) obtained in step 3 of Example 9 was dissolved by adding ethyl acetate (50 mL) and water (50 mL), sodium hydrogencarbonate (5.0 g) and bromoacetyl chloride (1.81 mL) were added at room temperature, and the mixture was stirred at room temperature for 1 hr. The reaction mixture was partitioned, and the obtained ethyl acetate layer was washed successively with water and saturated brine, and dried over anhydrous sodium sulfate. The solvent was evaporated to give the title compound (5.4 g) as an ocher solid.

### step 2

### Production of 8-isopropyl-4H-benzo[1,4]oxazin-3-one

2-Bromo-N-(2-hydroxy-3-isopropylphenyl)acetamide (5.4 g) was dissolved in N,N-dimethylformamide (27 mL), potassium carbonate (3.69 g) was added, and the mixture was stirred overnight at room temperature. Water and 1N hydrochloric acid was added to the reaction mixture, and the precipitated solid was collected by filtration. The obtained solid was crystallized from n-hexane-diisopropyl ether to give the title compound (2.73 g) as a pale-pink solid.

### step 3

### Production of (8-isopropyl-3-oxo-2,3-dihydrobenzo[1,4]oxazin-4-yl)acetic acid methyl ester

8-Isopropyl-4H-benzo[1,4]oxazin-3-one (1.50 g) was dissolved in N,N-dimethylformamide (15 mL), and 60% sodium hydride (376 mg) was added. After stirring the mixture at room temperature for 1 hr, methyl bromoacetate (0.867 mL) was added. After stirring the mixture at room temperature for 3 hr, water and 1N hydrochloric acid were added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=4:1), and crystallized from n-hexane-ethyl acetate to give the title compound (1.53 g) as a solid.

### step 4

### Production of (8-isopropyl-3-oxo-2,3-dihydrobenzo[1,4]oxazin-4-yl)acetic acid

(8-Isopropyl-3-oxo-2,3-dihydrobenzo[1,4]oxazin-4-yl)acetic acid methyl ester (300 mg) was dissolved in methanol (5 mL), and a 4N aqueous sodium hydroxide solution (1 mL) was added. After stirring the mixture overnight at room temperature, the solvent was evaporated. The mixture was acidified with water and 1N hydrochloric acid, and the precipitated solid was collected by filtration to give the title compound (218 mg) as white crystals.

### Example 11

### Production of (2,2-dimethyl-3-thioxo-2,3-dihydrobenzo[1,4]oxazin-4-yl)acetic acid

### step 1

### Production of (2,2-dimethyl-3-thioxo-2,3-dihydrobenzo[1,4]oxazin-4-yl)acetic acid methyl ester

(2,2-Dimethyl-3-oxo-2,3-dihydrobenzo[1,4]oxazin-4-yl)acetic acid methyl ester (1.00 g) obtained in step 3 of Example 5 was dissolved in toluene (10 mL), and Lawesson reagent (973 mg) was added. After heating under reflux for 7 hr, water and saturated aqueous sodium hydrogencarbonate solution were added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=9:1) to give the title compound (957 mg) as a lemon-colored solid.

### step 2

### Production of (2,2-dimethyl-3-thioxo-2,3-dihydrobenzo[1,4]oxazin-4-yl)acetic acid

(2,2-Dimethyl-3-thioxo-2,3-dihydrobenzo[1,4]oxazin-4-yl)acetic acid methyl ester (300 mg) was dissolved in methanol (1 mL) and 1,4-dioxane (2 mL), and a 2N aqueous sodium hydroxide solution (0.678 mL) was added. After stirring the mixture overnight at room temperature, the mixture was acidified with 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was purified by preparative silica gel chromatography (chloroform -methanol=9:1) to give the title compound (165 mg) as a lemon-colored solid.

### Example 12

### Production of (3-thioxo-2,3-dihydrobenzo[1,4]thiazin-4-yl)acetic acid

### step 1

### Production of (3-thioxo-2,3-dihydrobenzo[1,4]thiazin-4-yl)acetic acid methyl ester

(3-Oxo-2,3-dihydrobenzo[1,4]thiazin-4-yl)acetic acid methyl ester obtained in Example 2 (1.00 g) was dissolved in tetrahydrofuran (10 mL), and Lawesson reagent (1.02 g) was added. After heating under reflux for 9 hr, water and saturated aqueous sodium hydrogencarbonate solution were added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=4:1) to give the title compound (934 mg) as a lemon-colored solid.

### step 2

### Production of (3-thioxo-2,3-dihydrobenzo[1,4]thiazin-4-yl)acetic acid

3-Thioxo-2,3-dihydrobenzo[1,4]thiazin-4-yl)acetic acid methyl ester (300 mg) was dissolved in methanol (1 mL) and 1,4-dioxane (2 mL), and a 1N aqueous sodium hydroxide solution (1.42 mL) was added. After stirring the mixture at room temperature for 9 hr, the mixture was acidified with water and 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was crystallized from ethyl acetate to give the title compound (94 mg) as yellow crystals.

### Example 13

### Production of (2-isopropyl-3-thioxo-2,3-dihydrobenzo[1,4]oxazin-4-yl)acetic acid

### step 1

### Production of (2-isopropyl-3-thioxo-2,3-dihydrobenzo[1,4]oxazin-4-yl)acetic acid methyl ester

(2-Isopropyl-3-oxo-2,3-dihydrobenzo[1,4]oxazin-4-yl)acetic acid methyl ester (1.00 g) obtained in step 4 of Example 6 was dissolved in toluene (10 mL), and Lawesson reagent (922 mg) was added. After heating under reflux for 10 hr, saturated aqueous sodium hydrogencarbonate solution was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=9:1) to give the title compound (663 mg) as a yellow oil.

### step 2

### Production of (2-isopropyl-3-thioxo-2,3-dihydrobenzo[1,4]oxazin-4-yl)acetic acid

(2-Isopropyl-3-thioxo-2,3-dihydrobenzo[1,4]oxazin-4-yl)acetic acid methyl ester (300 mg) was dissolved in methanol (1 mL) and 1,4-dioxane (2 mL), and a 1N aqueous sodium hydroxide solution (1.25 mL) was added. After stirring the mixture at room temperature for 5 hr, the mixture was acidified with water and 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was purified by preparative silica gel chromatography (chloroform -methanol=12:1) to give the title compound (229 mg) as a yellow oil.

### Example 14

### Production of 3-(2,8-diisopropyl-3-oxo-2,3-dihydrobenzo[1,4]oxazin-4-yl)propionic acid

### step 1

### Production of 3-(2,8-diisopropyl-3-oxo-2,3-dihydrobenzo[1,4]oxazin-4-yl)propionic acid methyl ester

2,8-Diisopropyl-4H-benzo[1,4]oxazin-3-one (1.5 g) obtained in step 5 of Example 9 was dissolved in N,N-dimethylformamide (15 mL), and potassium carbonate (1.78 g) and methyl acrylate (1.16 mL) were added. After stirring with heating overnight at 100°C, water and 1N hydrochloric acid were added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=6:1) to give the title compound (2.09 g) as a colorless oil.

### step 2

Production of 3-(2,8-diisopropyl-3-oxo-2,3-dihydrobenzo[1,4]oxazin-4-yl)propionic acid 3-(2,8-Diisopropyl-3-oxo-2,3-dihydrobenzo[1,4]oxazin-4-yl)propionic acid methyl ester (300 mg) was dissolved in methanol (5 mL), and a 2N aqueous sodium hydroxide solution (0.939 mL) was added. After stirring the mixture overnight at room temperature, the mixture was acidified with water and 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (202 mg) as white crystals.

### Example 15

### Production of (8-isopropyl-3-thioxo-2,3-dihydrobenzo[1,4]oxazin-4-yl)acetic acid

### step 1

### Production of (8-isopropyl-3-oxo-2,3-dihydrobenzo[1,4]oxazin-4-yl)acetic acid tert-butyl ester

8-Isopropyl-4H-benzo[1,4]oxazin-3-one (1.00 g) obtained in step 2 of Example 10 was dissolved in N,N-dimethylformamide (10 mL), and 60% sodium hydride (251 mg) was added. After stirring the mixture at room temperature for 1 hr, bromoacetic acid tert-butyl ester (0.927 mL) was added. After stirring the mixture at room temperature for 2 hr, water and a 10% aqueous citric acid solution were added to the reaction mixture, and the mixture was extracted with ethyl acetate. The obtained ethyl acetate layer was washed successively with water and saturated brine, and dried over anhydrous sodium sulfate. The solvent was evaporated to give the title compound (1.83 g) as an orange oil.

### step 2

### Production of (8-isopropyl-3-thioxo-2,3-dihydrobenzo[1,4]oxazin-4-yl)acetic acid tert-butyl ester

(8-Isopropyl-3-oxo-2,3-dihydrobenzo[1,4]oxazin-4-yl)acetic acid tert-butyl ester (1.27 g) was dissolved in toluene (10 mL), and Lawesson reagent (1.27 g) was added. After heating under reflux overnight, saturated aqueous sodium hydrogencarbonate solution and water were added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=9:1) to give the title compound (852 mg) as a brown solid.

### step 3

### Production of (8-isopropyl-3-thioxo-2,3-dihydrobenzo[1,4]oxazin-4-yl)acetic acid

(8-Isopropyl-3-thioxo-2,3-dihydrobenzo[1,4]oxazin-4-yl)acetic acid tert-butyl ester (300 mg) was dissolved in toluene (1.5 mL) and trifluoroacetic acid (1.5 mL), and the mixture was stirred at room temperature for 3 hr. n-Hexane was added to the reaction mixture, and the precipitated solid was collected by filtration to give the title compound (168 mg) as white crystals.

### Example 16

### Production of 3-(2,8-diisopropyl-3-thioxo-2,3-dihydrobenzo[1,4]oxazin-4-yl)propionic acid

### step 1

Production of 3-(2,8-diisopropyl-3-thioxo-2,3-dihydrobenzo[1,4]oxazin-4-yl)propionic acid methyl ester 3-(2,8-Diisopropyl-3-oxo-2,3-dihydrobenzo[1,4]oxazin-4-yl)propionic acid methyl ester (1.00 g) obtained in step 1 of Example 14 was dissolved in toluene (10 mL), and Lawesson reagent (0.759 g) was added. After heating under reflux overnight, saturated aqueous sodium hydrogencarbonate solution was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=9:1) to give the title compound (916 mg) as a yellow oil.

### step 2

### Production of 3-(2,8-diisopropyl-3-thioxo-2,3-dihydrobenzo[1,4]oxazin-4-yl)propionic acid

3-(2,8-Diisopropyl-3-thioxo-2,3-dihydrobenzo[1,4]oxazin-4-yl)propionic acid methyl ester (300 mg) was dissolved in methanol (5 mL), and a 1N aqueous sodium hydroxide solution (1.07 mL) was added. After stirring the mixture overnight at room temperature, the mixture was acidified with water and 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was purified by preparative silica gel chromatography (chloroform -methanol=15:1) to give the title compound (250 mg) as a yellow oil.

### Example 17

### Production of (3-thioxo-2,3-dihydrobenzo[1,4]oxazin-4-yl)acetic acid

### step 1

### Production of (3-oxo-2,3-dihydrobenzo[1,4]oxazin-4-yl)acetic acid tert-butyl ester

2H-1,4-Benzoxazin-3(4H)-one (1.00 g) was dissolved in N,N-dimethylformamide (10 mL), and 60% sodium hydride (0.322 g) was added. After stirring the mixture at room temperature for 1 hr, tert-butyl bromoacetate (1.19 mL) was added. After stirring the mixture overnight at room temperature, a 10% aqueous citric acid solution was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=4:1) to give the title compound (1.63 g) as a white solid.

### step 2

### Production of (3-thioxo-2,3-dihydrobenzo[1,4]oxazin-4-yl)acetic acid tert-butyl ester

(3-Oxo-2,3-dihydrobenzo[1,4]oxazin-4-yl)acetic acid tert-butyl ester (1.63 g) was dissolved in toluene (15 mL), and Lawesson reagent (1.5 g) was added. After heating under reflux overnight, saturated aqueous sodium hydrogencarbonate solution was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=17:3) to give the title compound (1.68 g) as a brown oil.

### step 3

### Production of (3-thioxo-2,3-dihydrobenzo[1,4]oxazin-4-yl)acetic acid

(3-Thioxo-2,3-dihydrobenzo[1,4]oxazin-4-yl)acetic acid tert-butyl ester (300 mg) was dissolved in toluene (1.5 mL) and trifluoroacetic acid (1.5 mL), and the mixture was stirred overnight at room temperature. The solvent was evaporated and azeotroped with toluene. Toluene was added to the obtained residue, and the precipitated solid was collected by filtration to give the title compound (161 mg) as an ocher solid.

### Example 18

### Production of 4-(2,8-diisopropyl-3-oxo-2,3-dihydrobenzo[1,4]oxazin-4-yl)butyric acid

### step 1

### Production of 4-(2,8-diisopropyl-3-oxo-2,3-dihydrobenzo[1,4]oxazin-4-yl)butyric acid ethyl ester

2,8-Diisopropyl-4H-benzo[1,4]oxazin-3-one (734 mg) obtained in step 5 of Example 9 was dissolved in N,N-dimethylformamide (8 mL), and 60% sodium hydride (151 mg) was added. After stirring the mixture at room temperature for 2 hr, and ethyl 4-bromobutyrare (0.546 mL) was added. After stirring the mixture at room temperature, 1N hydrochloric acid was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography to give the title compound (0.958 g) as a colorless oil.

### step 2

### Production of 4-(2,8-diisopropyl-3-oxo-2,3-dihydrobenzo[1,4]oxazin-4-yl)butyric acid

4-(2,8-Diisopropyl-3-oxo-2,3-dihydrobenzo[1,4]oxazin-4-yl)butyric acid ethyl ester (300 mg) was dissolved in methanol (5 mL), and a 1N aqueous sodium hydroxide solution (1.5 mL) was added. After stirring the mixture at room temperature for 3.5 hr, the solvent was evaporated. The mixture was acidified with water and 1N hydrochloric acid, and the precipitated solid was collected by filtration to give the title compound (225 mg) as white crystals.

### Example 19

3-(3-oxo-2,3-dihydrobenzo[1,4]oxazin-4-yl)propionic acid was purchased from Aldrich.

### Example 20

### Production of 4-(2,8-diisopropyl-3-thioxo-2,3-dihydrobenzo[1,4]oxazin-4-yl)butyric acid

### step 1

### Production of 4-(2,8-diisopropyl-3-thioxo-2,3-dihydrobenzo[1,4]oxazin-4-yl)butyric acid ethyl ester

4-(2,8-Diisopropyl-3-oxo-2,3-dihydrobenzo[1,4]oxazin-4-yl)butyric acid ethyl ester (690 mg) obtained in step 1 of Example 18 was dissolved in toluene (10 mL), and Lawesson reagent (522 mg) was added. After heating under reflux for 8.5 hr, saturated aqueous sodium hydrogencarbonate solution was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=97:1) to give the title compound (670 mg) as a lemon-colored oil.

### step 2

### Production of 4-(2,8-diisopropyl-3-thioxo-2,3-dihydrobenzo[1,4]oxazin-4-yl)butyric acid

4-(2,8-Diisopropyl-3-thioxo-2,3-dihydrobenzo[1,4]oxazin-4-yl)butyric acid ethyl ester (300 mg) was dissolved in methanol (5 mL) and tetrahydrofuran (2 mL), and a 1N aqueous sodium hydroxide solution (0.99 mL) was added. After stirring the mixture overnight at room temperature, the solvent was evaporated. The mixture was acidified with water and 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was purified by preparative silica gel chromatography (chloroform -methanol=15:1) to give the title compound (230 mg) as a yellow oil.

### Example 21

### Production of 4-(2,8-diisopropyl-2,3-dihydrobenzo[1,4]oxazin-4-yl)-4-oxobutyric acid

### step 1

### Production of 2,8-diisopropyl-3,4-dihydro-2H-benzo[1,4]oxazine

2,8-Diisopropyl-4H-benzo[1,4]oxazin-3-one (4.0 g) obtained in step 5 of Example 9 was dissolved in tetrahydrofuran (40 mL), and lithium aluminum hydride (1.30 g) was added. After heating under reflux for 15 hr, water (1.3 mL), 15% aqueous sodium hydroxide solution (1.3 mL) and water (3.9 mL) were successively added under ice-cooling, and the mixture was stirred at room temperature. Anhydrous magnesium sulfate was added, and the mixture was dried and concentrated. The obtained residue was purified by silica gel chromatography (n-hexane-ethyl acetate=3:1) to give the title compound (3.53 g) as a green oil.

### step 2

### Production of 4-(2,8-diisopropyl-2,3-dihydrobenzo[1,4]oxazin-4-yl)-4-oxobutyric acid methyl ester

2,8-Diisopropyl-3,4-dihydro-2H-benzo[1,4]oxazine (300 mg) was dissolved in chloroform (5 mL), and triethylamine (0.229 mL) and methyl 3-(chlorocarbonyl)propionate (0.202 mL) were added. After stirring the mixture overnight at room temperature, the reaction mixture was purified by silica gel chromatography (n-hexane-ethyl acetate=3:1) to give the title compound (451 mg) as a white solid.

### step 3

### Production of 4-(2,8-diisopropyl-2,3-dihydrobenzo[1,4]oxazin-4-yl)-4-oxobutyric acid

4-(2,8-Diisopropyl-2,3-dihydrobenzo[1,4]oxazin-4-yl)-4-oxobutyric acid methyl ester (440 mg) was dissolved in methanol (5 mL), and a 2N aqueous sodium hydroxide solution (1.32 mL) was added. After stirring the mixture at room temperature for 15 hr, the solvent was evaporated. The mixture was acidified with water and 1N hydrochloric acid, and the mixture was extracted with ethyl acetate. The obtained ethyl acetate layer was dried over anhydrous sodium sulfate. The solvent was evaporated, and the obtained residue was crystallized from n-hexane to give the title compound (340 mg) as white crystals.

### Example 22

### Production of 3-(2-isopropyl-3-oxo-2,3-dihydrobenzo[1,4]thiazin-4-yl)propionic acid

### step 1

### Production of 2-isopropyl-4H-benzo[1,4]thiazin-3-one

2-Aminobenzenethiol (1.63 g) and ethyl 2-bromo-3-methylbutyrate (2.09 g) were dissolved in dimethyl sulfoxide (20 mL), and potassium carbonate (1.78 g) was added. After stirring the mixture at room temperature for 1.5 hr, 1N hydrochloric acid was added to the reaction mixture, and the mixture was extracted with ethyl acetate. The obtained ethyl acetate layer was washed successively with water and saturated brine, and dried over anhydrous magnesium sulfate. The solvent was evaporated, and the obtained residue was dissolved in N,N-dimethylformamide (10 mL). Concentrated hydrochloric acid (0.13 mL) was added, and the mixture was stirred with heating at 80°C for 1.5 hr. Water was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was crystallized from n-hexane-ethyl acetate to give the title compound (850 mg) as a pale-yellow solid.

### step 2

### Production of 3-(2-isopropyl-3-oxo-2,3-dihydrobenzo[1,4]thiazin-4-yl)propionic acid methyl ester

2-Isopropyl-4H-benzo[1,4]thiazin-3-one (250 mg) was dissolved in N,N-dimethylformamide (5 mL), and potassium carbonate (346 mg) and methyl acrylate (0.225 mL) were added. After stirring with heating for 15 hr at 80°C, 1N hydrochloric acid was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=3:1) to give the title compound (257 mg) as a colorless oil.

### step 3

### Production of 3-(2-isopropyl-3-oxo-2,3-dihydrobenzo[1,4]thiazin-4-yl)propionic acid

3-(2-Isopropyl-3-oxo-2,3-dihydrobenzo[1,4]thiazin-4-yl)propionic acid methyl ester (250 mg) was dissolved in methanol (5 mL), and a 2N aqueous sodium hydroxide solution (0.85 mL) was added. The mixture was stirred at room temperature for 16 hr, and concentrated. The residue was acidified with water and 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (202 mg) as white crystals.

### Example 23

### Production of 4-(2,3-dihydrobenzo[1,4]oxazin-4-yl)-4-oxobutyric acid

### step 1

### Production of 3,4-dihydro-2H-benzo[1,4]oxazine

Lithium aluminum hydride (3 g) was suspended in tetrahydrofuran (120 mL), and 2H-1,4-benzoxazin-3(4H)-one (6 g) was added by small portions under ice-cooling,. After heating under reflux for 10 hr, water (3 mL), 15% aqueous sodium hydroxide solution (3 mL) and water (9 mL) were successively added under ice-cooling, and the mixture was stirred at room temperature. Anhydrous sodium sulfate was added, and the mixture was dried and concentrated. The obtained residue was purified by silica gel chromatography (n-hexane-ethyl acetate=4:1) to give the title compound (4.9239 g) as an orange oil.

### step 2

### Production of 4-(2,3-dihydrobenzo[1,4]oxazin-4-yl)-4-oxobutyric acid

3,4-Dihydro-2H-benzo[1,4]oxazine (300 mg) and succinic anhydride (222 mg) were dissolved in toluene (3 mL), the mixture was heated under reflux for 4 hr. the reaction mixture was concentrated, obtained residue was purified by silica gel chromatography (chloroform-methanol=97:3) to give the title compound (348 mg) as a solid.

### Example 24

### Production of 5-(2,3-dihydrobenzo[1,4]oxazin-4-yl)-5-oxovaleric acid

3,4-Dihydro-2H-benzo[1,4]oxazine (300 mg) obtained in step 1 of Example 23 and glutaric anhydride (266 mg) were dissolved in toluene (3 mL), and the mixture was heated under reflux for 3 hr, and concentrated. The obtained residue was purified by silica gel chromatography (chloroform-methanol=97:3) to give the title compound (335 mg) as an oil.

### Example 25

### Production of (Z)-4-(2,3-dihydrobenzo[1,4]oxazin-4-yl)-4-oxo-2-butenoic acid

3,4-Dihydro-2H-benzo[1,4]oxazine (300 mg) obtained in step 1 of Example 23 and maleic anhydride (218 mg) were dissolved in toluene (3 mL), and the mixture was heated under reflux for 4 hr. The precipitated solid was collected by filtration to give the title compound (476 mg) as crystals.

### Example 26

Production of (8-isopropyl-3-oxo-2-phenyl-2,3-dihydrobenzo[1,4]oxazin-4-yl)acetic acid

### step 1

### Production of 2-chloro-N-(2-hydroxy-3-isopropylphenyl)-2-phenylacetamide

2-Amino-6-isopropylphenol hydrobromide (2.00 g) obtained in step 3 of Example 9 was dissolved by adding ethyl acetate (20 mL) and water (20 mL), and sodium hydrogencarbonate (1.81 g) and α-chlorophenylacetyl chloride (1.82 mL) were added at room temperature, and the mixture was stirred at room temperature. The reaction mixture was partitioned, and the obtained ethyl acetate layer was washed successively with saturated aqueous sodium hydrogencarbonate solution, water and saturated brine, and dried over anhydrous sodium sulfate. The solvent was evaporated to give the title compound as a brown oil.

### step 2

### Production of 8-isopropyl-2-phenyl-4H-benzo[1,4]oxazin-3-one

2-Chloro-N-(2-hydroxy-3-isopropylphenyl)-2-phenylacetamide obtained in step 1 was dissolved in N,N-dimethylformamide (15 mL), and potassium carbonate (1.61 g) was added, and the mixture was stirred overnight at room temperature. 1N Hydrochloric acid was added to the reaction mixture, water was added, and the precipitated solid was collected by filtration. The obtained ocher solid was crystallized from n-hexane-diisopropyl ether to give the title compound (1.92 g) as an ocher solid.

### step 3

### Production of (8-isopropyl-3-oxo-2-phenyl-2,3-dihydrobenzo[1,4]oxazin-4-yl)acetic acid methyl ester

8-Isopropyl-2-phenyl-4H-benzo[1,4]oxazin-3-one (253 mg) was dissolved in N,N-dimethylformamide (5 mL), and 60% sodium hydride (48 mg) was added. After stirring the mixture at room temperature for 1.5 hr, methyl bromoacetate (0.111 mL) was added. After stirring the mixture at room temperature for 2 hr, a 10% aqueous citric acid solution was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=4:1) to give the title compound (307 mg) as a colorless oil.

### step 4

### Production of (8-isopropyl-3-oxo-2-phenyl-2,3-dihydrobenzo[1,4]oxazin-4-yl)acetic acid

(8-Isopropyl-3-oxo-2-phenyl-2,3-dihydrobenzo[1,4]oxazin-4-yl)acetic acid methyl ester (302 mg) was dissolved in methanol (5 mL), and a 1N aqueous sodium hydroxide solution (1.78 mL) was added. After stirring the mixture at room temperature for 2 hr, the solvent was evaporated, and the mixture was acidified with 1N hydrochloric acid, and extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (262 mg) as white crystals.

### Example 27

### Production of 6-(2,3-dihydrobenzo[1,4]oxazin-4-yl)-6-oxohexanoic acid

### step 1

### Production of 6-(2,3-dihydrobenzo[1,4]oxazin-4-yl)-6-oxohexanoic acid ethyl ester

3,4-Dihydro-2H-benzo[1,4]oxazine (300 mg) obtained in step 1 of Example 23 and adipic acid monoethyl ester (425 mg) were dissolved in chloroform (6 mL), 4-dimethylaminopyridine (DMAP) (271 mg) and water-soluble carbodiimide (WSC·HCl: 1-ethyl-3-(3'-dimethylaminopropyl)carbodiimide hydrochloride) (468 mg) were added under ice-cooling. After stirring the mixture at room temperature for 12 hr, the solvent was evaporated, and the residue was extracted with ethyl acetate. The obtained ethyl acetate layer was washed successively with 1N hydrochloric acid and saturated aqueous sodium hydrogencarbonate solution, and dried over anhydrous sodium sulfate. The solvent was evaporated, and the obtained residue was purified by silica gel chromatography (n-hexane-ethyl acetate=75:25) to give the title compound (491 mg) as a solid.

### step 2

### Production of 6-(2,3-dihydrobenzo[1,4]oxazin-4-yl)-6-oxohexanoic acid

6-(2,3-Dihydrobenzo[1,4]oxazin-4-yl)-6-oxohexanoic acid ethyl ester (453 mg) was dissolved in ethanol (4.5 mL), and a 1N aqueous sodium hydroxide solution (1.7 mL) was added. After stirring the mixture at room temperature for 2 hr, the solvent was evaporated. Water was added, and the mixture was washed with diethyl ether. The obtained aqueous layer was acidified with 1N hydrochloric acid, and the mixture was extracted with ethyl acetate. The obtained ethyl acetate layer was washed successively with water and saturated brine, and dried over anhydrous sodium sulfate. The solvent was evaporated to give the title compound (403.1 mg) as crystals.

### Example 28

### Production of (E)-4-(2,3-dihydrobenzo[1,4]oxazin-4-yl)-4-oxo-2-butenoic acid

### step 1

### Production of (E)-4-(2,3-dihydrobenzo[1,4]oxazin-4-yl)-4-oxo-2-butenoic acid ethyl ester

3,4-Dihydro-2H-benzo[1,4]oxazine (300 mg) obtained in step 1 of Example 23 and fumaric acid monoethyl ester (352 mg) were dissolved in chloroform (6 mL), and 4-dimethylaminopyridine (DMAP) (271 mg) and water-soluble carbodiimide (WSC·HCl: 1-ethyl-3-(3'-dimethylaminopropyl)carbodiimide hydrochloride) (468 mg) were added under ice-cooling. After stirring the mixture at room temperature for 12 hr, the solvent was evaporated, and the mixture was extracted with ethyl acetate. The obtained ethyl acetate layer was washed successively with 1N hydrochloric acid and saturated aqueous sodium hydrogencarbonate solution, and dried over anhydrous sodium sulfate. The solvent was evaporated, and the obtained residue was purified by silica gel chromatography (n-hexane-ethyl acetate=75:25) to give the title compound (240 mg).

### step 2

### Production of (E)-4-(2,3-dihydrobenzo[1,4]oxazin-4-yl)-4-oxo-2-butenoic acid

(E)-4-(2,3-Dihydrobenzo[1,4]oxazin-4-yl)-4-oxo-2-butenoic acid ethyl ester (225 mg) was dissolved in ethanol (3 mL), and a 1N aqueous sodium hydroxide solution (0.95 mL) was added. After stirring the mixture at room temperature for 2 hr, the solvent was evaporated. Water was added, and the mixture was washed with diethyl ether. The obtained aqueous layer was acidified with 1N hydrochloric acid, and the mixture was extracted with ethyl acetate. The obtained ethyl acetate layer was washed successively with water and saturated brine, and dried over anhydrous sodium sulfate. The solvent was evaporated to give the title compound (189 mg) as crystals.

### Example 29

### Production of (2,8-diisopropyl-2,3-dihydrobenzo[1,4]oxazin-4-yl)oxoacetic acid

### step 1

### Production of (2,8-diisopropyl-2,3-dihydrobenzo[1,4]oxazin-4-yl)oxoacetic acid methyl ester

2,8-Diisopropyl-3,4-dihydro-2H-benzo[1,4]oxazine (300 mg) obtained in step 1 of Example 21 was dissolved in chloroform (5 mL), and triethylamine (0.229 mL) and methyl chlorooxoacetate (0.164 mL) were added. After stirring the mixture overnight at room temperature, the solvent was evaporated, and the obtained residue was purified by silica gel chromatography (n-hexane-ethyl acetate=3:1) to give the title compound (419 mg) as a colorless oil.

### step 2

### Production of (2,8-diisopropyl-2,3-dihydrobenzo[1,4]oxazin-4-yl)oxoacetic acid

(2,8-Diisopropyl-2,3-dihydrobenzo[1,4]oxazin-4-yl)oxoacetic acid methyl ester (410 mg) was dissolved in methanol (5 mL), and a 2N aqueous sodium hydroxide solution (1.34 mL) was added. After stirring the mixture at room temperature for 14 hr, the solvent was evaporated. The obtained residue was acidified with water, ethyl acetate and 1N hydrochloric acid, and the mixture was extracted with ethyl acetate. The obtained ethyl acetate layer was dried over anhydrous sodium sulfate. The solvent was evaporated to give the title compound (325 mg) as white crystals.

### Example 30

### Production of (2-isopropyl-3-oxo-2,3-dihydrobenzo[1,4]thiazin-4-yl)acetic acid

### step 1

### Production of (2-isopropyl-3-oxo-2,3-dihydrobenzo[1,4]thiazin-4-yl)acetic acid methyl ester

2-Isopropyl-4H-benzo[1,4]thiazin-3-one (250 mg) obtained in step 1 of Example 22 was dissolved in N,N-dimethylformamide (3 mL), and 60% sodium hydride (60 mg) was added. After stirring the mixture at room temperature for 5min, methyl bromoacetate (0.138 mL) was added. After stirring the mixture at room temperature for 15 hr, 1N hydrochloric acid was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=3:1) to give the title compound (340 mg) as a colorless oil.

### step 2

### Production of (2-isopropyl-3-oxo-2,3-dihydrobenzo[1,4]thiazin-4-yl)acetic acid

(2-Isopropyl-3-oxo-2,3-dihydrobenzo[1,4]thiazin-4-yl)acetic acid methyl ester (340 mg) was dissolved in methanol (5 mL), and a 2N aqueous sodium hydroxide solution (1.22 mL) was added. After stirring the mixture at room temperature for 15 hr, the solvent was evaporated. The mixture was acidified with 1N hydrochloric acid, and extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (303 mg) as a white amorphous solid.

### Example 31

### Production of 3-(2,8-diisopropyl-2,3-dihydrobenzo[1,4]oxazin-4-yl)-3-oxopropionic acid

### step 1

### Production of 3-(2,8-diisopropyl-2,3-dihydrobenzo[1,4]oxazin-4-yl)-3-oxopropionic acid ethyl ester

2,8-Diisopropyl-3,4-dihydro-2H-benzo[1,4]oxazine (300 mg) obtained in step 1 of Example 21 was dissolved in chloroform (5 mL), and triethylamine (0.229 mL) and ethyl chlorocarbonyl acetate (0.210 mL) were added. After stirring the mixture at room temperature for 15 hr, water was added to the reaction mixture, and the mixture was extracted with chloroform. The obtained chloroform layer was dried over anhydrous magnesium sulfate. The solvent was evaporated, and the obtained residue was purified by silica gel chromatography (n-hexane-ethyl acetate=3:1) to give the title compound (465 mg) as a colorless oil.

### step 2

### Production of 3-(2,8-diisopropyl-2,3-dihydrobenzo[1,4]oxazin-4-yl)-3-oxopropionic acid

3-(2,8-Diisopropyl-2,3-dihydrobenzo[1,4]oxazin-4-yl)-3-oxopropionic acid ethyl ester (460 mg) was dissolved in methanol (5 mL), and a 2N aqueous sodium hydroxide solution (1.37 mL) was added. After stirring the mixture at room temperature for 15 hr, the solvent was evaporated. The obtained residue was acidified with ethyl acetate and 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (366 mg) as white crystals.

### Example 32

### Production of 3-(2-isopropyl-3-oxo-2,3-dihydrobenzo[1,4]oxazin-4-yl)propionic acid

### step 1

### Production of 3-(2-isopropyl-3-oxo-2,3-dihydrobenzo[1,4]oxazin-4-yl)propionic acid methyl ester

2-Isopropyl-4H-benzo[1,4]oxazin-3-one (500 mg) obtained in step 3 of Example 6 was dissolved in N,N-dimethylformamide (5 mL), and potassium carbonate (722 mg) and methyl acrylate (0.471 mL) were added. After stirring with heating overnight at 100°C, water was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=9:1) to give the title compound (669 mg) as a colorless oil.

### step 2

### Production of 3-(2-isopropyl-3-oxo-2,3-dihydrobenzo[1,4]oxazin-4-yl)propionic acid

3-(2-Isopropyl-3-oxo-2,3-dihydrobenzo[1,4]oxazin-4-yl)propionic acid methyl ester (300 mg) was dissolved in methanol (5 mL), and a 2N aqueous sodium hydroxide solution (1.08 mL) was added. After stirring the mixture at room temperature for 2 hr, the solvent was evaporated. The mixture was acidified with water and 1N hydrochloric acid, and extracted with ethyl acetate. The obtained ethyl acetate layer was washed successively with water and saturated brine, and dried over anhydrous sodium sulfate. The solvent was evaporated to give the title compound (287 mg) as a colorless oil.

### Example 33

### Production of 3-(8-isopropyl-2,2-dimethyl-3-oxo-2,3-dihydrobenzo[1,4]oxazin-4-yl)propionic acid

### step 1

### Production of 2-bromo-N-(2-hydroxy-3-isopropylphenyl)-2-methylpropionamide

2-Amino-6-isopropylphenol hydrobromide (2.00 g) obtained in step 3 of Example 9 was dissolved by adding ethyl acetate (20 mL) and water (20 mL), and sodium hydrogencarbonate (1.81 g) and 2-bromo-2-methylpropionylbromide (1.27 mL) were added at room temperature, and the mixture was stirred at room temperature for 1 hr. The reaction mixture was partitioned, and the obtained ethyl acetate layer was washed successively with water and saturated brine, and dried over anhydrous sodium sulfate. The solvent was evaporated to give the title compound (2.72 g) as a brown oil.

### step 2

### Production of 8-isopropyl-2,2-dimethyl-4H-benzo[1,4]oxazin-3-one

2-Bromo-N-(2-hydroxy-3-isopropylphenyl)-2-methylpropionamide (2.72 g) was dissolved in N,N-dimethylformamide (20 mL), and potassium carbonate (1.61 g) was added, and the mixture was stirred overnight at room temperature. 1N Hydrochloric acid was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=4:1), and crystallized from n-hexane to give the title compound (493 mg) as a white solid.

### step 3

### Production of 3-(8-isopropyl-2,2-dimethyl-3-oxo-2,3-dihydrobenzo[1,4]oxazin-4-yl)propionic acid methyl ester

8-Isopropyl-2,2-dimethyl-4H-benzo[1,4]oxazin-3-one (300 mg) was dissolved in N,N-dimethylformamide (3 mL), and potassium carbonate (378 mg) and methyl acrylate (0.246 mL) were added. After stirring with heating overnight at 100°C, water was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=9:1) to give the title compound (382 mg) as a colorless oil.

### step 4

### Production of 3-(8-isopropyl-2,2-dimethyl-3-oxo-2,3-dihydrobenzo[1,4]oxazin-4-yl)propionic acid

3-(8-Isopropyl-2,2-dimethyl-3-oxo-2,3-dihydrobenzo[1,4]oxazin-4-yl)propionic acid methyl ester (377 mg) was dissolved in methanol (5 mL), and a 2N aqueous sodium hydroxide solution (1.23 mL) was added. After stirring the mixture at room temperature, the solvent was evaporated, and the residue was acidified with 1N hydrochloric acid, and extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (323 mg) as white crystals.

### Example 34

### Production of (8-isopropyl-2,2-dimethyl-3-oxo-2,3-dihydrobenzo[1,4]oxazin-4-yl)acetic acid

### step 1

### Production of (8-isopropyl-2,2-dimethyl-3-oxo-2,3-dihydrobenzo[1,4]oxazin-4-yl)acetic acid methyl ester

8-Isopropyl-2,2-dimethyl-4H-benzo[1,4]oxazin-3-one (300 mg) obtained in step 2 of Example 33 was dissolved in N,N-dimethylformamide (5 mL), and 60% sodium hydride (66 mg) was added. After stirring the mixture at room temperature for 1 hr, methyl bromoacetate (0.151 mL) was added. After stirring the mixture overnight at room temperature, a 10% aqueous citric acid solution was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=9:1) to give the title compound (221 mg) as a brown oil.

### step 2

### Production of (8-isopropyl-2,2-dimethyl-3-oxo-2,3-dihydrobenzo[1,4]oxazin-4-yl)acetic acid

(8-Isopropyl-2,2-dimethyl-3-oxo-2,3-dihydrobenzo[1,4]oxazin-4-yl)acetic acid methyl ester (216 mg) was dissolved in methanol (5 mL), and a 2N aqueous sodium hydroxide solution (0.741 mL) was added. After stirring the mixture overnight at room temperature, the solvent was evaporated, and the mixture was acidified with 1N hydrochloric acid, and extracted with ethyl acetate. The obtained ethyl acetate layer was washed successively with water and saturated brine, and dried over anhydrous sodium sulfate. The solvent was evaporated to give the title compound (204 mg) as a brown oil.

### Example 35

### Production of 3-(8-isopropyl-3-oxo-2,3-dihydrobenzo[1,4]oxazin-4-yl)propionic acid

### step 1

### Production of 3-(8-isopropyl-3-oxo-2,3-dihydrobenzo[1,4]oxazin-4-yl)propionic acid methyl ester

8-Isopropyl-4H-benzo[1,4]oxazin-3-one (190 mg) obtained in step 2 of Example 10 was dissolved in N,N-dimethylformamide (3 mL), and potassium carbonate (274 mg) and methyl acrylate (0.179 mL) were added. After stirring with heating overnight at 100°C, water was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=9:1) to give the title compound (245 mg) as a colorless oil.

### step 2

### Production of 3-(8-isopropyl-3-oxo-2,3-dihydrobenzo[1,4]oxazin-4-yl)propionic acid

3-(8-Isopropyl-3-oxo-2,3-dihydrobenzo[1,4]oxazin-4-yl)propionic acid methyl ester (240 mg) was dissolved in methanol (5 mL), and a 2N aqueous sodium hydroxide solution (1.30 mL) was added. After stirring the mixture at room temperature for 1 hr, the solvent was evaporated. The residue was acidified with water and 1N hydrochloric acid, and the precipitated solid was collected by filtration to give the title compound (181 mg) as white crystals.

### Example 36

### Production of (E)-4-(2,8-diisopropyl-2,3-dihydrobenzo[1,4]oxazin-4-yl)-4-oxo-2-butenoic acid

### step 1

### Production of (E)-3-chlorocarbonyl acrylic acid ethyl ester

Fumaric acid monoethyl ester (4.32 g) was dissolved in methylene chloride (40 mL), and oxalyl chloride (3.93 mL) and N,N-dimethylformamide (one drop) were added. After stirring the mixture at room temperature for 1 hr, the mixture was concentrated, and azeotroped with toluene to give the title compound.

### step 2

### Production of (E)-4-(2,8-diisopropyl-2,3-dihydrobenzo[1,4]oxazin-4-yl)-4-oxo-2-butenoic acid ethyl ester

2,8-Diisopropyl-3,4-dihydro-2H-benzo[1,4]oxazine (219 mg) obtained in step 1 of Example 21 was dissolved in chloroform (3 mL), triethylamine (0.181 mL) and (E)-3-chlorocarbonyl acrylic acid ethyl ester (211 mg) obtained in step 1 were added. After stirring the mixture at room temperature for 15 hr, water was added to the reaction mixture, and the mixture was extracted with chloroform. The obtained chloroform layer was washed successively with water and saturated brine, and dried over anhydrous magnesium sulfate. The solvent was evaporated, and the obtained residue was purified by silica gel chromatography (n-hexane-ethyl acetate=4:1) to give the title compound (345 mg) as a colorless oil.

### step 3

### Production of (E)-4-(2,8-diisopropyl-2,3-dihydrobenzo[1,4]oxazin-4-yl)-4-oxo-2-butenoic acid

(E)-4-(2,8-Diisopropyl-2,3-dihydrobenzo[1,4]oxazin-4-yl)-4-oxo-2-butenoic acid ethyl ester (345 mg) was dissolved in methanol (5 mL), and a 2N aqueous sodium hydroxide solution (1.00 mL) was added. After stirring the mixture at room temperature for 15 hr, the solvent was evaporated. The obtained residue was acidified with ethyl acetate and 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (193 mg) as white crystals.

### Example 37

### Production of (Z)-4-(2,8-diisopropyl-2,3-dihydrobenzo[1,4]oxazin-4-yl)-4-oxo-2-butenoic acid

### step 1

### Production of (Z)-3-chlorocarbonyl acrylic acid methyl ester

Monomethyl maleate (3.90 g) was dissolved in methylene chloride (40 mL), and oxalyl chloride (3.93 mL) and N,N-dimethylformamide (one drop) were added. After stirring the mixture at room temperature for 1 hr, the mixture was concentrated, and azeotroped with toluene to give the title compound.

### step 2

### Production of (Z)-4-(2,8-diisopropyl-2,3-dihydrobenzo[1,4]oxazin-4-yl)-4-oxo-2-butenoic acid methyl ester

2,8-Diisopropyl-3,4-dihydro-2H-benzo[1,4]oxazine (219 mg) obtained in step 1 of Example 21 was dissolved in chloroform (3 mL), and triethylamine (0.181 mL) and (Z)-3-chlorocarbonyl acrylic acid methyl ester (193 mg) obtained in step 1 were added. After stirring the mixture at room temperature for 15 hr, water was added to the reaction mixture, and the mixture was extracted with chloroform. The obtained chloroform layer was washed successively with water and saturated brine, and dried over anhydrous magnesium sulfate. The solvent was evaporated, and the obtained residue was purified by silica gel chromatography (n-hexane-ethyl acetate=4:1) to give the title compound (241 mg) as a pale-yellow oil.

### step 3

### Production of (Z)-4-(2,8-diisopropyl-2,3-dihydrobenzo[1,4]oxazin-4-yl)-4-oxo-2-butenoic acid

(Z)-4-(2,8-Diisopropyl-2,3-dihydrobenzo[1,4]oxazin-4-yl)-4-oxo-2-butenoic acid methyl ester (241 mg) was dissolved in methanol (4 mL), and a 2N aqueous sodium hydroxide solution (0.73 mL) was added. After stirring the mixture at room temperature for 15 hr, the solvent was evaporated. The obtained residue was acidified with ethyl acetate and 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (116 mg) as white crystals.

### Example 38

### Production of 3-(8-isopropyl-3-oxo-2-phenyl-2,3-dihydrobenzo[1,4]oxazin-4-yl)propionic acid

### step 1

### Production of 4-[2-(1,3-dioxoran-2-yl)ethyl]-8-isopropyl-2-phenyl-4H-benzo[1,4]oxazin-3-one

8-Isopropyl-2-phenyl-4H-benzo[1,4]oxazin-3-one (300 mg) obtained in step 2 of Example 26 was dissolved in N,N-dimethylformamide (3 mL), and 60% sodium hydride (54 mg) was added. After stirring the mixture at room temperature for 1 hr, 2-(2-bromoethyl)-1,3-dioxorane (0.197 mL) was added. After stirring the mixture at room temperature for 2 hr, a 10% aqueous citric acid solution was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=3:1) to give the title compound (275 mg) as a colorless oil.

### step 2

### Production of 3-(8-isopropyl-3-oxo-2-phenyl-2,3-dihydrobenzo[1,4]oxazin-4-yl)propionaldehyde

4-[2-(1,3-Dioxoran-2-yl)ethyl]-8-isopropyl-2-phenyl-4H-benzo[1,4]oxazin-3-one (270 mg) was dissolved in tetrahydrofuran (5 mL), and 1N hydrochloric acid (3 mL) was added. After stirring the mixture overnight at room temperature, saturated aqueous sodium hydrogencarbonate solution was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=4:1) to give the title compound (248 mg) as a white solid.

### step 3

### Production of 3-(8-isopropyl-3-oxo-2-phenyl-2,3-dihydrobenzo[1,4]oxazin-4-yl)propionic acid

3-(8-Isopropyl-3-oxo-2-phenyl-2,3-dihydrobenzo[1,4]oxazin-4-yl)propionaldehyde (248 mg) was dissolved in tert-butanol (6 mL) and water (1.6 mL), and 2-methyl-2-butene (238 mg), sodium dihydrogenphosphate (92 mg) and sodium chlorite (297 mg) were added. After stirring the mixture at room temperature for 1.5 hr, the mixture was acidified with 1N hydrochloric acid under ice-cooling, and extracted with ethyl acetate. The obtained ethyl acetate layer was washed successively with water and saturated brine, and dried over anhydrous sodium sulfate. The solvent was evaporated, and the obtained residue was crystallized from n-hexane-ethyl acetate to give the title compound (96 mg) as pale-yellow crystals.

### Example 39

### Production of (2-ethyl-8-isopropyl-3-oxo-2,3-dihydrobenzo[1,4]oxazin-4-yl)acetic acid

### step 1

### Production of 2-bromo-N-(2-hydroxy-3-isopropylphenyl)butylamide

2-Amino-6-isopropylphenol hydrobromide (2.00 g) obtained in step 3 of Example 9 was dissolved by adding ethyl acetate (20 mL) and water (20 mL), sodium hydrogencarbonate (1.81 g) and 2-bromobutyrylbromide (1.25 mL) were added at room temperature, and the mixture was stirred at room temperature for 3 hr. The reaction mixture was partitioned, and the obtained ethyl acetate layer was washed successively with water and saturated brine, and dried over anhydrous sodium sulfate. The solvent was evaporated to give the title compound (2.82 g) as a brown oil.

### step 2

### Production of 2-ethyl-8-isopropyl-4H-benzo[1,4]oxazin-3-one

2-Bromo-N-(2-hydroxy-3-isopropylphenyl)butylamide (2.82 g) was dissolved in N,N-dimethylformamide (15 mL), and potassium carbonate (1.61 g) was added, and the mixture was stirred overnight at room temperature. 1N Hydrochloric acid was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=3:1) to give the title compound (1.82 g) as a pale-pink solid.

### step 3

### Production of (2-ethyl-8-isopropyl-3-oxo-2,3-dihydrobenzo[1,4]oxazin-4-yl)acetic acid methyl ester

2-Ethyl-8-isopropyl-4H-benzo[1,4]oxazin-3-one (300 mg) was dissolved in N,N-dimethylformamide (3 mL), and 60% sodium hydride (66 mg) was added. After stirring the mixture at room temperature for 1 hr, methyl bromoacetate (0.151 mL) was added. After stirring the mixture overnight at room temperature, a 10% aqueous citric acid solution was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=2:1) to give the title compound (390 mg) as a pale-orange oil.

### step 4

### Production of (2-ethyl-8-isopropyl-3-oxo-2,3-dihydrobenzo[1,4]oxazin-4-yl)acetic acid

(2-Ethyl-8-isopropyl-3-oxo-2,3-dihydrobenzo[1,4]oxazin-4-yl)acetic acid methyl ester (385 mg) was dissolved in methanol (5 mL), and a 2N aqueous sodium hydroxide solution (1.32 mL) was added. After stirring the mixture overnight at room temperature, the solvent was evaporated, and the mixture was acidified with 1N hydrochloric acid, and extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (345 mg) as white crystals.

### Example 40

### Production of 3-(2-ethyl-8-isopropyl-3-oxo-2,3-dihydrobenzo[1,4]oxazin-4-yl)propionic acid

### step 1

### Production of 3-(2-ethyl-8-isopropyl-3-oxo-2,3-dihydrobenzo[1,4]oxazin-4-yl)propionic acid methyl ester

2-Ethyl-8-isopropyl-4H-benzo[1,4]oxazin-3-one (300 mg) obtained in step 2 of Example 39 was dissolved in N,N-dimethylformamide (3 mL), and potassium carbonate (378 mg) and methyl acrylate (0.246 mL) were added. After stirring with heating overnight at 100°C, a 10% aqueous citric acid solution was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=2:1) to give the title compound (412 mg) as a pale-orange oil.

### step 2

### Production of 3-(2-ethyl-8-isopropyl-3-oxo-2,3-dihydrobenzo[1,4]oxazin-4-yl)propionic acid

3-(2-Ethyl-8-isopropyl-3-oxo-2,3-dihydrobenzo[1,4]oxazin-4-yl)propionic acid methyl ester (407 mg) was dissolved in methanol (5 mL), and a 2N aqueous sodium hydroxide solution (1.30 mL) was added. After stirring the mixture overnight at room temperature, the solvent was evaporated. The mixture was acidified with water and 1N hydrochloric acid, and extracted with ethyl acetate. The obtained ethyl acetate layer was washed successively with water and saturated brine, and dried over anhydrous sodium sulfate. The solvent was evaporated, and the obtained residue was purified by silica gel chromatography (chloroform -methanol=30:1), and crystallized from n-hexane to give the title compound (192 mg) as a white solid.

### Example 41

### Production of 2-[(2,8-diisopropyl-3-oxo-2,3-dihydrobenzo[1,4]oxazin-4-yl)methyl]benzoic acid

### step 1

### Production of 2-bromomethylbenzoic acid methyl ester

2-Methylbenzoic acid methyl ester (5 g) was dissolved in carbon tetrachloride (50 mL), N-bromosuccinimide (5.93 g) and α,α'-azobisisobutyronitrile (273 mg) were added, and the mixture was stirred with heating at 90°C for 1.5 hr. The precipitated solid was removed by filtration, and the filtrate was concentrated. The obtained residue was purified by silica gel chromatography (n-hexane-ethyl acetate=20:1) to give the title compound (4.69 g) as a colorless oil.

### step 2

### Production of 2-[(2,8-diisopropyl-3-oxo-2,3-dihydrobenzo[1,4]oxazin-4-yl)methyl]benzoic acid methyl ester

2,8-Diisopropyl-4H-benzo[1,4]oxazin-3-one (200 mg) obtained in step 5 of Example 9 was dissolved in N,N-dimethylformamide (3 mL), and 60% sodium hydride (41 mg) was added. After stirring the mixture at room temperature for 1 hr, 2-bromomethylbenzoic acid methyl ester (236 mg) obtained in step 1 was added. After stirring the mixture overnight at room temperature, a 10% aqueous citric acid solution was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=9:1) to give the title compound (314 mg) as a colorless oil.

### step 3

### Production of 2-[(2,8-diisopropyl-3-oxo-2,3-dihydrobenzo[1,4]oxazin-4-yl)methyl]benzoic acid

2-[(2,8-Diisopropyl-3-oxo-2,3-dihydrobenzo[1,4]oxazin-4-yl)methyl]benzoic acid methyl ester (309 mg) was dissolved in methanol (5 mL), and a 2N aqueous sodium hydroxide solution (0.81 mL) was added. After stirring the mixture overnight at room temperature, the solvent was evaporated. The residue was acidified with water and 1N hydrochloric acid, and the precipitated solid was collected by filtration to give the title compound (270 mg) as a white amorphous solid.

### Example 42

### Production of 3-(2,8-diisopropyl-3-oxo-2,3-dihydrobenzo[1,4]oxazin-4-yl)-2,2-dimethylpropionic acid

### step 1

### Production of 3-methanesulfonyloxy-2,2-dimethylpropionic acid methyl ester

3-Hydroxy-2,2-dimethylpropionic acid methyl ester (5.00 g) was dissolved in chloroform (50 mL), pyridine (3.97 mL) was added, and methanesulfonylchloride (3.51 mL) was added under ice-cooling. After stirring at room temperature for 6.5 hr, water was added to the reaction mixture, and the mixture was extracted with chloroform. The obtained chloroform layer was washed successively with 1N hydrochloric acid, water and saturated brine, and dried over anhydrous sodium sulfate. The solvent was evaporated, and the obtained residue was purified by distillation under reduced pressure to give the title compound (5.46 g) as a colorless oil.

### step 2

### Production of 3-(2,8-diisopropyl-3-oxo-2,3-dihydrobenzo[1,4]oxazin-4-yl)-2,2-dimethylpropionic acid methyl ester

2,8-Diisopropyl-4H-benzo[1,4]oxazin-3-one (374 mg) obtained in step 5 of Example 9 was dissolved in N,N-dimethylformamide (10 mL), and potassium carbonate (662 mg), 3-methanesulfonyloxy-2,2-dimethylpropionic acid methyl ester (674 mg) obtained in step 1 and sodium iodide(480 mg) were added. After stirring overnight at 140°C, a 10% aqueous citric acid solution was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=20:1) to give the title compound (190 mg) as a colorless oil.

### step 3

### Production of 3-(2,8-diisopropyl-3-oxo-2,3-dihydrobenzo[1,4]oxazin-4-yl)-2,2-dimethylpropionic acid

3-(2,8-Diisopropyl-3-oxo-2,3-dihydrobenzo[1,4]oxazin-4-yl)-2,2-dimethylpropionic acid methyl ester (185 mg) was dissolved in methanol (5 mL), and a 2N aqueous sodium hydroxide solution (0.532 mL) was added. After stirring with heating overnight at 60°C, the solvent was evaporated. The residue was acidified with water and 1N hydrochloric acid, and extracted with ethyl acetate. The obtained ethyl acetate layer was washed successively with water and saturated brine, and dried over anhydrous sodium sulfate. The solvent was evaporated to give the title compound (160 mg) as a pale-orange oil.

### Example 43

### Production of 3-(2-isopropyl-7-methyl-3-oxo-2,3-dihydrobenzo[1,4]oxazin-4-yl)propionic acid

### step 1

### Production of 2-bromo-N-(2-hydroxy-4-methylphenyl)-3-methylbutylamide

2-Amino-5-methylphenol (1.23 g) was dissolved by adding ethyl acetate (25 mL) and water (30 mL), sodium hydrogencarbonate (2.1 g) and 2-bromo-3-methylbutyryl chloride (2.99 g) obtained in step 1 of Example 6 were added under ice-cooling, and the mixture was stirred at room temperature for 1 hr. The reaction mixture was partitioned, and the obtained ethyl acetate layer was washed successively with water and saturated brine, and dried over anhydrous sodium sulfate. The solvent was evaporated to give the title compound.

### step 2

### Production of 2-isopropyl-7-methyl-4H-benzo[1,4]oxazin-3-one

2-Bromo-N-(2-hydroxy-4-methylphenyl)-3-methylbutylamide obtained in step 1 was dissolved in N,N-dimethylformamide (15 mL), potassium carbonate (2.07 g) was added, and the mixture was stirred at room temperature for 14 hr. Water and 1N hydrochloric acid were added to the reaction mixture, and the precipitated solid was collected by filtration. The obtained solid was crystallized from n-hexane-diisopropyl ether to give the title compound (984 mg) as a yellow solid.

### step 3

### Production of 3-(2-isopropyl-7-methyl-3-oxo-2,3-dihydrobenzo[1,4]oxazin-4-yl)propionic acid methyl ester

2-Isopropyl-7-methyl-4H-benzo[1,4]oxazin-3-one (246 mg) was dissolved in N,N-dimethylformamide (5 mL), and potassium carbonate (346 mg) and methyl acrylate (0.225 mL) were added. After stirring with heating at 80°C for 15 hr, ethyl acetate and 1N hydrochloric acid were added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=5:1) to give the title compound (320 mg) as a colorless oil.

### step 4

### Production of 3-(2-isopropyl-7-methyl-3-oxo-2,3-dihydrobenzo[1,4]oxazin-4-yl)propionic acid

3-(2-Isopropyl-7-methyl-3-oxo-2,3-dihydrobenzo[1,4]oxazin-4-yl)propionic acid methyl ester (310 mg) was dissolved in methanol (5 mL), and a 2N aqueous sodium hydroxide solution (1.06 mL) was added. After stirring the mixture overnight at room temperature, the solvent was evaporated, water was added and the mixture was washed with diethyl ether. The obtained aqueous layer was acidified with water and 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (252 mg) as white crystals.

### Example 44

### Production of 3-(2-isopropyl-8-methyl-3-oxo-2,3-dihydrobenzo[1,4]oxazin-4-yl)propionic acid

### step 1

### Production of 4-bromo-2-methylphenol

2-Methylphenol (5 g) was dissolved in acetic acid (50 mL) and 48% hydrobromic acid (25 mL), and dimethyl sulfoxide (25 mL) was added dropwise at room temperature. After stirring the mixture at room temperature for 1 hr, the reaction mixture was neutralized with sodium carbonate, water was added, and the mixture was extracted with diethyl ether. The obtained diethyl ether layer was washed successively with water and saturated brine, and dried over anhydrous magnesium sulfate. The solvent was evaporated to give the title compound.

### step 2

### Production of 4-bromo-2-methyl-6-nitrophenol

To a mixture of 4-bromo-2-methylphenol obtained in step 1 and sodium nitrite (10.4 g) were added n-hexane (75 mL), diisopropyl ether (35 mL) and water (50 mL), and 4.5N sulfuric acid (110 mL) was added dropwise at room temperature. After stirring at room temperature for 15 hr, the reaction mixture was washed successively with saturated aqueous sodium hydrogencarbonate solution, water and saturated brine, and dried over anhydrous sodium sulfate. The solvent was evaporated, and the obtained residue was purified by silica gel chromatography (n-hexane-ethyl acetate=5:1) to give the title compound (7.14 g) as a yellow solid.

### step 3

### Production of 2-amino-6-methylphenol hydrobromide

4-Bromo-2-methyl-6-nitrophenol (7.1 g) was dissolved in methanol (50 mL). To this solution was added 7.5% palladium-carbon (1.5 g) under hydrogen atmosphere (2 kgf/cm²) and the mixture was stirred at room temperature for 18 hr. The reaction mixture was filtered through celite, and the filtrate was concentrated under reduced pressure. The obtained solid was crystallized from ethyl acetate to give the title compound (5.40 g) as a brown solid.

### step 4

### Production of 2-bromo-N-(2-hydroxy-3-methylphenyl)-3-methylbutylamide

2-Amino-6-methylphenol hydrobromide (2.04 g) was dissolved by adding ethyl acetate (30 mL) and water (35 mL), sodium hydrogencarbonate (2.77 g) and 2-bromo-3-methylbutyryl chloride (2.99 g) obtained in step 1 of Example 6 were added at room temperature, and the mixture was stirred at room temperature for 1 hr. The reaction mixture was partitioned, and the obtained ethyl acetate layer was washed successively with water and saturated brine, and dried over anhydrous sodium sulfate. The solvent was evaporated to give the title compound.

### step 5

### Production of 2-isopropyl-8-methyl-4H-benzo[1,4]oxazin-3-one

2-Bromo-N-(2-hydroxy-3-methylphenyl)-3-methylbutylamide obtained in step 4 was dissolved in N,N-dimethylformamide (15 mL), potassium carbonate (2.07 g) was added, and the mixture was stirred overnight at room temperature. 1N Hydrochloric acid was added to the reaction mixture, water was added, and the precipitated solid was collected by filtration. The obtained solid was crystallized from n-hexane-diisopropyl ether to give the title compound (1.04 g) as a brown solid.

### step 6

### Production of 3-(2-isopropyl-8-methyl-3-oxo-2,3-dihydrobenzo[1,4]oxazin-4-yl)propionic acid methyl ester

2-Isopropyl-8-methyl-4H-benzo[1,4]oxazin-3-one (246 mg) was dissolved in N,N-dimethylformamide (5 mL), and potassium carbonate (346 mg) and methyl acrylate (0.225 mL) were added. After stirring with heating at 80°C for 15 hr, ethyl acetate and 1N hydrochloric acid were added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=3:1) to give the title compound (328 mg) as a colorless oil.

### step 7

### Production of 3-(2-isopropyl-8-methyl-3-oxo-2,3-dihydrobenzo[1,4]oxazin-4-yl)propionic acid

3-(2-Isopropyl-8-methyl-3-oxo-2,3-dihydrobenzo[1,4]oxazin-4-yl)propionic acid methyl ester (320 mg) was dissolved in methanol (5 mL), and a 2N aqueous sodium hydroxide solution (1.10 mL) was added. After stirring the mixture at room temperature for 15 hr, the solvent was evaporated, and water and ethyl acetate were added. The mixture was acidified with 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (283 mg) as a white solid.

### Example 45

### Production of (E)-4-(8-isopropyl-2-phenyl-2,3-dihydrobenzo[1,4]oxazin-4-yl)-4-oxo-2-butenoic acid

### step 1

### Production of 8-isopropyl-2-phenyl-3,4-dihydro-2H-benzo[1,4]oxazine

8-Isopropyl-2-phenyl-4H-benzo[1,4]oxazin-3-one (149 mg) obtained in step 2 of Example 26 was dissolved in tetrahydrofuran (5 mL), and borane-tetrahydrofuran complex (1.0 M tetrahydrofuran solution, 0.836 mL) was added. After heating under reflux for 3 hr, water and saturated aqueous sodium hydrogencarbonate solution were added to the reaction mixture, and the mixture was extracted with ethyl acetate. The obtained ethyl acetate layer was washed successively with water and saturated brine, and dried over anhydrous sodium sulfate. The solvent was evaporated to give the title compound.

### step 2

### Production of (E)-4-(8-isopropyl-2-phenyl-2,3-dihydrobenzo[1,4]oxazin-4-yl)-4-oxo-2-butenoic acid ethyl ester

8-Isopropyl-2-phenyl-3,4-dihydro-2H-benzo[1,4]oxazine obtained in step 1 was dissolved in ethyl acetate (6 mL), and pyridine (0.067 mL) and (E)-3-chlorocarbonyl acrylic acid ethyl ester (109 mg) obtained in step 1 of Example 36 were added. After stirring the mixture at room temperature for 2 hr, a 10% aqueous citric acid solution was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=10:1) to give the title compound (191 mg) as a yellow oil.

### step 3

### Production of (E)-4-(8-isopropyl-2-phenyl-2,3-dihydrobenzo[1,4]oxazin-4-yl)-4-oxo-2-butenoic acid

(E)-4-(8-Isopropyl-2-phenyl-2,3-dihydrobenzo[1,4]oxazin-4-yl)-4-oxo-2-butenoic acid ethyl ester (185 mg) was dissolved in methanol (5 mL), and a 1N aqueous sodium hydroxide solution (0.586 mL) was added. After stirring the mixture at room temperature for 1 hr, the solvent was evaporated, and water was added to the obtained residue and washed with diethyl ether. The obtained aqueous layer was acidified with 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (139 mg) as a pale-yellow solid.

### Example 46

### Production of 3-(8-tert-butyl-2-isopropyl-3-oxo-2,3-dihydrobenzo[1,4]oxazin-4-yl)propionic acid

### step 1

### Production of 4-bromo-2-tert-butylphenol

2-tert-Butylphenol (24.7 g) was dissolved in acetic acid (220 mL) and 48% hydrobromic acid (110 mL), and dimethyl sulfoxide (110 mL) was added dropwise under ice-cooling. After stirring the mixture at room temperature for 1 hr, the reaction mixture was neutralized with water and sodium hydrogencarbonate (257 g) and extracted with diethyl ether. The obtained diethyl ether layer was washed successively with saturated aqueous sodium hydrogencarbonate solution, water and saturated brine, and dried over anhydrous sodium sulfate. The solvent was evaporated to give the title compound (41.6 g) as a colorless oil.

### step 2

### Production of 4-bromo-2-tert-butyl-6-nitrophenol

To a mixture of 4-bromo-2-tert-butylphenol (41.6 g) and sodium nitrite (33.9 g) were added n-hexane (250 mL), diisopropyl ether (110 mL) and water (170 mL), and 4.5N sulfuric acid (350 mL) was added dropwise under ice-cooling. After stirring the mixture at room temperature for 1 hr, the reaction mixture was extracted with diisopropyl ether. The obtained diisopropyl ether layer was washed successively with water and saturated brine, and dried over anhydrous sodium sulfate. The solvent was evaporated, and the obtained residue was crystallized from n-hexane to give the title compound (23.0 g) as a yellow solid.

### step 3

### Production of 2-amino-6-tert-butylphenol hydrobromide

4-Bromo-2-tert-butyl-6-nitrophenol (23.0 g) was dissolved in methanol (160 mL) and tetrahydrofuran (50 mL). To this solution was added 7.5% palladium-carbon (4 g), and the mixture was stirred under hydrogen atmosphere (2 kgf/cm²) at room temperature for 4 hr. The reaction mixture was filtered through celite, and the filtrate was concentrated under reduced pressure. The obtained solid was crystallized from diisopropyl ether to give the title compound (11.3 g) as a pink solid.

### step 4

### Production of 2-bromo-N-(3-tert-butyl-2-hydroxyphenyl)-3-methylbutylamide

2-Amino-6-tert-butylphenol hydrobromide (3.00 g) was dissolved by adding ethyl acetate (20 mL) and water (20 mL), sodium hydrogencarbonate (2.56 g) and 2-bromo-3-methylbutyryl chloride (2.92 g) obtained in step 1 of Example 6 were added at room temperature, and the mixture was stirred at room temperature for 3 hr. The reaction mixture was partitioned, and the obtained ethyl acetate layer was washed successively with water and saturated brine, dried over anhydrous sodium sulfate, and the solvent was evaporated to give the title compound (4.56 g) as a bright red oil.

### step 5

### Production of 8-tert-butyl-2-isopropyl-4H-benzo[1,4]oxazin-3-one

2-Bromo-N-(3-tert-butyl-2-hydroxyphenyl)-3-methylbutylamide (4.56 g) was dissolved in N,N-dimethylformamide (20 mL), potassium carbonate (2.27 g) was added, and the mixture was stirred overnight at room temperature. 1N Hydrochloric acid was added to the reaction mixture, water was added, and the precipitated solid was collected by filtration. The obtained ocher solid was crystallized from n-hexane-diisopropyl ether to give the title compound (2.02 g) as a gray solid.

### step 6

### Production of 3-(8-tert-butyl-2-isopropyl-3-oxo-2,3-dihydrobenzo[1,4]oxazin-4-yl)propionic acid methyl ester

8-tert-Butyl-2-isopropyl-4H-benzo[1,4]oxazin-3-one (300 mg) was dissolved in N,N-dimethylformamide (3 mL), and potassium carbonate (335 mg) and methyl acrylate (0.218 mL) were added. After stirring with heating overnight at 100°C, 10% aqueous citric acid solution and water were added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=9:1) to give the title compound (381 mg) as a colorless oil.

### step 7

### Production of 3-(8-tert-butyl-2-isopropyl-3-oxo-2,3-dihydrobenzo[1,4]oxazin-4-yl)propionic acid

3-(8-tert-Butyl-2-isopropyl-3-oxo-2,3-dihydrobenzo[1,4]oxazin-4-yl)propionic acid methyl ester (375 mg) was dissolved in methanol (5 mL), and a 2N aqueous sodium hydroxide solution (1.12 mL) was added. After stirring the mixture overnight at room temperature, the solvent was evaporated. The mixture was acidified with water and 1N hydrochloric acid, and extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (166 mg) as white crystals.

### Example 47

### Production of 3-(8-methyl-3-oxo-2-phenyl-2,3-dihydrobenzo[1,4]oxazin-4-yl)propionic acid

### step 1

### Production of 2-bromo-N-(2-hydroxy-3-methylphenyl)-2-phenylacetamide

2-Amino-6-methylphenol hydrobromide (2.04 g) obtained in step 3 of Example 44 was dissolved by adding ethyl acetate (30 mL) and water (35 mL), at room temperature sodium hydrogencarbonate (2.77 g) and α-bromophenylacetylbromide (2.42 mL) were added, and the mixture was stirred at room temperature for 1 hr. The reaction mixture was partitioned, and the obtained ethyl acetate layer was washed successively with water and saturated brine, and dried over anhydrous sodium sulfate. The solvent was evaporated to give the title compound.

### step 2

### Production of 8-methyl-2-phenyl-4H-benzo[1,4]oxazin-3-one

2-Bromo-N-(2-hydroxy-3-methylphenyl)-2-phenylacetamide obtained in step 1 was dissolved in N,N-dimethylformamide (15 mL), and potassium carbonate (2.07 g) was added, and the mixture was stirred overnight at room temperature. 1N Hydrochloric acid was added to the reaction mixture, water was added, and the precipitated solid was collected by filtration. The obtained solid was crystallized from n-hexane-diisopropyl ether to give the title compound (1.97 g) as a brown solid.

### step 3

### Production of 4-[2-(1,3-dioxoran-2-yl)ethyl]-8-methyl-2-phenyl-4H-benzo[1,4]oxazin-3-one

8-Methyl-2-phenyl-4H-benzo[1,4]oxazin-3-one (400 mg) was dissolved in N,N-dimethylformamide (4 mL), and 60% sodium hydride (80 mg) was added. The mixture was stirred at room temperature and 2-(2-bromoethyl)-1,3-dioxorane (0.293 mL) was added. After stirring the mixture at room temperature for 15 hr, water and ethyl acetate were added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=3:1) to give the title compound (330 mg) as a yellow oil.

### step 4

### Production of 3-(8-methyl-3-oxo-2-phenyl-2,3-dihydrobenzo[1,4]oxazin-4-yl)propionaldehyde

4-[2-(1,3-Dioxoran-2-yl)ethyl]-8-methyl-2-phenyl-4H-benzo[1,4]oxazin-3-one (320 mg) was dissolved in tetrahydrofuran (5 mL), and 6N hydrochloric acid (2 mL) was added. After stirring at room temperature for 18 hr, ethyl acetate and saturated aqueous sodium hydrogencarbonate solution were added to the reaction mixture, and the mixture was extracted with ethyl acetate. The obtained ethyl acetate layer was washed with saturated brine, and dried over anhydrous magnesium sulfate. The solvent was evaporated to give the title compound.

### step 5

### Production of 3-(8-methyl-3-oxo-2-phenyl-2,3-dihydrobenzo[1,4]oxazin-4-yl)propionic acid

3-(8-Methyl-3-oxo-2-phenyl-2,3-dihydrobenzo[1,4]oxazin-4-yl)propionaldehyde obtained in step 4 was dissolved in tert-butanol (8 mL) and water (2 mL), and 2-methyl-2-butene (297 mg), sodium dihydrogenphosphate (113 mg) and sodium chlorite (372 mg) were added. After stirring the mixture at room temperature for 15 hr, 1N hydrochloric acid was added to the reaction mixture, and the mixture was extracted with ethyl acetate. The obtained ethyl acetate layer was extracted with a 1N aqueous sodium hydroxide solution. The obtained aqueous layer was acidified with 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (167 mg) as white crystals.

### Example 48

### Production of 3-(7-methyl-3-oxo-2-phenyl-2,3-dihydrobenzo[1,4]oxazin-4-yl)propionic acid

### step 1

### Production of 2-bromo-N-(2-hydroxy-4-methylphenyl)-2-phenylacetamide

2-Amino-5-methylphenol (1.23 g) was dissolved by adding ethyl acetate (25 mL) and water (30 mL), sodium hydrogencarbonate (2.1 g) and α-bromophenylacetylbromide (2.42 g) were added under ice-cooling, and the mixture was stirred at room temperature for 1 hr. The reaction mixture was partitioned, and the obtained ethyl acetate layer was washed successively with water and saturated brine, and dried over anhydrous sodium sulfate. The solvent was evaporated to give the title compound.

### step 2

### Production of 7-methyl-2-phenyl-4H-benzo[1,4]oxazin-3-one

2-Bromo-N-(2-hydroxy-4-methylphenyl)-2-phenylacetamide obtained in step 1 was dissolved in N,N-dimethylformamide (15 mL), potassium carbonate (2.07 g) was added, and the mixture was stirred at room temperature for 15 hr. Water and 1N hydrochloric acid were added to the reaction mixture, and the precipitated solid was collected by filtration. The obtained solid was crystallized from n-hexane-diisopropyl ether to give the title compound (1.75 g) as a brown solid.

### step 3

### Production of 4-[2-(1,3-dioxoran-2-yl)ethyl]-7-methyl-2-phenyl-4H-benzo[1,4]oxazin-3-one

7-Methyl-2-phenyl-4H-benzo[1,4]oxazin-3-one (400 mg) was dissolved in N,N-dimethylformamide (4 mL), 60% sodium hydride (80 mg) was added, and the mixture was stirred at room temperature. 2-(2-Bromoethyl)-1,3-dioxorane (0.293 mL) was added and the mixture was stirred at room temperature for 15 hr. Water and ethyl acetate were added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=3:1) to give the title compound (258 mg) as a yellow oil.

### step 4

### Production of 3-(7-methyl-3-oxo-2-phenyl-2,3-dihydrobenzo[1,4]oxazin-4-yl)propionaldehyde

4-[2-(1,3-Dioxoran-2-yl)ethyl]-7-methyl-2-phenyl-4H-benzo[1,4]oxazin-3-one (250 mg) was dissolved in tetrahydrofuran (5 mL), and 6N hydrochloric acid (2 mL) was added. After stirring at room temperature for 17 hr, ethyl acetate and saturated aqueous sodium hydrogencarbonate solution were added to the reaction mixture, and the mixture was extracted with ethyl acetate. The obtained ethyl acetate layer was washed with saturated brine, and dried over anhydrous magnesium sulfate. The solvent was evaporated to give the title compound.

### step 5

### Production of 3-(7-methyl-3-oxo-2-phenyl-2,3-dihydrobenzo[1,4]oxazin-4-yl)propionic acid

3-(7-Methyl-3-oxo-2-phenyl-2,3-dihydrobenzo[1,4]oxazin-4-yl)propionaldehyde obtained in step 4 was dissolved in tert-butanol (6 mL) and water (1.6 mL), and 2-methyl-2-butene (234 mg), sodium dihydrogenphosphate (88 mg) and sodium chlorite (293 mg) were added. After stirring the mixture at room temperature for 15 hr, 1N hydrochloric acid was added to the reaction mixture, and the mixture was extracted with ethyl acetate. The obtained ethyl acetate layer was extracted with a 1N aqueous sodium hydroxide solution. The obtained aqueous layer was acidified with 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (80 mg) as white crystals.

### Example 49

### Production of 3-(8-tert-butyl-3-oxo-2-phenyl-2,3-dihydrobenzo[1,4]oxazin-4-yl)propionic acid

### step 1

### Production of N-(3-tert-butyl-2-hydroxyphenyl)-2-chloro-2-phenylacetamide

2-Amino-6-tert-butylphenol hydrobromide (3.00 g) obtained in step 3 of Example 46 was dissolved by adding ethyl acetate (20 mL) and water (20 mL), sodium hydrogencarbonate (2.56 g) and α-chlorophenylacetyl chloride (2.56 mL) were added at room temperature, and the mixture was stirred at room temperature for 4.5 hr. The reaction mixture was partitioned, and the obtained ethyl acetate layer was washed successively with water and saturated brine, and dried over anhydrous sodium sulfate. The solvent was evaporated to give the title compound.

### step 2

### Production of 8-tert-butyl-2-phenyl-4H-benzo[1,4]oxazin-3-one

N-(3-tert-Butyl-2-hydroxyphenyl)-2-chloro-2-phenylacetamide obtained in step 1 was dissolved in N,N-dimethylformamide (20 mL), potassium carbonate (2.27 g) was added, and the mixture was stirred overnight at room temperature. 1N Hydrochloric acid was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (2.6 g) as a pink solid.

### step 3

### Production of 8-tert-butyl-4-[2-(1,3-dioxoran-2-yl)ethyl]-2-phenyl-4H-benzo[1,4]oxazin-3-one

8-tert-Butyl-2-phenyl-4H-benzo[1,4]oxazin-3-one (400 mg) was dissolved in N,N-dimethylformamide (5 mL), and 60% sodium hydride (68 mg) was added. After stirring the mixture at room temperature for 1 hr, 2-(2-bromoethyl)-1,3-dioxorane (0.228 mL) was added. After stirring at room temperature for 4 hr, a 10% aqueous citric acid solution was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=4:1) to give the title compound (459 mg) as a pale-yellow oil.

### step 4

### Production of 3-(8-tert-butyl-3-oxo-2-phenyl-2,3-dihydrobenzo[1,4]oxazin-4-yl)propionaldehyde

8-tert-Butyl-4-[2-(1,3-dioxoran-2-yl)ethyl]-2-phenyl-4H-benzo[1,4]oxazin-3-one (455 mg) was dissolved in tetrahydrofuran (5 mL), and 3N hydrochloric acid (4 mL) was added. After stirring the mixture overnight at room temperature, a saturated aqueous sodium hydrogencarbonate solution was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=4:1) to give the title compound (263 mg) as a colorless oil.

### step 5

### Production of 3-(8-tert-butyl-3-oxo-2-phenyl-2,3-dihydrobenzo[1,4]oxazin-4-yl)propionic acid

3-(8-tert-Butyl-3-oxo-2-phenyl-2,3-dihydrobenzo[1,4]oxazin-4-yl)propionaldehyde (260 mg) was dissolved in tert-butanol (6 mL) and water (1.6 mL), and 2-methyl-2-butene (238 mg), sodium dihydrogenphosphate (92.5 mg) and sodium chlorite (299 mg) were added. After stirring the mixture at room temperature, the mixture was acidified with 1N hydrochloric acid, and extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (239 mg) as a white amorphous solid.

### Example 50

### Production of 3-(8-cyclohexyl-2-isopropyl-3-oxo-2,3-dihydrobenzo[1,4]oxazin-4-yl)propionic acid

### step 1

### Production of 4-chloro-2-cyclohexyl-6-nitrophenol

To a mixture of 4-chloro-2-cyclohexylphenol (35.3 g) and sodium nitrite (34.7 g) were added n-hexane (250 mL), diisopropyl ether (110 mL) and water (170 mL), and 4.5N sulfuric acid (350 mL) was added dropwise. After stirring the mixture at room temperature for 1 hr, the reaction mixture was extracted with diisopropyl ether. The obtained diisopropyl ether layer was washed successively with water and saturated brine, and dried over anhydrous sodium sulfate. The solvent was evaporated, and the obtained residue was purified by silica gel chromatography (n-hexane-ethyl acetate=20:1) to give the title compound (39.3 g) as a yellow solid.

### step 2

### Production of 2-amino-6-cyclohexylphenol hydrochloride

4-Chloro-2-cyclohexyl-6-nitrophenol (23.0 g) was dissolved in methanol (150 mL) and tetrahydrofuran (50 mL). To this solution was added 7.5% palladium-carbon (4 g) and the mixture was stirred under hydrogen atmosphere (2 kgf/cm²) at room temperature for 3 hr. The reaction mixture was filtered through celite, and the filtrate was concentrated under reduced pressure. The obtained solid was crystallized from ethyl acetate to give the title compound (6.42 g) as a cream-colored solid.

### step 3

### Production of 2-bromo-N-(3-cyclohexyl-2-hydroxyphenyl)-3-methylbutylamide

2-Amino-6-cyclohexylphenol hydrochloride (3.00 g) was dissolved by adding ethyl acetate (20 mL) and water (20 mL), sodium hydrogencarbonate (2.77 g) and 2-bromo-3-methylbutyryl chloride (3.16 g) obtained in step 1 of Example 6 were added at room temperature, and the mixture was stirred at room temperature for 2.5 hr. The reaction mixture was partitioned, and the obtained ethyl acetate layer was washed successively with water and saturated brine, and dried over anhydrous sodium sulfate. The solvent was evaporated to give the title compound as a brown oil.

### step 4

### Production of 8-cyclohexyl-2-isopropyl-4H-benzo[1,4]oxazin-3-one

2-Bromo-N-(3-cyclohexyl-2-hydroxyphenyl)-3-methylbutylamide obtained in step 3 was dissolved in N,N-dimethylformamide (20 mL), potassium carbonate (2.46 g) was added, and the mixture was stirred overnight at room temperature. 1N Hydrochloric acid was added to the reaction mixture, water was added, and the precipitated solid was collected by filtration to give the title compound (3.04 g) as an ocher solid.

### step 5

### Production of 3-(8-cyclohexyl-2-isopropyl-3-oxo-2,3-dihydrobenzo[1,4]oxazin-4-yl)propionic acid methyl ester

8-Cyclohexyl-2-isopropyl-4H-benzo[1,4]oxazin-3-one (300 mg) was dissolved in N,N-dimethylformamide (5 mL), and potassium carbonate (302 mg) and methyl acrylate (0.198 mL) were added. After stirring with heating overnight at 100°C, 10% aqueous citric acid solution and water were added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=9:1) to give the title compound (358 mg) as a pale-yellow oil.

### step 6

### Production of 3-(8-cyclohexyl-2-isopropyl-3-oxo-2,3-dihydrobenzo[1,4]oxazin-4-yl)propionic acid

3-(8-Cyclohexyl-2-isopropyl-3-oxo-2,3-dihydrobenzo[1,4]oxazin-4-yl)propionic acid methyl ester (353 mg) was dissolved in methanol (5 mL), and a 2N aqueous sodium hydroxide solution (1 mL) was added. After stirring the mixture overnight at room temperature, the solvent was evaporated. The residue was acidified with water and 1N hydrochloric acid, and extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (294 mg) as white crystals.

### Example 51

### Production of 3-(8-bromo-2-isopropyl-3-oxo-2,3-dihydrobenzo[1,4]oxazin-4-yl)propionic acid

### step 1

### Production of 2-amino-6-bromophenol hydrochloride

2-Bromo-6-nitrophenol (12 g) was dissolved in tetrahydrofuran (40 mL), ethanol (70 mL) and water (60 mL), ammonium chloride (14.7 g) and iron (15.4 g) were added, and the mixture was stirred with heating at 100°C for 3.5 hr. The insoluble material was removed by celite filtration, and the filtrate was concentrated and extracted with ethyl acetate and tetrahydrofuran. The obtained organic layer was washed successively with water and saturated brine, and dried over anhydrous sodium sulfate. The solvent was evaporated, and the obtained residue was dissolved in 4N hydrogen chloride (ethyl acetate solution). The precipitated solid was collected by filtration to give the title compound (6.56 g) as an ocher solid.

### step 2

### Production of 2-bromo-N-(3-bromo-2-hydroxyphenyl)-3-methylbutylamide

2-Amino-6-bromophenol hydrochloride (3.00 g) was dissolved by adding ethyl acetate (20 mL) and water (20 mL), sodium hydrogencarbonate (2.81 g) and 2-bromo-3-methylbutyryl chloride (3.2 g) obtained in step 1 of Example 6 were added at room temperature, and the mixture was stirred at room temperature. The reaction mixture was partitioned, and the obtained ethyl acetate layer was washed successively with water and saturated brine, and dried over anhydrous sodium sulfate. The solvent was evaporated. The obtained residue was purified by silica gel chromatography (n-hexane-ethyl acetate=6:1) to give the title compound (3.57 g) as an orange oil.

### step 3

### Production of 8-bromo-2-isopropyl-4H-benzo[1,4]oxazin-3-one

2-Bromo-N-(3-bromo-2-hydroxyphenyl)-3-methylbutylamide (3.57 g) was dissolved in N,N-dimethylformamide (30 mL), potassium carbonate (1.82 g) was added, and the mixture was stirred overnight at room temperature. 1N Hydrochloric acid was added to the reaction mixture, water was added, and the precipitated solid was collected by filtration to give the title compound (2.46 g) as a white solid.

### step 4

### Production of 3-(8-bromo-2-isopropyl-3-oxo-2,3-dihydrobenzo[1,4]oxazin-4-yl)propionic acid methyl ester

8-Bromo-2-isopropyl-4H-benzo[1,4]oxazin-3-one (1.0 g) was dissolved in N,N-dimethylformamide (10 mL), and potassium carbonate (1.02 g) and methyl acrylate (0.667 mL) were added. After stirring with heating overnight at 100°C, a 10% aqueous citric acid solution was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=6:1) to give the title compound (1.28 g) as a colorless oil.

### step 5

### Production of 3-(8-bromo-2-isopropyl-3-oxo-2,3-dihydrobenzo[1,4]oxazin-4-yl)propionic acid

3-(8-Bromo-2-isopropyl-3-oxo-2,3-dihydrobenzo[1,4]oxazin-4-yl)propionic acid methyl ester (250 mg) was dissolved in methanol (5 mL), and a 2N aqueous sodium hydroxide solution (0.7 mL) was added. After stirring the mixture at room temperature for 3.5 hr, the solvent was evaporated. The residue was acidified with water and 1N hydrochloric acid, and extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (226 mg) as white crystals.

### Example 52

### Production of (E)-4-(8-tert-butyl-2-isopropyl-2,3-dihydrobenzo[1,4]oxazin-4-yl)-4-oxo-2-butenoic acid

### step 1

### Production of 8-tert-butyl-2-isopropyl-3,4-dihydro-2H-benzo[1,4]oxazine

8-tert-Butyl-2-isopropyl-4H-benzo[1,4]oxazin-3-one (500 mg) obtained in step 5 of Example 46 was dissolved in tetrahydrofuran (6 mL), and a borane-tetrahydrofuran complex (1.0 M tetrahydrofuran solution, 3 mL) was added. After heating under reflux for 9 hr, 6N hydrochloric acid was added to the reaction mixture, and the mixture was heated under reflux for additional 7 hr. Water was added to the reaction mixture, and the mixture was washed with diethyl ether. A saturated aqueous sodium hydrogencarbonate solution was added to the obtained aqueous layer, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=6:1) to give the title compound (129 mg) as a pale-pink oil.

### step 2

### Production of (E)-4-(8-tert-butyl-2-isopropyl-2,3-dihydrobenzo[1,4]oxazin-4-yl)-4-oxo-2-butenoic acid ethyl ester

8-tert-Butyl-2-isopropyl-3,4-dihydro-2H-benzo[1,4]oxazine (125 mg) was dissolved in ethyl acetate (5 mL), and pyridine (0.065 mL) and (E)-3-chlorocarbonyl acrylic acid ethyl ester (105 mg) obtained in step 1 of Example 36 were added. After stirring the mixture at room temperature for 0.5 hr, water was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=8:1) to give the title compound (196 mg) as a yellow oil.

### step 3

### Production of (E)-4-(8-tert-butyl-2-isopropyl-2,3-dihydrobenzo[1,4]oxazin-4-yl)-4-oxo-2-butenoic acid

(E)-4-(8-tert-Butyl-2-isopropyl-2,3-dihydrobenzo[1,4]oxazin-4-yl)-4-oxo-2-butenoic acid ethyl ester (195 mg) was dissolved in ethanol (5 mL), and a 1N aqueous sodium hydroxide solution (0.643 mL) was added. After stirring the mixture at room temperature for 1.5 hr, the solvent was evaporated. The obtained residue was acidified with water and 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was crystallized from n-hexane-diisopropyl ether to give the title compound (111 mg) as egg-colored crystals.

### Example 53

### Production of 3-(8-ethyl-2-isopropyl-3-oxo-2,3-dihydrobenzo[1,4]oxazin-4-yl)propionic acid

### step 1

### Production of 4-bromo-2-(1-hydroxyethyl)-6-nitrophenol

1-(5-Bromo-2-hydroxy-3-nitrophenyl)ethanone (25.0 g) was dissolved in methanol (250 mL), and sodium borohydride (4.5 g) was added under ice-cooling. After stirring the mixture at room temperature, acetone and water were added to the reaction mixture, and the mixture was concentrated. A 10% aqueous citric acid solution was added to the obtained residue, and the mixture was extracted with ethyl acetate. The obtained ethyl acetate layer was washed successively with water and saturated brine, and dried over anhydrous sodium sulfate. The solvent was evaporated to give the title compound (27.5 g) as a yellow oil.

### step 2

### Production of 2-amino-6-ethylphenol hydrobromide

4-Bromo-2-(1-hydroxyethyl)-6-nitrophenol (27.5 g) was dissolved in methanol (200 mL). 7.5% Palladium-carbon (4.5 g) was added to this solution, and the mixture was stirred under hydrogen atmosphere (2 kgf/cm²) at room temperature for 6 hr. The reaction mixture was filtered through celite, and the filtrate was concentrated under reduced pressure. The obtained residue was dissolved in methanol (200 mL). To this solution was added palladium hydroxide (3 g) under hydrogen atmosphere (4 kgf/cm²) and the mixture was stirred overnight at room temperature. The reaction mixture was filtered through celite, and the filtrate was concentrated under reduced pressure. The obtained residue was crystallized from ethyl acetate to give the title compound (5.25 g) as a red-purple solid.

### step 3

### Production of 2-bromo-N-(3-ethyl-2-hydroxyphenyl)-3-methylbutylamide

2-Amino-6-ethylphenol hydrobromide (2.5 g) was dissolved by adding ethyl acetate (25 mL) and water (25 mL), sodium hydrogencarbonate (2.90 g) and 2-bromo-3-methylbutyryl chloride (2.74 g) obtained in step 1 of Example 6 were added at room temperature, and the mixture was stirred at room temperature. The reaction mixture was partitioned, and the obtained ethyl acetate layer was washed successively with water and saturated brine, and dried over anhydrous sodium sulfate. The solvent was evaporated, and the obtained residue was purified by silica gel chromatography (n-hexane-ethyl acetate=4:1) to give the title compound (1.51 g) as a pink solid.

### step 4

### Production of 8-ethyl-2-isopropyl-4H-benzo[1,4]oxazin-3-one

2-Bromo-N-(3-ethyl-2-hydroxyphenyl)-3-methylbutylamide (1.51 g) was dissolved in N,N-dimethylformamide (10 mL), potassium carbonate (0.902 g) was added, and the mixture was stirred overnight at room temperature. 1N Hydrochloric acid was added to the reaction mixture, water was added, and the precipitated solid was collected by filtration to give the title compound (1.03 g) as an egg-colored solid.

### step 5

### Production of 3-(8-ethyl-2-isopropyl-3-oxo-2,3-dihydrobenzo[1,4]oxazin-4-yl)propionic acid methyl ester

8-Ethyl-2-isopropyl-4H-benzo[1,4]oxazin-3-one (300 mg) was dissolved in N,N-dimethylformamide (5 mL), and potassium carbonate (378 mg) and methyl acrylate (0.246 mL) were added. After stirring with heating at 100°C for 7 hr, 10% aqueous citric acid solution and water were added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=6:1) to give the title compound (414 mg) as a colorless oil.

### step 6

### Production of 3-(8-ethyl-2-isopropyl-3-oxo-2,3-dihydrobenzo[1,4]oxazin-4-yl)propionic acid

3-(8-Ethyl-2-isopropyl-3-oxo-2,3-dihydrobenzo[1,4]oxazin-4-yl)propionic acid methyl ester (410 mg) was dissolved in methanol (5 mL), and a 2N aqueous sodium hydroxide solution (1.34 mL) was added. After stirring the mixture overnight at room temperature, the solvent was evaporated. Water was added and the mixture was washed with diethyl ether. The obtained aqueous layer was acidified with 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (322 mg) as white crystals.

### Example 54

### Production of (E)-4-(2-isopropyl-8-methyl-2,3-dihydrobenzo[1,4]oxazin-4-yl)-4-oxo-2-butenoic acid

### step 1

### Production of 2-isopropyl-8-methyl-3,4-dihydro-2H-benzo[1,4]oxazine

2-Isopropyl-8-methyl-4H-benzo[1,4]oxazin-3-one (410 mg) obtained in step 5 of Example 44 was dissolved in tetrahydrofuran (5 mL), and lithium aluminum hydride (152 mg) was added. After heating under reflux for 2 hr, water (0.15 mL), 15% aqueous sodium hydroxide solution (0.15 mL) and water (0.6 mL) were successively added under ice-cooling, and the mixture was stirred at room temperature. Anhydrous magnesium sulfate was added, and the mixture was dried and concentrated. The obtained residue was purified by silica gel chromatography (n-hexane-ethyl acetate=5:1) to give the title compound (316 mg) as a yellow oil.

### step 2

### Production of (E)-4-(2-isopropyl-8-methyl-2,3-dihydrobenzo[1,4]oxazin-4-yl)-4-oxo-2-butenoic acid ethyl ester

2-Isopropyl-8-methyl-3,4-dihydro-2H-benzo[1,4]oxazine (316 mg) was dissolved in chloroform (5 mL), and triethylamine (0.3 mL) and (E)-3-chlorocarbonyl acrylic acid ethyl ester (350 mg) obtained in step 1 of Example 36 were added. After stirring the mixture at room temperature for 14 hr, the reaction mixture was washed successively with water and saturated brine, and dried over anhydrous magnesium sulfate. The solvent was evaporated, and the obtained residue was purified by silica gel chromatography (n-hexane-ethyl acetate=5:1) to give the title compound (461 mg) as a pale-yellow oil.

### step 3

### Production of (E)-4-(2-isopropyl-8-methyl-2,3-dihydrobenzo[1,4]oxazin-4-yl)-4-oxo-2-butenoic acid

(E)-4-(2-Isopropyl-8-methyl-2,3-dihydrobenzo[1,4]oxazin-4-yl)-4-oxo-2-butenoic acid ethyl ester (461 mg) was dissolved in methanol (5 mL), and a 1N aqueous sodium hydroxide solution (2.18 mL) was added. After stirring the mixture at room temperature for 0.5 hr, the solvent was evaporated. The obtained residue was acidified with water, ethyl acetate and 1N hydrochloric acid, and the mixture was extracted with ethyl acetate. The obtained ethyl acetate layer was washed successively with water and saturated brine, and dried over anhydrous sodium sulfate. The solvent was evaporated to give the title compound (341 mg) as yellow crystals.

### Example 55

### Production of (E)-4-(8-methyl-2-phenyl-2,3-dihydrobenzo[1,4]oxazin-4-yl)-4-oxo-2-butenoic acid

### step 1

### Production of 8-methyl-2-phenyl-3,4-dihydro-2H-benzo[1,4]oxazine

8-Methyl-2-phenyl-4H-benzo[1,4]oxazin-3-one (479 mg) obtained in step 2 of Example 47 was dissolved in tetrahydrofuran (5 mL), and lithium aluminum hydride (152 mg) was added. After heating under reflux for 2 hr, water (0.15 mL), 15% aqueous sodium hydroxide solution (0.15 mL) and water (0.6 mL) were successively added under ice-cooling, and the mixture was stirred at room temperature. Anhydrous magnesium sulfate was added, and the mixture was dried and concentrated. The obtained residue was purified by silica gel chromatography (n-hexane-ethyl acetate=5:1) to give the title compound (345 mg) as a yellow oil.

### step 2

### Production of (E)-4-(8-methyl-2-phenyl-2,3-dihydrobenzo[1,4]oxazin-4-yl)-4-oxo-2-butenoic acid ethyl ester

8-Methyl-2-phenyl-3,4-dihydro-2H-benzo[1,4]oxazine (345 mg) was dissolved in chloroform (5 mL), and triethylamine (0.279 mL) and (E)-3-chlorocarbonyl acrylic acid ethyl ester (325 mg) obtained in step 1 of Example 36 were added. After stirring the mixture at room temperature for 15 hr, the reaction mixture was washed successively with water and saturated brine, and dried over anhydrous magnesium sulfate. The solvent was evaporated, and the obtained residue was purified by silica gel chromatography (n-hexane-ethyl acetate=5:1) to give the title compound (427 mg) as a pale-yellow oil.

### step 3

### Production of (E)-4-(8-methyl-2-phenyl-2,3-dihydrobenzo[1,4]oxazin-4-yl)-4-oxo-2-butenoic acid

(E)-4-(8-Methyl-2-phenyl-2,3-dihydrobenzo[1,4]oxazin-4-yl)-4-oxo-2-butenoic acid ethyl ester (427 mg) was dissolved in methanol (5 mL), and a 1N aqueous sodium hydroxide solution (1.83 mL) was added. After stirring the mixture at room temperature for 0.5 hr, the solvent was evaporated. The obtained residue was acidified with water, ethyl acetate and 1N hydrochloric acid, and the mixture was extracted with ethyl acetate. The obtained ethyl acetate layer was washed successively with water and saturated brine, and dried over anhydrous sodium sulfate. The solvent was evaporated to give the title compound (326 mg) as an amorphous yellow solid.

### Example 56

### Production of (E)-4-(2-isopropyl-7-methyl-2,3-dihydrobenzo[1,4]oxazin-4-yl)-4-oxo-2-butenoic acid

### step 1

### Production of 2-isopropyl-7-methyl-3,4-dihydro-2H-benzo[1,4]oxazine

2-Isopropyl-7-methyl-4H-benzo[1,4]oxazin-3-one (267 mg) obtained in step 2 of Example 43 was dissolved in tetrahydrofuran (5 mL), and lithium aluminum hydride (99 mg) was added. After stirring with heating at 60°C for 1 hr, water (0.1 mL), 15% aqueous sodium hydroxide solution (0.1 mL) and water (0.4 mL) were successively added under ice-cooling, and the mixture was stirred at room temperature. Anhydrous magnesium sulfate was added, and the mixture was dried and concentrated. The obtained residue was purified by silica gel chromatography (n-hexane-ethyl acetate=5:1) to give the title compound (185 mg) as a green oil.

### step 2

### Production of (E)-4-(2-isopropyl-7-methyl-2,3-dihydrobenzo[1,4]oxazin-4-yl)-4-oxo-2-butenoic acid ethyl ester

2-Isopropyl-7-methyl-3,4-dihydro-2H-benzo[1,4]oxazine (185 mg) was dissolved in chloroform (4 mL), and triethylamine (0.176 mL) and (E)-3-chlorocarbonyl acrylic acid ethyl ester (205 mg) obtained in step 1 of Example 36 were added. After stirring the mixture at room temperature for 5 hr, the reaction mixture was washed successively with water and saturated brine, and dried over anhydrous magnesium sulfate. The solvent was evaporated, and the obtained residue was purified by silica gel chromatography (n-hexane-ethyl acetate=5:1) to give the title compound (289 mg) as a brown oil.

### step 3

### Production of (E)-4-(2-isopropyl-7-methyl-2,3-dihydrobenzo[1,4]oxazin-4-yl)-4-oxo-2-butenoic acid

(E)-4-(2-Isopropyl-7-methyl-2,3-dihydrobenzo[1,4]oxazin-4-yl)-4-oxo-2-butenoic acid ethyl ester (289 mg) was dissolved in methanol (5 mL), and a 1N aqueous sodium hydroxide solution (1.37 mL) was added. After stirring the mixture at room temperature for 0.5 hr, the solvent was evaporated. The obtained residue was acidified with water, ethyl acetate and 1N hydrochloric acid, and the mixture was extracted with ethyl acetate. The obtained ethyl acetate layer was washed successively with water and saturated brine, and dried over anhydrous sodium sulfate. The solvent was evaporated to give the title compound (196 mg) as yellow crystals.

### Example 57

### Production of (E)-4-(7-methyl-2-phenyl-2,3-dihydrobenzo[1,4]oxazin-4-yl)-4-oxo-2-butenoic acid

### step 1

### Production of 7-methyl-2-phenyl-3,4-dihydro-2H-benzo[1,4]oxazine

7-Methyl-2-phenyl-4H-benzo[1,4]oxazin-3-one (311 mg) obtained in step 2 of Example 48 was dissolved in tetrahydrofuran (5 mL), and lithium aluminum hydride (99 mg) was added. After stirring with heating at 60°C for 1 hr, water (0.1 mL), 15% aqueous sodium hydroxide solution (0.1 mL) and water (0.4 mL) were successively added under ice-cooling, and the mixture was stirred at room temperature. Anhydrous magnesium sulfate was added, and the mixture was dried and concentrated. The obtained residue was purified by silica gel chromatography (n-hexane-ethyl acetate=5:1) to give the title compound (217 mg) as a green oil.

### step 2

### Production of (E)-4-(7-methyl-2-phenyl-2,3-dihydrobenzo[1,4]oxazin-4-yl)-4-oxo-2-butenoic acid ethyl ester

7-Methyl-2-phenyl-3,4-dihydro-2H-benzo[1,4]oxazine (217 mg) was dissolved in chloroform (4 mL), and triethylamine (0.176 mL) and (E)-3-chlorocarbonyl acrylic acid ethyl ester (205 mg) obtained in step 1 of Example 36 were added. After stirring at room temperature for 4 hr, the reaction mixture was washed successively with water and saturated brine, and dried over anhydrous magnesium sulfate. The solvent was evaporated, and the obtained residue was purified by silica gel chromatography (n-hexane-ethyl acetate=5:1) to give the title compound (309 mg) as a brown oil.

### step 3

### Production of (E)-4-(7-methyl-2-phenyl-2,3-dihydrobenzo[1,4]oxazin-4-yl)-4-oxo-2-butenoic acid

(E)-4-(7-Methyl-2-phenyl-2,3-dihydrobenzo[1,4]oxazin-4-yl)-4-oxo-2-butenoic acid ethyl ester (309 mg) was dissolved in methanol (5 mL), and a 1N aqueous sodium hydroxide solution (1.32 mL) was added. After stirring the mixture at room temperature for 0.5 hr, the solvent was evaporated. The obtained residue was acidified with water, ethyl acetate and 1N hydrochloric acid, and the mixture was extracted with ethyl acetate. The obtained ethyl acetate layer was washed successively with water and saturated brine, and dried over anhydrous sodium sulfate. The solvent was evaporated to give the title compound (236 mg) as an amorphous yellow solid.

### Example 58

### Production of 3-(2-isopropyl-6-methyl-3-oxo-2,3-dihydrobenzo[1,4]oxazin-4-yl)propionic acid

### step 1

### Production of 2-bromo-N-(2-hydroxy-5-methylphenyl)-3-methylbutylamide

2-Amino-4-methylphenol (1.23 g) was dissolved by adding ethyl acetate (25 mL) and water (30 mL), sodium hydrogencarbonate (2.10 g) and 2-bromo-3-methylbutyryl chloride (2.99 g) obtained in step 1 of Example 6 were added at room temperature, and the mixture was stirred at room temperature for 1.5 hr. The reaction mixture was partitioned, and the obtained ethyl acetate layer was washed successively with water and saturated brine, and dried over anhydrous sodium sulfate. The solvent was evaporated to give the title compound.

### step 2

### Production of 2-isopropyl-6-methyl-4H-benzo[1,4]oxazin-3-one

2-Bromo-N-(2-hydroxy-5-methylphenyl)-3-methylbutylamide obtained in step 1 was dissolved in N,N-dimethylformamide (15 mL), potassium carbonate (2.07 g) was added, and the mixture was stirred at room temperature for 16 hr. 1N Hydrochloric acid was added to the reaction mixture, water was added, and the precipitated solid was collected by filtration. The obtained solid was crystallized from n-hexane to give the title compound (1.74 g) as a pale-yellow solid.

### step 3

### Production of 3-(2-isopropyl-6-methyl-3-oxo-2,3-dihydrobenzo[1,4]oxazin-4-yl)propionic acid methyl ester

2-Isopropyl-6-methyl-4H-benzo[1,4]oxazin-3-one (246 mg) was dissolved in N,N-dimethylformamide (5 mL), and potassium carbonate (346 mg) and methyl acrylate (0.225 mL) were added. After stirring with heating at 80°C for 15 hr, water was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=4:1) to give the title compound (267 mg) as a pale-yellow oil.

### step 4

### Production of 3-(2-isopropyl-6-methyl-3-oxo-2,3-dihydrobenzo[1,4]oxazin-4-yl)propionic acid

3-(2-Isopropyl-6-methyl-3-oxo-2,3-dihydrobenzo[1,4]oxazin-4-yl)propionic acid methyl ester (260 mg) was dissolved in methanol (5 mL), and a 1N aqueous sodium hydroxide solution (1.3 mL) was added. After stirring the mixture at room temperature for 2 hr, the solvent was evaporated. Water and ethyl acetate were added to the obtained residue, and the mixture was acidified with 1N hydrochloric acid, and extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (252 mg) as white crystals.

### Example 59

### Production of 3-(8-ethyl-3-oxo-2-phenyl-2,3-dihydrobenzo[1,4]oxazin-4-yl)propionic acid

### step 1

### Production of 2-chloro-N-(3-ethyl-2-hydroxyphenyl)-2-phenylacetamide

2-Amino-6-ethylphenol hydrobromide (2.5 g) obtained in step 2 of Example 53 was dissolved by adding ethyl acetate (25 mL) and water (25 mL), sodium hydrogencarbonate (2.90 g) and α-chlorophenylacetyl chloride (2.42 mL) were added at room temperature, and the mixture was stirred at room temperature for 1 hr. The reaction mixture was partitioned, and the obtained ethyl acetate layer was washed successively with water and saturated brine, and dried over anhydrous sodium sulfate. The solvent was evaporated, and the obtained residue was purified by silica gel chromatography (n-hexane-ethyl acetate=4:1) to give the title compound (1.83 g) as a pink solid.

### step 2

### Production of 8-ethyl-2-phenyl-4H-benzo[1,4]oxazin-3-one

2-Chloro-N-(3-ethyl-2-hydroxyphenyl)-2-phenylacetamide (1.83 g) was dissolved in N,N-dimethylformamide (15 mL), potassium carbonate (1.13 g) was added, and the mixture was stirred overnight at room temperature. 1N Hydrochloric acid was added to the reaction mixture, water was added, and the precipitated solid was collected by filtration to give the title compound (1.4 g) as an ocher solid.

### step 3

### Production of 4-[2-(1,3-dioxoran-2-yl)ethyl]-8-ethyl-2-phenyl-4H-benzo[1,4]oxazin-3-one

8-Ethyl-2-phenyl-4H-benzo[1,4]oxazin-3-one (400 mg) was dissolved in N,N-dimethylformamide (5 mL), and 60% sodium hydride (76 mg) was added. After stirring the mixture at room temperature for 1 hr, 2-(2-bromoethyl)-1,3-dioxorane (0.273 mL) was added. After stirring the mixture overnight at room temperature, a 10% aqueous citric acid solution was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=3:1) to give the title compound (510 mg) as a pale-yellow oil.

### step 4

### Production of 3-(8-ethyl-3-oxo-2-phenyl-2,3-dihydrobenzo[1,4]oxazin-4-yl)propionaldehyde

4-[2-(1,3-Dioxoran-2-yl)ethyl]-8-ethyl-2-phenyl-4H-benzo[1,4]oxazin-3-one (510 mg) was dissolved in tetrahydrofuran (5 mL), and 3N hydrochloric acid (3 mL) was added. After stirring the mixture overnight at room temperature, water and saturated brine were added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=3:1) to give the title compound (286 mg) as a colorless oil.

### step 5

### Production of 3-(8-ethyl-3-oxo-2-phenyl-2,3-dihydrobenzo[1,4]oxazin-4-yl)propionic acid

3-(8-Ethyl-3-oxo-2-phenyl-2,3-dihydrobenzo[1,4]oxazin-4-yl)propionaldehyde (281 mg) was dissolved in tert-butanol (7.5 mL) and water (1.9 mL), and 2-methyl-2-butene (282 mg), sodium dihydrogenphosphate (109 mg) and sodium chlorite (352 mg) were added. After stirring the mixture overnight at room temperature, a 1N aqueous sodium hydroxide solution was added and the mixture was washed with diethyl ether. The obtained aqueous layer was acidified with 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (249 mg) as an egg-colored solid.

### Example 60

### Production of (E)-4-(8-ethyl-2-isopropyl-2,3-dihydrobenzo[1,4]oxazin-4-yl)-4-oxo-2-butenoic acid

### step 1

### Production of 8-ethyl-2-isopropyl-3,4-dihydro-2H-benzo[1,4]oxazin

8-Ethyl-2-isopropyl-4H-benzo[1,4]oxazin-3-one (300 mg) obtained in step 4 of Example 53 was dissolved in tetrahydrofuran (3 mL), and a borane-tetrahydrofuran complex (1.0 M tetrahydrofuran solution, 2 mL) was added. After heating under reflux overnight, 1N hydrochloric acid was added to the reaction mixture, and a saturated aqueous sodium hydrogencarbonate solution was further added. The mixture was extracted with ethyl acetate and the obtained ethyl acetate layer was washed successively with water and saturated brine, and dried over anhydrous sodium sulfate. The solvent was evaporated, and the obtained residue was purified by silica gel chromatography (n-hexane-ethyl acetate=9:1) to give the title compound (213 mg) as a colorless oil.

### step 2

### Production of (E)-4-(8-ethyl-2-isopropyl-2,3-dihydrobenzo[1,4]oxazin-4-yl)-4-oxo-2-butenoic acid ethyl ester

8-Ethyl-2-isopropyl-3,4-dihydro-2H-benzo[1,4]oxazine (208 mg) was dissolved in ethyl acetate (5 mL), and pyridine (0.123 mL) and (E)-3-chlorocarbonyl acrylic acid ethyl ester (198 mg) obtained in step 1 of Example 36 were added. After stirring at room temperature for 3 hr, water was added to the reaction mixture, and the mixture was extracted with ethyl acetate. The obtained ethyl acetate layer was washed successively with 1N hydrochloric acid, water and saturated brine, and dried over anhydrous sodium sulfate. The solvent was evaporated, and the obtained residue was purified by silica gel chromatography (n-hexane-ethyl acetate=9:1) to give the title compound (360 mg) as a yellow oil.

### step 3

### Production of (E)-4-(8-ethyl-2-isopropyl-2,3-dihydrobenzo[1,4]oxazin-4-yl)-4-oxo-2-butenoic acid

(E)-4-(8-Ethyl-2-isopropyl-2,3-dihydrobenzo[1,4]oxazin-4-yl)-4-oxo-2-butenoic acid ethyl ester (360 mg) was dissolved in tert-butanol, and a 1N aqueous sodium hydroxide solution (1.3 mL) was added. After stirring the mixture at room temperature, the mixture was acidified with 1N hydrochloric acid. The solvent was evaporated, and water and 1N hydrochloric acid were added to the obtained residue. The precipitated solid was collected by filtration to give the title compound (289 mg) as a lemon-colored solid.

### Example 61

### Production of 3-(2-isopropyl-6,8-dimethyl-3-oxo-2,3-dihydrobenzo[1,4]oxazin-4-yl)propionic acid

### step 1

### Production of 2-bromo-N-(2-hydroxy-3,5-dimethylphenyl)-3-methylbutylamide

2-Amino-4,6-dimethylphenol (1.5 g) was dissolved by adding ethyl acetate (15 mL) and water (15 mL), and sodium hydrogencarbonate (1.83 g) and 2-bromo-3-methylbutyryl chloride (2.62 g) obtained in step 1 of Example 6 were added at room temperature and stirred at room temperature for 1.5 hr. The reaction mixture was partitioned, and the obtained ethyl acetate layer was washed successively with water and saturated brine, and dried over anhydrous sodium sulfate. The solvent was evaporated, and the obtained residue was purified by silica gel chromatography (n-hexane-ethyl acetate=4:1) to give the title compound (2.56 g) as an orange solid.

### step 2

### Production of 2-isopropyl-6,8-dimethyl-4H-benzo[1,4]oxazin-3-one

2-Bromo-N-(2-hydroxy-3,5-dimethylphenyl)-3-methylbutylamide (2.56 g) was dissolved in N,N-dimethylformamide (20 mL), potassium carbonate (1.53 g) was added, and the mixture was stirred overnight at room temperature. 1N Hydrochloric acid was added to the reaction mixture, water was added, and the precipitated solid was collected by filtration to give the title compound (1.72 g) as an orange solid.

### step 3

### Production of 3-(2-isopropyl-6,8-dimethyl-3-oxo-2,3-dihydrobenzo[1,4]oxazin-4-yl)propionic acid methyl ester

2-Isopropyl-6,8-dimethyl-4H-benzo[1,4]oxazin-3-one (300 mg) was dissolved in N,N-dimethylformamide (5 mL), and potassium carbonate (378 mg) and methyl acrylate (0.247 mL) were added. After stirring with heating overnight at 100°C, a 10% aqueous citric acid solution was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=6:1) to give the title compound (410 mg) as an orange oil.

### step 4

### Production of 3-(2-isopropyl-6,8-dimethyl-3-oxo-2,3-dihydrobenzo[1,4]oxazin-4-yl)propionic acid

3-(2-Isopropyl-6,8-dimethyl-3-oxo-2,3-dihydrobenzo[1,4]oxazin-4-yl)propionic acid methyl ester (405 mg) was dissolved in methanol (6 mL), and a 2N aqueous sodium hydroxide solution (1.33 mL) was added. After stirring the mixture overnight at room temperature, the solvent was evaporated, water was added and the mixture was washed with diethyl ether. The obtained aqueous layer was acidified with 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (352 mg) as pale-pink crystals.

### Example 62

### Production of 3-(7-chloro-2-isopropyl-3-oxo-2,3-dihydrobenzo[1,4]oxazin-4-yl)propionic acid

### step 1

### Production of 2-bromo-N-(4-chloro-2-hydroxyphenyl)-3-methylbutylamide

2-Amino-5-chlorophenol (720 mg) was dissolved by adding ethyl acetate (15 mL) and water (15 mL), sodium hydrogencarbonate (1.05 g) and 2-bromo-3-methylbutyryl chloride (1.50 g) obtained in step 1 of Example 6 were added, and the mixture was stirred at room temperature for 1.5 hr. The reaction mixture was partitioned, and the obtained ethyl acetate layer was washed successively with water and saturated brine, and dried over anhydrous sodium sulfate. The solvent was evaporated to give the title compound.

### step 2

### Production of 7-chloro-2-isopropyl-4H-benzo[1,4]oxazin-3-one

2-Bromo-N-(4-chloro-2-hydroxyphenyl)-3-methylbutylamide obtained in step 1 was dissolved in N,N-dimethylformamide (10 mL), potassium carbonate (1.04 g) was added, and the mixture was stirred at room temperature for 3 days. Water and 1N hydrochloric acid were added to the reaction mixture, and the precipitated solid was collected by filtration. The obtained solid was crystallized from n-hexane to give the title compound (1.08 g) as a yellow solid.

### step 3

### Production of 3-(7-chloro-2-isopropyl-3-oxo-2,3-dihydrobenzo[1,4]oxazin-4-yl)propionic acid methyl ester

7-Chloro-2-isopropyl-4H-benzo[1,4]oxazin-3-one (271 mg) was dissolved in N,N-dimethylformamide (5 mL), and potassium carbonate (346 mg) and methyl acrylate (0.225 mL) were added. After stirring with heating overnight at 80°C, ethyl acetate and water were added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=5:1) to give the title compound (375 mg) as a colorless oil.

### step 4

### Production of 3-(7-chloro-2-isopropyl-3-oxo-2,3-dihydrobenzo[1,4]oxazin-4-yl)propionic acid

3-(7-Chloro-2-isopropyl-3-oxo-2,3-dihydrobenzo[1,4]oxazin-4-yl)propionic acid methyl ester (365 mg) was dissolved in methanol (5 mL), and a 1N aqueous sodium hydroxide solution (1.76 mL) was added. After stirring the mixture overnight at room temperature, the solvent was evaporated, water was added, and the mixture was washed with ethyl acetate. The obtained aqueous layer was acidified with 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (313 mg) as white crystals.

### Example 63

### Production of 3-(6-chloro-2-isopropyl-3-oxo-2,3-dihydrobenzo[1,4]oxazin-4-yl)propionic acid

### step 1

### Production of 2-bromo-N-(5-chloro-2-hydroxyphenyl)-3-methylbutylamide

2-Amino-4-chlorophenol (720 mg) was dissolved by adding ethyl acetate (15 mL) and water (15 mL), sodium hydrogencarbonate (1.05 g) and 2-bromo-3-methylbutyryl chloride (1.50 g) obtained in step 1 of Example 6 were added, and the mixture was stirred at room temperature for 1.5 hr. The reaction mixture was partitioned, and the obtained ethyl acetate layer was washed successively with water and saturated brine, and dried over anhydrous sodium sulfate. The solvent was evaporated to give the title compound.

### step 2

### Production of 6-chloro-2-isopropyl-4H-benzo[1,4]oxazin-3-one

2-Bromo-N-(5-chloro-2-hydroxyphenyl)-3-methylbutylamide obtained in step 1 was dissolved in N,N-dimethylformamide (10 mL), potassium carbonate (1.04 g) was added, and the mixture was stirred at room temperature for 14 hr. Water and 1N hydrochloric acid were added to the reaction mixture, and the precipitated solid was collected by filtration. The obtained solid was crystallized from n-hexane to give the title compound (871 mg) as a brown solid.

### step 3

### Production of 3-(6-chloro-2-isopropyl-3-oxo-2,3-dihydrobenzo[1,4]oxazin-4-yl)propionic acid methyl ester

6-Chloro-2-isopropyl-4H-benzo[1,4]oxazin-3-one (271 mg) was dissolved in N,N-dimethylformamide (5 mL), and potassium carbonate (346 mg) and methyl acrylate (0.225 mL) were added. After stirring with heating overnight at 80°C, ethyl acetate and water were added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=5:1) to give the title compound (375 mg) as a colorless oil.

### step 4

### Production of 3-(6-chloro-2-isopropyl-3-oxo-2,3-dihydrobenzo[1,4]oxazin-4-yl)propionic acid

3-(6-Chloro-2-isopropyl-3-oxo-2,3-dihydrobenzo[1,4]oxazin-4-yl)propionic acid methyl ester (365 mg) was dissolved in methanol (5 mL), and a 1N aqueous sodium hydroxide solution (1.76 mL) was added. After stirring the mixture overnight at room temperature, the solvent was evaporated, water was added and the mixture was washed with ethyl acetate. The obtained aqueous layer was acidified with 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (295 mg) as white crystals.

### Example 64

### Production of (E)-4-(2-isopropyl-6-methyl-2,3-dihydrobenzo[1,4]oxazin-4-yl)-4-oxo-2-butenoic acid

### step 1

### Production of 2-isopropyl-6-methyl-3,4-dihydro-2H-benzo[1,4]oxazine

2-Isopropyl-6-methyl-4H-benzo[1,4]oxazin-3-one (267 mg) obtained in step 2 of Example 58 was dissolved in tetrahydrofuran (5 mL), and lithium aluminum hydride (99 mg) was added. After stirring with heating at 80°C for 1 hr, water (0.1 mL), a 4N aqueous sodium hydroxide solution and water (0.4 mL) were successively added under ice-cooling, and the mixture was stirred at room temperature. Anhydrous magnesium sulfate was added, and the mixture was dried and concentrated. The obtained residue was purified by silica gel chromatography (n-hexane-ethyl acetate=5:1) to give the title compound (205 mg) as a green oil.

### step 2

### Production of (E)-4-(2-isopropyl-6-methyl-2,3-dihydrobenzo[1,4]oxazin-4-yl)-4-oxo-2-butenoic acid ethyl ester

2-Isopropyl-6-methyl-3,4-dihydro-2H-benzo[1,4]oxazine (205 mg) was dissolved in chloroform (4 mL), and triethylamine (0.194 mL) and (E)-3-chlorocarbonyl acrylic acid ethyl ester (226 mg) obtained in step 1 of Example 36 were added. After stirring the mixture overnight at room temperature, water was added to the reaction mixture, and the mixture was extracted with chloroform. The obtained chloroform layer was dried over anhydrous magnesium sulfate. The solvent was evaporated, and the obtained residue was purified by silica gel chromatography (n-hexane-ethyl acetate=5:1) to give the title compound (324 mg) as a yellow oil.

### step 3

### Production of (E)-4-(2-isopropyl-6-methyl-2,3-dihydrobenzo[1,4]oxazin-4-yl)-4-oxo-2-butenoic acid

(E)-4-(2-Isopropyl-6-methyl-2,3-dihydrobenzo[1,4]oxazin-4-yl)-4-oxo-2-butenoic acid ethyl ester (317 mg) was dissolved in methanol (5 mL), and a 1N aqueous sodium hydroxide solution (1.2 mL) was added. After stirring the mixture overnight at room temperature, the solvent was evaporated, water was added to the obtained residue, and the mixture was washed with ethyl acetate. The obtained aqueous layer was acidified with 1N hydrochloric acid, and the mixture was extracted with ethyl acetate. The obtained ethyl acetate layer was washed successively with water and saturated brine, and dried over anhydrous sodium sulfate. The solvent was evaporated to give the title compound (261 mg) as a white amorphous solid.

### Example 65

### Production of (E)-4-(6-methyl-2-phenyl-2,3-dihydrobenzo[1,4]oxazin-4-yl)-4-oxo-2-butenoic acid

### step 1

### Production of 2-bromo-N-(2-hydroxy-5-methylphenyl)-2-phenylacetamide

2-Amino-4-methylphenol (1.23 g) was dissolved by adding ethyl acetate (25 mL) and water (30 mL), sodium hydrogencarbonate (2.1 g) and α-bromophenylacetylbromide (2.42 g) were added under ice-cooling and the mixture was stirred at room temperature for 1.5 hr. The reaction mixture was partitioned, and the obtained ethyl acetate layer was washed successively with water and saturated brine, and dried over anhydrous sodium sulfate. The solvent was evaporated to give the title compound.

### step 2

### Production of 6-methyl-2-phenyl-4H-benzo[1,4]oxazin-3-one

2-Bromo-N-(2-hydroxy-5-methylphenyl)-2-phenylacetamide obtained in step 1 was dissolved in N,N-dimethylformamide (15 mL), potassium carbonate (2.07 g) was added, and the mixture was stirred at room temperature for 16 hr. Water and 1N hydrochloric acid were added to the reaction mixture, and the precipitated solid was collected by filtration. The obtained solid was crystallized from n-hexane-diisopropyl ether to give the title compound (1.86 g) as a brown solid.

### step 3

### Production of 6-methyl-2-phenyl-3,4-dihydro-2H-benzo[1,4]oxazine

6-Methyl-2-phenyl-4H-benzo[1,4]oxazin-3-one (311 mg) was dissolved in tetrahydrofuran (5 mL), and lithium aluminum hydride (99 mg) was added. After stirring with heating at 80°C for 1 hr, water (0.1 mL), a 4N aqueous sodium hydroxide solution (0.1 mL) and water (0.4 mL) were successively added under ice-cooling, and the mixture was stirred at room temperature. Anhydrous magnesium sulfate was added, and the mixture was dried and concentrated. The obtained residue was purified by silica gel chromatography (n-hexane-ethyl acetate=5:1) to give the title compound (277 mg) as a green oil.

### step 4

### Production of (E)-4-(6-methyl-2-phenyl-2,3-dihydrobenzo[1,4]oxazin-4-yl)-4-oxo-2-butenoic acid ethyl ester

6-Methyl-2-phenyl-3,4-dihydro-2H-benzo[1,4]oxazine (277 mg) was dissolved in chloroform (4 mL), triethylamine (0.223 mL) and (E)-3-chlorocarbonyl acrylic acid ethyl ester (260 mg) obtained in step 1 of Example 36 were added. After stirring the mixture overnight at room temperature, water was added to the reaction mixture, and the mixture was extracted with chloroform. The obtained chloroform layer was dried over anhydrous magnesium sulfate. The solvent was evaporated, and the obtained residue was purified by silica gel chromatography (n-hexane-ethyl acetate=5:1) to give the title compound (385 mg) as a yellow oil.

### step 5

### Production of (E)-4-(6-methyl-2-phenyl-2,3-dihydrobenzo[1,4]oxazin-4-yl)-4-oxo-2-butenoic acid

(E)-4-(6-Methyl-2-phenyl-2,3-dihydrobenzo[1,4]oxazin-4-yl)-4-oxo-2-butenoic acid ethyl ester (375 mg) was dissolved in methanol (5 mL), and a 1N aqueous sodium hydroxide solution (1.28 mL) was added. After stirring the mixture overnight at room temperature, the solvent was evaporated, water was added to the obtained residue, and the mixture was washed with ethyl acetate. The obtained aqueous layer was acidified with 1N hydrochloric acid, and the mixture was extracted with ethyl acetate. The obtained ethyl acetate layer was washed successively with water and saturated brine, and dried over anhydrous sodium sulfate. The solvent was evaporated to give the title compound (223 mg) as a white amorphous solid.

### Example 66

### Production of 3-(8-bromo-3-oxo-2-phenyl-2,3-dihydrobenzo[1,4]oxazin-4-yl)propionic acid

### step 1

### Production of N-(3-bromo-2-hydroxyphenyl)-2-chloro-2-phenylacetamide

2-Amino-6-bromophenol hydrochloride (1.5 g) obtained in step 1 of Example 51 was dissolved by adding ethyl acetate (15 mL) and water (15 mL), sodium hydrogencarbonate (1.7 g) and α-chlorophenylacetyl chloride (1.42 mL) were added at room temperature, and the mixture was stirred at room temperature for 2 hr. The reaction mixture was partitioned, and the obtained ethyl acetate layer was washed successively with water and saturated brine, and dried over anhydrous sodium sulfate. The solvent was evaporated, and the obtained residue was purified by silica gel chromatography (n-hexane-ethyl acetate=4:1) to give the title compound (2.08 g) as a bright yellow solid.

### step 2

### Production of 8-bromo-2-phenyl-4H-benzo[1,4]oxazin-3-one

N-(3-Bromo-2-hydroxyphenyl)-2-chloro-2-phenylacetamide (2.08 g) was dissolved in N,N-dimethylformamide (15 mL), potassium carbonate (1.10 g) was added, and the mixture was stirred overnight at room temperature. 1N Hydrochloric acid was added to the reaction mixture, water was added, and the precipitated solid was collected by filtration to give the title compound (1.57 g) as a flesh-colored solid.

### step 3

### Production of 8-bromo-4-[2-(1,3-dioxoran-2-yl)ethyl]-2-phenyl-4H-benzo[1,4]oxazin-3-one

8-Bromo-2-phenyl-4H-benzo[1,4]oxazin-3-one (400 mg) was dissolved in N,N-dimethylformamide (5 mL), and 60% sodium hydride (63 mg) was added. After stirring the mixture at room temperature for 1 hr, 2-(2-bromoethyl)-1,3-dioxorane (0.228 mL) was added. After stirring at room temperature for 4 hr, a 10% aqueous citric acid solution was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=3:1) to give the title compound (407 mg) as a colorless oil.

### step 4

### Production of 3-(8-bromo-3-oxo-2-phenyl-2,3-dihydrobenzo[1,4]oxazin-4-yl)propionaldehyde

8-Bromo-4-[2-(1,3-dioxoran-2-yl)ethyl]-2-phenyl-4H-benzo[1,4]oxazin-3-one (407 mg) was dissolved in tetrahydrofuran (5 mL), 3N hydrochloric acid (3 mL) was added. After stirring the mixture overnight at room temperature, water and saturated brine were added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=3:1) to give the title compound (267 mg) as a colorless oil.

### step 5

### Production of 3-(8-bromo-3-oxo-2-phenyl-2,3-dihydrobenzo[1,4]oxazin-4-yl)propionic acid

3-(8-Bromo-3-oxo-2-phenyl-2,3-dihydrobenzo[1,4]oxazin-4-yl)propionaldehyde (262 mg) was dissolved in tert-butanol (6 mL) and water (1.5 mL), and 2-methyl-2-butene (226 mg), sodium dihydrogenphosphate (87 mg) and sodium chlorite (281 mg) were added. After stirring the mixture overnight at room temperature, the solvent was evaporated, and a 1N aqueous sodium hydroxide solution was added to the obtained residue and the mixture was washed with diethyl ether. The obtained aqueous layer was acidified with 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (236 mg) as a cream-colored solid.

### Example 67

### Production of (E)-4-(2-ethyl-8-isopropyl-2,3-dihydrobenzo[1,4]oxazin-4-yl)-4-oxo-2-butenoic acid

### step 1

### Production of 2-ethyl-8-isopropyl-3,4-dihydro-2H-benzo[1,4]oxazine

2-Ethyl-8-isopropyl-4H-benzo[1,4]oxazin-3-one (300 mg) obtained in step 2 of Example 39 was dissolved in tetrahydrofuran (3 mL), and borane-tetrahydrofuran complex (1.0 M tetrahydrofuran solution, 2.05 mL) was added. The mixture was heated under reflux, water and 1N hydrochloric acid were added to the reaction mixture, and a saturated aqueous sodium hydrogencarbonate solution was further added. The mixture was extracted with ethyl acetate and the obtained ethyl acetate layer was washed successively with water and saturated brine, and dried over anhydrous sodium sulfate. The solvent was evaporated to give the title compound.

### step 2

### Production of (E)-4-(2-ethyl-8-isopropyl-2,3-dihydrobenzo[1,4]oxazin-4-yl)-4-oxo-2-butenoic acid ethyl ester

2-Ethyl-8-isopropyl-3,4-dihydro-2H-benzo[1,4]oxazine obtained in step 1 was dissolved in ethyl acetate (5 mL), and pyridine (0.166 mL) and (E)-3-chlorocarbonyl acrylic acid ethyl ester (267 mg) obtained in step 1 of Example 36 were added. After stirring the mixture at room temperature for 1 hr, water was added to the reaction mixture, and the mixture was extracted with ethyl acetate. The obtained ethyl acetate layer was washed successively with 1N hydrochloric acid, water and saturated brine, dried over anhydrous sodium sulfate. The solvent was evaporated, and the obtained residue was purified by silica gel chromatography (n-hexane-ethyl acetate=5:1) to give the title compound (451 mg) as a yellow oil.

### step 3

### Production of (E)-4-(2-ethyl-8-isopropyl-2,3-dihydrobenzo[1,4]oxazin-4-yl)-4-oxo-2-butenoic acid

(E)-4-(2-Ethyl-8-isopropyl-2,3-dihydrobenzo[1,4]oxazin-4-yl)-4-oxo-2-butenoic acid ethyl ester (446 mg) was dissolved in tert-butanol (20 mL), and a 1N aqueous sodium hydroxide solution (1.61 mL) was added. After stirring the mixture overnight at room temperature, the mixture was acidified with 1N hydrochloric acid. The solvent was evaporated, water was added to the obtained residue, and the precipitated solid was collected by filtration to give the title compound (369 mg) as a lemon-colored solid.

### Example 68

### Production of (E)-4-(7-chloro-2-isopropyl-2,3-dihydrobenzo[1,4]oxazin-4-yl)-4-oxo-2-butenoic acid

### step 1

### Production of 7-chloro-2-isopropyl-3,4-dihydro-2H-benzo[1,4]oxazine

7-Chloro-2-isopropyl-4H-benzo[1,4]oxazin-3-one (271 mg) obtained in step 2 of Example 62 was dissolved in tetrahydrofuran (3 mL), and a borane-tetrahydrofuran complex (1.0 M tetrahydrofuran solution, 1.8 mL) was added. After heating under reflux for 1 hr, 1N hydrochloric acid (3 mL) was added to the reaction mixture, and the mixture was further stirred with heating at 60°C for 1 hr, water and ethyl acetate were added, and the mixture was extracted with ethyl acetate. The obtained ethyl acetate layer was washed successively with water, saturated aqueous sodium hydrogencarbonate solution and saturated brine, and dried over anhydrous sodium sulfate. The solvent was evaporated, and the obtained residue was purified by silica gel chromatography (n-hexane-ethyl acetate=5:1) to give the title compound (208 mg) as a yellow oil.

### step 2

### Production of (E)-4-(7-chloro-2-isopropyl-2,3-dihydrobenzo[1,4]oxazin-4-yl)-4-oxo-2-butenoic acid ethyl ester

7-Chloro-2-isopropyl-3,4-dihydro-2H-benzo[1,4]oxazine (208 mg) was dissolved in chloroform (4 mL), and triethylamine (0.177 mL) and (E)-3-chlorocarbonyl acrylic acid ethyl ester (206 mg) obtained in step 1 of Example 36 were added. After stirring the mixture at room temperature for 1 hr, water and saturated brine were added to the reaction mixture, and the mixture was extracted with chloroform. The obtained chloroform layer was dried over anhydrous magnesium sulfate. The solvent was evaporated, and the obtained residue was purified by silica gel chromatography (n-hexane-ethyl acetate=5:1) to give the title compound (322 mg) as a colorless oil.

### step 3

### Production of (E)-4-(7-chloro-2-isopropyl-2,3-dihydrobenzo[1,4]oxazin-4-yl)-4-oxo-2-butenoic acid

(E)-4-(7-Chloro-2-isopropyl-2,3-dihydrobenzo[1,4]oxazin-4-yl)-4-oxo-2-butenoic acid ethyl ester (322 mg) was dissolved in tert-butanol (10 mL), a 1N aqueous sodium hydroxide solution (1.14 mL) was added and the mixture was stirred at room temperature for 2.5 hr. 1N Hydrochloric acid was added to the reaction mixture, and the solvent was evaporated. A 1N aqueous sodium hydroxide solution was added to the obtained residue, and the mixture was washed with ethyl acetate. The obtained aqueous layer was acidified with 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (180 mg) as white crystals.

### Example 69

### Production of (E)-4-(6-chloro-2-isopropyl-2,3-dihydrobenzo[1,4]oxazin-4-yl)-4-oxo-2-butenoic acid

### step 1

### Production of 6-chloro-2-isopropyl-3,4-dihydro-2H-benzo[1,4]oxazine

6-Chloro-2-isopropyl-4H-benzo[1,4]oxazin-3-one (271 mg) obtained in step 2 of Example 63 was dissolved in tetrahydrofuran (3 mL), and a borane-tetrahydrofuran complex (1.0 M tetrahydrofuran solution, 1.8 mL) was added. After heating under reflux for 1 hr, 1N hydrochloric acid (3 mL) was added to the reaction mixture, and the mixture was further stirred with heating at 60°C for 1 hr. Water and ethyl acetate were added to the reaction mixture, and the mixture was extracted with ethyl acetate. The obtained ethyl acetate layer was washed successively with water, saturated aqueous sodium hydrogencarbonate solution and saturated brine, and dried over anhydrous magnesium sulfate. The solvent was evaporated, and the obtained residue was purified by silica gel chromatography (n-hexane-ethyl acetate=5:1) to give the title compound (205 mg) as a yellow oil.

### step 2

### Production of (E)-4-(6-chloro-2-isopropyl-2,3-dihydrobenzo[1,4]oxazin-4-yl)-4-oxo-2-butenoic acid ethyl ester

6-Chloro-2-isopropyl-3,4-dihydro-2H-benzo[1,4]oxazine (205 mg) was dissolved in chloroform (4 mL), and triethylamine (0.177 mL) and (E)-3-chlorocarbonyl acrylic acid ethyl ester (206 mg) obtained in step 1 of Example 36 were added. After stirring the mixture at room temperature for 1 hr, water and saturated brine were added to the reaction mixture, and the mixture was extracted with chloroform. The obtained chloroform layer was dried over anhydrous magnesium sulfate. The solvent was evaporated, and the obtained residue was purified by silica gel chromatography (n-hexane-ethyl acetate=5:1) to give the title compound (304 mg) as a colorless oil.

### step 3

### Production of (E)-4-(6-chloro-2-isopropyl-2,3-dihydrobenzo[1,4]oxazin-4-yl)-4-oxo-2-butenoic acid

(E)-4-(6-Chloro-2-isopropyl-2,3-dihydrobenzo[1,4]oxazin-4-yl)-4-oxo-2-butenoic acid ethyl ester (304 mg) was dissolved in tert-butanol (10 mL), and a 1N aqueous sodium hydroxide solution (1.08 mL) was added. The mixture was stirred at room temperature for 2.5 hr, 1N hydrochloric acid was added to the reaction mixture, and the solvent was evaporated. A 1N aqueous sodium hydroxide solution was added to the obtained residue and the mixture was washed with ethyl acetate. The obtained aqueous layer was acidified with 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (220 mg) as a white amorphous solid.

### Example 70

### Production of (E)-4-(7-chloro-2-phenyl-2,3-dihydrobenzo[1,4]oxazin-4-yl)-4-oxo-2-butenoic acid

### step 1

### Production of 2-chloro-N-(4-chloro-2-hydroxyphenyl)-2-phenylacetamide

2-Amino-5-chlorophenol (720 mg) was dissolved by adding ethyl acetate (15 mL) and water (15 mL), sodium hydrogencarbonate (1.05 g) and α-chlorophenylacetyl chloride (1.21 mL) were added, and the mixture was stirred at room temperature for 1.5 hr. The reaction mixture was partitioned, and the obtained ethyl acetate layer was washed successively with water and saturated brine, and dried over anhydrous sodium sulfate. The solvent was evaporated to give the title compound.

### step 2

### Production of 7-chloro-2-phenyl-4H-benzo[1,4]oxazin-3-one

2-Chloro-N-(4-chloro-2-hydroxyphenyl)-2-phenylacetamide obtained in step 1 was dissolved in N,N-dimethylformamide (10 mL), potassium carbonate (1.04 g) was added, and the mixture was stirred at room temperature for 3 days. Water and 1N hydrochloric acid were added to the reaction mixture, and the precipitated solid was collected by filtration. The obtained solid was crystallized from n-hexane-diisopropyl ether to give the title compound (1.10 g) as a brown solid.

### step 3

### Production of 7-chloro-2-phenyl-3,4-dihydro-2H-benzo[1,4]oxazine

7-Chloro-2-phenyl-4H-benzo[1,4]oxazin-3-one (338 mg) was dissolved in tetrahydrofuran (5 mL), and lithium aluminum hydride (99 mg) was added. After stirring with heating at 60°C for 1 hr, water (0.1 mL), a 4N aqueous sodium hydroxide solution (0.1 mL) and water (0.4 mL) were successively added under ice-cooling, and the mixture was stirred at room temperature. Anhydrous magnesium sulfate was added, and the mixture was dried and concentrated. The obtained residue was purified by silica gel chromatography (n-hexane-ethyl acetate=5:1) to give the title compound (220 mg).

### step 4

### Production of (E)-4-(7-chloro-2-phenyl-2,3-dihydrobenzo[1,4]oxazin-4-yl)-4-oxo-2-butenoic acid ethyl ester

7-Chloro-2-phenyl-3,4-dihydro-2H-benzo[1,4]oxazine (220 mg) was dissolved in chloroform (4 mL), and triethylamine (0.162 mL) and (E)-3-chlorocarbonyl acrylic acid ethyl ester (189 mg) obtained in step 1 of Example 36 were added. After stirring the mixture overnight at room temperature, water and saturated brine were added to the reaction mixture, and the mixture was extracted with chloroform. The obtained chloroform layer was dried over anhydrous magnesium sulfate. The solvent was evaporated, and the obtained residue was purified by silica gel chromatography (n-hexane-ethyl acetate=5:1) to give the title compound (352 mg).

### step 5

### Production of (E)-4-(7-chloro-2-phenyl-2,3-dihydrobenzo[1,4]oxazin-4-yl)-4-oxo-2-butenoic acid

(E)-4-(7-Chloro-2-phenyl-2,3-dihydrobenzo[1,4]oxazin-4-yl)-4-oxo-2-butenoic acid ethyl ester (205 mg) was dissolved in tert-butanol (8 mL), a 1N aqueous sodium hydroxide solution (0.66 mL) was added and the mixture was stirred at room temperature for 2.5 hr. 1N Hydrochloric acid was added to the reaction mixture, and the solvent was evaporated. A 1N aqueous sodium hydroxide solution was added to the obtained residue, and the mixture was washed with ethyl acetate. The obtained aqueous layer was acidified with 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (130 mg) as a white amorphous solid.

### Example 71

### Production of (E)-4-(6-chloro-2-phenyl-2,3-dihydrobenzo[1,4]oxazin-4-yl)-4-oxo-2-butenoic acid

### step 1

### Production of 2-chloro-N-(5-chloro-2-hydroxyphenyl)-2-phenylacetamide

2-Amino-4-chlorophenol (720 mg) was dissolved by adding ethyl acetate (15 mL) and water (15 mL), sodium hydrogencarbonate (1.05 g) and α-chlorophenylacetyl chloride (1.21 mL) were added, and the mixture was stirred at room temperature for 1.5 hr. The reaction mixture was partitioned, and the obtained ethyl acetate layer was washed successively with water and saturated brine, and dried over anhydrous sodium sulfate. The solvent was evaporated to give the title compound.

### step 2

### Production of 6-chloro-2-phenyl-4H-benzo[1,4]oxazin-3-one

2-Chloro-N-(5-chloro-2-hydroxyphenyl)-2-phenylacetamide obtained in step 1 was dissolved in N,N-dimethylformamide (10 mL), potassium carbonate (1.04 g) was added, and the mixture was stirred at room temperature for 3 days. Water and 1N hydrochloric acid were added to the reaction mixture, and the precipitated solid was collected by filtration. The obtained solid was crystallized from n-hexane-diisopropyl ether to give the title compound (939 mg) as a brown solid.

### step 3

### Production of 6-chloro-2-phenyl-3,4-dihydro-2H-benzo[1,4]oxazine

6-Chloro-2-phenyl-4H-benzo[1,4]oxazin-3-one (338 mg) was dissolved in tetrahydrofuran (5 mL), and lithium aluminum hydride (99 mg) was added. After stirring with heating at 60°C for 1 hr, water (0.1 mL), a 4N aqueous sodium hydroxide solution (0.1 mL) and water (0.4 mL) were successively added under ice-cooling, and the mixture was stirred at room temperature. Anhydrous magnesium sulfate was added, and the mixture was dried and concentrated. The obtained residue was purified by silica gel chromatography (n-hexane-ethyl acetate=5:1) to give the title compound (223 mg).

### step 4

### Production of (E)-4-(6-chloro-2-phenyl-2,3-dihydrobenzo[1,4]oxazin-4-yl)-4-oxo-2-butenoic acid ethyl ester

6-Chloro-2-phenyl-3,4-dihydro-2H-benzo[1,4]oxazine (223 mg) was dissolved in chloroform (4 mL), and triethylamine (0.164 mL) and (E)-3-chlorocarbonyl acrylic acid ethyl ester (192 mg) obtained in step 1 of Example 36 were added. After stirring the mixture overnight at room temperature, water and saturated brine were added to the reaction mixture, and the mixture was extracted with chloroform. The obtained chloroform layer was dried over anhydrous magnesium sulfate. The solvent was evaporated, and the obtained residue was purified by silica gel chromatography (n-hexane-ethyl acetate=5:1) to give the title compound (296 mg).

### step 5

### Production of (E)-4-(6-chloro-2-phenyl-2,3-dihydrobenzo[1,4]oxazin-4-yl)-4-oxo-2-butenoic acid

(E)-4-(6-Chloro-2-phenyl-2,3-dihydrobenzo[1,4]oxazin-4-yl)-4-oxo-2-butenoic acid ethyl ester (267 mg) was dissolved in tert-butanol (8 mL), a 1N aqueous sodium hydroxide solution (0.86 mL) was added and the mixture was stirred at room temperature for 2.5 hr. 1N Hydrochloric acid was added to the reaction mixture, and the solvent was evaporated. A 1N aqueous sodium hydroxide solution was added to the obtained residue, and the mixture was washed with ethyl acetate. The obtained aqueous layer was acidified with 1N hydrochloric acid, and the mixture was extracted with ethyl acetate. The obtained ethyl acetate layer was washed successively with water and saturated brine, and dried over anhydrous sodium sulfate. The solvent was evaporated to give the title compound (80 mg) as a white amorphous solid.

### Example 72

### Production of 3-(6,8-dimethyl-3-oxo-2-phenyl-2,3-dihydrobenzo[1,4]oxazin-4-yl)propionic acid

### step 1

### Production of 2-chloro-N-(2-hydroxy-3,5-dimethylphenyl)-2-phenylacetamide

2-Amino-4,6-dimethylphenol (1.5 g) was dissolved by adding ethyl acetate (20 mL) and water (20 mL), sodium hydrogencarbonate (1.83 g) and α-chlorophenylacetyl chloride (2.3 mL) were added at room temperature, and the mixture was stirred overnight at room temperature. The reaction mixture was partitioned, and the obtained ethyl acetate layer was washed successively with water and saturated brine, and dried over anhydrous sodium sulfate. The solvent was evaporated, and the obtained residue was purified by silica gel chromatography (n-hexane-ethyl acetate=7:3) to give the title compound (2.65 g) as an orange solid.

### step 2

### Production of 6,8-dimethyl-2-phenyl-4H-benzo[1,4]oxazin-3-one

2-Chloro-N-(2-hydroxy-3,5-dimethylphenyl)-2-phenylacetamide (2.65 g) was dissolved in N,N-dimethylformamide (20 mL), potassium carbonate (1.51 g) was added, and the mixture was stirred at room temperature for 3 hr. 1N Hydrochloric acid was added to the reaction mixture, water was added, and the precipitated solid was collected by filtration to give the title compound (2.00 g) as a dark brown solid.

### step 3

### Production of 4-[2-(1,3-dioxoran-2-yl)ethyl]-6,8-dimethyl-2-phenyl-4H-benzo[1,4]oxazin-3-one

6,8-Dimethyl-2-phenyl-4H-benzo[1,4]oxazin-3-one (400 mg) was dissolved in N,N-dimethylformamide (5 mL), and 60% sodium hydride (75.8 mg) was added. After stirring the mixture at room temperature for 1 hr, 2-(2-bromoethyl)-1,3-dioxorane (0.253 mL) was added. After stirring the mixture overnight at room temperature, a 10% aqueous citric acid solution was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=3:1) to give the title compound (500 mg) as an orange oil.

### step 4

### Production of 3-(6,8-dimethyl-3-oxo-2-phenyl-2,3-dihydrobenzo[1,4]oxazin-4-yl)propionaldehyde

4-[2-(1,3-Dioxoran-2-yl)ethyl]-6,8-dimethyl-2-phenyl-4H-benzo[1,4]oxazin-3-one (500 mg) was dissolved in tetrahydrofuran (5 mL), and 3N hydrochloric acid (3 mL) was added. After stirring the mixture overnight at room temperature, a saturated aqueous sodium hydrogencarbonate solution was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate) to give the title compound (280 mg) as an orange solid.

### step 5

### Production of 3-(6,8-dimethyl-3-oxo-2-phenyl-2,3-dihydrobenzo[1,4]oxazin-4-yl)propionic acid

3-(6,8-Dimethyl-3-oxo-2-phenyl-2,3-dihydrobenzo[1,4]oxazin-4-yl)propionaldehyde (280 mg) was dissolved in tert-butanol (7.2 mL) and water (1.8 mL), and 2-methyl-2-butene (281 mg), sodium dihydrogenphosphate (109 mg) and sodium chlorite (351 mg) were added. After stirring at room temperature for 4 hr, the solvent was evaporated, a 1N aqueous sodium hydroxide solution was added to the obtained residue, and the mixture was washed with diethyl ether. The obtained aqueous layer was acidified with 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (236 mg) as a white solid.

### Example 73

### Production of 3-((S)-2-isopropyl-6,8-dimethyl-3-oxo-2,3-dihydrobenzo[1,4]oxazin-4-yl)propionic acid

### step 1

### Production of (R)-2-bromo-3-methylbutyryl chloride

(R)-2-Bromo-3-methylbutyric acid (5 g) was dissolved in chloroform (25 mL), N,N-dimethylformamide (one drop) and oxalyl chloride (3.13 mL) were added, and the mixture was stirred overnight at room temperature. The solvent was evaporated, and the residue was azeotroped with toluene to give the title compound as a yellow oil.

### step 2

### Production of (R)-2-bromo-N-(2-hydroxy-3,5-dimethylphenyl)-3-methylbutylamide

2-Amino-4,6-dimethylphenol (1.5 g) was dissolved by adding ethyl acetate (15 mL) and water (15 mL), sodium hydrogencarbonate (1.83 g) and (R)-2-bromo-3-methylbutyryl chloride (2.62 g) obtained in step 1 were added at room temperature, and the mixture was stirred at room temperature for 1.5 hr. The reaction mixture was partitioned, and the obtained ethyl acetate layer was washed successively with water and saturated brine, and dried over anhydrous sodium sulfate. The solvent was evaporated, and the obtained residue was purified by silica gel chromatography (n-hexane-ethyl acetate=3:1) to give the title compound (3.05 g) as a brown solid.

### step 3

### Production of (S)-2-isopropyl-6,8-dimethyl-4H-benzo[1,4]oxazin-3-one

(R)-2-Bromo-N-(2-hydroxy-3,5-dimethylphenyl)-3-methylbutylamide (3.05 g) was dissolved in N,N-dimethylformamide (20 mL), potassium carbonate (1.82 g) was added, and the mixture was stirred at room temperature for 5 hr. 1N Hydrochloric acid was added to the reaction mixture, water was added, and the precipitated solid was collected by filtration to give the title compound (2.09 g) as an orange solid.

### step 4

### Production of 3-((S)-2-isopropyl-6,8-dimethyl-3-oxo-2,3-dihydrobenzo[1,4]oxazin-4-yl)propionic acid methyl ester

(S)-2-Isopropyl-6,8-dimethyl-4H-benzo[1,4]oxazin-3-one (400 mg) was dissolved in N,N-dimethylformamide (5 mL), and potassium carbonate (504 mg) and methyl acrylate (0.329 mL) were added. After stirring with heating at 100°C for 6.5 hr, a 10% aqueous citric acid solution was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=6:1) to give the title compound (502 mg) as an orange oil.

### step 5

### Production of 3-((S)-2-isopropyl-6,8-dimethyl-3-oxo-2,3-dihydrobenzo[1,4]oxazin-4-yl)propionic acid

3-((S)-2-Isopropyl-6,8-dimethyl-3-oxo-2,3-dihydrobenzo[1,4]oxazin-4-yl)propionic acid methyl ester (500 mg) was dissolved in methanol (5 mL), and a 2N aqueous sodium hydroxide solution (1.64 mL) was added. After stirring at room temperature for 4 hr, the solvent was evaporated, water was added and the mixture was washed with diethyl ether. The obtained aqueous layer was acidified with 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was crystallized from n-hexane-ethyl acetate to give the title compound (371 mg) as pale-pink crystals.

### Example 74

### Production of 3-((R)-2-isopropyl-6,8-dimethyl-3-oxo-2,3-dihydrobenzo[1,4]oxazin-4-yl)propionic acid

### step 1

### Production of (S)-2-bromo-3-methylbutyryl chloride

(S)-2-Bromo-3-methylbutyric acid (5 g) was dissolved in chloroform (25 mL), N,N-dimethylformamide (one drop) and oxalyl chloride (4.15 mL) were added, and the mixture was stirred overnight at room temperature. The solvent was evaporated, and the residue was azeotroped with toluene to give the title compound as a yellow oil.

### step 2

### Production of (S)-2-bromo-N-(2-hydroxy-3,5-dimethylphenyl)-3-methylbutylamide

2-Amino-4,6-dimethylphenol (1.5 g) was dissolved by adding ethyl acetate (15 mL) and water (15 mL), sodium hydrogencarbonate (1.83 g) and (S)-2-bromo-3-methylbutyryl chloride (2.03 g) obtained in step 1 were added at room temperature, and the mixture was stirred at room temperature for 2.5 hr. The reaction mixture was partitioned, and the obtained ethyl acetate layer was washed successively with water and saturated brine, and dried over anhydrous sodium sulfate. The solvent was evaporated, and the obtained residue was purified by silica gel chromatography (n-hexane-ethyl acetate=3:1) to give the title compound (1.97 g) as a orange-brown solid.

### step 3

### Production of (R)-2-isopropyl-6,8-dimethyl-4H-benzo[1,4]oxazin-3-one

(S)-2-Bromo-N-(2-hydroxy-3,5-dimethylphenyl)-3-methylbutylamide (1.97 g) was dissolved in N,N-dimethylformamide (15 mL), potassium carbonate (1.38 g) was added, and the mixture was stirred overnight at room temperature. 1N Hydrochloric acid was added to the reaction mixture, water was added, and the precipitated solid was collected by filtration to give the title compound (1.54 g) as a pale-russet solid.

### step 4

### Production of 3-((R)-2-isopropyl-6,8-dimethyl-3-oxo-2,3-dihydrobenzo[1,4]oxazin-4-yl)propionic acid methyl ester

(R)-2-Isopropyl-6,8-dimethyl-4H-benzo[1,4]oxazin-3-one (400 mg) was dissolved in N,N-dimethylformamide (5 mL), and potassium carbonate (504 mg) and methyl acrylate (0.329 mL) were added. After stirring with heating at 100°C for 7 hr, a 10% aqueous citric acid solution was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=6:1) to give the title compound (539 mg) as an orange oil.

### step 5

### Production of 3-((R)-2-isopropyl-6,8-dimethyl-3-oxo-2,3-dihydrobenzo[1,4]oxazin-4-yl)propionic acid

3-((R)-2-Isopropyl-6,8-dimethyl-3-oxo-2,3-dihydrobenzo[1,4]oxazin-4-yl)propionic acid methyl ester (534 mg) was dissolved in methanol (6 mL), and a 2N aqueous sodium hydroxide solution (1.75 mL) was added. After stirring the mixture overnight at room temperature, the solvent was evaporated, water was added and the mixture was washed with diethyl ether. The obtained aqueous layer was acidified with 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was crystallized from n-hexane-ethyl acetate to give the title compound (332 mg) as white crystals.

### Example 75

### Production of 3-(6-methyl-3-oxo-2-phenyl-2,3-dihydrobenzo[1,4]oxazin-4-yl)propionic acid

### step 1

### Production of 4-[2-(1,3-dioxoran-2-yl)ethyl]-6-methyl-2-phenyl-4H-benzo[1,4]oxazin-3-one

6-Methyl-2-phenyl-4H-benzo[1,4]oxazin-3-one (239 mg) obtained in step 2 of Example 65 was dissolved in N,N-dimethylformamide (4 mL), and 60% sodium hydride (48 mg) was added. After stirring at room temperature, 2-(2-bromoethyl)-1,3-dioxorane (0.176 mL) was added. After stirring at room temperature for 3 days, water and ethyl acetate were added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=3:1) to give the title compound (218 mg) as a yellow oil.

### step 2

### Production of 3-(6-methyl-3-oxo-2-phenyl-2,3-dihydrobenzo[1,4]oxazin-4-yl)propionaldehyde

4-[2-(1,3-Dioxoran-2-yl)ethyl]-6-methyl-2-phenyl-4H-benzo[1,4]oxazin-3-one (218 mg) was dissolved in tetrahydrofuran (5 mL), and 6N hydrochloric acid (2 mL) was added. After stirring the mixture overnight at room temperature, ethyl acetate and saturated brine were added to the reaction mixture, and the mixture was extracted with ethyl acetate. The obtained ethyl acetate layer was washed with saturated brine, and dried over anhydrous magnesium sulfate. The solvent was evaporated to give the title compound.

### step 3

### Production of 3-(6-methyl-3-oxo-2-phenyl-2,3-dihydrobenzo[1,4]oxazin-4-yl)propionic acid

3-(6-Methyl-3-oxo-2-phenyl-2,3-dihydrobenzo[1,4]oxazin-4-yl)propionaldehyde obtained in step 2 was dissolved in tert-butanol (6 mL) and water (1.5 mL), and 2-methyl-2-butene (221 mg), sodium dihydrogenphosphate (85.2 mg) and sodium chlorite (276 mg) were added. After stirring the mixture overnight at room temperature, the solvent was evaporated, a 1N aqueous sodium hydroxide solution was added to the obtained residue, and the mixture was washed with ethyl acetate. The obtained aqueous layer was acidified with 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (124 mg) as white crystals.

### Example 76

### Production of 3-(7-chloro-3-oxo-2-phenyl-2,3-dihydrobenzo[1,4]oxazin-4-yl)propionic acid

### step 1

### Production of 7-chloro-4-[2-(1,3-dioxoran-2-yl)ethyl]-2-phenyl-4H-benzo[1,4]oxazin-3-one

7-Chloro-2-phenyl-4H-benzo[1,4]oxazin-3-one (260 mg) obtained in step 2 of Example 70 was dissolved in N,N-dimethylformamide (4 mL), and 60% sodium hydride (48 mg) was added. After stirring at room temperature, 2-(2-bromoethyl)-1,3-dioxorane (0.176 mL) was added. After stirring at room temperature for 3 days, water and ethyl acetate were added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=3:1) to give the title compound (246 mg) as a yellow oil.

### step 2

### Production of 3-(7-chloro-3-oxo-2-phenyl-2,3-dihydrobenzo[1,4]oxazin-4-yl)propionaldehyde

7-Chloro-4-[2-(1,3-dioxoran-2-yl)ethyl]-2-phenyl-4H-benzo[1,4]oxazin-3-one (246 mg) was dissolved in tetrahydrofuran (5 mL), and 6N hydrochloric acid (2 mL) was added. After stirring the mixture overnight at room temperature, ethyl acetate and saturated brine were added to the reaction mixture, and the mixture was extracted with ethyl acetate. The obtained ethyl acetate layer was washed with saturated brine, and dried over anhydrous magnesium sulfate. The solvent was evaporated to give the title compound.

### step 3

### Production of 3-(7-chloro-3-oxo-2-phenyl-2,3-dihydrobenzo[1,4]oxazin-4-yl)propionic acid

3-(7-Chloro-3-oxo-2-phenyl-2,3-dihydrobenzo[1,4]oxazin-4-yl)propionaldehyde obtained in step 2 was dissolved in tert-butanol (6 mL) and water (1.5 mL), and 2-methyl-2-butene (236 mg), sodium dihydrogenphosphate (91.2 mg) and sodium chlorite (295 mg) were added. After stirring the mixture overnight at room temperature, the solvent was evaporated, and a 1N aqueous sodium hydroxide solution was added to the obtained residue, and the mixture was washed with ethyl acetate. The obtained aqueous layer was acidified with 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (162 mg) as white crystals.

### Example 77

### Production of 3-(6-chloro-3-oxo-2-phenyl-2,3-dihydrobenzo[1,4]oxazin-4-yl)propionic acid

### step 1

### Production of 6-chloro-4-[2-(1,3-dioxoran-2-yl)ethyl]-2-phenyl-4H-benzo[1,4]oxazin-3-one

6-Chloro-2-phenyl-4H-benzo[1,4]oxazin-3-one (260 mg) obtained in step 2 of Example 71 was dissolved in N,N-dimethylformamide (4 mL), and 60% sodium hydride (48 mg) was added. After stirring at room temperature, 2-(2-bromoethyl)-1,3-dioxorane (0.176 mL) was added. After stirring at room temperature for 3 days, water and ethyl acetate were added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=2:1) to give the title compound (232 mg) as a yellow oil.

### step 2

### Production of 3-(6-chloro-3-oxo-2-phenyl-2,3-dihydrobenzo[1,4]oxazin-4-yl)propionaldehyde

6-Chloro-4-[2-(1,3-dioxoran-2-yl)ethyl]-2-phenyl-4H-benzo[1,4]oxazin-3-one (232 mg) was dissolved in tetrahydrofuran (5 mL), and 6N hydrochloric acid (2 mL) was added. After stirring the mixture overnight at room temperature, ethyl acetate and saturated brine were added to the reaction mixture, and the mixture was extracted with ethyl acetate. The obtained ethyl acetate layer was washed with saturated brine, and dried over anhydrous magnesium sulfate. The solvent was evaporated to give the title compound.

### step 3

### Production of 3-(6-chloro-3-oxo-2-phenyl-2,3-dihydrobenzo[1,4]oxazin-4-yl)propionic acid

3-(6-Chloro-3-oxo-2-phenyl-2,3-dihydrobenzo[1,4]oxazin-4-yl)propionaldehyde obtained in step 2 was dissolved in tert-butanol (6 mL) and water (1.5 mL), and 2-methyl-2-butene (221 mg), sodium dihydrogenphosphate (85.2 mg) and sodium chlorite (276 mg) were added. After stirring the mixture overnight at room temperature, the solvent was evaporated, and a 1N aqueous sodium hydroxide solution was added to the obtained residue, and the mixture was washed with diethyl ether. The obtained aqueous layer was acidified with 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (142 mg) as white crystals.

### Example 78

### Production of (E)-4-(8-bromo-2-isopropyl-2,3-dihydrobenzo[1,4]oxazin-4-yl)-4-oxo-2-butenoic acid

### step 1

### Production of 8-bromo-2-isopropyl-3,4-dihydro-2H-benzo[1,4]oxazine

8-Bromo-2-isopropyl-4H-benzo[1,4]oxazin-3-one (300 mg) obtained in step 3 of Example 51 was dissolved in tetrahydrofuran (5 mL), and a borane-tetrahydrofuran complex (1.0 M tetrahydrofuran solution, 1.65 mL) was added. After heating under reflux for 5.5 hr, 1N hydrochloric acid was added to the reaction mixture, and a saturated aqueous sodium hydrogencarbonate solution was further added. The mixture was extracted with ethyl acetate and the obtained ethyl acetate layer was washed successively with water and saturated brine, and dried over anhydrous sodium sulfate. The solvent was evaporated to give the title compound.

### step 2

### Production of (E)-4-(8-bromo-2-isopropyl-2,3-dihydrobenzo[1,4]oxazin-4-yl)-4-oxo-2-butenoic acid ethyl ester

8-Bromo-2-isopropyl-3,4-dihydro-2H-benzo[1,4]oxazine obtained in step 1 was dissolved in ethyl acetate (10 mL), and pyridine (0.133 mL) and (E)-3-chlorocarbonyl acrylic acid ethyl ester (215 mg) obtained in step 1 of Example 36 were added. After stirring the mixture overnight at room temperature, water was added to the reaction mixture, and the mixture was extracted with ethyl acetate. The obtained ethyl acetate layer was washed with saturated brine, dried over anhydrous sodium sulfate. The solvent was evaporated, and the obtained residue was purified by silica gel chromatography (n-hexane-ethyl acetate=6:1) to give the title compound (360 mg) as a lemon-colored oil.

### step 3

### Production of (E)-4-(8-bromo-2-isopropyl-2,3-dihydrobenzo[1,4]oxazin-4-yl)-4-oxo-2-butenoic acid

(E)-4-(8-Bromo-2-isopropyl-2,3-dihydrobenzo[1,4]oxazin-4-yl)-4-oxo-2-butenoic acid ethyl ester (355 mg) was dissolved in tert-butanol and a 1N aqueous sodium hydroxide solution (1.11 mL) was added. After stirring the mixture overnight at room temperature, the solvent was evaporated. Water was added, and the mixture was washed with diethyl ether. The obtained aqueous layer was acidified with 1N hydrochloric acid, and the mixture was extracted with ethyl acetate. The obtained ethyl acetate layer was washed successively with water and saturated brine, and dried over anhydrous sodium sulfate. The solvent was evaporated, and water was added to the obtained residue, and the precipitated solid was collected by filtration to give the title compound (258 mg) as a lemon-colored solid.

### Example 79

### Production of (E)-4-(8-cyclohexyl-2-isopropyl-2,3-dihydrobenzo[1,4]oxazin-4-yl)-4-oxo-2-butenoic acid

### step 1

### Production of 8-cyclohexyl-2-isopropyl-3,4-dihydro-2H-benzo[1,4]oxazine

8-Cyclohexyl-2-isopropyl-4H-benzo[1,4]oxazin-3-one (300 mg) obtained in step 4 of Example 50 was dissolved in tetrahydrofuran (5 mL), and a borane-tetrahydrofuran complex (1.0 M tetrahydrofuran solution, 1.65 mL) was added. The mixture was heated under reflux and 1N hydrochloric acid was added to the reaction mixture. A saturated aqueous sodium hydrogencarbonate solution was further added and the mixture was extracted with ethyl acetate. The obtained ethyl acetate layer was washed successively with water and saturated brine, and dried over anhydrous sodium sulfate. The solvent was evaporated to give the title compound.

### step 2

### Production of (E)-4-(8-cyclohexyl-2-isopropyl-2,3-dihydrobenzo[1,4]oxazin-4-yl)-4-oxo-2-butenoic acid ethyl ester

8-Cyclohexyl-2-isopropyl-3,4-dihydro-2H-benzo[1,4]oxazine obtained in step 1 was dissolved in ethyl acetate (10 mL), pyridine (0.133 mL) and (E)-3-chlorocarbonyl acrylic acid ethyl ester (215 mg) obtained in step 1 of Example 36 were added. After stirring the mixture overnight at room temperature, water was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=6:1) to give the title compound (311 mg) as a yellow oil.

### step 3

### Production of (E)-4-(8-cyclohexyl-2-isopropyl-2,3-dihydrobenzo[1,4]oxazin-4-yl)-4-oxo-2-butenoic acid

(E)-4-(8-Cyclohexyl-2-isopropyl-2,3-dihydrobenzo[1,4]oxazin-4-yl)-4-oxo-2-butenoic acid ethyl ester (306 mg) was dissolved in tert-butanol, and a 1N aqueous sodium hydroxide solution (0.953 mL) was added. After stirring the mixture overnight at room temperature, the solvent was evaporated. Water was added, and the mixture was washed with diethyl ether. The obtained aqueous layer was acidified with 1N hydrochloric acid, and the mixture was extracted with ethyl acetate. The obtained ethyl acetate layer was washed successively with water and saturated brine, and dried over anhydrous sodium sulfate. The solvent was evaporated, and water was added to the obtained residue, and the precipitated solid was collected by filtration to give the title compound (247 mg) as a yellow solid.

### Example 80

### Production of 3-(6,8-dichloro-2-isopropyl-3-oxo-2,3-dihydrobenzo[1,4]oxazin-4-yl)propionic acid

### step 1

### Production of 2-amino-4,6-dichlorophenol hydrochloride

2,4-Dichloro-6-nitrophenol (12 g) was dissolved in tetrahydrofuran (40 mL) and ethanol (70 mL) and water (60 mL), ammonium chloride (15.4 g) and iron (16.1 g) were added, and the mixture was stirred with heating at 90°C. The insoluble material was removed by celite filtration, and the filtrate was concentrated and extracted with ethyl acetate. Anhydrous sodium sulfate, silica gel and activated carbon were added to the obtained ethyl acetate layer and the mixture was filtered through celite. The filtrate was concentrated, and the obtained residue was dissolved in ethyl acetate, 4N hydrogen chloride (ethyl acetate solution) was added, and the precipitated solid was collected by filtration to give the title compound (9.30 g) as a gray solid.

### step 2

### Production of 2-bromo-N-(3,5-dichloro-2-hydroxyphenyl)-3-methylbutylamide

2-Amino-4,6-dichlorophenol hydrochloride (1.2 g) was dissolved by adding ethyl acetate (20 mL) and water (20 mL), sodium hydrogencarbonate (1.41 g) and 2-bromo-3-methylbutyryl chloride (1.34 g) obtained in step 1 of Example 6 were added at room temperature, and the mixture was stirred at room temperature for 2.5 hr. The reaction mixture was partitioned, and the obtained ethyl acetate layer was washed successively with water and saturated brine, and dried over anhydrous sodium sulfate. The solvent was evaporated to give the title compound (2.17 g) as an ocher solid.

### step 3

### Production of 6,8-dichloro-2-isopropyl-4H-benzo[1,4]oxazin-3-one

2-Bromo-N-(3,5-dichloro-2-hydroxyphenyl)-3-methylbutylamide (2.17 g) was dissolved in N,N-dimethylformamide (10 mL), potassium carbonate (1.00 g) was added, and the mixture was stirred overnight at room temperature. 1N Hydrochloric acid was added to the reaction mixture, water was added, and the precipitated solid was collected by filtration to give the title compound (1.39 g) as an ocher solid.

### step 4

### Production of 3-(6,8-dichloro-2-isopropyl-3-oxo-2,3-dihydrobenzo[1,4]oxazin-4-yl)propionic acid methyl ester

6,8-Dichloro-2-isopropyl-4H-benzo[1,4]oxazin-3-one (300 mg) was dissolved in N,N-dimethylformamide (5 mL), and potassium carbonate (319 mg) and methyl acrylate (0.208 mL) were added. After stirring with heating at 100°C for 9.5 hr, a 10% aqueous citric acid solution was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=6:1) to give the title compound (352 mg) as a colorless oil.

### step 5

### Production of 3-(6,8-dichloro-2-isopropyl-3-oxo-2,3-dihydrobenzo[1,4]oxazin-4-yl)propionic acid

3-(6,8-Dichloro-2-isopropyl-3-oxo-2,3-dihydrobenzo[1,4]oxazin-4-yl)propionic acid methyl ester (350 mg) was dissolved in methanol (5 mL), and a 2N aqueous sodium hydroxide solution (1 mL) was added. After stirring the mixture at room temperature for 1.5 hr, the solvent was evaporated. Water was added, and the mixture was washed with diethyl ether. The obtained aqueous layer was acidified with 1N hydrochloric acid, and extracted with ethyl acetate. The obtained ethyl acetate layer was washed successively with water and saturated brine, and dried over anhydrous sodium sulfate. The solvent was evaporated, and the obtained residue was crystallized from n-hexane-ethyl acetate to give the title compound (286 mg) as white crystals.

### Example 81

### Production of 3-(6-chloro-2-isopropyl-8-methyl-3-oxo-2,3-dihydrobenzo[1,4]oxazin-4-yl)propionic acid

### step 1

### Production of 4-chloro-2-methyl-6-nitrophenol

To a mixture of 4-chloro-2-methylphenol (6.42 g) and sodium nitrite (10.4 g) were added n-hexane (75 mL) and diisopropyl ether (35 mL) and water (50 mL), and 4.5N sulfuric acid (110 mL) was added dropwise at room temperature. After stirring at room temperature for 2 hr, solid precipitated in the reaction mixture was collected by filtration to give the title compound (5.32 g) as a yellow solid.

### step 2

### Production of 2-amino-4-chloro-6-methylphenol

4-Chloro-2-methyl-6-nitrophenol (3.0 g) and ammonium chloride (4.28 g) were dissolved in tetrahydrofuran (16 mL) and ethanol (32 mL) and water (16 mL). To this solution was added iron (4.47 g), and the mixture was heated under reflux for 2 hr. The reaction mixture was filtered through celite, concentrated under reduced pressure to give the title compound (1.69 g) as a brown solid.

### step 3

### Production of 2-bromo-N-(5-chloro-2-hydroxy-3-methylphenyl)-3-methylbutylamide

2-Amino-4-chloro-6-methylphenol (800 mg) was dissolved by adding ethyl acetate (15 mL) and water (15 mL), sodium hydrogencarbonate (1.05 g) and 2-bromo-3-methylbutyryl chloride (1.50 g) obtained in step 1 of Example 6 were added at room temperature, and the mixture was stirred at room temperature for 1.5 hr. The reaction mixture was partitioned, and the obtained ethyl acetate layer was washed successively with water and saturated brine, and dried over anhydrous sodium sulfate. The solvent was evaporated to give the title compound.

### step 4

### Production of 6-chloro-2-isopropyl-8-methyl-4H-benzo[1,4]oxazin-3-one

2-Bromo-N-(5-chloro-2-hydroxy-3-methylphenyl)-3-methylbutylamide obtained in step 3 was dissolved in N,N-dimethylformamide (10 mL), potassium carbonate (1.04 g) was added, and the mixture was stirred at room temperature for 3 days. 1N Hydrochloric acid was added to the reaction mixture, water was added, and the precipitated solid was collected by filtration. The obtained solid was crystallized from n-hexane to give the title compound (870 mg) as a pale-yellow solid.

### step 5

### Production of 3-(6-chloro-2-isopropyl-8-methyl-3-oxo-2,3-dihydrobenzo[1,4]oxazin-4-yl)propionic acid methyl ester

6-Chloro-2-isopropyl-8-methyl-4H-benzo[1,4]oxazin-3-one (288 mg) was dissolved in N,N-dimethylformamide (5 mL), and potassium carbonate (346 mg) and methyl acrylate (0.225 mL) were added. After stirring with heating overnight at 80°C, ethyl acetate and 1N hydrochloric acid were added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=4:1) to give the title compound (386 mg) as a colorless oil.

### step 6

### Production of 3-(6-chloro-2-isopropyl-8-methyl-3-oxo-2,3-dihydrobenzo[1,4]oxazin-4-yl)propionic acid

3-(6-Chloro-2-isopropyl-8-methyl-3-oxo-2,3-dihydrobenzo[1,4]oxazin-4-yl)propionic acid methyl ester (386 mg) was dissolved in methanol (5 mL), and a 1N aqueous sodium hydroxide solution (2.36 mL) was added. After stirring the mixture at room temperature for 1 hr, the solvent was evaporated, and water and ethyl acetate were added. The mixture was acidified with 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (335 mg) as white crystals.

### Example 82

### Production of 3-(6,8-dimethyl-3-oxo-2,3-dihydrobenzo[1,4]oxazin-4-yl)propionic acid

### step 1

### Production of 2-chloro-N-(2-hydroxy-3,5-dimethylphenyl)acetamide

2-Amino-4,6-dimethylphenol (1.006 g) was dissolved in ethyl acetate (10 mL), water (10 mL) and sodium hydrogencarbonate (1.2331 g) were added, and chloroacetyl chloride (0.7 mL) was added dropwise under ice-cooling. After stirring the mixture overnight at room temperature, the reaction mixture was extracted with ethyl acetate. The obtained ethyl acetate layer was washed successively with water and saturated brine, and dried over anhydrous sodium sulfate. The solvent was evaporated to give the title compound (1.6180 g) as a brown solid.

### step 2

### Production of 6,8-dimethyl-4H-benzo[1,4]oxazin-3-one

2-Chloro-N-(2-hydroxy-3,5-dimethylphenyl)acetamide (500.4 mg) was dissolved in N,N-dimethylformamide (5 mL), and potassium carbonate (421.7 mg) was added at room temperature. After stirring the mixture overnight at room temperature, 1N hydrochloric acid and water (20 mL) were added under ice-cooling and the mixture was extracted with ethyl acetate. The obtained ethyl acetate layer was washed successively with water and saturated brine, and dried over anhydrous sodium sulfate. The solvent was evaporated to give the title compound (386.6 mg) as an orange solid.

### step 3

### Production of 3-(6,8-dimethyl-3-oxo-2,3-dihydrobenzo[1,4]oxazin-4-yl)propionic acid methyl ester

6,8-Dimethyl-4H-benzo[1,4]oxazin-3-one (213 mg) was dissolved in N,N-dimethylformamide (5 mL), and potassium carbonate (346 mg) and methyl acrylate (0.225 mL) were added. After stirring with heating overnight at 80°C, ethyl acetate and water were added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=4:1) to give the title compound (286 mg) as a colorless oil.

### step 4

### Production of 3-(6,8-dimethyl-3-oxo-2,3-dihydrobenzo[1,4]oxazin-4-yl)propionic acid

3-(6,8-Dimethyl-3-oxo-2,3-dihydrobenzo[1,4]oxazin-4-yl)propionic acid methyl ester (286 mg) was dissolved in methanol (5 mL), and a 1N aqueous sodium hydroxide solution (2 mL) was added. After stirring the mixture at room temperature for 1 hr, the solvent was evaporated, water and ethyl acetate were added, and the mixture was washed with ethyl acetate. The obtained aqueous layer was acidified with 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (242 mg) as white crystals.

### Example 83

### Production of (E)-4-(6-chloro-2-isopropyl-8-methyl-2,3-dihydrobenzo[1,4]oxazin-4-yl)-4-oxo-2-butenoic acid

### step 1

### Production of 6-chloro-2-isopropyl-8-methyl-3,4-dihydro-2H-benzo[1,4]oxazine

6-Chloro-2-isopropyl-8-methyl-4H-benzo[1,4]oxazin-3-one (300 mg) obtained in step 4 of Example 81 was dissolved in tetrahydrofuran (4 mL), and a borane-tetrahydrofuran complex (1.0 M tetrahydrofuran solution, 2 mL) was added. After heating under reflux for 2 hr, 1N hydrochloric acid was added to the reaction mixture, and the mixture was further stirred with heating at 50°C for 1 hr. Water and ethyl acetate were added to the reaction mixture, and the mixture was extracted with ethyl acetate. The obtained ethyl acetate layer was washed successively with water, saturated aqueous sodium hydrogencarbonate solution and saturated brine, and dried over anhydrous magnesium sulfate. The solvent was evaporated, and the obtained residue was purified by silica gel chromatography (n-hexane-ethyl acetate=5:1) to give the title compound (235 mg) as a yellow oil.

### step 2

### Production of (E)-4-(6-chloro-2-isopropyl-8-methyl-2,3-dihydrobenzo[1,4]oxazin-4-yl)-4-oxo-2-butenoic acid ethyl ester

6-Chloro-2-isopropyl-8-methyl-3,4-dihydro-2H-benzo[1,4]oxazine (226 mg) was dissolved in ethyl acetate (4 mL), and pyridine (0.121 mL) and (E)-3-chlorocarbonyl acrylic acid ethyl ester (244 mg) obtained in step 1 of Example 36 were added. After stirring at room temperature for 5 days, the reaction mixture was washed successively with water, 1N hydrochloric acid, saturated aqueous sodium hydrogencarbonate solution and saturated brine, and dried over anhydrous sodium sulfate. The solvent was evaporated, and the obtained residue was purified by silica gel chromatography (n-hexane-ethyl acetate=3:1) to give the title compound (328 mg) as a colorless oil.

### step 3

### Production of (E)-4-(6-chloro-2-isopropyl-8-methyl-2,3-dihydrobenzo[1,4]oxazin-4-yl)-4-oxo-2-butenoic acid

(E)-4-(6-Chloro-2-isopropyl-8-methyl-2,3-dihydrobenzo[1,4]oxazin-4-yl)-4-oxo-2-butenoic acid ethyl ester (328 mg) was dissolved in tert-butanol (5 mL), and a 1N aqueous sodium hydroxide solution (1.12 mL) was added. After stirring the mixture overnight at room temperature, the reaction mixture was concentrated under reduced pressure. Water and ethyl acetate were added to the obtained residue, and the mixture was washed with ethyl acetate. The obtained aqueous layer was acidified with 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (213 mg) as white crystals.

### Example 84

### Production of (E)-4-(6-chloro-8-methyl-2-phenyl-2,3-dihydrobenzo[1,4]oxazin-4-yl)-4-oxo-2-butenoic acid

### step 1

### Production of 2-chloro-N-(5-chloro-2-hydroxy-3-methylphenyl)-2-phenylacetamide

2-Amino-4-chloro-6-methylphenol (800 mg) obtained in step 2 of Example 81 was dissolved by adding ethyl acetate (15 mL) and water (15 mL), sodium hydrogencarbonate (1.05 g) and α-chlorophenylacetyl chloride (1.21 mL) were added, and the mixture was stirred at room temperature for 1.5 hr. The reaction mixture was partitioned, and the obtained ethyl acetate layer was washed successively with water and saturated brine, and dried over anhydrous sodium sulfate. The solvent was evaporated to give the title compound.

### step 2

### Production of 6-chloro-8-methyl-2-phenyl-4H-benzo[1,4]oxazin-3-one

2-Chloro-N-(5-chloro-2-hydroxy-3-methylphenyl)-2-phenylacetamide obtained in step 1 was dissolved in N,N-dimethylformamide (10 mL), potassium carbonate (1.04 g) was added, and the mixture was stirred at room temperature for 3 days. Water and 1N hydrochloric acid were added to the reaction mixture, and the precipitated solid was collected by filtration. The obtained solid was crystallized from n-hexane-diisopropyl ether to give the title compound (910 mg) as a brown solid.

### step 3

### Production of 6-chloro-8-methyl-2-phenyl-3,4-dihydro-2H-benzo[1,4]oxazine

6-Chloro-8-methyl-2-phenyl-4H-benzo[1,4]oxazin-3-one (342 mg) was dissolved in tetrahydrofuran (4 mL), and a borane-tetrahydrofuran complex (1.0 M tetrahydrofuran solution, 2 mL) was added. After heating under reflux for 2 hr, 1N hydrochloric acid was added to the reaction mixture, and the mixture was further stirred with heating at 50°C for 1 hr. Water and ethyl acetate were added to the reaction mixture, and the mixture was extracted with ethyl acetate. The obtained ethyl acetate layer was washed successively with water, saturated aqueous sodium hydrogencarbonate solution and saturated brine, and dried over anhydrous magnesium sulfate. The solvent was evaporated, and the obtained residue was purified by silica gel chromatography (n-hexane-ethyl acetate=5:1) to give the title compound (235 mg) as a yellow oil.

### step 4

### Production of (E)-4-(6-chloro-8-methyl-2-phenyl-2,3-dihydrobenzo[1,4]oxazin-4-yl)-4-oxo-2-butenoic acid ethyl ester

6-Chloro-8-methyl-2-phenyl-3,4-dihydro-2H-benzo[1,4]oxazine (227 mg) was dissolved in ethyl acetate (4 mL), and pyridine (0.106 mL) and (E)-3-chlorocarbonyl acrylic acid ethyl ester (213 mg) obtained in step 1 of Example 36 were added. After stirring at room temperature for 4 days, the reaction mixture was washed successively with water and saturated brine, and dried over anhydrous sodium sulfate. The solvent was evaporated, and the obtained residue was purified by silica gel chromatography (n-hexane-ethyl acetate=4:1) to give the title compound (338 mg) as a colorless oil.

### step 5

### Production of (E)-4-(6-chloro-8-methyl-2-phenyl-2,3-dihydrobenzo[1,4]oxazin-4-yl)-4-oxo-2-butenoic acid

(E)-4-(6-Chloro-8-methyl-2-phenyl-2,3-dihydrobenzo[1,4]oxazin-4-yl)-4-oxo-2-butenoic acid ethyl ester (338 mg) was dissolved in tert-butanol (5 mL), and a 1N aqueous sodium hydroxide solution (1.06 mL) was added. After stirring the mixture overnight at room temperature, the reaction mixture was concentrated under reduced pressure. Water and ethyl acetate were added to the obtained residue, and the mixture was washed with ethyl acetate. The obtained aqueous layer was acidified with 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (234 mg) as white crystals.

### Example 85

### Production of (E)-4-(6,8-dimethyl-2,3-dihydrobenzo[1,4]oxazin-4-yl)-4-oxo-2-butenoic acid

### step 1

### Production of 6,8-dimethyl-3,4-dihydro-2H-benzo[1,4]oxazine

6,8-Dimethyl-4H-benzo[1,4]oxazin-3-one (266 mg) obtained in step 2 of Example 82 was dissolved in tetrahydrofuran (5 mL), and a borane-tetrahydrofuran complex (1.0 M tetrahydrofuran solution, 2.25 mL) was added. After heating under reflux for 1 hr, 1N hydrochloric acid was added to the reaction mixture, and the mixture was further stirred with heating at 50°C for 1 hr. Water and ethyl acetate were added to the reaction mixture, and the mixture was extracted with ethyl acetate. The obtained ethyl acetate layer was washed with saturated brine, and dried over anhydrous magnesium sulfate. The solvent was evaporated, and the obtained residue was purified by silica gel chromatography (n-hexane-ethyl acetate=5:1) to give the title compound (213 mg) as a yellow oil.

### step 2

### Production of (E)-4-(6,8-dimethyl-2,3-dihydrobenzo[1,4]oxazin-4-yl)-4-oxo-2-butenoic acid ethyl ester

6,8-Dimethyl-3,4-dihydro-2H-benzo[1,4]oxazine (208 mg) was dissolved in ethyl acetate (4 mL), and pyridine (0.155 mL) and (E)-3-chlorocarbonyl acrylic acid ethyl ester (311 mg) obtained in step 1 of Example 36 were added. After stirring at room temperature for 5 days, the reaction mixture was washed successively with water and saturated brine, and dried over anhydrous sodium sulfate. The solvent was evaporated, and the obtained residue was purified by silica gel chromatography (n-hexane-ethyl acetate=4:1) to give the title compound (312 mg) as a colorless oil.

### step 3

### Production of (E)-4-(6,8-dimethyl-2,3-dihydrobenzo[1,4]oxazin-4-yl)-4-oxo-2-butenoic acid

(E)-4-(6,8-Dimethyl-2,3-dihydrobenzo[1,4]oxazin-4-yl)-4-oxo-2-butenoic acid ethyl ester (312 mg) was dissolved in tert-butanol (5 mL), and a 1N aqueous sodium hydroxide solution (1.3 mL) was added. After stirring the mixture overnight at room temperature, the reaction mixture was concentrated under reduced pressure. Water and ethyl acetate were added to the obtained residue, and the mixture was washed with ethyl acetate. The obtained aqueous layer was acidified with 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (249 mg) as white crystals.

### Example 86

### Production of 3-(2,6,8-trimethyl-3-oxo-2,3-dihydrobenzo[1,4]oxazin-4-yl)propionic acid

### step 1

### Production of 2-bromo-N-(2-hydroxy-3,5-dimethylphenyl)propionamide

2-Amino-4,6-dimethylphenol (1.5 g) was dissolved by adding ethyl acetate (15 mL) and water (15 mL), sodium hydrogencarbonate (1.84 g) and 2-bromopropionylbromide (1.39 mL) were added at room temperature, and the mixture was stirred at room temperature. The reaction mixture was partitioned, and the obtained ethyl acetate layer was washed successively with water and saturated brine, and dried over anhydrous sodium sulfate. The solvent was evaporated to give the title compound (2.8 g) as a brown solid.

### step 2

### Production of 2,6,8-trimethyl-4H-benzo[1,4]oxazin-3-one

2-Bromo-N-(2-hydroxy-3,5-dimethylphenyl)propionamide (2.8 g) was dissolved in N,N-dimethylformamide (20 mL), potassium carbonate (2.11 g) was added, and the mixture was stirred overnight at room temperature. Water was added to the reaction mixture, and the precipitated solid was collected by filtration to give the title compound (1.74 g) as an orange solid.

### step 3

### Production of 3-(2,6,8-trimethyl-3-oxo-2,3-dihydrobenzo[1,4]oxazin-4-yl)propionic acid methyl ester

2,6,8-Trimethyl-4H-benzo[1,4]oxazin-3-one (300 mg) was dissolved in N,N-dimethylformamide (5 mL), and potassium carbonate (433 mg) and methyl acrylate (0.283 mL) were added. After stirring with heating overnight at 100°C, a 10% aqueous citric acid solution was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=5:1) to give the title compound (358 mg) as an orange solid.

### step 4

### Production of 3-(2,6,8-trimethyl-3-oxo-2,3-dihydrobenzo[1,4]oxazin-4-yl)propionic acid

3-(2,6,8-Trimethyl-3-oxo-2,3-dihydrobenzo[1,4]oxazin-4-yl)propionic acid methyl ester (353 mg) was dissolved in methanol (5 mL), and a 2N aqueous sodium hydroxide solution (1.27 mL) was added. After stirring at room temperature for 3 hr, the solvent was evaporated. Water was added, and the mixture was washed with diethyl ether. The obtained aqueous layer was filtered through celite to remove insoluble materials. The filtrate was acidified with 1N hydrochloric acid. The precipitated solid was collected by filtration to give the title compound (303 mg) as pale-pink crystals.

### Example 87

### Production of 3-(2-ethyl-6,8-dimethyl-3-oxo-2,3-dihydrobenzo[1,4]oxazin-4-yl)propionic acid

### step 1

### Production of 2-bromo-N-(2-hydroxy-3,5-dimethylphenyl)butylamide

2-Amino-4,6-dimethylphenol (1.5 g) was dissolved by adding ethyl acetate (15 mL) and water (15 mL), sodium hydrogencarbonate (1.84 g) and 2-bromobutyrylbromide (1.59 mL) were added at room temperature, and the mixture was stirred at room temperature. The reaction mixture was partitioned, and the obtained ethyl acetate layer was washed successively with water and saturated brine, and dried over anhydrous sodium sulfate. The solvent was evaporated to give the title compound (2.81 g) as a brown oil.

### step 2

### Production of 2-ethyl-6,8-dimethyl-4H-benzo[1,4]oxazin-3-one

2-Bromo-N-(2-hydroxy-3,5-dimethylphenyl)butylamide (2.81 g) was dissolved in N,N-dimethylformamide (20 mL), potassium carbonate (2.11 g) was added, and the mixture was stirred overnight at room temperature. Water was added to the reaction mixture, and the precipitated solid was collected by filtration to give the title compound (1.66 g) as an orange solid.

### step 3

### Production of 3-(2-ethyl-6,8-dimethyl-3-oxo-2,3-dihydrobenzo[1,4]oxazin-4-yl)propionic acid methyl ester

2-Ethyl-6,8-dimethyl-4H-benzo[1,4]oxazin-3-one (300 mg) was dissolved in N,N-dimethylformamide (5 mL), and potassium carbonate (403 mg) and methyl acrylate (0.263 mL) were added. After stirring with heating overnight at 100°C, a 10% aqueous citric acid solution was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=5:1) to give the title compound (352 mg) as an orange oil.

### step 4

### Production of 3-(2-ethyl-6,8-dimethyl-3-oxo-2,3-dihydrobenzo[1,4]oxazin-4-yl)propionic acid

3-(2-Ethyl-6,8-dimethyl-3-oxo-2,3-dihydrobenzo[1,4]oxazin-4-yl)propionic acid methyl ester (347 mg) was dissolved in methanol (5 mL), and a 2N aqueous sodium hydroxide solution (1.19 mL) was added. After stirring the mixture at room temperature, the solvent was evaporated. Water was added, and the mixture was washed with diethyl ether. The obtained aqueous layer was acidified with 1N hydrochloric acid. The precipitated solid was collected by filtration to give the title compound (296 mg) as pale-pink crystals.

### Example 88

### Production of 3-(6-tert-butyl-2-isopropyl-3-oxo-2,3-dihydrobenzo[1,4]oxazin-4-yl)propionic acid

### step 1

### Production of 2-bromo-N-(5-tert-butyl-2-hydroxyphenyl)-3-methylbutylamide

2-Amino-4-tert-butylphenol (1.5 g) was dissolved by adding ethyl acetate (15 mL) and water (15 mL), sodium hydrogencarbonate (1.53 g) and 2-bromo-3-methylbutyryl chloride (2.17 g) obtained in step 1 of Example 6 were added at room temperature, and the mixture was stirred at room temperature for 5.5 hr. The reaction mixture was partitioned, and the obtained ethyl acetate layer was washed successively with water and saturated brine, and dried over anhydrous sodium sulfate. The solvent was evaporated to give the title compound.

### step 2

### Production of 6-tert-butyl-2-isopropyl-4H-benzo[1,4]oxazin-3-one

2-Bromo-N-(5-tert-butyl-2-hydroxyphenyl)-3-methylbutylamide obtained in step 1 was dissolved in N,N-dimethylformamide (20 mL), potassium carbonate (1.75 g) was added, and the mixture was stirred overnight at room temperature. Water was added to the reaction mixture, and the precipitated solid was collected by filtration to give the title compound (2.17 g) as a cream-colored solid.

### step 3

### Production of 3-(6-tert-butyl-2-isopropyl-3-oxo-2,3-dihydrobenzo[1,4]oxazin-4-yl)propionic acid methyl ester

6-tert-Butyl-2-isopropyl-4H-benzo[1,4]oxazin-3-one (300 mg) was dissolved in N,N-dimethylformamide (5 mL), and potassium carbonate (334 mg) and methyl acrylate (0.218 mL) were added. After stirring with heating at 100°C for 10 hr, a 10% aqueous citric acid solution was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=4:1) to give the title compound (397 mg) as a pale-yellow oil.

### step 4

### Production of 3-(6-tert-butyl-2-isopropyl-3-oxo-2,3-dihydrobenzo[1,4]oxazin-4-yl)propionic acid

3-(6-tert-Butyl-2-isopropyl-3-oxo-2,3-dihydrobenzo[1,4]oxazin-4-yl)propionic acid methyl ester (392 mg) was dissolved in methanol (6 mL), and a 2N aqueous sodium hydroxide solution (1.18 mL) was added. After stirring at room temperature for 3 hr, the mixture was acidified with water and 1N hydrochloric acid, and extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (315 mg) as white crystals.

### Example 89

### Production of 3-(2,2,6,8-tetramethyl-3-oxo-2,3-dihydrobenzo[1,4]oxazin-4-yl)propionic acid

### step 1

### Production of 2-bromo-N-(2-hydroxy-3,5-dimethylphenyl)-2-methylpropionamide

2-Amino-4,6-dimethylphenol (2 g) was dissolved by adding ethyl acetate (20 mL) and water (20 mL), sodium hydrogencarbonate (2.45 g) and 2-bromo-2-methylpropionylbromide (2.0 mL) were added at room temperature, and the mixture was stirred at room temperature for 7 hr. The reaction mixture was partitioned, and the obtained ethyl acetate layer was washed successively with saturated aqueous sodium hydrogencarbonate solution, water and saturated brine, and dried over anhydrous sodium sulfate. The solvent was evaporated to give the title compound (4.0707 g) as a red-brown solid.

### step 2

### Production of 2,2,6,8-tetramethyl-4H-benzo[1,4]oxazin-3-one

2-Bromo-N-(2-hydroxy-3,5-dimethylphenyl)-2-methylpropionamide (4.07 g) was dissolved in N,N-dimethylformamide (20 mL), potassium carbonate (2.75 g) was added, and the mixture was stirred overnight at room temperature. Water was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=4:1) to give the title compound (757.6 mg) as a bright red solid.

### step 3

### Production of 3-(2,2,6,8-tetramethyl-3-oxo-2,3-dihydrobenzo[1,4]oxazin-4-yl)propionic acid methyl ester

2,2,6,8-Tetramethyl-4H-benzo[1,4]oxazin-3-one (757.6 mg) was dissolved in N,N-dimethylformamide (7 mL), and potassium carbonate (1.02 g) and methyl acrylate (0.66 mL) were added. After stirring with heating overnight at 90°C, a 10% aqueous citric acid solution was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=5:1) to give the title compound (402.3 mg) as an orange oil.

### step 4

### Production of 3-(2,2,6,8-tetramethyl-3-oxo-2,3-dihydrobenzo[1,4]oxazin-4-yl)propionic acid

3-(2,2,6,8-Tetramethyl-3-oxo-2,3-dihydrobenzo[1,4]oxazin-4-yl)propionic acid methyl ester (402.3 mg) was dissolved in methanol (5 mL) and tetrahydrofuran (3 mL), and 4N aqueous lithium hydroxide solution (1.4 mL) was added. After stirring the mixture overnight at room temperature, the solvent was evaporated. Water was added, and the mixture was washed with diethyl ether. The obtained aqueous layer was acidified with 1N hydrochloric acid. The precipitated solid was collected by filtration to give the title compound (251.5 mg) as pale-pink crystals.

### Example 90

### Production of 3-(2-isobutyl-6,8-dimethyl-3-oxo-2,3-dihydrobenzo[1,4]oxazin-4-yl)propionic acid

### step 1

### Production of 2-chloro-4-methylpentanoylchloride

Thionyl chloride (5.5 mL) was added to DL-leucine (10 g), and the mixture was heated under reflux for 1.5 hr. The reaction mixture was concentrated, and the obtained residue was dissolved in methylene chloride, and oxalyl chloride (7.0 mL) and N,N-dimethylformamide (one drop) were added. After stirring the mixture at room temperature for 2 hr, the mixture was concentrated, and azeotroped with toluene to give the title compound.

### step 2

### Production of 2-chloro-N-(2-hydroxy-3,5-dimethylphenyl)-4-methylvaleramide

2-Amino-4,6-dimethylphenol (686 mg) was dissolved by adding ethyl acetate (15 mL) and water (15 mL), sodium hydrogencarbonate (1.05 g) and 2-chloro-4-methylpentanoylchloride (1.23 g) were added at room temperature, and the mixture was stirred at room temperature for 2 hr. The reaction mixture was partitioned, and the obtained ethyl acetate layer was washed successively with water and saturated brine, and dried over anhydrous sodium sulfate. The solvent was evaporated to give the title compound.

### step 3

### Production of 2-isobutyl-6,8-dimethyl-4H-benzo[1,4]oxazin-3-one

2-Chloro-N-(2-hydroxy-3,5-dimethylphenyl)-4-methylvaleramide obtained in step 2 was dissolved in N,N-dimethylformamide (10 mL), potassium carbonate (1.04 g) was added, and the mixture was stirred overnight at room temperature. Ethyl acetate and 1N hydrochloric acid were added to the reaction mixture, and the mixture was extracted with ethyl acetate. The obtained ethyl acetate layer was washed successively with saturated aqueous sodium hydrogencarbonate solution and saturated brine, and dried over anhydrous sodium sulfate. The solvent was evaporated, and the obtained residue was purified by silica gel chromatography (n-hexane-ethyl acetate=3:1) and further crystallized from n-hexane to give the title compound (520 mg) as a yellow solid.

### step 4

### Production of 3-(2-isobutyl-6,8-dimethyl-3-oxo-2,3-dihydrobenzo[1,4]oxazin-4-yl)propionic acid methyl ester

2-Isobutyl-6,8-dimethyl-4H-benzo[1,4]oxazin-3-one (350 mg) was dissolved in N,N-dimethylformamide (5 mL), and potassium carbonate (415 mg) and methyl acrylate (0.270 mL) were added. After stirring with heating overnight at 80°C, ethyl acetate and water were added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=3:1) to give the title compound (455 mg) as a colorless oil.

### step 5

### Production of 3-(2-isobutyl-6,8-dimethyl-3-oxo-2,3-dihydrobenzo[1,4]oxazin-4-yl)propionic acid

3-(2-Isobutyl-6,8-dimethyl-3-oxo-2,3-dihydrobenzo[1,4]oxazin-4-yl)propionic acid methyl ester (455 mg) was dissolved in methanol (5 mL), and a 1N aqueous sodium hydroxide solution (2.13 mL) was added. After stirring the mixture at room temperature for 1 hr, the solvent was evaporated, water was added and the mixture was washed with ethyl acetate. The obtained aqueous layer was acidified with 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (392 mg) as white crystals.

### Example 91

### Production of 3-((R)-2-isobutyl-6,8-dimethyl-3-oxo-2,3-dihydrobenzo[1,4]oxazin-4-yl)propionic acid

### step 1

Production of (S)-2-chloro-4-methylpentanoylchloride (S)-2-Chloro-4-methylvaleric acid (5 g) was dissolved in methylene chloride (50 mL), and oxalyl chloride (4.29 mL) and N,N-dimethylformamide (one drop) were added. After stirring the mixture at room temperature for 2 hr, the mixture was concentrated and azeotroped with toluene to give the title compound.

### step 2

### Production of (S)-2-chloro-N-(2-hydroxy-3,5-dimethylphenyl)-4-methylvaleramide

2-Amino-4,6-dimethylphenol (1.37 g) was dissolved by adding ethyl acetate (30 mL) and water (30 mL), sodium hydrogencarbonate (2.10 g) and (S)-2-chloro-4-methylpentanoylchloride (2.46 g) obtained in step 1 were added at room temperature, and the mixture was stirred at room temperature for 2 hr. The reaction mixture was partitioned, and the obtained ethyl acetate layer was washed successively with water and saturated brine, and dried over anhydrous sodium sulfate. The solvent was evaporated to give the title compound.

### step 3

### Production of (R)-2-isobutyl-6,8-dimethyl-4H-benzo[1,4]oxazin-3-one

(S)-2-Chloro-N-(2-hydroxy-3,5-dimethylphenyl)-4-methylvaleramide obtained in step 2 was dissolved in N,N-dimethylformamide (15 mL), potassium carbonate (2.07 g) was added, and the mixture was stirred overnight at room temperature. Water and 1N hydrochloric acid were added to the reaction mixture, and the precipitated solid was collected by filtration. The obtained solid was crystallized from n-hexane to give the title compound (990 mg) as a yellow solid.

### step 4

### Production of 3-((R)-2-isobutyl-6,8-dimethyl-3-oxo-2,3-dihydrobenzo[1,4]oxazin-4-yl)propionic acid methyl ester

(R)-2-Isobutyl-6,8-dimethyl-4H-benzo[1,4]oxazin-3-one (817 mg) was dissolved in N,N-dimethylformamide (10 mL), and potassium carbonate (965 mg) and methyl acrylate (0.630 mL) were added. After stirring with heating at 80°C for 12 hr, ethyl acetate and water were added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=3:1) to give the title compound (1.10 g) as a colorless oil.

### step 5

### Production of 3-((R)-2-isobutyl-6,8-dimethyl-3-oxo-2,3-dihydrobenzo[1,4]oxazin-4-yl)propionic acid

3-((R)-2-Isobutyl-6,8-dimethyl-3-oxo-2,3-dihydrobenzo[1,4]oxazin-4-yl)propionic acid methyl ester (1.10 g) was dissolved in methanol (20 mL), and a 1N aqueous sodium hydroxide solution (6.88 mL) was added. After stirring the mixture at room temperature for 0.5 hr, the solvent was evaporated, water was added and the mixture was washed with ethyl acetate. The obtained aqueous layer was acidified with 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (920 mg) as white crystals.

### Example 92

### Production of 3-(2-benzyl-6,8-dimethyl-3-oxo-2,3-dihydrobenzo[1,4]oxazin-4-yl)propionic acid

### step 1

### Production of 2-bromo-3-phenylpropionic acid

DL-Phenylalanine (22.2 g) was suspended in 48% hydrobromic acid (80 mL), sodium nitrite (11.1 g) dissolved in water (27 mL) was added dropwise under ice-cooling. Diisopropyl ether was added, and the mixture was stirred under ice-cooling. Water was added to the reaction mixture, and the mixture was extracted with ethyl acetate. The obtained ethyl acetate layer was washed successively with water, aqueous sodium sulfite solution, water and saturated brine, and dried over anhydrous sodium sulfate. The solvent was evaporated, and the obtained residue was purified by silica gel chromatography (n-hexane-ethyl acetate=1:1) to give the title compound (18.6 g) as a pale-yellow oil.

### step 2

### Production of 2-bromo-3-phenylpropionylchloride

2-Bromo-3-phenylpropionic acid (6.0 g) was dissolved in chloroform (25 mL), and oxalyl chloride (2.97 mL) and N,N-dimethylformamide (one drop) were added. After stirring the mixture overnight at room temperature, the mixture was concentrated and azeotroped with toluene to give the title compound as a yellow oil.

### step 3

### Production of 2-bromo-N-(2-hydroxy-3,5-dimethylphenyl)-3-phenylpropionamide

2-Amino-4,6-dimethylphenol (1.5 g) was dissolved by adding ethyl acetate (15 mL) and water (15 mL), sodium hydrogencarbonate (1.83 g) and 2-bromo-3-phenylpropionylchloride (3.24 g) obtained in step 2 were added at room temperature, and the mixture was stirred at room temperature for 0.5 hr. The reaction mixture was partitioned, and the obtained ethyl acetate layer was washed successively with water and saturated brine, and dried over anhydrous sodium sulfate. The solvent was evaporated to give the title compound as a brown oil.

### step 4

### Production of 2-benzyl-6,8-dimethyl-4H-benzo[1,4]oxazin-3-one

2-Bromo-N-(2-hydroxy-3,5-dimethylphenyl)-3-phenylpropionamide obtained in step 3 was dissolved in N,N-dimethylformamide (20 mL), potassium carbonate (2.11 g) was added, and the mixture was stirred overnight at room temperature. Water was added to the reaction mixture, and the precipitated solid was collected by filtration to give the title compound (2.20 g) as an orange solid.

### step 5

### Production of 3-(2-benzyl-6,8-dimethyl-3-oxo-2,3-dihydrobenzo[1,4]oxazin-4-yl)propionic acid methyl ester

2-Benzyl-6,8-dimethyl-4H-benzo[1,4]oxazin-3-one (300 mg) was dissolved in N,N-dimethylformamide (5 mL), and potassium carbonate (309 mg) and methyl acrylate (0.201 mL) were added. After stirring with heating overnight at 90°C, a 10% aqueous citric acid solution was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=4:1) to give the title compound (356 mg) as an orange oil.

### step 6

### Production of 3-(2-benzyl-6,8-dimethyl-3-oxo-2,3-dihydrobenzo[1,4]oxazin-4-yl)propionic acid

3-(2-Benzyl-6,8-dimethyl-3-oxo-2,3-dihydrobenzo[1,4]oxazin-4-yl)propionic acid methyl ester (351 mg) was dissolved in methanol (6 mL), and a 2N aqueous sodium hydroxide solution (1 mL) was added. After stirring the mixture overnight at room temperature, the solvent was evaporated, a 4N aqueous sodium hydroxide solution and water were added, and the mixture was washed with diethyl ether. The aqueous layer was filtered through celite to remove insoluble materials. The obtained aqueous layer was acidified with 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (290 mg) as pale-orange crystals.

### Example 93

### Production of 3-(2-butyl-6,8-dimethyl-3-oxo-2,3-dihydrobenzo[1,4]oxazin-4-yl)propionic acid

### step 1

### Production of 2-bromo-N-(2-hydroxy-3,5-dimethylphenyl)hexanamide

2-Amino-4,6-dimethylphenol (1.5 g) was dissolved by adding ethyl acetate (15 mL) and water (15 mL), sodium hydrogencarbonate (1.83 g) and 2-bromohexanoylbromide (2.18 mL) were added at room temperature, and the mixture was stirred at room temperature. The reaction mixture was partitioned, and the obtained ethyl acetate layer was washed successively with water and saturated brine, and dried over anhydrous sodium sulfate. The solvent was evaporated to give the title compound.

### step 2

### Production of 2-butyl-6,8-dimethyl-4H-benzo[1,4]oxazin-3-one

2-Bromo-N-(2-hydroxy-3,5-dimethylphenyl)hexanamide obtained in step 1 was dissolved in N,N-dimethylformamide (20 mL), potassium carbonate (2.26 g) was added, and the mixture was stirred overnight at room temperature. Water was added to the reaction mixture, and the precipitated solid was collected by filtration to give the title compound (2.37 g) as an orange solid.

### step 3

### Production of 3-(2-butyl-6,8-dimethyl-3-oxo-2,3-dihydrobenzo[1,4]oxazin-4-yl)propionic acid methyl ester

2-Butyl-6,8-dimethyl-4H-benzo[1,4]oxazin-3-one (300 mg) was dissolved in N,N-dimethylformamide (5 mL), and potassium carbonate (355 mg) and methyl acrylate (0.232 mL) were added. After stirring with heating at 90°C for 6.5 hr, a 10% aqueous citric acid solution was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=6:1) to give the title compound (400 mg) as an orange oil.

### step 4

### Production of 3-(2-butyl-6,8-dimethyl-3-oxo-2,3-dihydrobenzo[1,4]oxazin-4-yl)propionic acid

3-(2-Butyl-6,8-dimethyl-3-oxo-2,3-dihydrobenzo[1,4]oxazin-4-yl)propionic acid methyl ester (395 mg) was dissolved in methanol (6 mL), and a 2N aqueous sodium hydroxide solution (1.24 mL) was added. After stirring the mixture overnight at room temperature, the solvent was evaporated. Water was added, and the mixture was washed with diethyl ether. The obtained aqueous layer was acidified with 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (312 mg) as white crystals.

### Examples 94

### Production of 3-(6,8-dichloro-2-isopropyl-7-methyl-3-oxo-2,3-dihydrobenzo[1,4]oxazin-4-yl)propionic acid

### step 1

### Production of 2-bromo-N-(3,5-dichloro-2-hydroxy-4-methylphenyl)-3-methylbutylamide

6-Amino-2,4-dichloro-3-methylphenol hydrochloride (1.5 g) was dissolved by adding ethyl acetate (20 mL) and water (20 mL), sodium hydrogencarbonate (1.49 g) and 2-bromo-3-methylbutyryl chloride (1.41 g) obtained in step 1 of Example 6 were added at room temperature, and the mixture was stirred at room temperature for 1.5 hr. Insoluble materials in the reaction mixture were removed by filtration, and the filtrate was partitioned. The obtained ethyl acetate layer was washed successively with water and saturated brine, and dried over anhydrous sodium sulfate. The solvent was evaporated to give the title compound.

### step 2

### Production of 6,8-dichloro-2-isopropyl-7-methyl-4H-benzo[1,4]oxazin-3-one

2-Bromo-N-(3,5-dichloro-2-hydroxy-4-methylphenyl)-3-methylbutylamide obtained in step 1 was dissolved in N,N-dimethylformamide (15 mL), potassium carbonate (1.22 g) was added, and the mixture was stirred overnight at room temperature. Water and n-hexane were added to the reaction mixture, and the precipitated solid was collected by filtration to give the title compound (1.70 g) as an ocher solid.

### step 3

### Production of 3-(6,8-dichloro-2-isopropyl-7-methyl-3-oxo-2,3-dihydrobenzo[1,4]oxazin-4-yl)propionic acid methyl ester

6,8-Dichloro-2-isopropyl-7-methyl-4H-benzo[1,4]oxazin-3-one (300 mg) was dissolved in N,N-dimethylformamide (5 mL), and potassium carbonate (302 mg) and methyl acrylate (0.197 mL) were added. After stirring with heating at 90°C for 5 hr, a 10% aqueous citric acid solution was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=6:1) to give the title compound (386 mg) as a colorless oil.

### step 4

### Production of 3-(6,8-dichloro-2-isopropyl-7-methyl-3-oxo-2,3-dihydrobenzo[1,4]oxazin-4-yl)propionic acid

3-(6,8-Dichloro-2-isopropyl-7-methyl-3-oxo-2,3-dihydrobenzo[1,4]oxazin-4-yl)propionic acid methyl ester (380 mg) was dissolved in methanol (6 mL), a 2N aqueous sodium hydroxide solution (1.05 mL) was added, and the mixture was stirred overnight at room temperature. Water was added to the reaction mixture, the insoluble materials were removed by filtration, and the aqueous layer was washed with diethyl ether. The obtained aqueous layer was acidified with 1N hydrochloric acid. The precipitated solid was collected by filtration to give the title compound (291 mg) as white crystals.

### Example 95

### Production of 3-(2-isopropyl-7-nitro-3-oxo-2,3-dihydrobenzo[1,4]oxazin-4-yl)propionic acid

### step 1

### Production of 2-bromo-N-(2-hydroxy-4-nitrophenyl)-3-methylbutylamide

2-Amino-5-nitrophenol (1.001 g) was dissolved by adding ethyl acetate (12 mL) and water (10 mL), sodium hydrogencarbonate (1.0968 g) and 2-bromo-3-methylbutyryl chloride (1.4848 g) obtained in step 1 of Example 6 were added at room temperature, and the mixture was stirred overnight at room temperature. The reaction mixture was partitioned, and the obtained ethyl acetate layer was washed successively with water and saturated brine, and dried over anhydrous sodium sulfate. The solvent was evaporated to give the title compound (2.0159 g) as a yellow solid.

### step 2

### Production of 2-isopropyl-7-nitro-4H-benzo[1,4]oxazin-3-one

2-Bromo-N-(2-hydroxy-4-nitrophenyl)-3-methylbutylamide (2.0085 g) was dissolved in N,N-dimethylformamide (20 mL), potassium carbonate (1.1303 g) was added, and the mixture was stirred overnight at room temperature. Ethyl acetate and water were added to the reaction mixture, and the mixture was extracted with ethyl acetate. The obtained ethyl acetate layer was washed successively with water and saturated brine, and dried over anhydrous sodium sulfate. The solvent was evaporated to give the title compound (1.4391 g) as a yellow solid.

### step 3

### Production of 3-(2-isopropyl-7-nitro-3-oxo-2,3-dihydrobenzo[1,4]oxazin-4-yl)propionic acid methyl ester

2-Isopropyl-7-nitro-4H-benzo[1,4]oxazin-3-one (332.4 mg) was dissolved in N,N-dimethylformamide (5 mL), and potassium carbonate (388.4 mg) and methyl acrylate (0.253 mL) were added. After stirring with heating overnight at 100°C, ethyl acetate and water were added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (chloroform -methanol=97:3) to give the title compound (248.2 mg) as a yellow oil.

### step 4

### Production of 3-(2-isopropyl-7-nitro-3-oxo-2,3-dihydrobenzo[1,4]oxazin-4-yl)propionic acid

3-(2-Isopropyl-7-nitro-3-oxo-2,3-dihydrobenzo[1,4]oxazin-4-yl)propionic acid methyl ester (237.6 mg) was dissolved in methanol (2.5 mL), and a 2N aqueous sodium hydroxide solution (0.738 mL) was added. After stirring the mixture at room temperature, the solvent was evaporated, water was added and the mixture was washed with diethyl ether. The obtained aqueous layer was acidified with 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (chloroform -methanol=95:5) to give the title compound (118.7 mg) as yellow crystals.

### Example 96

### Production of 3-(2-isopropyl-6-nitro-3-oxo-2,3-dihydrobenzo[1,4]oxazin-4-yl)propionic acid

### step 1

### Production of 2-bromo-N-(2-hydroxy-5-nitrophenyl)-3-methylbutylamide

2-Amino-4-nitrophenol (1.0027 g) was dissolved by adding ethyl acetate (10 mL) and water (10 mL), sodium hydrogencarbonate (1.0969 g) and 2-bromo-3-methylbutyryl chloride (1.5032 g) obtained in step 1 of Example 6 were added at room temperature, and the mixture was stirred overnight at room temperature. The reaction mixture was partitioned, and the obtained ethyl acetate layer was washed successively with water and saturated brine, and dried over anhydrous sodium sulfate. The solvent was evaporated to give the title compound (1.9585 g) as a yellow solid.

### step 2

### Production of 2-isopropyl-6-nitro-4H-benzo[1,4]oxazin-3-one

2-Bromo-N-(2-hydroxy-5-nitrophenyl)-3-methylbutylamide (1.9511 g) was dissolved in N,N-dimethylformamide (20 mL), potassium carbonate (1.1015 g) was added, and the mixture was stirred overnight at room temperature. Ethyl acetate and water were added to the reaction mixture, and the mixture was extracted with ethyl acetate. The obtained ethyl acetate layer was washed successively with water and saturated brine, and dried over anhydrous sodium sulfate. The solvent was evaporated to give the title compound (1.4462 g) as a yellow solid.

### step 3

### Production of 3-(2-isopropyl-6-nitro-3-oxo-2,3-dihydrobenzo[1,4]oxazin-4-yl)propionic acid methyl ester

2-Isopropyl-6-nitro-4H-benzo[1,4]oxazin-3-one (331.1 mg) was dissolved in N,N-dimethylformamide (5 mL), and potassium carbonate (392.1 mg) and methyl acrylate (0.253 mL) were added. After stirring with heating overnight at 100°C, ethyl acetate and water were added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (chloroform -methanol=97:3) to give the title compound (386.4 mg) as a yellow oil.

### step 4

### Production of 3-(2-isopropyl-6-nitro-3-oxo-2,3-dihydrobenzo[1,4]oxazin-4-yl)propionic acid

3-(2-Isopropyl-6-nitro-3-oxo-2,3-dihydrobenzo[1,4]oxazin-4-yl)propionic acid methyl ester (379.6 mg) was dissolved in methanol (4 mL), and a 2N aqueous sodium hydroxide solution (1.178 mL) was added. After stirring the mixture overnight at room temperature, the solvent was evaporated. The mixture was acidified with water and 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (chloroform -methanol=95:5) to give the title compound (277.4 mg) as pale-yellow crystals.

### Example 97

### Production of 3-(2-isopropyl-3-oxo-6-trifluoromethyl-2,3-dihydrobenzo[1,4]oxazin-4-yl)propionic acid

### step 1

### Production of 2-amino-4-trifluoromethylphenol

2-Nitro-4-trifluoromethylphenol (3.0951 g) was dissolved in tetrahydrofuran (15 mL). To this solution was added 7.5% palladium-carbon (299.8 mg), and the mixture was stirred overnight at room temperature under hydrogen atmosphere. The reaction mixture was filtered through celite, and concentrated under reduced pressure to give the title compound (2.6346 g) as a gray solid.

### step 2

### Production of 2-bromo-N-(2-hydroxy-5-trifluoromethylphenyl)-3-methylbutylamide

2-Amino-4-trifluoromethylphenol (1.0090 g) was dissolved by adding ethyl acetate (10 mL) and water (10 mL), sodium hydrogencarbonate (0.9558 g) and 2-bromo-3-methylbutyryl chloride (1.4406 g) obtained in step 1 of Example 6 were added at room temperature, and the mixture was stirred overnight at room temperature. The reaction mixture was partitioned, and the obtained ethyl acetate layer was washed successively with water and saturated brine, and dried over anhydrous sodium sulfate. The solvent was evaporated to give the title compound (2.0145 g).

### step 3

### Production of 2-isopropyl-6-trifluoromethyl-4H-benzo[1,4]oxazin-3-one

2-Bromo-N-(2-hydroxy-5-trifluoromethylphenyl)-3-methylbutylamide (2.0051 g) was dissolved in N,N-dimethylformamide (20 mL), potassium carbonate (1.0272 g) was added, and the mixture was stirred overnight at room temperature. Ethyl acetate and water were added to the reaction mixture, and the mixture was extracted with ethyl acetate. The obtained ethyl acetate layer was washed successively with water and saturated brine, and dried over anhydrous sodium sulfate. The solvent was evaporated to give the title compound (1.4858 g) as a pale-yellow solid.

### step 4

### Production of 3-(2-isopropyl-3-oxo-6-trifluoromethyl-2,3-dihydrobenzo[1,4]oxazin-4-yl)propionic acid methyl ester

2-Isopropyl-6-trifluoromethyl-4H-benzo[1,4]oxazin-3-one (338.8 mg) was dissolved in N,N-dimethylformamide (5 mL), and potassium carbonate (362.0 mg) and methyl acrylate (0.24 mL) were added. After stirring with heating overnight at 100°C, ethyl acetate and water were added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (chloroform -methanol=97:3) to give the title compound (437.4 mg) as a pale-yellow oil.

### step 5

### Production of 3-(2-isopropyl-3-oxo-6-trifluoromethyl-2,3-dihydrobenzo[1,4]oxazin-4-yl)propionic acid

3-(2-Isopropyl-3-oxo-6-trifluoromethyl-2,3-dihydrobenzo[1,4]oxazin-4-yl)propionic acid methyl ester (418.5 mg) was dissolved in methanol (4 mL), and a 2N aqueous sodium hydroxide solution (1.212 mL) was added. After stirring the mixture overnight at room temperature, the solvent was evaporated. The mixture was acidified with water and 1N hydrochloric acid, and the mixture was extracted with ethyl acetate. The obtained ethyl acetate layer was washed successively with water and saturated brine, and dried over anhydrous sodium sulfate. The solvent was evaporated to give the title compound (361.2 mg) as a pale-yellow solid.

### Example 98

### Production of 3-(8-chloro-2-isopropyl-6-methyl-3-oxo-2,3-dihydrobenzo[1,4]oxazin-4-yl)propionic acid

### step 1

### Production of 2-chloro-4-methyl-6-nitrophenol

To a mixture of 2-chloro-4-methylphenol (6.42 g) and sodium nitrite (10.4 g), were added n-hexane (75 mL) and diisopropyl ether (35 mL) and water (50 mL), 4.5N sulfuric acid (110 mL) was added dropwise at room temperature. After stirring at room temperature for 0.5 hr, the reaction mixture was partitioned, and the obtained organic layer was washed successively with water and saturated brine, and dried over anhydrous sodium sulfate. The solvent was evaporated, and the obtained residue crystallized from water to give the title compound (8.04 g) as a pale-yellow solid.

### step 2

### Production of 2-amino-6-chloro-4-methylphenol

2-Chloro-4-methyl-6-nitrophenol (4.0 g) was dissolved in ethanol (40 mL). To this solution was added platinum oxide (34 mg), and the mixture was stirred under hydrogen atmosphere at room temperature for 3 days. The reaction mixture was filtered through celite, and the filtrate was concentrated under reduced pressure. The obtained residue was purified by silica gel chromatography (n-hexane-ethyl acetate=3:1) to give the title compound (2.49 g) as a brown oil.

### step 3

### Production of 2-bromo-N-(3-chloro-2-hydroxy-5-methylphenyl)-3-methylbutylamide

2-Amino-6-chloro-4-methylphenol (630 mg) was dissolved by adding ethyl acetate (10 mL) and water (10 mL), sodium hydrogencarbonate (840 mg) and 2-bromo-3-methylbutyryl chloride (1.20 g) obtained in step 1 of Example 6 were added at room temperature, and the mixture was stirred at room temperature for 0.5 hr. The reaction mixture was partitioned, and the obtained ethyl acetate layer was washed successively with water and saturated brine, and dried over anhydrous sodium sulfate. The solvent was evaporated to give the title compound.

### step 4

### Production of 8-chloro-2-isopropyl-6-methyl-4H-benzo[1,4]oxazin-3-one

2-Bromo-N-(3-chloro-2-hydroxy-5-methylphenyl)-3-methylbutylamide obtained in step 3 was dissolved in N,N-dimethylformamide (6 mL), potassium carbonate (829 mg) was added, and the mixture was stirred at room temperature for 4 days. To the reaction mixture were added 1N hydrochloric acid and water, and the precipitated solid was collected by filtration. The obtained solid was crystallized from n-hexane to give the title compound (759 mg) as a yellow solid.

### step 5

### Production of 3-(8-chloro-2-isopropyl-6-methyl-3-oxo-2,3-dihydrobenzo[1,4]oxazin-4-yl)propionic acid methyl ester

8-Chloro-2-isopropyl-6-methyl-4H-benzo[1,4]oxazin-3-one (360 mg) was dissolved in N,N-dimethylformamide (5 mL), and potassium carbonate (415 mg) and methyl acrylate (0.27 mL) were added. After stirring with heating overnight at 80°C, ethyl acetate and water were added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=3:1) to give the title compound (489 mg) as a colorless oil.

### step 6

### Production of 3-(8-chloro-2-isopropyl-6-methyl-3-oxo-2,3-dihydrobenzo[1,4]oxazin-4-yl)propionic acid

3-(8-Chloro-2-isopropyl-6-methyl-3-oxo-2,3-dihydrobenzo[1,4]oxazin-4-yl)propionic acid methyl ester (489 mg) was dissolved in methanol (10 mL), and a 1N aqueous sodium hydroxide solution (3.0 mL) was added. After stirring the mixture at room temperature for 0.5 hr, the solvent was evaporated, water was added to the obtained residue, and the mixture was washed with ethyl acetate. The obtained aqueous layer was acidified with 1N hydrochloric acid, and the mixture was extracted with ethyl acetate. The obtained ethyl acetate layer was washed successively with water and saturated brine, and dried over anhydrous sodium sulfate. The solvent was evaporated, and the residue was crystallized from n-hexane to give the title compound (420 mg) as white crystals.

### Example 99

### Production of 3-(2-isopropyl-3-oxo-2,3-dihydronaphtho[1,2-b][1,4]oxazin-4-yl)propionic acid

### step 1

### Production of 2-bromo-N-(1-hydroxynaphthalen-2-yl)-3-methylbutylamide

2-Amino-1-naphthol hydrochloride(1.0062 g) was dissolved by adding ethyl acetate (10 mL) and water (10 mL), sodium hydrogencarbonate (1.2999 g) and 2-bromo-3-methylbutyryl chloride (1.2585 g) obtained in step 1 of Example 6 were added at room temperature, and the mixture was stirred overnight at room temperature. The reaction mixture was partitioned, and the obtained ethyl acetate layer was washed successively with water and saturated brine, and dried over anhydrous sodium sulfate. The solvent was evaporated to give the title compound (1.7268 g) as a brown solid.

### step 2

### Production of 2-isopropyl-4H-naphtho[1,2-b][1,4]oxazin-3-one

2-Bromo-N-(1-hydroxynaphthalen-2-yl)-3-methylbutylamide (1.7268 g) was dissolved in N,N-dimethylformamide (18 mL), potassium carbonate (0.9293 g) was added, and the mixture was stirred overnight at room temperature. Ethyl acetate and water were added to the reaction mixture, and the mixture was extracted with ethyl acetate. The obtained ethyl acetate layer was washed successively with water and saturated brine, and dried over anhydrous sodium sulfate. The solvent was evaporated to give the title compound (1.2357 g) as a brown solid.

### step 3

### Production of 3-(2-isopropyl-3-oxo-2,3-dihydronaphtho[1,2-b][1,4]oxazin-4-yl)propionic acid methyl ester

2-Isopropyl-4H-naphtho[1,2-b][1,4]oxazin-3-one (997.1 mg) was dissolved in N,N-dimethylformamide (10 mL), and potassium carbonate (1.1480 g) and methyl acrylate (0.75 mL) were added. After stirring with heating overnight at 85°C, ethyl acetate and water were added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=4:1) to give the title compound (1.1871 g) as an orange oil.

### step 4

### Production of 3-(2-isopropyl-3-oxo-2,3-dihydronaphtho[1,2-b][1,4]oxazin-4-yl)propionic acid

3-(2-Isopropyl-3-oxo-2,3-dihydronaphtho[1,2-b][1,4]oxazin-4-yl)propionic acid methyl ester (1.1572 g) was dissolved in methanol (10 mL), and a 2N aqueous sodium hydroxide solution (3.5 mL) was added. After stirring the mixture at room temperature for 2 hr, the solvent was evaporated. Water was added, and the mixture was washed with diethyl ether. The obtained aqueous layer was acidified with 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (chloroform -methanol=95:5), and crystallized from ethanol to give the title compound (592.1 mg) as crystals.

### Example 100

### Production of 3-(8-chloro-2-isopropyl-3-oxo-2,3-dihydrobenzo[1,4]oxazin-4-yl)propionic acid

### step 1

### Production of 2-amino-6-chlorophenol

2-Chloro-6-nitrophenol (1.0023 g) was dissolved in ethanol (10 mL). To this solution was added platinum oxide (12 mg), and the mixture was stirred under hydrogen atmosphere at room temperature for 7 hr. The reaction mixture was filtered through celite, concentrated under reduced pressure, and the obtained residue was purified by silica gel chromatography (chloroform - methanol=95:5) to give the title compound (615.3 mg) as a brown solid.

### step 2

### Production of 2-bromo-N-(3-chloro-2-hydroxyphenyl)-3-methylbutylamide

2-Amino-6-chlorophenol (615.3 mg) was dissolved by adding ethyl acetate (6 mL) and water (6 mL), sodium hydrogencarbonate (124.5 mg) and 2-bromo-3-methylbutyryl chloride (1.1329 g) obtained in step 1 of Example 6 were added at room temperature, and the mixture was stirred overnight at room temperature. The reaction mixture was partitioned, and the obtained ethyl acetate layer was washed successively with water and saturated brine, and dried over anhydrous sodium sulfate. The solvent was evaporated to give the title compound (1.4579 g) as a brown oil.

### step 3

### Production of 8-chloro-2-isopropyl-4H-benzo[1,4]oxazin-3-one

2-Bromo-N-(3-chloro-2-hydroxyphenyl)-3-methylbutylamide (1.3663 g) was dissolved in N,N-dimethylformamide (15 mL), and potassium carbonate (801.3 mg) was added, and the mixture was stirred overnight at room temperature. Ethyl acetate and water were added to the reaction mixture, and the mixture was extracted with ethyl acetate. The obtained ethyl acetate layer was washed successively with water and saturated brine, and dried over anhydrous sodium sulfate. The solvent was evaporated to give the title compound (943.5 mg) as an ocher solid.

### step 4

### Production of 3-(8-chloro-2-isopropyl-3-oxo-2,3-dihydrobenzo[1,4]oxazin-4-yl)propionic acid methyl ester

8-Chloro-2-isopropyl-4H-benzo[1,4]oxazin-3-one (927.8 mg) was dissolved in N,N-dimethylformamide (10 mL), and potassium carbonate (1.1404 g) and methyl acrylate (0.75 mL) were added. After stirring with heating overnight at 85°C, ethyl acetate and water were added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=4:1) to give the title compound (1.2007 g) as a yellow oil.

### step 5

### Production of 3-(8-chloro-2-isopropyl-3-oxo-2,3-dihydrobenzo[1,4]oxazin-4-yl)propionic acid

3-(8-Chloro-2-isopropyl-3-oxo-2,3-dihydrobenzo[1,4]oxazin-4-yl)propionic acid methyl ester (1.1813 g) was dissolved in methanol (10 mL), and a 2N aqueous sodium hydroxide solution (3.8 mL) was added. After stirring the mixture overnight at room temperature, the solvent was evaporated. Water was added, and the mixture was washed with diethyl ether. The obtained aqueous layer was acidified with 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (chloroform -methanol=95:5) and crystallized from n-hexane-ethanol to give the title compound (804.3 mg) as white crystals.

### Example 101

### Production of 3-(6-ethyl-2-isopropyl-8-methyl-3-oxo-2,3-dihydrobenzo[1,4]oxazin-4-yl)propionic acid

### step 1

### Production of 4-ethyl-2-methylphenol

4-Acetyl-2-methylphenol (10.0 g) was dissolved in methanol (100 mL), and concentrated hydrochloric acid (10 drops) was added. To this solution was added palladium hydroxide (1.5 g), and the mixture was stirred under hydrogen atmosphere (3.8 kgf/cm²) at room temperature for 3.5 hr. The reaction mixture was filtered through celite, and concentrated under reduced pressure to give the title compound (9.2 g) as a pale-yellow oil.

### step 2

### Production of 4-ethyl-2-methyl-6-nitrophenol

To a mixture of 4-ethyl-2-methylphenol (9.2 g) and sodium nitrite (13.8 g), were added n-hexane (97 mL), diisopropyl ether (42 mL) and water (69 mL) and 4.5N sulfuric acid (140 mL) was added dropwise. After stirring the mixture at room temperature, the reaction mixture was extracted with diisopropyl ether. The obtained organic layer was washed successively with water and saturated brine, and dried over anhydrous sodium sulfate. The solvent was evaporated, and the obtained residue was purified by silica gel chromatography (n-hexane-ethyl acetate=9:1) to give the title compound (10.6 g) as a yellow solid.

### step 3

### Production of 2-amino-4-ethyl-6-methylphenol

4-Ethyl-2-methyl-6-nitrophenol (5.0 g) was dissolved in methanol (50 mL). To this solution was added 7.5% palladium-carbon (800 mg), and the mixture was stirred under hydrogen atmosphere overnight at room temperature. The reaction mixture was filtered through celite, and concentrated under reduced pressure to give the title compound (4.17 g) as an ocher solid.

### step 4

### Production of 2-bromo-N-(5-ethyl-2-hydroxy-3-methylphenyl)-3-methylbutylamide

2-Amino-4-ethyl-6-methylphenol (1.0 g) was dissolved by adding ethyl acetate (15 mL) and water (15 mL), sodium hydrogencarbonate (1.11 g) and 2-bromo-3-methylbutyryl chloride (1.58 g) obtained in step 1 of Example 6 were added at room temperature, and the mixture was stirred at room temperature for 1.5 hr. The reaction mixture was partitioned, and the obtained ethyl acetate layer was washed successively with water and saturated brine, and dried over anhydrous sodium sulfate. The solvent was evaporated to give the title compound as a yellow solid.

### step 5

### Production of 6-ethyl-2-isopropyl-8-methyl-4H-benzo[1,4]oxazin-3-one

2-Bromo-N-(5-ethyl-2-hydroxy-3-methylphenyl)-3-methylbutylamide obtained in step 4 was dissolved in N,N-dimethylformamide (15 mL), potassium carbonate (1.37 g) was added, and the mixture was stirred overnight at room temperature. Water was added to the reaction mixture, and the precipitated solid was collected by filtration to give the title compound (1.44 g) as an ocher solid.

### step 6

### Production of 3-(6-ethyl-2-isopropyl-8-methyl-3-oxo-2,3-dihydrobenzo[1,4]oxazin-4-yl)propionic acid methyl ester

6-Ethyl-2-isopropyl-8-methyl-4H-benzo[1,4]oxazin-3-one (400 mg) was dissolved in N,N-dimethylformamide (5 mL), and potassium carbonate (473 mg) and methyl acrylate (0.309 mL) were added. After stirring with heating overnight at 90°C, a 10% aqueous citric acid solution was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=6:1) to give the title compound (510 mg) as a pale-yellow oil.

### step 7

### Production of 3-(6-ethyl-2-isopropyl-8-methyl-3-oxo-2,3-dihydrobenzo[1,4]oxazin-4-yl)propionic acid

3-(6-Ethyl-2-isopropyl-8-methyl-3-oxo-2,3-dihydrobenzo[1,4]oxazin-4-yl)propionic acid methyl ester (505 mg) was dissolved in methanol (7 mL), and a 2N aqueous sodium hydroxide solution (1.58 mL) was added. After stirring the mixture overnight at room temperature, the solvent was evaporated. The mixture was acidified with water and 1N hydrochloric acid, and extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (433 mg) as a white solid.

### Example 102

### Production of 3-(6,8-dimethyl-3-oxo-2-propyl-2,3-dihydrobenzo[1,4]oxazin-4-yl)propionic acid

### step 1

### Production of 2-bromopentanoylchloride

2-Bromovaleric acid (5.0666 g) was dissolved in chloroform (25 mL), and oxalyl chloride (4.8 mL) and N,N-dimethylformamide (2 drops) were added under ice-cooling. After stirring the mixture at room temperature, the mixture was concentrated to give the title compound (5.5392 g).

### step 2

### Production of 2-bromo-N-(2-hydroxy-3,5-dimethylphenyl)valeramide

2-Amino-4,6-dimethylphenol (1.0032 g) was dissolved by adding ethyl acetate (10 mL) and water (10 mL), sodium hydrogencarbonate (1.2381 g) and 2-bromopentanoylchloride (1.7713 g) obtained in step 1 were added under ice-cooling, and the mixture was stirred at room temperature for 0.5 hr. The reaction mixture was partitioned, and the obtained ethyl acetate layer was washed successively with water and saturated brine, and dried over anhydrous sodium sulfate. The solvent was evaporated to give the title compound (2.368 g) as a brown oil.

### step 3

### Production of 6,8-dimethyl-2-propyl-4H-benzo[1,4]oxazin-3-one

2-Bromo-N-(2-hydroxy-3,5-dimethylphenyl)valeramide (2.309 g) was dissolved in N,N-dimethylformamide (20 mL), potassium carbonate (1.3141 g) was added, and the mixture was stirred overnight at room temperature. To the reaction mixture were added ethyl acetate (30 mL) and water (30 mL), and the mixture was extracted with ethyl acetate. The obtained ethyl acetate layer was washed successively with water and saturated brine, and dried over anhydrous sodium sulfate. The solvent was evaporated, and the residue was crystallized from n-hexane-ethyl acetate to give the title compound (1.0768 g) as a brown solid.

### step 4

### Production of 3-(6,8-dimethyl-3-oxo-2-propyl-2,3-dihydrobenzo[1,4]oxazin-4-yl)propionic acid methyl ester

6,8-Dimethyl-2-propyl-4H-benzo[1,4]oxazin-3-one (461.8 mg) was dissolved in N,N-dimethylformamide (5 mL), and potassium carbonate (586.5 mg) and methyl acrylate (0.38 mL) were added. After stirring with heating overnight at 85°C, to the reaction mixture were added ethyl acetate (15 mL) and water (15 mL), and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=4:1) to give the title compound (504.6 mg) as an orange oil.

### step 5

### Production of 3-(6,8-dimethyl-3-oxo-2-propyl-2,3-dihydrobenzo[1,4]oxazin-4-yl)propionic acid

3-(6,8-Dimethyl-3-oxo-2-propyl-2,3-dihydrobenzo[1,4]oxazin-4-yl)propionic acid methyl ester (483.1 mg) was dissolved in methanol (5 mL), and a 2N aqueous sodium hydroxide solution (1.5 mL) was added. After stirring the mixture overnight at room temperature, the solvent was evaporated. Water was added, and the mixture was washed with diethyl ether. The obtained aqueous layer was acidified with 1N hydrochloric acid, and the mixture was extracted with ethyl acetate. The obtained ethyl acetate layer was washed successively with water and saturated brine, and dried over anhydrous sodium sulfate. The solvent was evaporated to give the title compound (461.1 mg) as pale-pink crystals.

### Example 103

### Production of 3-[2-isopropyl-3-oxo-6,8-bis(trifluoromethyl)-2,3-dihydrobenzo[1,4]oxazin-4-yl]propionic acid

### step 1

### Production of 2-bromo-N-[2-hydroxy-3,5-bis(trifluoromethyl)phenyl]-3-methylbutylamide

2-Amino-4,6-bis(trifluoromethyl)phenol (1.0033 g) was dissolved by adding ethyl acetate (10 mL) and water (10 mL), sodium hydrogencarbonate (689.2 mg) and 2-bromo-3-methylbutyryl chloride (1.0374 g) obtained in step 1 of Example 6 were added at room temperature, and the mixture was stirred overnight at room temperature. The reaction mixture was partitioned, and the obtained ethyl acetate layer was washed successively with water and saturated brine, and dried over anhydrous sodium sulfate. The solvent was evaporated to give the title compound (2.0234 g).

### step 2

### Production of 2-isopropyl-6,8-bis(trifluoromethyl)-4H-benzo[1,4]oxazin-3-one

2-Bromo-N-[2-hydroxy-3,5-bis(trifluoromethyl)phenyl]-3-methylbutylamide (2.0234 g) was dissolved in N,N-dimethylformamide (15 mL), potassium carbonate (740 mg) was added, and the mixture was stirred at room temperature for 3 hr. To the reaction mixture were added ethyl acetate (20 mL) and water (20 mL), and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=4:1) to give the title compound (1.1993 g) as a yellow solid.

### step 3

### Production of 3-[2-isopropyl-3-oxo-6,8-bis(trifluoromethyl)-2,3-dihydrobenzo[1,4]oxazin-4-yl]propionic acid methyl ester

2-Isopropyl-6,8-bis(trifluoromethyl)-4H-benzo[1,4]oxazin-3-one (575.6 mg) was dissolved in N,N-dimethylformamide (5 mL), and potassium carbonate (486.5 mg) and methyl acrylate (0.32 mL) were added. After stirring with heating overnight at 85°C, ethyl acetate and water were added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=4:1) to give the title compound (431.3 mg) as a yellow oil.

### step 4

### Production of 3-[2-isopropyl-3-oxo-6,8-bis(trifluoromethyl)-2,3-dihydrobenzo[1,4]oxazin-4-yl]propionic acid

3-[2-Isopropyl-3-oxo-6,8-bis(trifluoromethyl)-2,3-dihydrobenzo[1,4]oxazin-4-yl]propionic acid methyl ester (414.6 mg) was dissolved in methanol (4 mL), and a 2N aqueous sodium hydroxide solution (1 mL) was added. After stirring the mixture at room temperature for 2 hr, the solvent was evaporated, water (15 mL) was added, and the mixture was washed with diethyl ether. The obtained aqueous layer was acidified with 1N hydrochloric acid, and the mixture was extracted with ethyl acetate. The obtained ethyl acetate layer was washed successively with water and saturated brine, and dried over anhydrous sodium sulfate. The solvent was evaporated to give the title compound (363.6 mg) as a white solid.

### Example 104

### Production of 3-(2-isopropyl-6-methoxy-8-methyl-3-oxo-2,3-dihydrobenzo[1,4]oxazin-4-yl)propionic acid

### step 1

### Production of 2-hydroxymethyl-4-methoxy-6-nitrophenol

2-Hydroxy-5-methoxy-3-nitrobenzaldehyde (2.0 g) was suspended in methanol (20 mL) and tetrahydrofuran (20 mL), and sodium borohydride (384 mg) was added under ice-cooling. After stirring the mixture at room temperature, water and 1N hydrochloric acid were added to the reaction mixture. The precipitated solid was collected by filtration to give the title compound (1.87 g) as a yellow solid. The filtrate was extracted with ethyl acetate. The obtained ethyl acetate layer was washed successively with water and saturated brine, and dried over anhydrous sodium sulfate. The solvent was further evaporated to give the title compound (147 mg) as a yellow solid.

### step 2

### Production of 2-amino-6-hydroxymethyl-4-methoxyphenol

2-Hydroxymethyl-4-methoxy-6-nitrophenol (2.01 g) was dissolved in methanol (20 mL) and tetrahydrofuran (20 mL). To this solution was added 7.5% palladium-carbon (400 mg), and the mixture was stirred overnight under hydrogen atmosphere at room temperature. The reaction mixture was filtered through celite, and concentrated under reduced pressure to give the title compound as a dark brown solid.

### step 3

### Production of 2-bromo-N-(2-hydroxy-3-hydroxymethyl-5-methoxyphenyl)-3-methylbutylamide

2-Amino-6-hydroxymethyl-4-methoxyphenol (765 mg) was dissolved by adding ethyl acetate (10 mL), water (10 mL) and tetrahydrofuran (5 mL), sodium hydrogencarbonate (760 mg) and 2-bromo-3-methylbutyryl chloride (902 mg) obtained in step 1 of Example 6 were added at room temperature, and the mixture was stirred overnight at room temperature. The reaction mixture was partitioned, and the obtained ethyl acetate layer was washed successively with water and saturated brine, and dried over anhydrous sodium sulfate. The solvent was evaporated to give the title compound.

### step 4

### Production of 8-hydroxymethyl-2-isopropyl-6-methoxy-4H-benzo[1,4]oxazin-3-one

2-Bromo-N-(2-hydroxy-3-hydroxymethyl-5-methoxyphenyl)-3-methylbutylamide obtained in step 3 was dissolved in N,N-dimethylformamide (10 mL), potassium carbonate (934 mg) was added, and the mixture was stirred at room temperature for 6 hr. A 10% aqueous citric acid solution was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=1:1) to give the title compound (890 mg) as a brown oil.

### step 5

### Production of 8-acetoxymethyl-2-isopropyl-6-methoxy-4H-benzo[1,4]oxazin-3-one

8-Hydroxymethyl-2-isopropyl-6-methoxy-4H-benzo[1,4]oxazin-3-one was dissolved in chloroform (10 mL), pyridine (0.852 mL) and acetic anhydride (0.798 mL) were added, and the mixture was stirred overnight at room temperature. Water was added to the reaction mixture, and the mixture was extracted with chloroform. The obtained chloroform layer was washed successively with 1N hydrochloric acid and saturated brine, and dried over anhydrous sodium sulfate. The solvent was evaporated to give the title compound (1.13 g) as an orange oil.

### step 6

### Production of 2-isopropyl-6-methoxy-8-methyl-4H-benzo[1,4]oxazin-3-one

8-Acetoxymethyl-2-isopropyl-6-methoxy-4H-benzo[1,4]oxazin-3-one (1.13 g) was dissolved in methanol (20 mL). To this solution was added palladium hydroxide (150 mg), and the mixture was stirred under hydrogen atmosphere (3 kgf/cm²) at room temperature for 4 hr. The reaction mixture was filtered through celite and concentrated under reduced pressure. The obtained residue was purified by silica gel chromatography (n-hexane-ethyl acetate=3:1) to give the title compound (779 mg) as a cream-colored solid.

### step 7

### Production of 3-(2-isopropyl-6-methoxy-8-methyl-3-oxo-2,3-dihydrobenzo[1,4]oxazin-4-yl)propionic acid methyl ester

2-Isopropyl-6-methoxy-8-methyl-4H-benzo[1,4]oxazin-3-one (300 mg) was dissolved in N,N-dimethylformamide (5 mL), and potassium carbonate (352 mg) and methyl acrylate (0.230 mL) were added. After stirring with heating at 90°C for 11 hr, to the reaction mixture were added a 10% aqueous citric acid solution and water, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=4:1) to give the title compound (361 mg) as a colorless oil.

### step 8

### Production of 3-(2-isopropyl-6-methoxy-8-methyl-3-oxo-2,3-dihydrobenzo[1,4]oxazin-4-yl)propionic acid

3-(2-Isopropyl-6-methoxy-8-methyl-3-oxo-2,3-dihydrobenzo[1,4]oxazin-4-yl)propionic acid methyl ester (355 mg) was dissolved in methanol (6 mL), and a 2N aqueous sodium hydroxide solution (1.10 mL) was added. After stirring at room temperature for 4.5 hr, the solvent was evaporated. The residue was acidified with water and 1N hydrochloric acid, and the mixture was extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (298 mg) as a white solid.

### Example 105

### Production of 3-[(R)-2-((S)-sec-butyl)-6,8-dimethyl-3-oxo-2,3-dihydrobenzo[1,4]oxazin-4-yl]propionic acid

### step 1

### Production of (2S,3S)-2-chloro-3-methylpentanoylchloride

(2S,3S)-2-Chloro-3-methylvaleric acid (5 g) was dissolved in chloroform (25 mL), and oxalyl chloride (3.74 mL) and N,N-dimethylformamide (one drop) were added under ice-cooling. After stirring the mixture at room temperature, the mixture was concentrated and azeotroped with chloroform and toluene to give the title compound (5.775 g).

### step 2

### Production of (2S,3S)-2-chloro-N-(2-hydroxy-3,5-dimethylphenyl)-3-methylvaleramide

2-Amino-4,6-dimethylphenol (3.794 g) was dissolved by adding ethyl acetate (38 mL) and water (38 mL), sodium hydrogencarbonate (4.649 g) and (2S,3S)-2-chloro-3-methylpentanoylchloride (5.775 g) obtained in step 1 were added at room temperature, and the mixture was stirred at room temperature for 0.5 hr. The reaction mixture was partitioned, and the obtained ethyl acetate layer was washed successively with water and saturated brine, and dried over anhydrous sodium sulfate. The solvent was evaporated to give the title compound (7.844 g) as a brown oil.

### step 3

### Production of (R)-2-((S)-sec-butyl)-6,8-dimethyl-4H-benzo[1,4]oxazin-3-one

((2S,3S)-2-Chloro-N-(2-hydroxy-3,5-dimethylphenyl)-3-methylvaleramide (7.84 g) was dissolved in N,N-dimethylformamide (78 mL), potassium carbonate (5.346 g) was added, and the mixture was stirred at room temperature. Water (200 mL) was added to the reaction mixture, and the mixture was extracted with diethyl ether. The obtained diethyl ether layer was washed successively with water and saturated brine, and dried over anhydrous sodium sulfate. The solvent was evaporated to give the title compound (6.034 g).

### step 4

### Production of 3-[(R)-2-((S)-sec-butyl)-6,8-dimethyl-3-oxo-2,3-dihydrobenzo[1,4]oxazin-4-yl]propionic acid methyl ester

(R)-2-((S)-sec-Butyl)-6,8-dimethyl-4H-benzo[1,4]oxazin-3-one (3.025 g) was dissolved in N,N-dimethylformamide (50 mL), and potassium carbonate (3.593 g) and methyl acrylate (2.34 mL) were added. After stirring with heating at 90°C for 12 hr, a 10% aqueous citric acid solution was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=9:1) to give the title compound (3.134 g) as a pale-orange oil.

### step 5

### Production of 3-[(R)-2-((S)-sec-butyl)-6,8-dimethyl-3-oxo-2,3-dihydrobenzo[1,4]oxazin-4-yl]propionic acid

3-[(R)-2-((S)-sec-Butyl)-6,8-dimethyl-3-oxo-2,3-dihydrobenzo[1,4]oxazin-4-yl]propionic acid methyl ester (3.12 g) was dissolved in methanol (30 mL), and a 4N aqueous sodium hydroxide solution (4.88 mL) was added. After stirring at room temperature for 3 hr, the solvent was evaporated. Water was added, and the mixture was washed with diethyl ether. The obtained aqueous layer was acidified with 1N hydrochloric acid, and the mixture was extracted with ethyl acetate. The obtained ethyl acetate layer was washed successively with water and saturated brine, and dried over anhydrous sodium sulfate. The solvent was evaporated to give the title compound (3.009 g) as a pale-brown oil.

### Example 106

### Production of 3-((R)-8-chloro-2-isopropyl-6-methyl-3-oxo-2,3-dihydrobenzo[1,4]oxazin-4-yl)propionic acid

### step 1

### Production of (S)-2-chloro-3-methylbutyryl chloride

(S)-2-Chloro-3-methylbutyric acid (20 g) was dissolved in chloroform (100 mL), N,N-dimethylformamide (4 drops) and oxalyl chloride (16.6 mL) were added, and the mixture was stirred overnight at room temperature. The solvent was evaporated, and the residue was azeotroped with chloroform to give the title compound (20.2 g) as a yellow oil.

### step 2

### Production of (S)-2-chloro-N-(3-chloro-2-hydroxy-5-methylphenyl)-3-methylbutylamide

2-Amino-6-chloro-4-methylphenol (1.80 g) obtained in step 2 of Example 98 was dissolved by adding ethyl acetate (25 mL) and water (25 mL), sodium hydrogencarbonate (2.39 g) and (S)-2-chloro-3-methylbutyryl chloride (2.65 g) obtained in step 1 were added at room temperature, and the mixture was stirred at room temperature for 2 hr. The reaction mixture was partitioned, and the obtained ethyl acetate layer was washed successively with water and saturated brine, and dried over anhydrous sodium sulfate. The solvent was evaporated to give the title compound.

### step 3

### Production of (R)-8-chloro-2-isopropyl-6-methyl-4H-benzo[1,4]oxazin-3-one

(S)-2-Chloro-N-(3-chloro-2-hydroxy-5-methylphenyl)-3-methylbutylamide obtained in step 2 was dissolved in N,N-dimethylformamide (12 mL), potassium carbonate (2.36 g) was added, and the mixture was stirred at room temperature for 2 days. Water and 1N hydrochloric acid were added to the reaction mixture, and the precipitated solid was collected by filtration, and crystallized from n-hexane to give the title compound (2.13 g) as a yellow solid.

### step 4

### Production of 3-((R)-8-chloro-2-isopropyl-6-methyl-3-oxo-2,3-dihydrobenzo[1,4]oxazin-4-yl)propionic acid methyl ester

(R)-8-Chloro-2-isopropyl-6-methyl-4H-benzo[1,4]oxazin-3-one (2.13 g) was dissolved in N,N-dimethylformamide (20 mL), and potassium carbonate (2.46 g) and methyl acrylate (1.60 mL) were added. After stirring with heating overnight at 80°C, water was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=3:1) to give the title compound (2.49 g) as a colorless oil.

### step 5

### Production of 3-((R)-8-chloro-2-isopropyl-6-methyl-3-oxo-2,3-dihydrobenzo[1,4]oxazin-4-yl)propionic acid

3-((R)-8-Chloro-2-isopropyl-6-methyl-3-oxo-2,3-dihydrobenzo[1,4]oxazin-4-yl)propionic acid methyl ester (2.49 g) was dissolved in methanol (35 mL), and a 1N aqueous sodium hydroxide solution (11.5 mL) was added. After stirring at room temperature for 3 days, the solvent was evaporated, water was added, and the mixture was washed with ethyl acetate. The obtained aqueous layer was acidified with 1N hydrochloric acid, and extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (2.09 g) as white crystals.

### Example 107

### Production of 3-((R)-6-chloro-2-isopropyl-8-methyl-3-oxo-2,3-dihydrobenzo[1,4]oxazin-4-yl)propionic acid

### step 1

### Production of (S)-2-chloro-N-(5-chloro-2-hydroxy-3-methylphenyl)-3-methylbutylamide

2-Amino-4-chloro-6-methylphenol (1.40 g) obtained in step 2 of Example 81 was dissolved by adding ethyl acetate (20 mL) and water (20 mL), sodium hydrogencarbonate (1.86 g) and (S)-2-chloro-3-methylbutyryl chloride (2.06 g) obtained in step 1 of Example 106 were added at room temperature, and the mixture was stirred at room temperature for 2 hr. The reaction mixture was partitioned, and the obtained ethyl acetate layer was washed successively with water and saturated brine, and dried over anhydrous sodium sulfate. The solvent was evaporated to give the title compound.

### step 2

### Production of (R)-6-chloro-2-isopropyl-8-methyl-4H-benzo[1,4]oxazin-3-one

(S)-2-Chloro-N-(5-chloro-2-hydroxy-3-methylphenyl)-3-methylbutylamide obtained in step 1 was dissolved in N,N-dimethylformamide (10 mL), potassium carbonate (1.84 g) was added, and the mixture was stirred at room temperature for 2 days. Water and 1N hydrochloric acid were added to the reaction mixture, and the precipitated solid was collected by filtration to give the title compound (1.58 g) as a yellow solid.

### step 3

### Production of 3-((R)-6-chloro-2-isopropyl-8-methyl-3-oxo-2,3-dihydrobenzo[1,4]oxazin-4-yl)propionic acid methyl ester

(R)-6-Chloro-2-isopropyl-8-methyl-4H-benzo[1,4]oxazin-3-one (1.58 g) was dissolved in N,N-dimethylformamide (15 mL), and potassium carbonate (1.82 g) and methyl acrylate (1.19 mL) were added. After stirring with heating overnight at 80°C, water was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=3:1) to give the title compound (2.08 g) as a colorless oil.

### step 4

### Production of 3-((R)-6-chloro-2-isopropyl-8-methyl-3-oxo-2,3-dihydrobenzo[1,4]oxazin-4-yl)propionic acid

3-((R)-6-Chloro-2-isopropyl-8-methyl-3-oxo-2,3-dihydrobenzo[1,4]oxazin-4-yl)propionic acid methyl ester (2.08 g) was dissolved in methanol (30 mL), and a 1N aqueous sodium hydroxide solution (9.57 mL) was added. After stirring at room temperature for 3 days, the solvent was evaporated, water was added, and the mixture was washed with ethyl acetate. The obtained aqueous layer was acidified with 1N hydrochloric acid, and extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (1.78 g) as a white solid.

### step 5

### Production of 3-((R)-6-chloro-2-isopropyl-8-methyl-3-oxo-2,3-dihydrobenzo[1,4]oxazin-4-yl)propionic acid· (R)-1-phenylethylamine salt

3-((R)-6-Chloro-2-isopropyl-8-methyl-3-oxo-2,3-dihydrobenzo[1,4]oxazin-4-yl)propionic acid (1.45 g) was dissolved in ethyl acetate (29 mL), (R)-1-phenylethylamine (563 mg) was added, and the mixture was heated under reflux. After allowing to cool to room temperature, the precipitated solid was collected by filtration to give the title compound (1.68 g) as a white solid.

### step 6

### Production of 3-((R)-6-chloro-2-isopropyl-8-methyl-3-oxo-2,3-dihydrobenzo[1,4]oxazin-4-yl)propionic acid

To 3-((R)-6-chloro-2-isopropyl-8-methyl-3-oxo-2,3-dihydrobenzo[1,4]oxazin-4-yl)propionic acid· (R)-1-phenylethylamine salt (1.60 g) were added ethyl acetate (30 mL) and 1N hydrochloric acid (30 mL). After stirring the mixture at room temperature, the mixture was extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (1.13 g) as white crystals.

### Example 108

### Production of 3-((R)-6,8-dichloro-2-isopropyl-3-oxo-2,3-dihydrobenzo[1,4]oxazin-4-yl)propionic acid

### step 1

### Production of (S)-2-chloro-N-(3,5-dichloro-2-hydroxyphenyl)-3-methylbutylamide

2-Amino-4,6-dichlorophenol hydrochloride (2.5 g) obtained in step 1 of Example 80 was dissolved by adding ethyl acetate (30 mL) and tetrahydrofuran (20 mL) and water (30 mL), sodium hydrogencarbonate (2.95 g) and (S)-2-chloro-3-methylbutyryl chloride (2.17 g) obtained in step 1 of Example 106 were added at room temperature, and the mixture was stirred at room temperature. The reaction mixture was partitioned, and the obtained ethyl acetate layer was washed successively with water and saturated brine, and dried over anhydrous sodium sulfate. The solvent was evaporated, and the obtained residue was purified by silica gel chromatography (n-hexane-ethyl acetate=6:1) to give the title compound (2.52 g) as a cream-colored solid.

### step 2

### Production of (R)-6,8-dichloro-2-isopropyl-4H-benzo[1,4]oxazin-3-one

(S)-2-Chloro-N-(3,5-dichloro-2-hydroxyphenyl)-3-methylbutylamide (2.52 g) was dissolved in N,N-dimethylformamide (25 mL), potassium carbonate (1.76 g) was added, and the mixture was stirred overnight at room temperature. Water was added to the reaction mixture, and the precipitated solid was collected by filtration to give the title compound (1.99 g) as a pale-pink solid.

### step 3

### Production of 3-((R)-6,8-dichloro-2-isopropyl-3-oxo-2,3-dihydrobenzo[1,4]oxazin-4-yl)propionic acid methyl ester

(R)-6,8-Dichloro-2-isopropyl-4H-benzo[1,4]oxazin-3-one (1.94 g) was dissolved in N,N-dimethylformamide (20 mL), and potassium carbonate (2.06 g) and methyl acrylate (1.34 mL) were added. After stirring with heating overnight at 90°C, 10% aqueous citric acid solution and water were added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=6:1) to give the title compound (2.63 g) as a pale-yellow oil.

### step 4

### Production of 3-((R)-6,8-dichloro-2-isopropyl-3-oxo-2,3-dihydrobenzo[1,4]oxazin-4-yl)propionic acid

3-((R)-6,8-Dichloro-2-isopropyl-3-oxo-2,3-dihydrobenzo[1,4]oxazin-4-yl)propionic acid methyl ester (2.63 g) was dissolved in methanol (25 mL), and a 2N aqueous sodium hydroxide solution (7.6 mL) was added. After stirring the mixture overnight at room temperature, the solvent was evaporated. Water was added, and the mixture was washed with diethyl ether. The obtained aqueous layer was acidified with 1N hydrochloric acid, and extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (2.05 g) as a white solid.

### step 5

### Production of 3-((R)-6,8-dichloro-2-isopropyl-3-oxo-2,3-dihydrobenzo[1,4]oxazin-4-yl)propionic acid· (R)-1-phenylethylamine salt

3-((R)-6,8-Dichloro-2-isopropyl-3-oxo-2,3-dihydrobenzo[1,4]oxazin-4-yl)propionic acid (2.01 g) was dissolved in ethyl acetate (50 mL), (R)-1-phenylethylamine (0.772 mL) was added, and the mixture was heated under reflux. After confirmation that a uniform solution was obtained, the mixture was allowed to cool to room temperature. The precipitated solid was collected by filtration, and the white solid (1.87 g) was recrystallized from ethyl acetate (47 mL). The precipitated solid was collected by filtration to give the title compound (1.44 g) as a white solid.

### step 6

### Production of 3-((R)-6,8-dichloro-2-isopropyl-3-oxo-2,3-dihydrobenzo[1,4]oxazin-4-yl)propionic acid

Ethyl acetate (15 mL), 1N hydrochloric acid (6.26 mL) and water were added to 3-((R)-6,8-dichloro-2-isopropyl-3-oxo-2,3-dihydrobenzo[1,4]oxazin-4-yl)propionic acid· (R)-1-phenylethylamine salt (1.42 g). After stirring the mixture at room temperature, the mixture was extracted with ethyl acetate. The obtained ethyl acetate layer was washed successively with water and saturated brine, and dried over anhydrous sodium sulfate. The solvent was evaporated, and the obtained residue was crystallized from n-hexane-ethyl acetate to give the title compound (990 mg) as white crystals.

### Example 109

### Production of 3-((S)-8-chloro-2-isopropyl-6-methyl-3-oxo-2,3-dihydrobenzo[1,4]oxazin-4-yl)propionic acid

### step 1

### Production of (R)-2-bromo-3-methylbutyryl chloride

(R)-2-Bromo-3-methylbutyric acid (2 g) was dissolved in chloroform (10 mL), N,N-dimethylformamide (one drop) and oxalyl chloride (1.26 mL) were added under ice-cooling, and the mixture was stirred overnight at room temperature. The solvent was evaporated, and the obtained residue was azeotroped with chloroform to give the title compound (1.1303 g) as a yellow oil.

### step 2

### Production of 2-chloro-4-methyl-6-nitrophenol

To 2-chloro-4-methylphenol (60.0 g) were added diisopropyl ether (300 mL) and 9N sulfuric acid (335 mL). Sodium nitrite (90.0 g) dissolved in water (240 mL) was added dropwise at room temperature over 1 hr. After stirring the mixture at room temperature for 1 hr, the reaction mixture was washed successively with water and saturated brine, and dried over anhydrous magnesium sulfate. The solvent was evaporated, and the obtained residue was crystallized from methanol (200 mL) and water (600 mL) and stirred under ice-cooling for 1 hr and the crystals were collected by filtration to give the title compound (73.6 g).

### step 3

### Production of 2-amino-6-chloro-4-methylphenol hydrochloride

2-Chloro-4-methyl-6-nitrophenol (73.5 g) was dissolved in ethanol (700 mL). To this solution was added platinum oxide (1.37 g), and the mixture was stirred under hydrogen atmosphere at room temperature for 20 hr. The reaction mixture was filtered through celite, and the obtained filtrate was concentrated under reduced pressure to a liquid amount of about 500 mL. To the obtained residue were added 4N hydrogen chloride (ethyl acetate solution)(110 mL) and n-hexane (500 mL) and the precipitated solid was collected by filtration to give the title compound (61.5 g).

### step 4

### Production of (R)-2-bromo-N-(3-chloro-2-hydroxy-5-methylphenyl)-3-methylbutylamide

2-Amino-6-chloro-4-methylphenol hydrochloride (100 mg) was dissolved by adding ethyl acetate (0.5 mL) and water (2.5 mL) and sodium hydrogencarbonate (105 mg) and a solution of (R)-2-bromo-3-methylbutyryl chloride (106 mg) obtained in step 1 in ethyl acetate (2.0 mL) were added under ice-cooling and the mixture was stirred at room temperature for 5 hr. The reaction mixture was partitioned, and the obtained ethyl acetate layer was washed successively with water and saturated brine, and dried over anhydrous sodium sulfate. The solvent was evaporated to give the title compound (168.5 mg) as a beige solid.

### step 5

### Production of (S)-8-chloro-2-isopropyl-6-methyl-4H-benzo[1,4]oxazin-3-one

(R)-2-Bromo-N-(3-chloro-2-hydroxy-5-methylphenyl)-3-methylbutylamide (166 mg) was dissolved in N,N-dimethylformamide (3 mL), potassium carbonate (93 mg) was added, and the mixture was stirred at room temperature for 2 days. Water was added to the reaction mixture, and the precipitated solid was collected by filtration to give the title compound (108.5 mg) as a white solid.

### step 6

### Production of 3-((S)-8-chloro-2-isopropyl-6-methyl-3-oxo-2,3-dihydrobenzo[1,4]oxazin-4-yl)propionic acid methyl ester

(S)-8-Chloro-2-isopropyl-6-methyl-4H-benzo[1,4]oxazin-3-one (70 mg) was dissolved in N,N-dimethylformamide (1 mL), and potassium carbonate (81 mg) and methyl acrylate (0.053 mL) were added. After stirring with heating overnight at 90°C, a 10% aqueous citric acid solution was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=6:1) to give the title compound (87.5 mg) as a colorless oil.

### step 7

### Production of 3-((S)-8-chloro-2-isopropyl-6-methyl-3-oxo-2,3-dihydrobenzo[1,4]oxazin-4-yl)propionic acid

3-((S)-8-Chloro-2-isopropyl-6-methyl-3-oxo-2,3-dihydrobenzo[1,4]oxazin-4-yl)propionic acid methyl ester (85 mg) was dissolved in methanol (3 mL), and a 2N aqueous sodium hydroxide solution (0.26 mL) was added. After stirring the mixture overnight at room temperature, the solvent was evaporated, water was added to the obtained residue and the mixture was washed with diethyl ether. The obtained aqueous layer was acidified with 1N hydrochloric acid. The mixture was extracted with ethyl acetate, the obtained ethyl acetate layer was washed successively with water and saturated brine, and dried over anhydrous sodium sulfate. The solvent was evaporated, and the obtained residue was crystallized from n-hexane to give the title compound (67.4 mg) as white crystals.

### Example 110

### Production of 3-((S)-6-chloro-2-isopropyl-8-methyl-3-oxo-2,3-dihydrobenzo[1,4]oxazin-4-yl)propionic acid

### step 1

### Production of 4-chloro-2-methyl-6-nitrophenol

4-Chloro-2-methylphenol (100 g) was dissolved in acetic acid (900 mL), concentrated nitric acid (100 mL) was added dropwise at room temperature over about 1 hr. After stirring the mixture at room temperature for 1 hr, water was added. The precipitated solid was collected by filtration to give the title compound (102 g) as a yellow solid.

### step 2

### Production of 2-amino-4-chloro-6-methylphenol hydrochloride

4-Chloro-2-methyl-6-nitrophenol (50.3 g) was dissolved in tetrahydrofuran (250 mL). To this solution was added platinum oxide (1 g), and the mixture was stirred overnight under hydrogen atmosphere at room temperature. The reaction mixture was filtered through celite, 4N hydrogen chloride (ethyl acetate solution)(134 mL) was added to the obtained filtrate, and the precipitated solid was collected by filtration to give the title compound (47.1 g) as a beige solid.

### step 3

### Production of (R)-2-bromo-N-(5-chloro-2-hydroxy-3-methylphenyl)-3-methylbutylamide

2-Amino-4-chloro-6-methylphenol hydrochloride (380 mg) was dissolved by adding ethyl acetate (3 mL) and water (10 mL), and sodium hydrogencarbonate (395 mg) and a solution of (R)-2-bromo-3-methylbutyryl chloride (400 mg) obtained in step 1 of Example 109 in ethyl acetate (7 mL) were added under ice-cooling and the mixture was stirred at room temperature stirred for 4 hr. The reaction mixture was partitioned, and the obtained ethyl acetate layer was washed successively with water and saturated brine, and dried over anhydrous sodium sulfate. The solvent was evaporated to give the title compound (629.1 mg) as a pale-brown solid.

### step 4

### Production of (S)-6-chloro-2-isopropyl-8-methyl-4H-benzo[1,4]oxazin-3-one

(R)-2-Bromo-N-(5-chloro-2-hydroxy-3-methylphenyl)-3-methylbutylamide (627 mg) was dissolved in N,N-dimethylformamide (5 mL), potassium carbonate (352 mg) was added, and the mixture was stirred at room temperature for 2 days. Water was added to the reaction mixture, and the precipitated solid was collected by filtration to give the title compound (434.1 mg) as a pale-beige solid.

### step 5

### Production of 3-((S)-6-chloro-2-isopropyl-8-methyl-3-oxo-2,3-dihydrobenzo[1,4]oxazin-4-yl)propionic acid methyl ester

(S)-6-Chloro-2-isopropyl-8-methyl-4H-benzo[1,4]oxazin-3-one (200 mg) was dissolved in N,N-dimethylformamide (2.5 mL), and potassium carbonate (229 mg) and methyl acrylate (0.15 mL) were added. After stirring with heating overnight at 90°C, a 10% aqueous citric acid solution was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=6:1) to give the title compound (257.3 mg) as a colorless oil.

### step 6

### Production of 3-((S)-6-chloro-2-isopropyl-8-methyl-3-oxo-2,3-dihydrobenzo[1,4]oxazin-4-yl)propionic acid

3-((S)-6-Chloro-2-isopropyl-8-methyl-3-oxo-2,3-dihydrobenzo[1,4]oxazin-4-yl)propionic acid methyl ester (255 mg) was dissolved in methanol (4 mL), and a 2N aqueous sodium hydroxide solution (0.78 mL) was added. After stirring at room temperature for 4 hr, the solvent was evaporated, water was added to the obtained residue and the mixture was washed with diethyl ether. The obtained aqueous layer was acidified with 1N hydrochloric acid under ice-cooling and the mixture was extracted with ethyl acetate. The obtained ethyl acetate layer was washed successively with water and saturated brine, and dried over anhydrous sodium sulfate. The solvent was evaporated, and the obtained residue was crystallized from n-hexane to give the title compound (199.2 g) as pale-pink crystals.

### Example 111

### Production of 3-((S)-6,8-dichloro-2-isopropyl-3-oxo-2,3-dihydrobenzo[1,4]oxazin-4-yl)propionic acid

### step 1

### Production of (R)-2-bromo-N-(3,5-dichloro-2-hydroxyphenyl)-3-methylbutylamide

2-Amino-4,6-dichlorophenol hydrochloride (420 mg) obtained in step 1 of Example 80 was dissolved by adding ethyl acetate (5 mL) and water (10 mL), sodium hydrogencarbonate (395 mg) and a solution of (R)-2-bromo-3-methylbutyryl chloride (400 mg) obtained in step 1 of Example 109 in ethyl acetate (5 mL) were added under ice-cooling, and the mixture was stirred at room temperature for 2 hr. The reaction mixture was partitioned, and the obtained ethyl acetate layer was washed successively with water and saturated brine, and dried over anhydrous sodium sulfate. The solvent was evaporated, and the obtained residue was purified by silica gel chromatography (n-hexane-ethyl acetate=6:1) to give the title compound (641.5 mg) as a cream-colored solid.

### step 2

### Production of (S)-6,8-dichloro-2-isopropyl-4H-benzo[1,4]oxazin-3-one

(R)-2-Bromo-N-(3,5-dichloro-2-hydroxyphenyl)-3-methylbutylamide (640 mg) was dissolved in N,N-dimethylformamide (4 mL), potassium carbonate (338 mg) was added, and the mixture was stirred at room temperature for 3 days. Water was added to the reaction mixture, and the precipitated solid was collected by filtration to give the title compound (465.6 mg) as a white solid.

### step 3

### Production of 3-((S)-6,8-dichloro-2-isopropyl-3-oxo-2,3-dihydrobenzo[1,4]oxazin-4-yl)propionic acid methyl ester

(S)-6,8-Dichloro-2-isopropyl-4H-benzo[1,4]oxazin-3-one (200 mg) was dissolved in N,N-dimethylformamide (2 mL), and potassium carbonate (213 mg) and methyl acrylate (0.14 mL) were added. After stirring with heating overnight at 90°C, a 10% aqueous citric acid solution was added to the reaction mixture, and the mixture was extracted with ethyl acetate. The obtained ethyl acetate layer was washed successively with water and saturated brine, and dried over anhydrous sodium sulfate. The solvent was evaporated, and the obtained residue was purified by silica gel chromatography (n-hexane-ethyl acetate=6:1) to give the title compound (187.3 mg) as a colorless oil.

### step 4

### Production of 3-((S)-6,8-dichloro-2-isopropyl-3-oxo-2,3-dihydrobenzo[1,4]oxazin-4-yl)propionic acid

3-((S)-6,8-Dichloro-2-isopropyl-3-oxo-2,3-dihydrobenzo[1,4]oxazin-4-yl)propionic acid methyl ester (185 mg) was dissolved in methanol (3 mL), and a 2N aqueous sodium hydroxide solution (0.53 mL) was added. After stirring the mixture overnight at room temperature, the solvent was evaporated, and water was added to the obtained residue. The mixture was washed with diethyl ether. The obtained aqueous layer was acidified with 1N hydrochloric acid and the mixture was extracted with ethyl acetate. The obtained ethyl acetate layer was washed successively with water and saturated brine, and dried over anhydrous sodium sulfate. The solvent was evaporated, and the obtained residue was crystallized from diethyl ether-n-hexane to give the title compound (100.2 mg) as white crystals.

The ¹H-NMR spectrum data of the compounds of Examples 1-111 are shown in Table 1-Table 23.
The ¹H-NMR spectrum was measured in CDCl₃ or DMSO-d₆, with tetramethylsilane as an inner standard, and the total δ value are shown in ppm.
The symbols in the tables mean the following.
s: singlet
d: doublet
t: triplet
q: quartet
quin: quintet
septet: septet
dd: double doublet
ddd: double double doublet
brs: broad singlet
m: multiplet
J: coupling constant

**[Table 1]**

| Ex. No. | Structural formula | NMR |
|---|---|---|
| 1 | 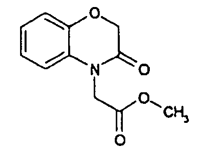 | (400 MHz, CDCl₃) 3.79 (s, 3H), 4.67 (s, 2H), 4.68 (s, 2H), 6.73 - 6.79 (m, 1H), 7.01 - 7.03 (m, 3H) |
| 2 | 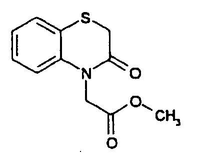 | (400 MHz, CDCl₃) 3.47 (s, 2H), 3.80 (s, 3H), 4.67 (s, 2H), 6.87 (dd, J=8.35, 1. 6Hz, 1H), 7.02 - 7.07 (m, 1H), 7.20 - 7.25 (m, 1H), 7.39 (dd, J=7.77, 1.51Hz, 1H) |
| 3 | 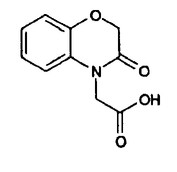 | (400 MHz, DMSO-d₆) 4.63 (s, 2H), 4.69 (s, 2H), 7.02 - 7. 6 (m, 4H), 13. 9 (brs, 1H) |
| 4 | 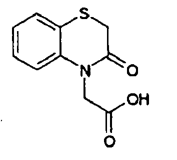 | (400 MHz, DMSO-d₆) 3.55 (s, 2H), 4.61 (s, 2H), 7.05 - 7.11 (m, 2H), 7.26 - 7.30 (m, 1H), 7.43 (dd, J=7.65, 1.62Hz, 1H), 12.99 (brs, 1H) |
| 5 | 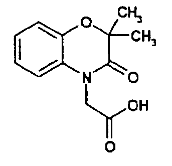 | (400 MHz, DMSO-d₆) 1. 41 (s, 6H), 4. 60 (s, 2H), 6. 98 - 7. 04 (m, 4H), 13. 05 (brs, 1H) |

**[Table 2]**

| Ex. No. | Structural formula | NMR |
|---|---|---|
| 6 | 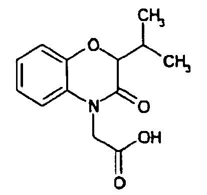 | (400 MHz, DMSO-d₆) 0. 94 (d, J=6. 72Hz, 3H), 0. 98 (d, J=6. 72Hz, 3H), 2. 06 - 2. 14 (m, 1H), 4. 40 (d, J=6. 72Hz, 1H), 4. 53 (d, J=17. 74Hz, 1H), 4. 68 (d, J=17. 74Hz, 1H), 6. 97 - 7. 05 (m, 4H), 13. 05 (brs, 1H) |
| 7 | 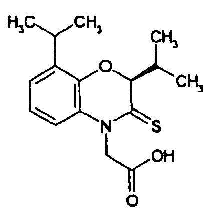 | (400 MHz, CDCl₃) 0.98 (d, J=6. 72Hz, 3H), 1. 12 (d, J=6. 96Hz, 3H), 1. 21 (d, J=6. 72Hz, 3H), 1. 27 (d, J=6. 92Hz, 3H), 2. 35 - 2. 48 (m, 1H), 3. 42 (septet, J=6. 96Hz, 1H), 4. 82 (d, J=6. 28Hz, 1H), 4. 85 - 4. 98 (m, 1H), 5. 55 - 5. 70 (m, 1H), 6. 75 (dd, J=8. 12, 1. 40Hz, 1H), 6. 98 (dd, J=5. 56, 5. 56Hz, 1H), 7. 06 (dd, J=7. 88, 1. 40Hz, 1H) |
| 8 | 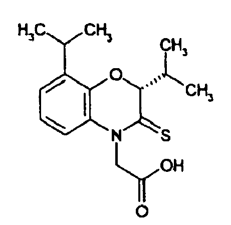 | (400 MHz, CDCl₃) 0. 99 (d, J=6. 72Hz, 3H), 1. 12 (d, J=6. 92Hz, 3H), 1. 21 (d, J=6. 96Hz, 3H), 1. 28 (d, J=6. 96Hz, 3H), 2.35 - 2. 50 (m, 1H), 3. 42 (septet, J=6. 96Hz, 1H), 4. 83 (d, J=6. 48Hz, 1H), 4. 85 - 5. 00 (m, 1H), 5. 55 - 5. 70 (m, 1H), 6. 75 (dd, J=7. 88, 1. 40Hz, 1H), 6. 99 (dd, J=8. 08, 8.08Hz, 1H), 7.07 (dd, J=7. 88, 1.12Hz, 1H) |
| 9 | 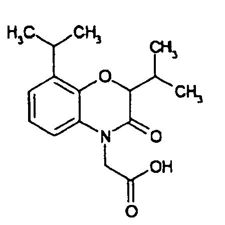 | (300 MHz, DMSO-d₆) 0.96 (d, J=6. 78Hz, 3H), 1. 05 (d, J=6. 78Hz, 3H), 1. 18 (d, J=6. 78Hz, 3H), 1.22 (d, J=6. 78Hz, 3H), 2. 15 - 2. 29 (m, 1H), 3. 24 - 3. 31 (m, 1H), 4.41 (d, J=5. 14Hz, 1H), 4. 54 (d, J=17. 79Hz, 1H), 4.62 (d, J=17. 79Hz, 1H), 6. 78 - 6.84 (m, 1H), 6. 94 - 7. 01 (m, 2H) |
| 10 | 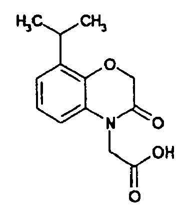 | (300 MHz, DMSO-d₆) 1. 18 (d, J=6. 60Hz, 6H), 3. 16 - 3. 32 (m, 1H), 4. 58 (s, 2H), 4. 69 (s, 2H), 6. 84 - 6. 88 (m, 1H), 6. 95 - 7. 03 (m, 2H), 13. 06 (brs, 1H) |

**[Table 3]**

| Ex. No. | Structural formula | NMR |
|---|---|---|
| 11 | 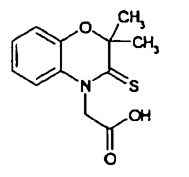 | (400 MHz, DMSO-d₆) 1.54 (s, 6H), 5.20 (brs, 2H), 7. 03 - 7.09 (m, 2H), 7. 12 - 7. 16 (m, 1H), 7.20 - 7.23 (m, 1H) |
| 12 | 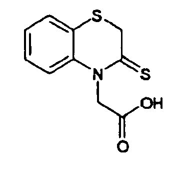 | (400 MHz, DMSO-d₆) 4.02 (s, 2H), 5.20 (s, 2H), 7. 14 - 7. 18 (m, 1H), 7. 30 - 7. 33 (m, 2H), 7. 45 - 7.48 (m, 1H), 13. 12 (brs, 1H) |
| 13 | 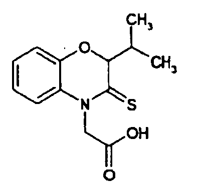 | (400 MHz, DMSO-d₆) 0.90 (d, J=6.84Hz, 3H), 1.01 (d, J=6. 84Hz, 3H), 2. 08 - 2. 17 (m, 1H), 4.79 (d, J=7. 42Hz, 1H), 4.96 - 5. 10 (m, 1H), 5. 38 (d, J=16. 46Hz, 1H), 7. 02 - 7. 16 (m, 3H), 7. 21 - 7. 24 (m, 1H) |
| 14 | 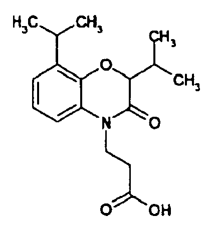 | (400 MHz, DMSO-d₆) 0. 95 (d, J=6. 72Hz, 3H), 1. 04 (d, J=6. 72Hz, 3H), 1. 17 (d, J=6. 72Hz, 3H), 1.21 (d, J=6. 72Hz, 3H), 2. 17 - 2. 27 (m, 1H), 2. 36 - 2. 59 (m, 2H), 3.22 - 3. 32 (m, 1H), 4. 01 - 4. 17 (m, 2H), 4.35 (d, J=5. 10Hz, 1H), 6. 95 - 7. 05 (m, 3H), 12. 40 (brs, 1H) |
| 15 | 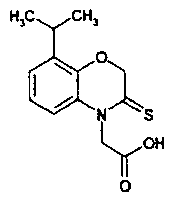 | (400 MHz, DMSO-d₆) 1. 19 (d, J=6.96Hz, 6H), 3.18 - 3.28 (m, 1H), 4.95 (s, 2H), 5.20 (s, 2H), 7. 02 - 7. 13 (m, 3H), 13. 21 (brs, 1H) |

**[Table 4]**

| Ex. No. | Structural formula | NMR |
|---|---|---|
| 16 | 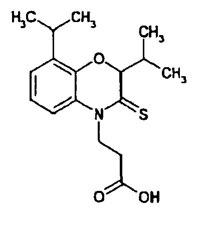 | (400 MHz, DMSO-d₆) 0.88 (d, J=6. 72Hz, 3H), 1. 03 (d, J=6. 72Hz, 3H), 1. 15 (d, J=6. 96Hz, 3H), 1. 23 (d, J=6. 96Hz, 3H), 2. 19 - 2. 28 (m, 1H), 2. 61 - 2. 77 (m, 2H), 3. 28 - 3. 36 (m, 1H), 4. 49 - 4. 58 (m, 1H), 4. 70 - 4. 80 (m, 1H), 4. 74 (d, J=6. 49Hz, 1H), 7. 02 - 7. 07 (m, 1H), 7. 10 - 7. 13 (m, 1H), 7. 23 - 7. 27 (m, 1H) |
| 17 | 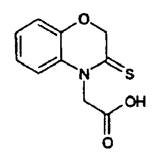 | (400 MHz, DMSO-d₆) 4. 96 (s, 2H), 5. 23 (s, 2H), 7. 06 - 7. 17 (m, 3H), 7. 27 - 7. 31 (m, 1H), 13. 21 (brs, 1H) |
| 18 | 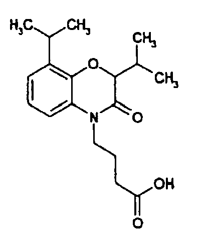 | (400 MHz, DMSO-d₆) 0.95 (d, J=6.96Hz, 3H), 1. 04 (d, J=6. 96Hz, 3H), 1. 17 (d, J=6. 96Hz, 3H), 1. 21 (d, J=6. 96Hz, 3H), 1. 69 - 1. 83 (m, 2H), 2. 17 - 2. 32 (m, 3H), 3. 24 - 3. 32 (m, 1H), 3. 79 - 3. 98 (m, 2H), 4. 36 (d, J=5. 10Hz, 1H), 6. 95 - 7. 02 (m, 2H), 7. 08 (dd, J=7. 65, 1. 62Hz, 1H), 12. 17 (brs, 1H) |
| 19 | 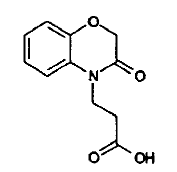 | (400 MHz, DMSO-d₆) 2.54 (t, J=7. 65Hz, 2H), 4. 11 (t, J=7. 65Hz, 2H), 4. 62 (s, 2H), 7. 00 - 7. 08 (m, 3H), 7. 24 (d, J=7. 20Hz, 1H), 12. 40 (brs, 1H) |
| 20 | 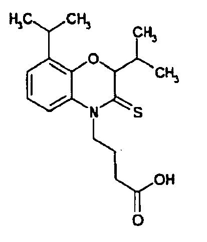 | (400 MHz, DMSO-d₆) 0.88 (d, J=6. 96Hz, 3H), 1. 03 (d, J=6. 96Hz, 3H), 1. 15 (d, J=6. 96Hz, 3H), 1.23 (d, J=6. 96Hz, 3H), 1. 85 - 1. 95 (m, 2H), 2. 18 - 2. 27 (m, 1H), 2. 34 - 2. 39 (m, 2H), 3. 28 - 3. 37 (m, 1H), 4. 29 - 4. 39 (m, 1H), 4. 53 - 4. 62 (m, 1H), 4. 76 (d, J=6. 72Hz, 1H), 7.0 3 - 7. 07 (m, 1H), 7. 12 (dd, J=7. 77, 1. 33Hz, 1H), 7. 32 (dd, J=8. 12, 1. 33Hz, 1H), 12. 23 (brs, 1H) |

**[Table 5]**

| Ex. No. | Structural formula | NMR |
|---|---|---|
| 21 | 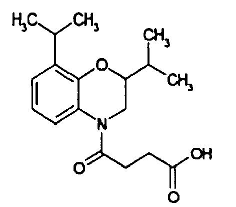 | (300 MHz, CDCl₃) 1. 04 - 1. 29 (m, 12H), 1. 87 - 1. 98 (m, 1H), 2. 70 - 3. 20 (m, 5H), 3. 22 - 3. 34 (m, 1H), 3. 87 - 3. 95 (m, 1H), 4. 61 (brs, 1H), 6.81 - 7. 26 (m, 3H) |
| 22 | 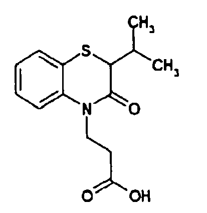 | (300 MHz, CDCl₃) 1. 00 (t, J=7. 33Hz, 6H), 1. 72 - 1. 80 (m, 1H), 2. 70 - 2. 84 (m, 2H), 3. 08 (d, J=9. 17Hz, 1H), 4. 17 - 4. 42 (m, 2H), 7. 00 - 7.37 (m, 4H) |
| 23 | 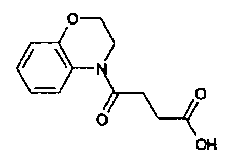 | (400 MHz, DMSO-d₆) 2. 45 - 2. 60 (m, 2H), 2. 78 (t, J=6. 40Hz, 2H), 3. 87 (t, J=4. 60Hz, 2H), 4. 24 (t, J=4. 60Hz, 2H), 6. 80 - 7. 30 (m, 3H), 7. 75 (brs, 1H), 12. 09 (s, 1H) |
| 24 | 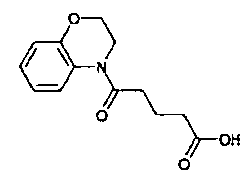 | (400 MHz, DMSO-d₆) 1. 78 (quin, J=7. 30Hz, 2H), 2. 27 (t, J=7. 30Hz, 2H), 2. 60 (t, J=7. 30Hz, 2H), 3. 85 (t, J=4. 70Hz, 2H), 4. 25 (t, J=4. 70Hz, 2H), 6. 80 - 6. 90 (m, 2H), 6. 95 - 7. 10 (m, 1H), 7. 75 (brs, 1H), 12. 04 (s, 1H) |
| 25 | 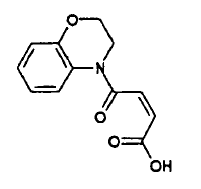 | (400 MHz, DMSO-d₆) 3. 60 - 4. 00 (m, 2H), 4. 15 - 4. 40 (m, 2H), 5. 80 - 6. 20 (m, 1H), 6. 70 - 7. 40 (m, 4H), 8. 16 (brs, 1H), 12. 75 (brs, 1H) |

**[Table 6]**

| Ex. No. | Structural formula | NMR |
|---|---|---|
| 26 | 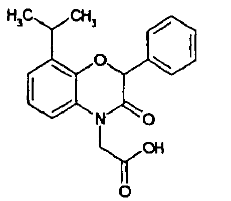 | (400 MHz, DMSO-d₆) 0. 95 (d, J=6.96Hz, 3H), 1.11 (d, J=6.96Hz, 3H), 3.19 (septet, J=6.96Hz, 1H), 4.59 (d, J=17.74Hz, 1H), 4.78 (d, J=17.74Hz, 1H), 5.91 (s, 1H), 6.87 - 7.00 (m, 3H), 7.32 - 7. 35 (m, 3H), 7.42 - 7.45 (m, 2H), 13. 13 (brs, 1H) |
| 27 | 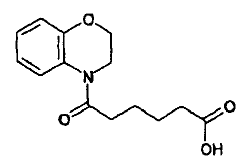 | (400 MHz, DMSO-d₆) 1.40 - 1. 65 (m, 4H), 2.21 (t, J=7.00Hz, 2H), 2.57 (t, J=7.00Hz, 2H), 3. 85 (t, J=4. 70Hz, 2H), 4. 24 (t, J=4.70Hz, 2H), 6.80 - 6. 90 (m, 2H), 6.95 - 7.10 (m, 1H), 7.75 (brs, 1H), 11.99 (s, 1H) |
| 28 | 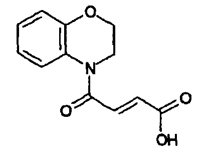 | (400 MHz, DMSO-d₆) 3. 94 (t, J=4.80Hz, 2H), 4. 32 (t, J=4.40Hz, 2H), 6. 55 - 6. 70 (m, 1H), 6.75-7.50 (m, 5H), 13.00 (s, 1H) |
| 29 | 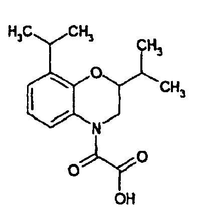 | (300 MHz, CDCl₃) 1.05 - 1.27 (m, 12H), 1. 95 - 2.01 (m, 1H), 3.21 - 3. 63 (m, 2H), 3.96 - 4.01 (m, 1H), 4.50 - 4.84 (m, 1H), 6. 76 - 7. 81 (m, 3H) |
| 30 | 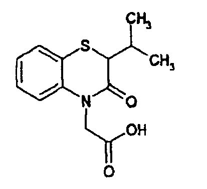 | (300 MHz, CDCl₃) 1.05 (d, J=14.70Hz, 6H), 1.75- 1. 87 (m, 1H), 3. 15 (d, J=9.53Hz, 1H), 4.31 (d, J=17.60Hz, 1H), 5.11 (d, J=18.00Hz, 1H), 6.87 (dd, J=8.07, 0.73Hz, 1H), 7.00 - 7.36 (m, 2H), 7. 37 (dd, J=7.70, 1.47Hz, 1H) |

**[Table 7]**

| Ex. No. | Structural formula | NMR |
|---|---|---|
| 31 | 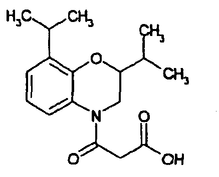 | (300 MHz, CDCl₃) 1.01 - 1. 24 (m, 12H), 1.90 - 2.01 (m, 1H), 3. 09 - 3. 36 (m, 2H), 3.68 (d, J=18.30Hz, 1H), 3.76 (d, J=18.30Hz, 1H), 3.95 - 4.01 (m, 1H), 4. 70 (dd, J=11.70, 2.93Hz, 1H), 6.86 - 6.91 (m, 2H), 7.13 - 7.25 (m, 1H) |
| 32 | 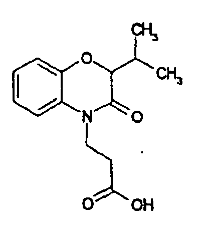 | (400 MHz, DMSO-d₆) 0.93 (d, J=6.84Hz, 3H), 0. 98 (d, J=6. 84Hz, 3H), 2.05 - 2. 13 (m, 1H), 2.45 - 2.59 (m, 2H), 4.00 - 4. 19 (m, 2H), 4.34 (d, J=6.26Hz, 1H), 7.00 - 7.07 (m, 3H), 7.19 - 7.21 (m, 1H), 12. 39 (brs, 1H) |
| 33 | 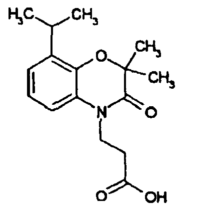 | (400 MHz, DMSO-d₆) 1. 17 (d, J=6. 96Hz, 6H), 1. 38 (s, 6H), 2.47 - 2. 53 (m, 2H), 3.19 - 3. 26 (m, 1H), 4. 09 (t, J=7. 65Hz, 2H), 6.96 - 7.05 (m, 3H), 12.37 (brs, 1H) |
| 34 | 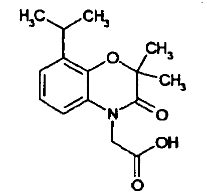 | (400 MHz, DMSO-d₆) 1. 18 (d, J=6.96Hz, 6H), 1. 41 (s, 6H), 3.21 - 3.28 (m, 1H), 4. 57 (s, 2H), 6.79 - 6. 84 (m, 1H), 6.96 - 6. 99 (m, 2H), 13.02 (brs, 1H) |
| 35 | 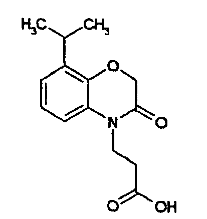 | (300 MHz, DMSO-d₆) 1.17 (d, J=6.97Hz, 6H), 2.49 - 2.56 (m, 2H), 3.21 (septet, J=6.97Hz, 1H), 4.07 - 4. 12 (m, 2H), 4.61 (s, 2H), 6.95 - 7.09 (m, 3H), 12.38 (brs, 1H) |

**[Table 8]**

| Ex. No. | Structural formula | NMR |
|---|---|---|
| 36 | 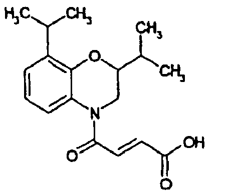 | (300 MHz, CDCl₃) 1.05 - 1. 20 (m, 6H), 1.24 (d, J=6.97Hz, 6H), 1.90 - 2.01 (m, 1H), 3.21 - 3. 37 (m, 2H), 3.93 - 3. 99 (m, 1H), 4. 63 (brs, 1H), 6.78 - 7.10 (m, 4H), 7. 55 (d, J=15.40Hz, 1H) |
| 37 | 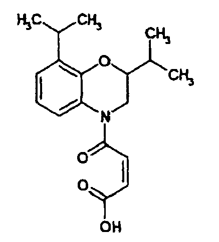 | (300 MHz, CDCl₃) 1.05 - 1. 12 (m, 6H), 1. 2 4(d, J=6. 97Hz, 6H), 1.90 - 2.01 (m, 1H), 3.21 - 3. 37 (m, 2H), 3.93 - 3. 99 (m, 1H), 4.61 - 4. 65 (m, 1H), 6.70 - 7. 10 (m, 4H), 7.55 (d, J=15. 40Hz, 1H) |
| 38 | 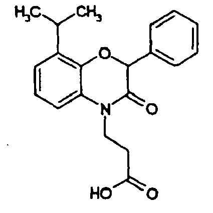 | (400 MHz, DMSO-d₆) 1.01 (d, J=6.96Hz, 3H), 1.12 (d, J=6.96Hz, 3H), 2.58 - 2. 62 (m, 2H), 3.22 (septet, J=6.96Hz, 1H), 4.12 - 4.25 (m, 2H), 5. 82 (s, 1H), 6.94 - 6. 96 (m, 1H), 6.99 - 7.04 (m, 1H), 7.09 - 7. 11 (m, 1H), 7.31 - 7.36 (m, 5H), 12.42 (brs, 1H) |
| 39 | 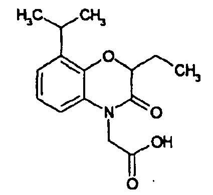 | (300 MHz, DMSO-d₆) 1.02 (t, J=7.34Hz, 3H), 1.19 (d, J=6.97Hz, 3H), 1.20 (d, J=6. 97Hz, 3H), 1.68 - 1. 95 (m, 2H), 3. 21 - 3. 32 (m, 1H), 4. 53 (dd, J=8. 25, 4.58Hz, 1H), 4. 58 (s, 2H), 6.80 - 6. 86 (m, 1H), 6. 96 - 7.02 (m, 2H), 12.99 (brs, 1H) |
| 40 | 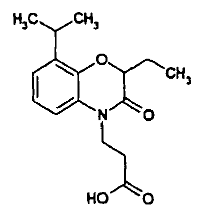 | (400 MHz, DMSO-d₆) 1.01 (t, J=7. 42Hz, 3H), 1.17 - 1. 20 (m, 6H), 1.67 - 1. 78 (m, 1H), 1. 82 - 1. 92 (m, 1H), 2.49 - 2. 53 (m, 2H), 3.20 - 3.29 (m, 1H), 4.08 (t, J=7.65Hz, 2H), 4. 46 (dd, J=8. 58, 4.41Hz, 1H), 6. 96 - 7.06 (m, 3H), 12.39 (brs, 1H) |

**[Table 9]**

| Ex. No. | Structural formula | NMR |
|---|---|---|
| 41 | 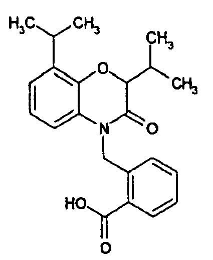 | (400 MHz, DMSO-d₆) 1. 04 (d, J=6. 72Hz, 3H), 1. 11 (d, J=6.96Hz, 3H), 1.20 (d, J=6.96Hz, 3H), 1.23 (d, J=6.96Hz, 3H), 2. 33 - 2.41 (m, 1H), 3.29 - 3.35 (m, 1H), 4. 59 (d, J=5.10Hz, 1H), 5. 40 (d, J=17.97Hz, 1H), 5.48 (d, J=17.97Hz, 1H), 6.58 (dd, J=7.88, 1.16Hz, 1H), 6.85 - 6.89 (m, 1H), 6.94 - 7. 01 (m, 2H), 7.36 - 7.40 (m, 1H), 7.46 - 7.50 (m, 1H), 7.99 (dd, J=7.88, 1. 16Hz, 1H) |
| 42 | 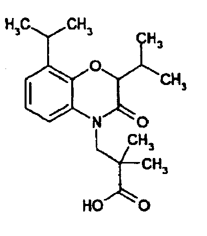 | (400 MHz, DMSO-d₆) 0.97 (d, J=6.72Hz, 3H), 1. 02 (s, 3H), 1. 06 (s, 3H), 1. 08 (d, J=6. 96Hz, 3H), 1. 17 (d, J=6. 96Hz, 3H), 1. 21 (d, J=6.72Hz, 3H), 2.25 - 2.33 (m, 1H), 3.24 - 3.32 (m, 1H), 4.02 (d, J=14.38Hz, 1H), 4. 27 (d, J=4.64Hz, 1H), 4. 31 (d, J=14.38Hz, 1H), 6.93 - 6.97 (m, 2H), 7.11 - 7. 15 (m, 1H), 12. 35 (brs, 1H) |
| 43 | 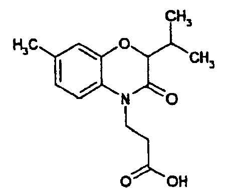 | (300 MHz, CDCl₃) 1.01 - 1. 05 (m, 6H), 2. 11 - 2.26 (m, 1H), 2.29 (s, 3H), 2. 74 (t, J=7.70Hz, 2H), 4.12 - 4. 30 (m, 3H), 6. 80 - 6.86 (m, 3H) |
| 44 | 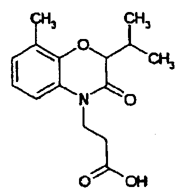 | (300 MHz, CDCl₃) 1.01 - 1. 10 (m, 6H), 2. 10 - 2.24 (m, 1H), 2.27 (s, 3H), 2.75 (t, J=7.70Hz, 2H), 4.13 - 4. 32 (m, 3H), 6. 80 - 6.93 (m, 3H) |
| 45 | 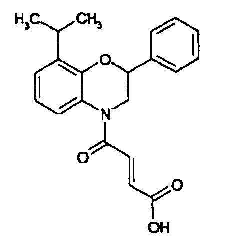 | (400 MHz, DMSO-d₆) 1.21 (d, J=6.96Hz, 3H), 1.22 (d, J=6.72Hz, 3H), 3.24 - 3. 32 (m, 1H), 3.72 - 3. 74 (m, 1H), 4.34 - 4. 36 (m, 1H), 5.52 - 5. 54 (m, 1H), 6.53 - 6. 56 (m, 1H), 6. 92 - 6.96 (m, 2H), 7.14 - 7. 15 (m, 1H), 7.23 - 7.26 (m, 1H), 7.33 - 7.45 (m, 5H), 12.97 (brs, 1H) |

**[Table 10]**

| Ex. No. | Structural formula | NMR |
|---|---|---|
| 46 | 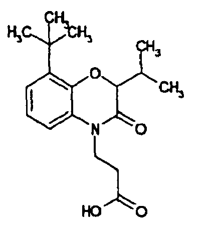 | (400 MHz, DMSO-d₆) 0. 99 (d, J=6. 72Hz, 3H), 1. 17 (d, J=6. 96Hz, 3H), 1. 35 (s, 9H), 2. 48 - 2. 59 (m, 3H), 4. 09 (t, J=7.65Hz, 2H), 4.37 (d, J=2.55Hz, 1H), 6.98 - 6. 99 (m, 2H), 7.06 - 7.10 (m, 1H), 12.38 (brs, 1H) |
| 47 | 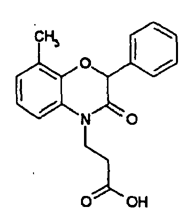 | (300 MHz, CDCl₃) 2. 28 (s, 3H), 2.68 - 2. 88 (m, 2H), 4.26 (t, J=7.70Hz, 2H), 5.75 (s, 1H), 6.81 - 6. 93 (m, 3H), 7.28 - 7. 38 (m, 5H) |
| 48 | 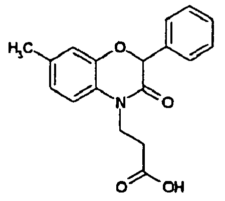 | (300 MHz, CDCl₃) 2.25 (s, 3H), 2.75 - 2.87 (m, 2H), 4. 27 (t, J=7. 70Hz, 2H), 5. 69 (s, 1H), 6.79 - 6. 88 (m, 3H), 7.27 - 7.39 (m, 5H) |
| 49 | 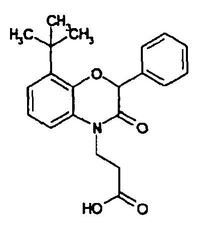 | (400 MHz, DMSO-d₆) 1. 22 (s, 9H), 2.58 - 2. 62 (m, 2H), 4.15 - 4. 19 (m, 2H), 5. 66 (s, 1H), 6.99 - 7. 07 (m, 2H), 7.19 - 7. 21 (m, 1H), 7.34 - 7. 42 (m, 5H), 12. 39 (brs, 1H) |
| 50 | 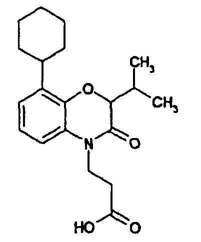 | (400 MHz, DMSO-d₆) 0. 94 (d, J=6. 72Hz, 3H), 1. 04 (d, J=6. 72Hz, 3H), 1. 18 - 1. 52 (m, 5H), 1.69 - 1. 84 (m, 5H), 2.18 - 2. 26 (m, 1H), 2.45 - 2. 58 (m, 2H), 2.86 - 2. 93 (m, 1H), 4.01 - 4. 16 (m, 2H), 4.33 (d, J=5.33Hz, 1H), 6.92 - 7.04 (m, 3H), 12.38 (brs, 1H) |

**[Table 11]**

| Ex. No. | Structural formula | NMR |
|---|---|---|
| 51 | 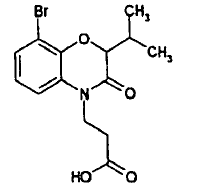 | (300 MHz, DMSO-d₆) 0.95 (d, J=6.60Hz, 3H), 1.01 (d, J=6.97Hz, 3H), 2.01 - 2.13 (m, 1H), 2.43 - 2. 61 (m, 2H), 4.02 - 4. 21 (m, 2H), 4.49 (d, J=6.24Hz, 1H), 6.96 - 7.02 (m, 1H), 7.22 - 7.25 (m, 1H), 7.27 - 7. 30 (m, 1H) |
| 52 | 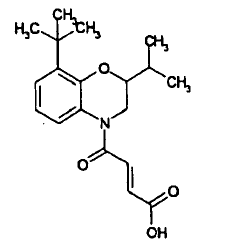 | (300 MHz, DMSO-d₆) 1. 04 (d, J=6. 97Hz, 3H), 1.08 (d, J=6. 60Hz, 3H), 1. 37 (s, 9H), 1.91 - 2. 02 (m, 1H), 3.22 - 3. 35 (m, 1H), 4.00 - 4.07 (m, 1H), 4.39 - 4.45 (m, 1H), 6.63 (d, J=15.04Hz, 1H), 6.82 - 6.88 (m, 1H), 6.90 - 7.09 (br m, 1H), 7.11 - 7.14 (m, 1H), 7.26 (d, J=15.04Hz, 1H), 12.96 (brs, 1H) |
| 53 | 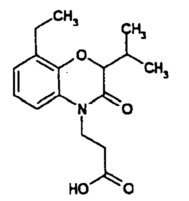 | (400 MHz, DMSO-d₆) 0. 94 (d, J=6.72Hz, 3H), 1. 02 (d, J=6. 72Hz, 3H), 1. 15 (t, J=7. 54Hz, 3H), 2.13 - 2. 22 (m, 1H), 2. 46 - 2. 70 (m, 4H), 4.01 - 4. 17 (m, 2H), 4.34 (d, J=5. 57Hz, 1H), 6.91 (dd, J=7.65, 1. 62Hz, 1H), 6.95 - 6.99 (m, 1H), 7. 04 (dd, J=7.88, 1.62Hz, 1H), 12.38 (brs, 1H) |
| 54 | 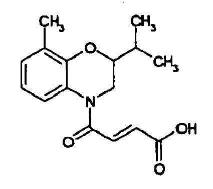 | (300 MHz, CDCl₃) 1.02 - 1. 11 (m, 6H), 1. 88 - 1.99 (m, 1H), 2.22 (s, 3H), 3.22 - 3.30 (m, 1H), 3.93 - 4.00 (m, 1H), 4.58 - 4.62 (m, 1H), 6.77 - 7.02 (m, 4H), 7. 53 (d, J=15.40Hz, 1H) |
| 55 | 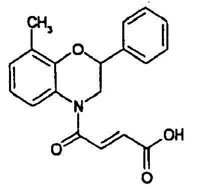 | (300 MHz, CDCl₃) 2.30 (s, 3H), 3.48 (brs, 1H), 4. 75 (brs, 1H), 5.35 (dd, J=8.44, 2.88Hz, 1H), 6.84 - 7.55 (m, 10H) |

**[Table 12]**

| Ex. No. | Structural formula | NMR |
|---|---|---|
| 56 | 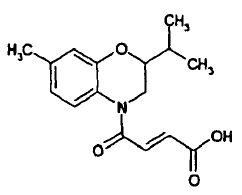 | (300 MHz, CDCl₃) 1.00 - 1. 09 (m, 6H), 1. 86 - 2.00 (m, 1H), 2.30 (s, 3H), 3.25 - 3.33 (m, 1H), 3.89 - 3.95 (m, 1H), 4.53 - 4. 57 (m, 1H), 6.67 - 7.00 (m, 4H), 7. 53 (d, J=15.40Hz, 1H) |
| 57 | 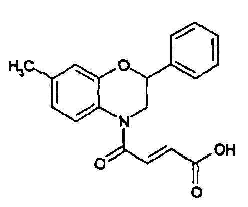 | (300 MHz, CDCl₃) 2.30 (s, 3H), 3. 50 (brs, 1H), 4.69 (brs, 1H), 5.29 (dd, J=8. 43, 2. 93Hz, 1H), 6.76 - 7. 55 (m, 10H) |
| 58 | 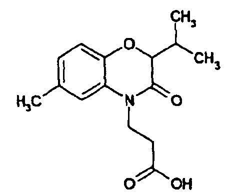 | (300 MHz, CDCl₃) 0. 99 - 1. 05 (m, 6H), 2. 19 - 2.28 (m, 1H), 2.33 (s, 3H), 2. 75 (t, J=7.70Hz, 2H), 4. 13 - 4. 31 (m, 3H), 6. 77 - 6. 90 (m, 3H) |
| 59 | 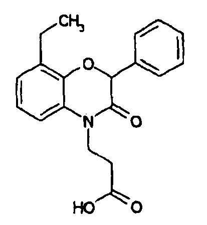 | (300 MHz, DMSO-d₆) 1.02 (t, J=7.52Hz, 3H), 2. 49 - 2. 62 (m, 4H), 4.16 - 4. 22 (m, 2H), 5.83 (s, 1H), 6.89 (dd, J=7.70, 1. 28Hz, 1H), 6.98 (dd, J=8.07, 7.70Hz, 1H), 7.10 (dd, J=8.07, 1.28Hz, 1H), 7.29 - 7.37 (m, 5H), 12.39 (brs, 1H) |
| 60 | 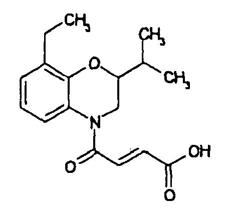 | (300 MHz, DMSO-d₆) 0. 99 (d, J=6. 60Hz, 3H), 1. 04 (d, J=6.60Hz, 3H), 1. 17 (t, J=7.49Hz, 3H), 1.84 - 1. 96 (m, 1H), 2. 60 (q, J=7.49Hz, 2H), 3.20 - 3. 41 (m, 1H), 3.99 - 4.05 (m, 1H), 4.32 - 4.45 (m, 1H), 6. 64 (d, J=15.23Hz, 1H), 6.80 - 7. 06 (m, 1H), 6.81 - 6. 86 (m, 1H), 7.02 - 7. 04 (m, 1H), 7.31 (d, J=15.23Hz, 1H) |

**[Table 13]**

| Ex. No. | Structural formula | NMR |
|---|---|---|
| 61 | 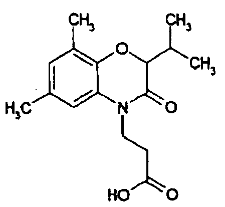 | (300 MHz, DMSO-d₆) 0.92 (d, J=6.60Hz, 3H), 0. 98 (d, J=6. 97Hz, 3H), 1. 99 - 2. 11 (m, 1H), 2. 16 (s, 3H), 2. 24 (s, 3H), 2.47 - 2.54 (m, 2H), 3. 97 - 4.16 (m, 2H), 4. 27 (d, J=6. 60Hz, 1H), 6.70 (s, 1H), 6. 85 (s, 1H), 12.36 (brs, 1H) |
| 62 | 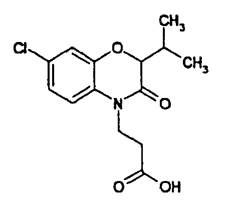 | (300 MHz, CDCl₃) 0.98 - 1.05 (m, 6H), 2. 15 - 2.26 (m, 1H), 2.72 (t, J=7.70Hz, 2H), 4.12 - 4. 31 (m, 3H), 6. 88 - 7.01 (m, 3H) |
| 63 | 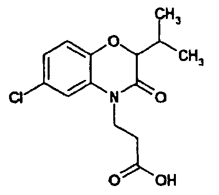 | (300 MHz, CDCl₃) 0.98 - 1.06 (m, 6H), 2. 14 - 2. 26 (m, 1H), 2. 74 (t, J=7.70Hz, 2H), 4.10 - 4. 30 (m, 3H), 6.91 - 6. 99 (m, 3H) |
| 64 | 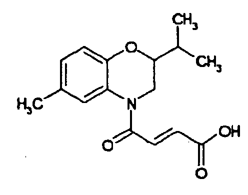 | (300 MHz, CDCl₃) 1.02 (d, J=6.60Hz, 3H), 1. 07 (d, J=6. 96Hz, 3H), 1.88 - 1. 95 (m, 1H), 2. 28 (s, 3H), 3.20 - 3.30 (m, 1H), 3.90 - 3. 95 (m, 1H), 4. 50 (brs, 1H), 6. 83 - 6.96 (m, 4H), 7.55 (d, J=15.40Hz, 1H) |
| 65 | 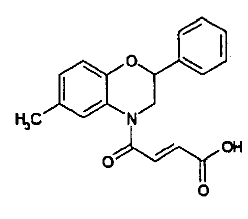 | (300 MHz, CDCl₃) 2.31 (s, 3H), 3.50 (brs, 1H), 4. 65 (brs, 1H), 5. 28 (dd, J=8. 43, 2.93Hz, 1H), 6. 70 - 7. 55 (m, 10H) |

**[Table 14]**

| Ex. No. | Structural formula | NMR |
|---|---|---|
| 66 | 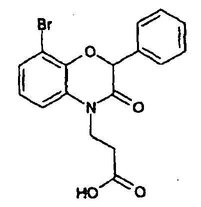 | (300 MHz, DMSO-d₆) 2.54 - 2. 61 (m, 2H), 4.17 - 4. 22 (m, 2H), 6. 02 (s, 1H), 6.96 - 7.02 (m, 1H), 7.26 - 7.30 (m, 2H), 7.34 - 7. 37 (m, 5H), 12.39 (brs, 1H) |
| 67 | 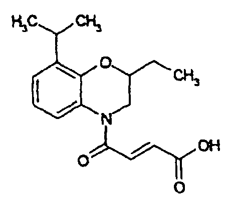 | (400 MHz, DMSO-d₆) 1. 02 (t, J=7.42Hz, 3H), 1. 19 (d, J=6. 84Hz, 3H), 1. 20 (d, J=6.84Hz, 3H), 1.59 - 1. 69 (m, 2H), 3. 23 (septet, J=6. 84Hz, 1H), 3.32 - 3. 45 (m, 1H), 4.19 - 4.31 (m, 2H), 6.64 (d, J=15.31Hz, 1H), 6.85 - 6.89 (m, 1H), 6.85 - 7. 08 (m, 1H), 7. 06 - 7.08 (m, 1H), 7.27 - 7.33 (m, 1H), 13. 00 (brs, 1H) |
| 68 | 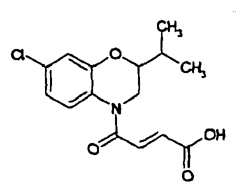 | (300 MHz, CDCl₃) 1.02 - 1.09 (m, 6H), 1.86 - 1. 98 (m, 1H), 3.20 - 3.40 (m, 1H), 3.92 - 3. 96 (m, 1H), 4.50 - 4.60 (m, 1H), 6.87 - 6. 99 (m, 4H), 7.48 (d, J=15.00Hz, 1H) |
| 69 | 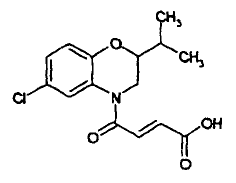 | (300 MHz, CDCl₃) 1.01 - 1. 09 (m, 6H), 1.87 - 1. 98 (m, 1H), 3.30 - 3. 45 (m, 1H), 3. 89 - 3.96 (m, 1H), 4.50 (brs, 1H), 6.88 - 7.11 (m, 4H), 7.51 (d, J=15.00Hz, 1H) |
| 70 | 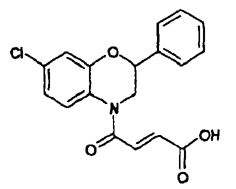 | (300 MHz, CDCl₃) 3. 55 (brs, 1H), 4. 60 (brs, 1H), 5.31 (dd, J=8.44, 2.93Hz, 1H), 6. 70 - 7.55 (m, 10H) |

**[Table 15]**

| Ex. No. | Structural formula | NMR |
|---|---|---|
| 71 | 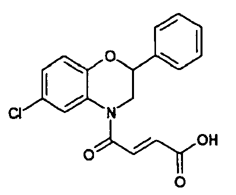 | (300 MHz, CDCl₃) 3. 55 (brs, 1H), 4. 60 (brs, 1H), 5.25 - 5.30 (m, 1H), 6.70 - 7.55 (m, 10H) |
| 72 | 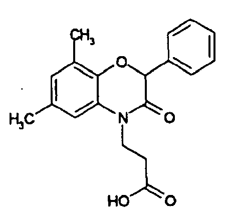 | (400 MHz, DMSO-d₆) 2. 14 (s, 3H), 2. 22 (s, 3H), 2.55 - 2. 59 (m, 2H), 4.12 - 4. 19 (m, 2H), 5.81 (s, 1H), 6.68 (s, 1H), 6.90 (s, 1H), 7.29 - 7.37 (m, 5H), 12.40 (brs, 1H) |
| 73 | 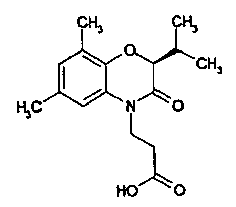 | (400 MHz, DMSO-d₆) 0.91 (d, J=6.72Hz, 3H), 0.98 (d, J=6.72Hz, 3H), 2.00 - 2.11 (m, 1H), 2.16 (s, 3H), 2.24 (s, 3H), 2.44 - 2.58 (m, 2H), 3.99 - 4.15 (m, 2H), 4.28 (d, J=6.49Hz, 1H), 6.70 (s, 1H), 6.86 (s, 1H), 12.37 (brs, 1H) |
| 74 | 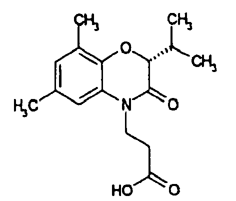 | (400 MHz, DMSO-d₆) 0. 91 (d, J=6. 72Hz, 3H), 0.98 (d, J=6. 72Hz, 3H), 2.00 - 2. 11 (m, 1H), 2.16 (s, 3H), 2. 24 (s, 3H), 2.43 - 2.57 (m, 2H), 3.99 - 4. 15 (m, 2H), 4. 27 (d, J=6.49Hz, 1H), 6. 70 (s, 1H), 6.85 (s, 1H), 12. 36 (brs, 1H) |
| 75 | 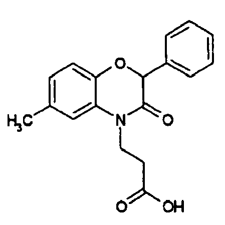 | (300 MHz, CDCl₃) 2.31 (s, 3H), 2.75 - 2.81 (m, 1H), 4.28 (t, J=8.06Hz, 2H), 5.67 (s, 1H), 6. 78 - 6. 96 (m, 3H), 7.30 - 7. 36 (m, 5H) |

**[Table 16]**

| Ex. No. | Structural formula | NMR |
|---|---|---|
| 76 | 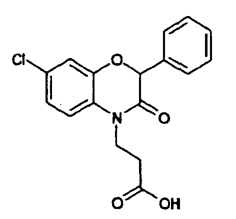 | (300 MHz, CDCl₃) 2.74 - 2.79 (m, 2H), 4.27 (t, J=7.33Hz, 2H), 5.72 (s, 1H), 6.90 - 7.36 (m, 8H) |
| 77 | 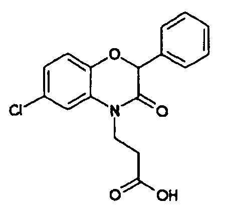 | (300 MHz, CDCl₃) 2.75 - 2.81 (m, 2H), 4.26 (t, J=7.70Hz, 2H), 5.70 (s, 1H), 6.97 - 6.99 (m, 3H), 7.31 - 7. 34 (m, 5H) |
| 78 | 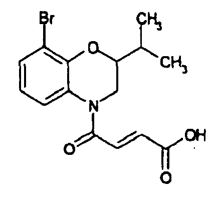 | (400 MHz, DMSO-d₆) 0. 99 (d, J=6. 72Hz, 3H), 1.05 (d, J=6.72Hz, 3H), 1. 86 - 1. 95 (m, 1H), 3.31 - 3.41 (m, 1H), 4.11 - 4. 16 (m, 1H), 4.34 - 4. 47 (m, 1H), 6. 64 (d, J=15.31Hz, 1H), 6.85 - 6.89 (m, 1H), 7.02 - 7.48 (m, 1H), 7.27 - 7.32 (m, 1H), 7.43 (d, J=8. 12Hz, 1H), 13.03 (brs, 1H) |
| 79 | 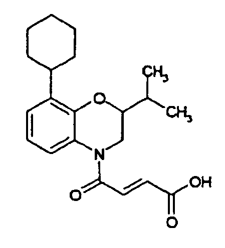 | (400 MHz, DMSO-d₆) 1.00 (d, J=6. 72Hz, 3H), 1. 05 (d, J=6. 72Hz, 3H), 1.17 - 1. 50 (m, 5H), 1. 68 - 1. 85 (m, 5H), 1.86 - 1. 95 (m, 1H), 2. 82 - 2. 89 (m, 1H), 3. 20 - 3. 39 (m, 1H), 3.96 - 4.02 (m, 1H), 4.35 - 4.52 (m, 1H), 6.63 (d, J=15.54Hz, 1H), 6.83 - 7.07 (m, 1H), 6.84 - 6. 88 (m, 1H), 7. 03 - 7.05 (m, 1H), 7.26 - 7.31 (m, 1H), 12.98 (brs, 1H) |
| 80 | 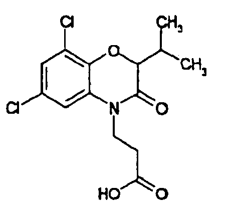 | (400 MHz, DMSO-d₆) 0.93 (d, J=6.72Hz, 3H), 0. 99 (d, J=6. 96Hz, 3H), 2.04 - 2.12 (m, 1H), 2.44 - 2.60 (m, 2H), 4.04 - 4. 19 (m, 2H), 4.54 (d, J=6.49Hz, 1H), 7.32 (d, J=2.32Hz, 1H), 7.37 (d, J=2.32Hz, 1H), 12.39 (brs, 1H) |

**[Table 17]**

| Ex. No. | Structural formula | NMR |
|---|---|---|
| 81 | 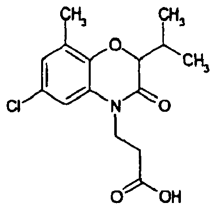 | (300 MHz, CDCl₃) 1.00 - 1. 11 (m, 6H), 2. 15 - 2. 27 (m, 4H), 2. 73 (t, J=7.70Hz, 2H), 4.09 - 4.31 (m, 3H), 6.82 - 6. 87 (m, 2H) |
| 82 | 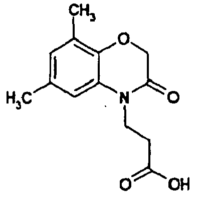 | (300 MHz, CDCl₃) 2.20 (s, 3H), 2. 30 (s, 3H), 2.73 - 2. 78 (m, 2H), 4. 22 (t, J=7.70Hz, 2H), 4. 57 (s, 2H), 6.66 - 6. 71 (m, 2H) |
| 83 | 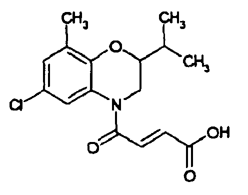 | (300 MHz, CDCl₃) 1.03 - 1. 11 (m, 6H), 1. 88 - 1. 99 (m, 1H), 2. 22 (s, 3H), 3.20 - 3. 35 (m, 1H), 3.92 - 3. 98 (m, 1H), 4. 54 (brs, 1H), 6.81 - 7. 00 (m, 3H), 7. 50 (d, J=15.40Hz, 1H) |
| 84 | 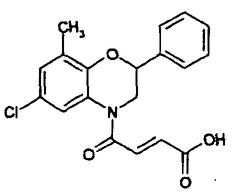 | (300 MHz, CDCl₃) 2. 29 (s, 3H), 3. 50 (brs, 1H), 4.60 (brs, 1H), 5.33 (dd, J=8.43, 2. 93Hz, 1H), 6.86 - 7. 06 (m, 3H), 7.35 - 7. 50 (m, 6H) |
| 85 | 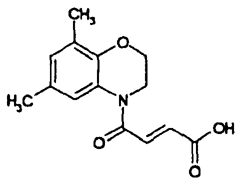 | (300 MHz, CDCl₃) 2. 18 (s, 3H), 2. 24 (s, 3H), 4. 02 (t, J=4. 80Hz, 2H), 4. 36 (t, J=4.40Hz, 2H), 6. 57 (brs, 1H), 6. 83 (s, 1H), 6.92 (d, J=15.40Hz, 1H), 7.56 (d, J=15.40Hz, 1H) |

**[Table 18]**

| Ex. No. | Structural formula | NMR |
|---|---|---|
| 86 | 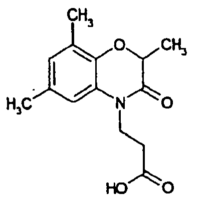 | (400 MHz, DMSO-d₆) 1.39 (d, J=6.72Hz, 3H), 2.14 (s, 3H), 2.25 (s, 3H), 2.48 - 2.52 (m, 2H), 4.04 - 4.09 (m, 2H), 4. 60 (q, J=6.72Hz, 1H), 6. 71 (s, 1H), 6.88 (s, 1H), 12. 38 (brs, 1H) |
| 87 | 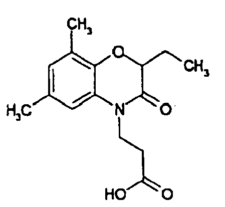 | (400 MHz, DMSO-d₆) 0.98 (t, J=7.30Hz, 3H), 1. 59 - 1. 82 (m, 2H), 2. 15 (s, 3H), 2. 24 (s, 3H), 2.48 - 2. 52 (m, 2H), 4.04 - 4. 08 (m, 2H), 4. 44 (dd, J=8. 93, 4. 29Hz, 1H), 6.71 (s, 1H), 6.87 (s, 1H), 12.38 (brs, 1H) |
| 88 | 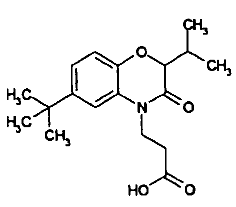 | (400 MHz, DMSO-d₆) 0. 93 (d, J=6. 72Hz, 3H), 0.97 (d, J=6. 96Hz, 3H), 1. 28 (s, 9H), 2.01 - 2. 13 (m, 1H), 2.45 - 2. 58 (m, 2H), 4.09 - 4. 22 (m, 2H), 4. 29 (d, J=6.49Hz, 1H), 6.92 (d, J=8.35Hz, 1H), 7.01 (dd, J=8. 35, 2. 09Hz, 1H), 7. 15 (d, J=2.09Hz, 1H), 12.40 (brs, 1H) |
| 89 | 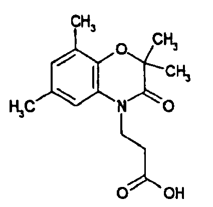 | (400 MHz, CDCl₃) 1. 47 (s, 6H), 2. 19 (s, 3H), 2.29 (s, 3H), 2. 73 (t, J=7. 64Hz, 2H), 4.20 (t, J=7.64Hz, 2H), 6.61 (s, 1H), 6.70 (s, 1H) |
| 90 | 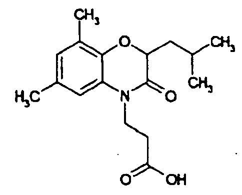 | (300 MHz, CDCl₃) 0.96 - 1. 00 (m, 6H), 1. 58 - 1. 78 (m, 2H), 1.87 - 1. 96 (m, 1H), 2.21 (s, 3H), 2.27 (s, 3H), 2.74 (t, J=7.70Hz, 2H), 4.20 (t, J=6.96Hz, 2H), 4.54 (dd, J=10. 30, 4.03Hz, 1H), 6. 64 (s, 1H), 6. 70 (s, 1H) |

**[Table 19]**

| Ex. No. | Structural formula | NMR |
|---|---|---|
| 91 | 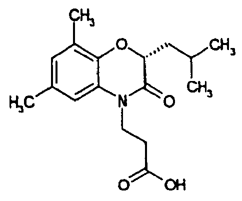 | (300 MHz, CDCl₃) 0. 96 (d, J=5.13Hz, 3H), 0.99 (d, J=5.13Hz, 3H), 1.58 - 1. 78 (m, 2H), 1.91 - 1. 96 (m, 1H), 2.21 (s, 3H), 2.29 (s, 3H), 2. 73 (dd, J=6. 23, 1.47Hz, 4H), 4.18 - 4. 23 (m, 2H), 4. 55 (dd, J=9. 89, 3.67Hz, 1H), 6.63 (s, 1H), 6. 70 (s, 1H) |
| 92 | 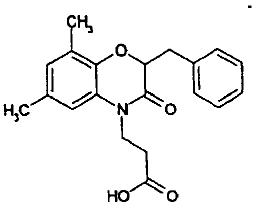 | (400 MHz, DMSO-d₆) 1. 90 (s, 3H), 2. 24 (s, 3H), 2.49 - 2. 54 (m, 2H), 2. 89 (dd, J=14.44, 9.97Hz, 1H), 3.12 (dd, J=14.44, 3. 65Hz, 1H), 4.06 - 4. 10 (m, 2H), 4. 79 (dd, J=9. 97, 3.65Hz, 1H), 6. 68 (s, 1H), 6.89 (s, 1H), 7.21 - 7. 25 (m, 3H), 7.28 - 7.32 (m, 2H) |
| 93 | 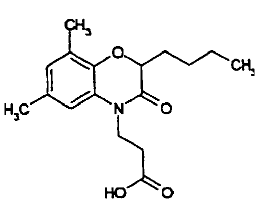 | (400 MHz, DMSO-d₆) 0. 86 (t, J=7.30Hz, 3H), 1.23 - 1. 51 (m, 4H), 1.58 - 1. 78 (m, 2H), 2.14 (s, 3H), 2. 24 (s, 3H), 2. 47 - 2. 52 (m, 2H), 4. 06 (t, J=7. 65Hz, 2H), 4. 49 (dd, J=9.04, 4.17Hz, 1H), 6.71 (s, 1H), 6.87 (s, 1H), 12.38 (brs, 1H) |
| 94 | 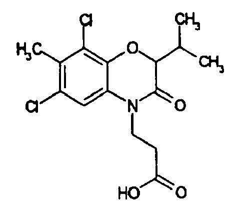 | (400 MHz, DMSO-d₆) 0.93 (d, J=6.72Hz, 3H), 0.99 (d, J=6.72Hz, 3H), 2.02 - 2.10 (m, 1H), 2. 37 (s, 3H), 2.44 - 2. 59 (m, 2H), 4. 03 - 4. 18 (m, 2H), 4. 52 (d, J=6. 72Hz, 1H), 7.39 (s, 1H), 12.40 (brs, 1H) |
| 95 | 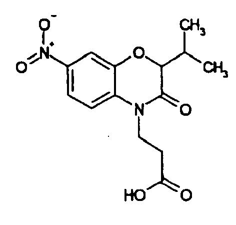 | (400 MHz, DMSO-d₆) 0.95 (d, J=6.49Hz, 3H), 1.05 (d, J=6.96Hz, 1H), 2.10 - 2.20 (m, 1H), 2.50 - 2. 67 (m, 2H), 4.10 - 4. 26 (m, 2H), 4. 57 (d, J=5. 57Hz, 1H), 7. 47 (d, J=8.81Hz, 1H), 7.83 (d, J=2.32Hz, 1H), 7.95 (dd, J=8.81, 2. 78Hz, 1H), 12.44 (s, 1H) |

**[Table 20]**

| Ex. No. | Structural formula | NMR |
|---|---|---|
| 96 | 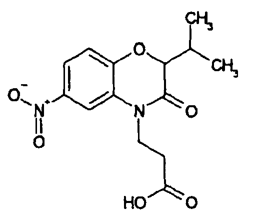 | (400 MHz, DMSO-d₆) 0.92 (d, J=6.96Hz, 3H), 1. 00 (d, J=6.49Hz, 1H), 2.12 - 2. 21 (m, 1H), 2.53 - 2. 64 (m, 2H), 4.13 - 4. 28 (m, 2H), 4.64 (d, J=5. 57Hz, 1H), 7. 25 (d, J=8.81Hz, 1H), 7. 94 (dd, J=8. 81, 2.78Hz, 1H), 8.05 (d, J=2.32Hz, 1H), 12. 44 (s, 1H) |
| 97 | 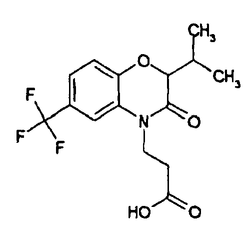 | (400 MHz, DMSO-d₆) 0.93 (d, J=6. 49Hz, 3H), 0.99 (d, J=6.96Hz, 1H), 2.09 - 2.17 (m, 1H), 2.47 - 2.61 (m, 2H), 4.10 - 4.24 (m, 2H), 4.51 (d, J=6. 03Hz, 1H), 7.21 (d, J=8. 35Hz, 1H), 7. 94 (dd, J=8. 35, 2.04Hz, 1H), 7.57 (d, J=1.86Hz, 1H), 12.39 (s, 1H) |
| 98 | 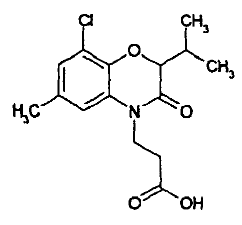 | (300 MHz, CDCl₃) 1.03 (d, J=6.60Hz, 3H), 1.06 (d, J=6.96Hz, 3H), 2. 07 - 2.18 (m, 1H), 2.31 (s, 3H), 2. 73 (t, J=7. 70Hz, 2H), 4.13 - 4.37 (m, 3H), 6.68 (s, 1H), 6.90 (d, J=1.10Hz, 1H) |
| 99 | 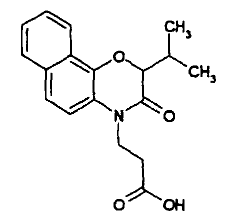 | (400 MHz, DMSO-d₆) 0. 97 (d, J=6. 49Hz, 3H), 1.06 (d, J=6.96Hz, 1H), 2.10 - 2.20 (m, 1H), 2.49 - 2.64 (m, 2H), 4.16 - 4.30 (m, 2H), 4.54 (d, J=6.49Hz, 1H), 7. 44 - 7.57 (m, 3H), 7.66 (d, J=8.81Hz, 1H), 7.90 (d, J=7.88Hz, 1H), 8.06 (d, J=8.35Hz, 1H), 12.41 (s, 1H) |
| 100 | 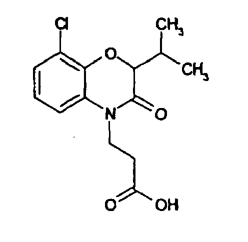 | (400 MHz, DMSO-d₆) 0.94 (d, J=6.96Hz, 3H), 1.00 (d, J=6.96Hz, 1H), 2.02 - 2. 11 (m, 1H), 2. 45 - 2.59 (m, 2H), 4. 04 - 4.19 (m, 2H), 4. 50 (d, J=6. 96Hz, 1H), 7. 05 (d, J=8.35Hz, 1H), 7.16 (dd, J=8.35, 1.39Hz, 1H), 7. 21 (dd, J=8. 35, 1. 39Hz, 1H), 12. 40 (s, 1H) |

**[Table 21]**

| Ex. No. | Structural formula | NMR |
|---|---|---|
| 101 | 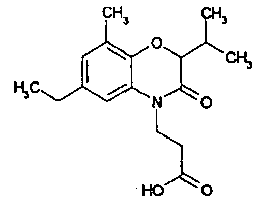 | (400 MHz, DMSO-d₆) 0. 92 (d, J=6. 72Hz, 3H), 0. 98 (d, J=6. 96Hz, 3H), 1.16 (t, J=7. 65Hz, 3H), 2.01 - 2. 10 (m, 1H), 2. 18 (s, 3H), 2.43 - 2. 56 (m, 4H), 4.01 - 4.16 (m, 2H), 4. 28 (d, J=6. 26Hz, 1H), 6. 74 (d, J=1.28Hz, 1H), 6. 88 (d, J=1.28Hz, 1H), 12.39 (brs, 1H) |
| 102 | 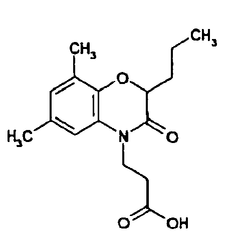 | (400 MHz, DMSO-d₆) 0.91 (t, J=7. 42Hz, 3H), 1.38 - 1. 54 (m, 1H), 1.58 - 1. 74 (m, 1H), 2. 14 (s, 3H), 2. 24 (s, 3H), 2.44 - 2. 56 (m, 2H), 4. 06 (t, J=7. 42Hz, 3H), 4. 51 (q, J=4.64Hz, 1H), 6. 71 (s, 1H), 6.87 (s, 1H), 12.37 (s, 1H) |
| 103 | 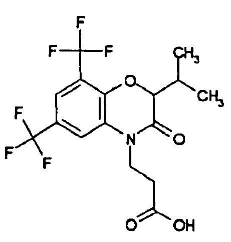 | (400 MHz, DMSO-d₆) 0. 96 (d, J=6. 96Hz, 3H), 1.03 (d, J=6.96Hz, 1H), 2.26 - 2.34 (m, 1H), 2.51 - 2.63 (m, 2H), 4.15 - 4. 29 (m, 2H), 4. 78 (d, J=4.17Hz, 1H), 7.64 (s, 1H), 7.91 (s, 1H), 12.41 (s, 1H) |
| 104 | 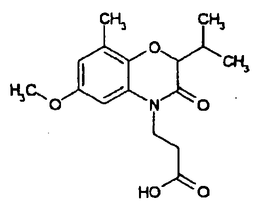 | (400 MHz, DMSO-d₆) 0.92 (d, J=6.72Hz, 3H), 0.98 (d, J=6.96Hz, 3H), 2.01 - 2.09 (m, 1H), 2. 19 (s, 3H), 2.46 - 2. 53 (m, 2H), 3.72 (s, 3H), 4.01 - 4.16 (m, 2H), 4. 24 (d, J=6.49Hz, 1H), 6. 50 (d, J=2. 55Hz, 1H), 6.61 (d, J=2.55Hz, 1H), 12.38 (brs, 1H) |
| 105 | 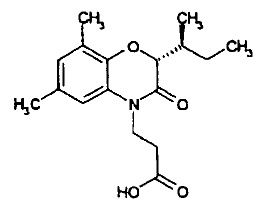 | (400 MHz, DMSO-d₆) 0.75 - 1. 00 (m, 6H), 1.10 - 1. 60 (m, 2H), 1.75 - 2. 00 (m, 1H), 2. 15 (s, 3H), 2. 24 (s, 3H), 2.45 - 2. 60 (m, 2H), 3.95 - 4. 20 (m, 2H), 4.30 - 4. 45 (m, 1H), 6.70 (s, 1H), 6.86 (s, 1H), 12.37 (s, 1H) |

**[Table 22]**

| Ex. No. | Structural formula | NMR |
|---|---|---|
| 106 | 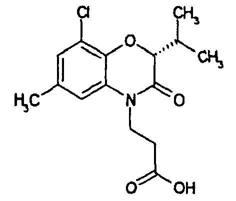 | (300 MHz, CDCl₃) 1. 03 (d, J=6. 96Hz, 3H), 1. 06 (d, J=6. 60Hz, 3H), 2.07 - 2. 18 (m, 1H), 2.27 (s, 3H), 2. 73 (t, J=7.70Hz, 2H), 4.12 - 4. 37 (m, 3H), 6. 68 (s, 1H), 6.90 (s, 1H) |
| 107 | 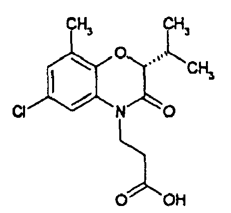 | (300 MHz, CDCl₃) 1. 00 (d, J=6. 96Hz, 3H), 1. 06 (d, J=6.96Hz, 3H), 2.15 - 2. 28 (m, 4H), 2. 74 (t, J=7. 70Hz, 2H), 4.09 - 4.31 (m, 3H), 6.81 (d, J=2.19Hz, 1H), 6.87 (d, J=1.83Hz, 1H) |
| 108 | 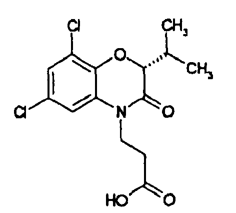 | (400 MHz, DMSO-d₆) 0. 93 (d, J=6.72Hz, 3H), 0.99 (d, J=6.96Hz, 3H), 2.04 - 2.12 (m, 1H), 2.45 - 2.59 (m, 2H), 4.02 - 4. 19 (m, 2H), 4. 54 (d, J=6.49Hz. 1H), 7. 32 (d, J=2.09Hz, 1H), 7.38 (d, J=2.09Hz, 1H), 12.40 (brs, 1H) |

**[Table 23]**

| Ex. No. | Structural formula | NMR |
|---|---|---|
| 109 | 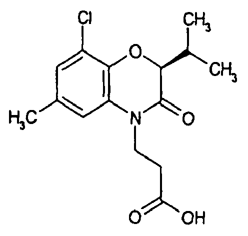 | (400 MHz, CDCl₃) 1. 04 (d, J=6. 48Hz, 3H), 1. 07 (d, J=6. 96Hz, 3H), 2. 09 - 2. 18 (m, 1H), 2. 32 (s, 3H), 2. 75 (t, J=7. 64Hz, 2H), 4. 15 - 4. 30 (m, 2H), 4. 37 (d, J=7.20Hz, 1H), 6. 69 (d, J=1.16Hz, 1H), 6. 92 (d, J=0.92Hz, 1H) |
| 110 | 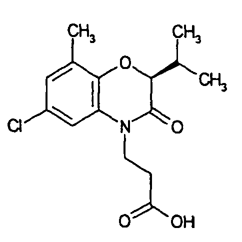 | (400 MHz, CDCl₃) 1. 01 (d, J=6. 48Hz, 3H), 1. 08 (d, J=6. 72Hz, 3H), 2.17 - 2. 26 (m, 1H), 2. 26 (s, 3H), 2. 75 (t, J=7. 68Hz, 2H), 4.12 - 4. 28 (m, 2H), 4. 31 (d, J=6. 48Hz, 1H), 6.83 (d, J=2. 32Hz, 1H), 6.88 (dd, J=2.32, 0.68Hz, 1H) |
| 111 | 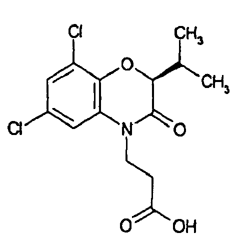 | (400 MHz, DMSO-d₆) 0. 94 (d, J=6. 48Hz, 3H), 1. 00 (d, J=6. 92Hz, 3H), 2. 05 - 2. 13 (m, 1H), 2.45 - 2. 59 (m, 2H), 4.05 - 4. 20 (m, 2H), 4. 55 (d, J=6. 48Hz, 1H), 7. 34 (d, J=2. 08Hz, 1H), 7. 39 (d, J=2. 32Hz, 1H), 12. 47 (brs, 1H) |

The present invention is explained in detail by referring to the following Preparation Examples, which are not to be construed as limitative.

### Formulation Example 1 (production of capsule)

| | | |
|---|---|---|
| 1) | compound of Example 1 | 30 mg |
| 2) | microcrystalline cellulose | 10 mg |
| 3) | lactose | 19 mg |
| 4) | magnesium stearate | 1 mg |

| | | |
|---|---|---|
| 1), 2), 3) and 4) are admixed and filled in a gelatin capsule. | | |

### Formulation Example 2 (production of tablet)

| | | |
|---|---|---|
| 1) | compound of Example 1 | 30 g |
| 2) | lactose | 50 g |
| 3) | cornstarch | 15 g |
| 4) | carboxymethylcellulose calcium | 44 g |
| 5) | magnesium stearate | 1 g |

The total amount of 1), 2) and 3) and 30 g of 4) are kneaded with water, vacuum dried and sized. This sized powder is admixed with 14 g of 4) and 1 g of 5) and the mixture is punched with a tableting machine. In this way, 1000 tablets containing 30 mg of the compound of Example 1 per tablet are obtained.
Capsule or tablet can be produced for the compounds of Examples 2 to 111 in the same manner as in the above-mentioned Formulation Example 1 or Formulation Example 2.

### Experimental Example 1 uric acid transport inhibitory test using human URAT1-expressing cells

Human URAT1 full length cDNA was subcloned to expression vector pcDNA3.1 and human URAT1 gene was transfected into human embryonic kidney derived cell line (HEK293 cells) by liposome method using Lipofectamine2000. Simultaneously, HEK293 cells transfected with expression vector pcDNA3.1 alone (hereinafter mock cells) were also produced. HEK293 cells expressing human URAT1 gene or mock cells were selected with geneticin resistance as an index. The functional expression of human URAT1 gene was confirmed by a method similar to the following method, using ¹⁴C-uric acid transport into the cells as an index.

Human URAT1-expressing HEK293 cells or mock cells were cultured in Dulbecco's modified Eagle's MEM medium (high glucose) containing 10% fetal bovine serum, 0.5 mg/mL geneticin sulfate, 100 units/mL penicillin and 100 µg/mL streptomycin under the conditions of 37°C and 5% CO₂ in an incubator. The cells were plated in a 96 well plate (poly-D-Lysine coated) at 1x10⁵ cells/well and the following uric acid transport inhibitory test was performed 24 hr later. This test was performed at room temperature.

After the medium was removed by aspiration from each well, the cells were washed once with Hank's Balanced Salt Solution (HBSS) and preincubated with HBSS (100 µL/well) for 5 min. HBSS was aspirated and an assay buffer (wherein NaCl in the above-mentioned HBSS had been substituted with Na-gluconate) containing various concentrations of the Example compound and a radioactive ligand (¹⁴C-uric acid; final concentration 50 µM) was added to each well at 50 µL/well and an uptake reaction was carried out for 5 min. After the reaction, the cells were washed twice with ice-cold HBSS (150 µL/well), and Microscinti TM 20 (PerkinElmer) was added at 50 µL/well. The cells were lysed by stirring and the radioactivity of each well was measured by a liquid scintillation counter (TOP COUNT, Packard).

The uric acid transport rate (%) of the Example compound at each concentration was calculated relative to the radioactivity (difference in radioactivity between human URAT1-expressing HEK293 cells and mock cells without addition of Example compound (DMSO addition)) showing URAT1 specific uric acid transport as 100%, and the concentration (IC₅₀) of the Example compound necessary for inhibiting the uric acid transport rate by 50% was determined. The results are shown in Table 24 - Table 27. In Table 24 - Table 27, "+++" means IC₅₀ value of less than 300 nM, "++" means IC₅₀ value of 300 nM to less than 3000 nM, and "+" means IC₅₀ value of not less than 3000 nM.

**[Table 24]**

| Example No. | hURAT1 IC₅₀ |
|---|---|
| 1 | + |
| 2 | + |
| 3 | + |
| 4 | + |
| 5 | + |
| 6 | + |
| 7 | ++ |
| 8 | ++ |
| 9 | + |
| 10 | + |
| 11 | + |
| 12 | + |
| 13 | + |
| 14 | ++ |
| 15 | + |
| 16 | ++ |
| 17 | + |
| 18 | + |
| 19 | + |
| 20 | + |
| 21 | + |
| 22 | ++ |
| 23 | + |
| 24 | + |
| 25 | + |
| 26 | + |
| 27 | + |
| 28 | + |
| 29 | + |
| 30 | + |

**[Table 25]**

| Example No. | hURAT1 IC₅₀ |
|---|---|
| 31 | + |
| 32 | + |
| 33 | + |
| 34 | + |
| 35 | + |
| 36 | ++ |
| 37 | ++ |
| 38 | +++ |
| 39 | + |
| 40 | ++ |
| 41 | + |
| 42 | ++ |
| 43 | ++ |
| 44 | +++ |
| 45 | ++ |
| 46 | + |
| 47 | ++ |
| 48 | + |
| 49 | ++ |
| 50 | + |
| 51 | ++ |
| 52 | + |
| 53 | ++ |
| 54 | + |
| 55 | + |
| 56 | + |
| 57 | + |
| 58 | ++ |
| 59 | ++ |
| 60 | ++ |

**[Table 26]**

| Example No. | hURAT1 IC₅₀ |
|---|---|
| 61 | +++ |
| 62 | ++ |
| 63 | +++ |
| 64 | ++ |
| 65 | + |
| 66 | ++ |
| 67 | ++ |
| 68 | ++ |
| 69 | + |
| 70 | + |
| 71 | + |
| 72 | +++ |
| 73 | +++ |
| 74 | +++ |
| 75 | ++ |
| 76 | + |
| 77 | + |
| 78 | + |
| 79 | + |
| 80 | +++ |
| 81 | +++ |
| 82 | + |
| 83 | ++ |
| 84 | ++ |
| 85 | + |
| 86 | + |
| 87 | +++ |
| 88 | ++ |
| 89 | + |
| 90 | +++ |

**[Table 27]**

| Example No. | hURAT1 IC₅₀ |
|---|---|
| 91 | +++ |
| 92 | + |
| 93 | ++ |
| 94 | +++ |
| 95 | + |
| 96 | ++ |
| 97 | ++ |
| 98 | +++ |
| 99 | + |
| 100 | +++ |
| 101 | +++ |
| 102 | +++ |
| 103 | ++ |
| 104 | +++ |
| 105 | +++ |
| 106 | +++ |
| 107 | +++ |
| 108 | +++ |
| 109 | ++ |
| 110 | ++ |
| 111 | ++ |

### Experimental Example 2 CYP inhibitory test using human liver microsome

Human liver microsome (20 mg protein/mL, 5 µL, Xenotech LLC, purchased from Lenexa KS) was suspended in 100 mM potassium phosphate buffer (70 µL, pH 7.4), mixed with a solution (0.5 µL) of the Example compound dissolved in DMSO and preincubated at 37°C for 5 min. NADPH producing system coenzyme solution (β-nicotinamide adenine dinucleotide phosphate: 5.2 mM, D-glucose-6-phosphate: 13.2 mM, magnesium chloride: 13.2 mM, glucose-6-phosphate dehydrogenase: 1.8 U/mL) 25 µL, and a model substrate (CYP3A4: midazolam 1 mM, CYP2D6: bufuralol 1 mM, CYP2C9: diclofenac 2 mM) 0.5 µL dissolved in DMSO were added to start the reaction. After incubation at 37°C for 10 min., acetonitrile (200 µL) containing an internal standard substance (propranolol 1 µM) was added and the mixture was centrifuged (room temperature, 3000 rpm, 20 min). The amount of the metabolite produced from each model substrate in the supernatant was measured by high performance liquid chromatography/mass spectrometry (LC/MS/MS) and each CYP enzyme activity was determined. The concentration showing 50% inhibition (IC₅₀) was calculated relative to the enzyme activity without addition of Example compound (DMSO 0.5 µL added) as 100%. The results are shown in Table 28.

As a comparative example, the CYP enzyme activity of the compound described in JP-A-3-135917 was examined by determining the IC₅₀ value in the same manner as above. The result is also shown in Table 28.

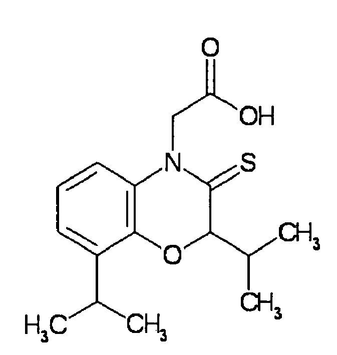

**[Table 28]**

| Example No. | CYP2C9 inhibition, IC₅₀ (µM) |
|---|---|
| 9 | 30 |
| 14 | >50 |
| 16 | >50 |
| 36 | >50 |
| 37 | >50 |
| 38 | >50 |
| 54 | >50 |
| 55 | >50 |
| 56 | >50 |
| 64 | >50 |
| 65 | >50 |
| 69 | >50 |
| 73 | >50 |
| 74 | >50 |
| 83 | >50 |
| 84 | >50 |
| 91 | >50 |
| 94 | >50 |
| 101 | >50 |
| 102 | >50 |
| 105 | >50 |
| 106 | >50 |
| 107 | >50 |
| 108 | >50 |
| 109 | >50 |
| 110 | >50 |
| 111 | >50 |
| Comparative Example | 16 |

As is clear from the above-mentioned Experimental Example 1 (uric acid transport inhibitory test using human URAT1 expression cells), the compound of the present invention, a pharmaceutically acceptable salt thereof and a solvate thereof have a superior inhibitory action on URAT1 activity. As is clear from Experimental Example 2 (CYP inhibitory test), the compound of the present invention, a pharmaceutically acceptable salt thereof and a solvate thereof have no or extremely low CYP inhibitory action as compared to the compound of the Comparative Example (compound described in JP-A-3-135917).

They indicate that the compound of the present invention, a pharmaceutically acceptable salt thereof and a solvate thereof have an effect of strong suppression of reabsorption of uric acid, and very low fear of side effect since they do not substantially inhibit CYP.

Therefore, the compound of the present invention inhibits reabsorption of uric acid by inhibiting URAT1 activity, whereby affords an agent for the prophylaxis or treatment of pathology showing involvement of uric acid, such as hyperuricemia, gouty tophus, acute gouty arthritis, chronic gouty arthritis, gouty kidney, urolithiasis, renal function disorder, coronary artery disease, ischemic heart disease and the like.

Moreover, as the causal disease of the above-mentioned pathology showing involvement of uric acid, complications or diseases highly possibly complicated, for example, gout arthritis, urolithiasis, hypertension or hypertensive complications, hyperlipidemia or hyperlipidemic complications, diabetes or diabetic complications, obesity or obesity complications, decreased uric acid excretion secondary hyperuricemia and the like can be mentioned. A combined use of an agent for the prophylaxis or treatment of these diseases including hyperuricemia and a pharmaceutically effective amount of the compound of the present invention or a pharmaceutically acceptable salt thereof or a solvate thereof is effective for the prophylaxis or treatment of these diseases. In addition, a combined use of a pharmaceutical agent that increases the blood uric acid level and a pharmaceutically effective amount of the compound of the present invention or a pharmaceutically acceptable salt thereof or a solvate thereof is effective for suppressing an increase in the blood uric acid level.

### [Industrial Applicability]

The present invention is useful for the prophylaxis or treatment of pathology showing involvement of uric acid, such as hyperuricemia, gouty tophus, acute gouty arthritis, chronic gouty arthritis, gouty kidney, urolithiasis, renal function disorder, coronary artery disease, ischemic heart disease and the like.

## Claims

1. A URAT1 activity inhibitor comprising a compound represented by the following formula [1] or a pharmaceutically acceptable salt thereof, or a solvate thereof 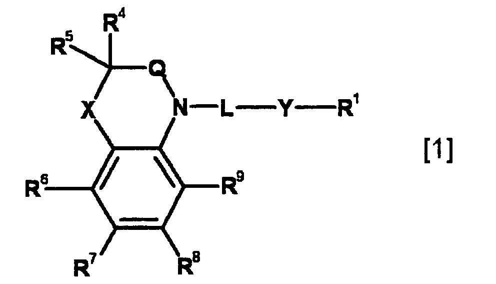 wherein
Q is
1) -CH2- or
2) -(C=Z)-:
Z is
1) an oxygen atom, or
2) a sulfur atom:
X is
1) an oxygen atom,
2) a sulfur atom,
3) -S(=O)- or
4) -S(=O)₂-:
L is
1) a single bond, or
2) -(C=O)-:
Y is
1) a single bond,
2) a C₁₋₄ alkylene group optionally substituted by the same or different one or more substituents selected from the following group A,
3) a C₂₋₄ alkenylene group optionally substituted by the same or different one or more substituents selected from the following group A,
4) a C₂₋₄ alkynylene group optionally substituted by the same or different one or more substituents selected from the following group A, or
5) a C₁₋₄ alkylene-C₆₋₁₄ arylene group optionally substituted by the same or different one or more substituents selected from the following group A:
R¹ is
1) -COOH, or
2) -COOR wherein R is
(a) a C₁₋₆ alkyl group optionally substituted by the same or different one or more substituents selected from the following group A,
(b) an allyl group optionally substituted by the same or different one or more substituents selected from the following group A,
(c) a phenyl group optionally substituted by the same or different one or more substituents selected from the following group A, or
(d) a benzyl group optionally substituted by the same or different one or more substituents selected from the following group A;
R⁴ and R⁵ are the same or different and each is
1) a hydrogen atom,
2) a C₁₋₆ alkyl group optionally substituted by the same or different one or more substituents selected from the following group A,
3) a C₂₋₆ alkenyl group optionally substituted by the same or different one or more substituents selected from the following group A,
4) a C₆₋₁₄ aryl group optionally substituted by the same or different one or more substituents selected from the following group A,
5) a C₇₋₁₂ aralkyl group optionally substituted by the same or different one or more substituents selected from the following group A, or
6) a halogen atom, or
7) R⁴ and R⁵ form, together with the carbon atom bonded thereto, a saturated carbocycle having 3 to 6 carbon atoms (said carbocycle is optionally substituted by the same or different one or more substituents selected from the following group A); R⁶, R⁷, R⁸ and R⁹ are the same or different and each is
1) a hydrogen atom,
2) a halogen atom,
3) a C₁₋₆ alkyl group optionally substituted by the same or different one or more substituents selected from the following group A,
4) a C₁₋₆ alkoxy group optionally substituted by the same or different one or more substituents selected from the following group A,
5) a C₃₋₈ cycloalkyl group optionally substituted by the same or -different one or more substituents selected from the following group A,
6) a C₆₋₁₄ aryl group optionally substituted by the same or different one or more substituents selected from the following group A,
7) a nitro group,
8) a cyano group,
9) -COOH, or
10) -COOR wherein R is as defined above, or
11) R⁶ and R⁷, R⁷ and R⁸ or R⁸ and R⁹ form, together with the carbon atom bonded thereto, a saturated or unsaturated carbocycle having 3 to 14 carbon atoms (said carbocycle is optionally substituted by the same or different one or more substituents selected from the following group A),
provided that when
Q is -(C=Z)-,
Z is a sulfur atom,
L is a single bond, and
one of R⁴ and R⁵ is a hydrogen atom, and the other is an isopropyl group or a sec-butyl group,
Y is
1) a single bond,
2) a C₂₋₄ alkylene group optionally substituted by the same or different one or more substituents selected from the following group A,
3) a C₂₋₄ alkenylene group optionally substituted by the same or different one or more substituents selected from the following group A,
4) a C₂₋₄ alkynylene group optionally substituted by the same or different one or more substituents selected from the following group A, or
5) a C₁₋₄ alkylene-C₆₋₁₄ arylene group optionally substituted by the same or different one or more substituents selected from the following group A:
[group A]
1) a halogen atom,
2) -OR¹⁰ wherein R¹⁰ is
(a) a hydrogen atom,
(b) a C₁₋₆ alkyl group optionally substituted by the same or different one or more substituents selected from the following group B, or
(c) -COR¹¹ wherein R¹¹ is
a) a hydrogen atom,
b) a hydroxyl group,
c) a C₁₋₆ alkyl group optionally substituted by the same or different one or more substituents selected from the following group B,
d) a C₁₋₆ alkoxy group (said C₁₋₆ alkoxy group is optionally substituted by the same or different one or more substituents selected from the following (i) and (ii),
(i) a substituent selected from the following group B,
(ii) a C₁₋₆ alkyl group optionally substituted by the same or different one or more substituents selected from the following group B),
e) a saturated or unsaturated carbocyclic group having 3 to 14 carbon atoms (said carbocyclic group is optionally substituted by the same or different one or more substituents selected from the following (i) and (ii),
(i) a substituent selected from the following group B,
(ii) a C₁₋₆ alkyl group optionally substituted by the same or different one or more substituents selected from the following group B),
f) a cycloalkylalkoxy group (said cycloalkylalkoxy group is optionally substituted by the same or different one or more substituents selected from the following (i) and (ii),
(i) a substituent selected from the following group B,
(ii) a C₁₋₆ alkyl group optionally substituted by the same or different one or more substituents selected from the following group B),
g) a C₇₋₁₂ aralkyl group (said C₇₋₁₂ aralkyl group is optionally substituted by the same or different one or more substituents selected from the following (i) and (ii),
(i) a substituent selected from the following group B,
(ii) a C₁₋₆ alkyl group optionally substituted by the same or different one or more substituents selected from the following group B), or
h) an aralkoxy group (said aralkoxy group is optionally substituted by the same or different one or more substituents selected from the following (i) and (ii),
(i) a substituent selected from the following group B,
(ii) a C₁₋₆ alkyl group optionally substituted by the same or different one or more substituents selected from the following group B),
3) a C₁₋₆ alkyl group optionally substituted by the same or different one or more substituents selected from the following group B,
4) a cycloalkylalkoxy group (said cycloalkylalkoxy group is optionally substituted by the same or different one or more substituents selected from the following (a) and (b),
(a) a substituent selected from the following group B,
(b) a C₁₋₆ alkyl group optionally substituted by the same or different one or more substituents selected from the following group B),
5) a C₇₋₁₂ aralkyl group (said C₇₋₁₂ aralkyl group is optionally substituted by the same or different one or more substituents selected from the following (a) and (b),
(a) a substituent selected from the following group B,
(b) a C₁₋₆ alkyl group optionally substituted by the same or different one or more substituents selected from the following group B),
6) an aralkoxy group (said aralkoxy group is optionally substituted by the same or different one or more substituents selected from the following (a) and (b),
(a) a substituent selected from the following group B,
(b) a C₁₋₆ alkyl group optionally substituted by the same or different one or more substituents selected from the following group B),
7) -COR¹¹ wherein R¹¹ is as defined above,
8) -NR¹²R¹³ wherein R¹² and R¹³ are the same or different and
each is
(a) a hydrogen atom, or
(b) a C₁₋₆ alkyl group optionally substituted by the same or different one or more substituents selected from the following group B, or
(c) R¹² and R¹³ optionally form, together with the nitrogen atom bonded thereto, monocyclic nitrogen-containing saturated heterocycle (said heterocycle is optionally substituted by the same or different one or more substituents selected from the following (i) and (ii),
(i) a substituent selected from the following group B,
(ii) a C₁₋₆ alkyl group optionally substituted by the same or different one or more substituents selected from the
following group B),
9) -CONR¹²R¹³ wherein R¹² and R¹³ is as defined above,
10) -NR¹⁴COR¹¹ wherein R¹¹ is as defined above, R¹⁴ is
(a) a hydrogen atom, or
(b) a C₁₋₆ alkyl group optionally substituted by the same or different one or more substituents selected from the following group B,
11) -NR¹⁴S(=O)₂R¹¹ wherein R¹¹ and R¹⁴ are as defined above,
12) -NR¹⁴CONR¹²R¹³ wherein R¹², R¹³ and R¹⁴ are as defined above,
13) -SR¹⁰ wherein R¹⁰ is as defined above,
14) -S(=O)R¹¹ wherein R¹¹ is as defined above,
15) -S(=O)₂R¹¹ wherein R¹¹ is as defined above,
16) -S(=O)₂NR¹²R¹³ wherein R¹² and R¹³ are as defined above,
17) a saturated or unsaturated carbocyclic group having 3 to 14 carbon atoms (said carbocyclic group is optionally substituted by the same or different one or more substituents selected from the following (a) and (b),
(a) a substituent selected from the following group B,
(b) a C₁₋₆ alkyl group optionally substituted by the same or different one or more substituents selected from the following group B),
18) a saturated or unsaturated heterocyclic group containing at least one hetero atom selected from a nitrogen atom, an oxygen atom and a sulfur atom (said heterocyclic group is optionally substituted by the same or different one or more substituents selected from the following (a) and (b),
(a) a substituent selected from the following group B,
(b) a C₁₋₆ alkyl group optionally substituted by the same or different one or more substituents selected from the following group B,
19) an aryloxy group (said aryloxy group is optionally substituted by the same or different one or more substituents selected from the following (a) and (b),
(a) a substituent selected from the following group B,
(b) a C₁₋₆ alkyl group optionally substituted by the same or different one or more substituents selected from the following group B,
20) a cyano group, and
21) a nitro group
[group B]
1) a halogen atom,
2) a hydroxyl group,
3) a C₁₋₆ alkoxy group,
4) -NR¹⁵R¹⁶ wherein R¹⁵ and R¹⁶ are the same or different and each is
(a) a hydrogen atom, or
(b) a C₁₋₆ alkyl group, or
(c) R¹⁵ and R¹⁶ optionally form, together with the nitrogen atom bonded thereto, monocyclic nitrogen-containing saturated heterocycle,
5) -CONR¹⁵R¹⁶ wherein R¹⁵ and R¹⁶ are as defined above,
6) -COR¹⁷ wherein R¹⁷ is
(a) a hydrogen atom,
(b) a hydroxyl group,
(c) a C₁₋₆ alkyl group, or
(d) a C₁₋₆ alkoxy group,
7) -NR¹⁸COR¹⁷ wherein R¹⁷ is as defined above, R¹⁸ is
(a) a hydrogen atom, or
(b) a C₁₋₆ alkyl group,
8) -NR¹⁸CONR¹⁵R¹⁶ wherein R¹⁵ R¹⁶ and R¹⁸ are as defined above,
9) -NR¹⁸S(=O)₂R¹⁹ wherein R¹⁸ is as defined above, R¹⁹ is a C₁₋₆ alkyl group, and
10) -S(=O)₂R¹⁹ wherein R¹⁹ is as defined above.

2. A compound represented by the following formula [1'] or a pharmaceutically acceptable salt thereof, or a solvate thereof. 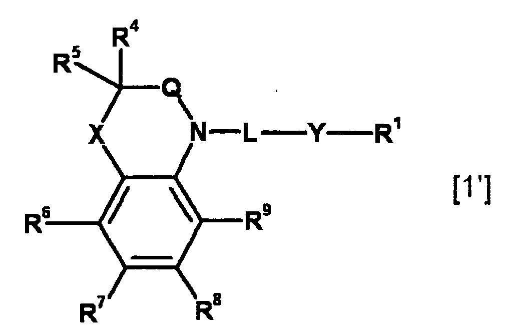 wherein
Q is
1) -CH₂- or
2) -(C=Z)-:
Z is
1) an oxygen atom, or
2) a sulfur atom:
X is
1) an oxygen atom,
2) a sulfur atom,
3) -S(=O)- or
4) -S(=O)₂-:
L is
1) a single bond, or
2) -(C=O)-:
Y is
1) a single bond,
2) a C₁₋₄ alkylene group optionally substituted by the same or different one or more substituents selected from the following group A,
3) a C₂₋₄ alkenylene group optionally substituted by the same or different one or more substituents selected from the following group A, or
4) a C₁₋₄ alkylene-C₆₋₁₄ arylene group optionally substituted by the same or different one or more substituents selected from the following group A:
R¹ is
1) -COOH, or
2) -COOR wherein R is
(a) a C₁₋₆ alkyl group optionally substituted by the same or different one or more substituents selected from the following group A,
(b) an allyl group optionally substituted by the same or different one or more substituents selected from the following group A,
(c) a phenyl group optionally substituted by the same or different one or more substituents selected from the following group A, or
(d) a benzyl group optionally substituted by the same or different one or more substituents selected from the following group A;
R⁴ and R⁵ are the same or different and each is
1) a hydrogen atom,
2) a C₁₋₆ alkyl group optionally substituted by the same or different one or more substituents selected from the following group A,
3) a C₂₋₆ alkenyl group optionally substituted by the same or different one or more substituents selected from the following group A,
4) a C₆₋₁₄ aryl group optionally substituted by the same or different one or more substituents selected from the following group A,
5) a C₇₋₁₂ aralkyl group optionally substituted by the same or different one or more substituents selected from the following group A, or
6) a halogen atom, or
7) R⁴ and R⁵ form, together with the carbon atom bonded thereto, a saturated carbocycle having 3 to 6 carbon atoms (said carbocycle is optionally substituted by the same or different one or more substituents selected from the following group A); R⁶, R⁷, R⁸ and R⁹ are the same or different and each is
1) a hydrogen atom,
2) a halogen atom,
3) a C₁₋₆ alkyl group optionally substituted by the same or different one or more substituents selected from the following group A,
4) a C₁₋₆ alkoxy group optionally substituted by the same or different one or more substituents selected from the following group A,
5) a C₃₋₈ cycloalkyl group optionally substituted by the same or different one or more substituents selected from the following group A,
6) a C₆₋₁₄ aryl group optionally substituted by the same or different one or more substituents selected from the following group A,
7) a nitro group,
8) a cyano group,
9) -COOH, or
10) -COOR wherein R is as defined above, or
11) R⁶ and R⁷, R⁷ and R⁸ or R⁸ and R⁹ form, together with the carbon atom bonded thereto, a saturated or unsaturated carbocycle having 3 to 14 carbon atoms (said carbocycle is optionally substituted by the same or different one or more substituents selected from the following group A), provided that when
(i) Q is -(C=Z)-, and
L is a single bond,
Y is
1) a single bond,
2) a C₂₋₄ alkylene group optionally substituted by the same or different one or more substituents selected from the following group A,
3) a C₂₋₄ alkenylene group optionally substituted by the same or different one or more substituents selected from the following group A, or
4) a C₁₋₄ alkylene-C₆₋₁₄ arylene group optionally substituted by the same or different one or more substituents selected from the following group A, and
R⁵ is
1) a C₂₋₆ alkyl group optionally substituted by the same or different one or more substituents selected from the following group A,
2) a C₂₋₆ alkenyl group optionally substituted by the same or different one or more substituents selected from the following group A,
3) a C₆₋₁₄ aryl group optionally substituted by the same or different one or more substituents selected from the following group A,
4) a C₇₋₁₂ aralkyl group optionally substituted by the same or different one or more substituents selected from the following group A, or
5) a halogen atom, or
6) R⁴ and R⁵ form, together with the carbon atom bonded thereto, a saturated carbocycle having 3 to 6 carbon atoms (said carbocycle is optionally substituted by the same or different one or more substituents selected from the following group A);
(ii) the following compounds are excluded:
3-[2-(4-methoxybenzyl)-3-oxo-2,3-dihydrobenzo[1,4]thiazin-4-yl]propionic acid,
(E)-4-(2,2-dimethyl-6-nitro-2,3-dihydrobenzo[1,4]oxazin-4-yl)-4-oxo-2-butenoic acid,
(6-methoxy-8-methyl-2,3-dihydrobenzo[1,4]oxazin-4-yl)acetic acid,
(2,2-dimethyl-6-nitro-2,3-dihydrobenzo[1,4]oxazin-4-yl)acetic acid, and
(2E,4E)-3-methyl-6-oxo-6-(6,6,9,9-tetramethyl-2,3,6,7,8,9-hexahydronaphtho[2,3-b][1,4]oxazin-4-yl)2,4-hexadienoic acid,
[group A]
1) a halogen atom,
2) -OR¹⁰ wherein R¹⁰ is
(a) a hydrogen atom,
(b) a C₁₋₆ alkyl group optionally substituted by the same or different one or more substituents selected from the following group B, or
(c) -COR¹¹ wherein R¹¹ is
a) a hydrogen atom,
b) a hydroxyl group,
c) a C₁₋₆ alkyl group optionally substituted by the same or different one or more substituents selected from the following group B,
d) a C₁₋₆ alkoxy group (said C₁₋₆ alkoxy group is optionally substituted by the same or different one or more substituents selected from the following (i) and (ii),
(i) a substituent selected from the following group B,
(ii) a C₁₋₆ alkyl group optionally substituted by the same or different one or more substituents selected from the following group B,
e) a saturated or unsaturated carbocyclic group having 3 to 14 carbon atoms (said carbocyclic group is optionally substituted by the same or different one or more substituents selected from the following (i) and (ii),
(i) a substituent selected from the following group B,
(ii) a C₁₋₆ alkyl group optionally substituted by the same or different one or more substituents selected from the following group B,
f) a cycloalkylalkoxy group (said cycloalkylalkoxy group is optionally substituted by the same or different one or more substituents selected from the following (i) and (ii),
(i) a substituent selected from the following group B,
(ii) a C₁₋₆ alkyl group optionally substituted by the same or different one or more substituents selected from the following group B,
g) a C₇₋₁₂ aralkyl group (said C₇₋₁₂ aralkyl group is optionally substituted by the same or different one or more substituents selected from the following (i) and (ii),
(i) a substituent selected from the following group B,
(ii) a C₁₋₆ alkyl group optionally substituted by the same or different one or more substituents selected from the following group B, or
h) an aralkoxy group (said aralkoxy group is optionally substituted by the same or different one or more substituents selected from the following (i) and (ii),
(i) a substituent selected from the following group B,
(ii) a C₁₋₆ alkyl group optionally substituted by the same or different one or more substituents selected from the following group B),
3) a C₁₋₆ alkyl group optionally substituted by the same or different one or more substituents selected from the following group B,
4) a cycloalkylalkoxy group (said cycloalkylalkoxy group is optionally substituted by the same or different one or more substituents selected from the following (a) and (b),
(a) a substituent selected from the following group B,
(b) a C₁₋₆ alkyl group optionally substituted by the same or different one or more substituents selected from the following group B,
5) a C₇₋₁₂ aralkyl group (said C₇₋₁₂ aralkyl group is optionally substituted by the same or different one or more substituents selected from the following (a) and (b),
(a) a substituent selected from the following group B,
(b) a C₁₋₆ alkyl group optionally substituted by the same or different one or more substituents selected from the following group B,
6) an aralkoxy group (said aralkoxy group is optionally substituted by the same or different one or more substituents selected from the following (a) and (b),
(a) a substituent selected from the following group B,
(b) a C₁₋₆ alkyl group optionally substituted by the same or different one or more substituents selected from the following group B,
7) -COR¹¹ wherein R¹¹ is as defined above,
8) -NR¹²R¹³ wherein R¹² and R¹³ are the same or different and each is
(a) a hydrogen atom, or
(b) a C₁₋₆ alkyl group optionally substituted by the same or different one or more substituents selected from the following group B, or
(c) R¹² and R¹³ optionally form, together with the nitrogen atom bonded thereto, monocyclic nitrogen-containing saturated heterocycle (said heterocycle is optionally substituted by the same or different one or more substituents selected from the following (i) and (ii),
(i) a substituent selected from the following group B,
(ii) a C₁₋₆ alkyl group optionally substituted by the same or different one or more substituents selected from the following group B),
9) -CONR¹²R¹³ wherein R¹² and R¹³ are as defined above,
10) -NR¹⁴COR¹¹ wherein R¹¹ is as defined above, R¹⁴ is
(a) a hydrogen atom, or
(b) a C₁₋₆ alkyl group optionally substituted by the same or different one or more substituents selected from the following group B,
11) -NR¹⁴S(=O)₂R¹¹ wherein R¹¹ and R¹⁴ are as defined above,
12) -NR¹⁴CONR¹²R¹³ wherein R¹², R¹³ and R¹⁴ are as defined above, 13) -SR¹⁰ wherein R¹⁰ is as defined above,
14) -S(=O)R¹¹ wherein R¹¹ is as defined above,
15) -S(=O)₂R¹¹ wherein R¹¹ is as defined above,
16) -S(=O)₂NR¹²R¹³ wherein R¹² and R¹³ are as defined above,
17) a saturated or unsaturated carbocyclic group having 3 to 14 carbon atoms (said carbocyclic group is optionally substituted by the same or different one or more substituents selected from the following (a) and (b),
(a) a substituent selected from the following group B,
(b) a C₁₋₆ alkyl group optionally substituted by the same or different one or more substituents selected from the following group B,
18) a saturated or unsaturated heterocyclic group containing at least one hetero atom selected from a nitrogen atom, an oxygen atom and a sulfur atom (said heterocyclic group is optionally substituted by the same or different one or more substituents selected from the following (a) and (b),
(a) a substituent selected from the following group B,
(b) a C₁₋₆ alkyl group optionally substituted by the same or different one or more substituents selected from the following group B,
19) an aryloxy group (said aryloxy group is optionally substituted by the same or different one or more substituents selected from the following (a) and (b),
(a) a substituent selected from the following group B,
(b) a C₁₋₆ alkyl group optionally substituted by the same or different one or more substituents selected from the following group B,
20) a cyano group, and
21) a nitro group,
[group B]
1) a halogen atom,
2) a hydroxyl group,
3) a C₁₋₆ alkoxy group,
4) -NR¹⁵R¹⁶ wherein R¹⁵ and R¹⁶ are the same or different and each is
(a) a hydrogen atom, or
(b) a C₁₋₆ alkyl group, or
(c) R¹⁵ and R¹⁶ optionally form, together with the nitrogen atom bonded thereto, monocyclic nitrogen-containing saturated heterocycle,
5) -CONR¹⁵R¹⁶ wherein R¹⁵ and R¹⁶ are as defined above,
6) -COR¹⁷ wherein R¹⁷ is
(a) a hydrogen atom,
(b) a hydroxyl group,
(c) a C₁₋₆ alkyl group, or
(d) a C₁₋₆ alkoxy group,
7) -NR¹⁸COR¹⁷ wherein R¹⁷ is as defined above, R¹⁸ is
(a) a hydrogen atom, or
(b) a C₁₋₆ alkyl group,
8) -NR¹⁸CONR¹⁵R¹⁶ wherein R¹⁵, R¹⁶ and R¹⁸ are as defined above,
9) -NR¹⁸S(=O)₂R¹⁹ wherein R¹⁸ is as defined above, R¹⁹ is a C₁₋₆ alkyl group, and
10) -S(=O)₂R¹⁹ wherein R¹⁹ is as defined above.

3. The compound of claim 2, which is represented by the following formula [A-1], or a pharmaceutically acceptable salt thereof, or a solvate thereof: 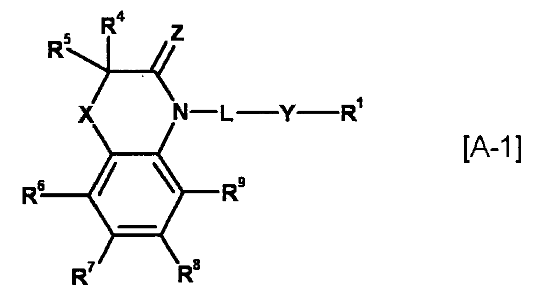 wherein
Z is
1) an oxygen atom, or
2) a sulfur atom:
X is
1) an oxygen atom,
2) a sulfur atom,
3) -S(=O)- or
4) -S(=O)₂-_{:}
L is
1) a single bond, or
2) -(C=O)-:
Y is
1) a single bond,
2) a C₁₋₄ alkylene group optionally substituted by the same or different one or more substituents selected from the aforementioned group A,
3) a C₂₋₄ alkenylene group optionally substituted by the same or different one or more substituents selected from the aforementioned group A, or
4) a C₁₋₄ alkylene-C₆₋₁₄ arylene group optionally substituted by the same or different one or more substituents selected from the aforementioned group A:
R¹ is
1) -COOH, or
2) -COOR wherein R is
(a) a C₁₋₆ alkyl group optionally substituted by the same or different one or more substituents selected from the aforementioned group A,
(b) an allyl group optionally substituted by the same or different one or more substituents selected from the aforementioned group A,
(c) a phenyl group optionally substituted by the same or different one or more substituents selected from the aforementioned group A, or
(d) a benzyl group optionally substituted by the same or different one or more substituents selected from the aforementioned group A;
R⁴ and R⁵ are the same or different and each is
1) a hydrogen atom,
2) a C₁₋₆ alkyl group optionally substituted by the same or different one or more substituents selected from the aforementioned group A,
3) a C₂₋₆ alkenyl group optionally substituted by the same or different one or more substituents selected from the aforementioned group A,
4) a C₆₋₁₄ aryl group optionally substituted by the same or different one or more substituents selected from the aforementioned group A,
5) a C₇₋₁₂ aralkyl group optionally substituted by the same or different one or more substituents selected from the aforementioned group A, or
6) a halogen atom, or
7) R⁴ and R⁵ form, together with the carbon atom bonded thereto, a saturated carbocycle having 3 to 6 carbon atoms (said carbocycle is optionally substituted by the same or different one or more substituents selected from the aforementioned group A);
R⁶, R⁷, R⁸ and R⁹ are the same or different and each is
1) a hydrogen atom,
2) a halogen atom,
3) a C₁₋₆ alkyl group optionally substituted by the same or different one or more substituents selected from the aforementioned group A,
4) a C₁₋₆ alkoxy group optionally substituted by the same or different one or more substituents selected from the aforementioned group A,
5) a C₃₋₈ cycloalkyl group optionally substituted by the same or different one or more substituents selected from the aforementioned group A,
6) a C₆₋₁₄ aryl group optionally substituted by the same or different one or more substituents selected from the aforementioned group A,
7) a nitro group,
8) a cyano group,
9) -COOH, or
10) -COOR wherein R is as defined above, or
11) R⁶ and R⁷, R⁷ and R⁸ or R⁸ and R⁹ form, together with the carbon atom bonded thereto, a saturated or unsaturated carbocycle having 3 to 14 carbon atoms (said carbocycle is optionally substituted by the same or different one or more substituents selected from the aforementioned group A), provided that when
(i) L is a single bond,
Y is
1) a single bond,
2) a C₂₋₄ alkylene group optionally substituted by the same or different one or more substituents selected from the aforementioned group A,
3) a C₂₋₄ alkenylene group optionally substituted by the same or different one or more substituents selected from the aforementioned group A, or
4) a C₁₋₄ alkylene-C₆₋₁₄ arylene group optionally substituted by the same or different one or more substituents selected from the aforementioned group A, and
R⁵ is
1) a C₂₋₆ alkyl group optionally substituted by the same or different one or more substituents selected from the aforementioned group A,
2) a C₂₋₆ alkenyl group optionally substituted by the same or different one or more substituents selected from the aforementioned group A,
3) a C₆₋₁₄ aryl group optionally substituted by the same or different one or more substituents selected from the aforementioned group A,
4) a C₇₋₁₂ aralkyl group optionally substituted by the same or different one or more substituents selected from the aforementioned group A, or
5) a halogen atom, or
6) R⁴ and R⁵ form, together with the carbon atom bonded thereto, a saturated carbocycle having 3 to 6 carbon atoms (said carbocycle is optionally substituted by the same or different one or more substituents selected from the aforementioned group A);
(ii) the following compound is excluded,
3-[2-(4-methoxybenzyl)-3-oxo-2,3-dihydrobenzo[1,4]thiazin-4-yl]propionic acid.

4. The compound of claim 2 or 3, which is represented by the following formula [A-2], or a pharmaceutically acceptable salt thereof, or a solvate thereof: 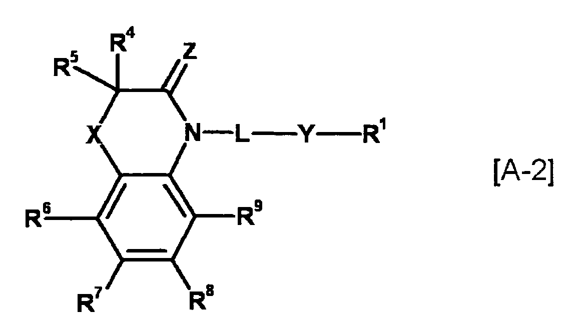 wherein
Z is
1) an oxygen atom, or
2) a sulfur atom:
X is
1) an oxygen atom, or
2) a sulfur atom:
L is
1) a single bond, or
2) -(C=O)-:
Y is
1) a single bond,
2) a C₁₋₄ alkylene group optionally substituted by the same or different one or more substituents selected from the aforementioned group A,
3) a C₂₋₄ alkenylene group optionally substituted by the same or different one or more substituents selected from the aforementioned group A, or
4) a C₁₋₄ alkylene-C₆₋₁₄ arylene group optionally substituted by the same or different one or more substituents selected from the aforementioned group A:
R¹ is
1) -COOH, or
2) -COOR wherein R is
(a) a C₁₋₆ alkyl group optionally substituted by the same or different one or more substituents selected from the aforementioned group A,
(b) an allyl group optionally substituted by the same or different one or more substituents selected from the aforementioned group A,
(c) a phenyl group optionally substituted by the same or different one or more substituents selected from the aforementioned group A, or
(d) a benzyl group optionally substituted by the same or different one or more substituents selected from the aforementioned group A;
R⁴ and R⁵ are the same or different and each is
1) a hydrogen atom,
2) a C₁₋₆ alkyl group,
3) a phenyl group, or
4) a benzyl group:
R⁶, R⁷, R⁸ and R⁹ are the same or different and each is
1) a hydrogen atom,
2) a halogen atom,
3) a trifluoromethyl group,
4) a C₁₋₆ alkyl group,
5) a C₁₋₆ alkoxy group,
6) a C₃₋₈ cycloalkyl group, or
7) a nitro group, or
8) R⁶ and R⁷ form, together with the carbon atom bonded thereto, a saturated or unsaturated carbocycle having 3 to 14 carbon atoms,
provided that when
L is a single bond,
Y is
1) a single bond,
2) a C₂₋₄ alkylene group optionally substituted by the same or different one or more substituents selected from the aforementioned group A,
3) a C₂₋₄ alkenylene group optionally substituted by the same or different one or more substituents selected from the aforementioned group A, or
4) a C₁₋₄ alkylene-C₆₋₁₄ arylene group optionally substituted by the same or different one or more substituents selected from the aforementioned group A, and
R⁵ is
1) a C₂₋₆ alkyl group,
2) a phenyl group, or
3) a benzyl group.

5. The compound of any one of claims 2 to 4, which is represented by the following formula [A-3], or a pharmaceutically acceptable salt thereof, or a solvate thereof: 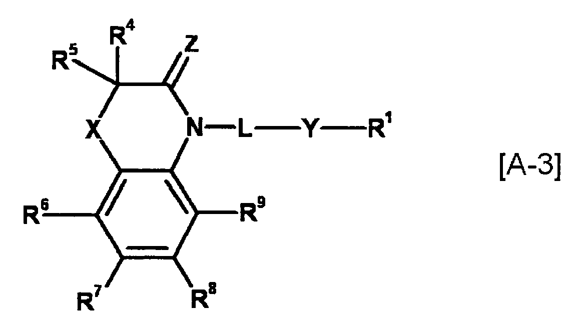 wherein
Z is an oxygen atom;
X is an oxygen atom;
L is a single bond;
Y is a C₂₋₄ alkylene group;
R¹ is
1) -COOH, or
2) -COOR wherein R is
(a) a C₁₋₆ alkyl group optionally substituted by the same or different one or more substituents selected from the aforementioned group A,
(b) an allyl group optionally substituted by the same or different one or more substituents selected from the aforementioned group A,
(c) a phenyl group optionally substituted by the same or different one or more substituents selected from the aforementioned group A, or
(d) a benzyl group optionally substituted by the same or different one or more substituents selected from the aforementioned group A;
R⁴ is
1) a hydrogen atom,
2) a C₂₋₆ alkyl group, or
3) a phenyl group:
R⁵ is
1) a C₂₋₆ alkyl group, or
2) a phenyl group:
R⁶ and R⁸ are the same or different and each is
1) a halogen atom,
2) a trifluoromethyl group,
3) a C₁₋₆ alkyl group,
4) a C₁₋₆ alkoxy group, or
5) a C₃₋₈ cycloalkyl group,
R⁷ and R⁹ are the same or different and each is
1) a hydrogen atom,
2) a halogen atom,
3) a trifluoromethyl group,
4) a C₁₋₆ alkyl group,
5) a C₁₋₆ alkoxy group,
6) a C₃₋₈ cycloalkyl group, or
7) a nitro group, or
R⁶ and R⁷ form, together with the carbon atom bonded thereto, a saturated or unsaturated carbocycle having 3 to 14 carbon atoms.

6. The compound of any one of claims 2 to 5, which is represented by the following formula [A-4], or a pharmaceutically acceptable salt thereof, or a solvate thereof: 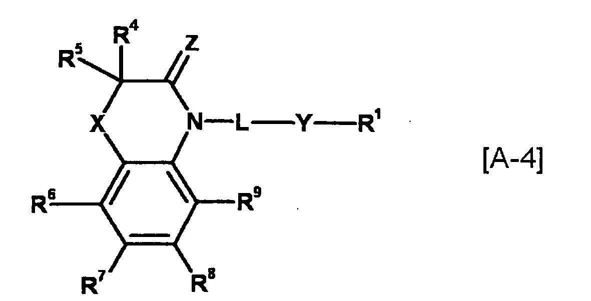 wherein
Z is an oxygen atom;
X is an oxygen atom;
L is a single bond;
Y is a C₂₋₃ alkylene group;
R¹ is
1) -COOH, or
2) -COOR wherein R is a C₁₋₆ alkyl group optionally substituted by the same or different one or more substituents selected from the aforementioned group A:
R⁴ is
1) a hydrogen atom,
2) a C₂₋₆ alkyl group, or
3) a phenyl group:
R⁵ is
1) a C₂₋₆ alkyl group, or
2) a phenyl group:
R⁶ and R⁸ are the same or different and each is
1) a halogen atom,
2) a trifluoromethyl group,
3) a C₁₋₆ alkyl group,
4) a C₁₋₆ alkoxy group, or
5) a C₃₋₈ cycloalkyl group,
R⁷ and R⁹ are the same or different and each is
1) a hydrogen atom,
2) a halogen atom,
3) a trifluoromethyl group,
4) a C₁₋₆ alkyl group,
5) a C₁₋₆ alkoxy group,
6) a C₃₋₈ cycloalkyl group, or
7) a nitro group.

7. The compound of any one of claims 2 to 6, which is represented by the following formula [A-5], or a pharmaceutically acceptable salt thereof, or a solvate thereof: 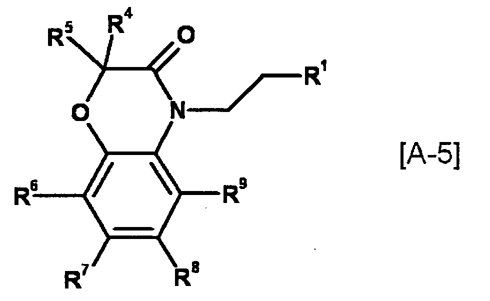 wherein
R¹ is
1) -COOH, or
2) a -COO-C₁₋₆ alkyl group:
R⁴ is
1) a hydrogen atom, or
2) a C₂₋₆ alkyl group:
R⁵ is
1) a C₂₋₆ alkyl group, or
2) a phenyl group:
R⁶ and R⁸ are the same or different and each is
1) a halogen atom,
2) a trifluoromethyl group,
3) a C₁₋₆ alkyl group,
4) a C₁₋₆ alkoxy group, or
5) a C₃₋₈ cycloalkyl group:
R⁷ and R⁹ are the same or different and each is
1) a hydrogen atom,
2) a halogen atom,
3) a trifluoromethyl group,
4) a C₁₋₆ alkyl group,
5) a C₁₋₆ alkoxy group,
6) a C₃₋₈ cycloalkyl group, or
7) a nitro group.

8. The compound of any one of claims 2 to 7, which is represented by the following formula [A-6], or a pharmaceutically acceptable salt thereof, or a solvate thereof: 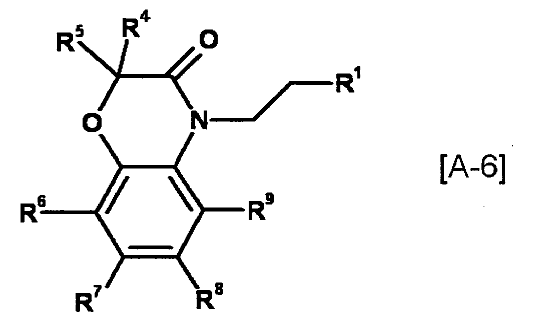 wherein
R¹ is
1) -COOH, or
2) a -COO-C₁₋₆ alkyl group:
R⁴ is
1) a hydrogen atom, or
2) a C₂₋₆ alkyl group:
R⁵ is
1) a C₂₋₆ alkyl group, or
2) a phenyl group:
R⁶ and R⁸ are the same or different and each is
1) a halogen atom,
2) a trifluoromethyl group,
3) a C₁₋₆ alkyl group,
4) a C₁₋₆ alkoxy group, or
5) a C₃₋₈ cycloalkyl group:
R⁷ and R⁹ are the same or different and each is
1) a hydrogen atom,
2) a halogen atom,
3) a trifluoromethyl group,
4) a C₁₋₆ alkyl group,
5) a C₁₋₆ alkoxy group,
6) a C₃₋₈ cycloalkyl group, or
7) a nitro group,
provided that at least one of R⁶, R⁷, R⁸ and R⁹ is a C₁₋₆ alkyl group, a trifluoromethyl group, a C₁₋₆ alkoxy group, or a C₃₋₈ cycloalkyl group.

9. The compound of any one of claims 2 to 8, which is represented by the following formula [A-7], or a pharmaceutically acceptable salt thereof, or a solvate thereof: 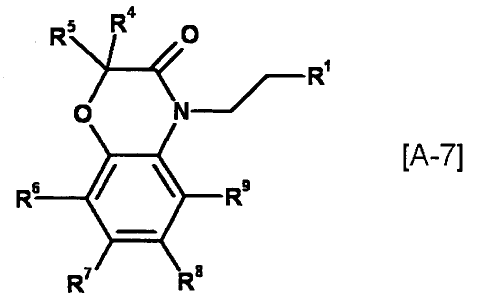 wherein
R¹ is
1) -COOH, or
2) -COO-C₁₋₂ alkyl group:
R⁴ is
1) a hydrogen atom, or
2) a C₃₋₄ alkyl group, :
R⁵ is a C₃₋₄ alkyl group;
R⁶ and R⁸ are the same or different and each is
1) a halogen atom,
2) a trifluoromethyl group,
3) a C₁₋₆ alkyl group, or
4) a C₅₋₆ cycloalkyl group:
R⁷ and R⁹ are the same or different and each is
1) a hydrogen atom,
2) a halogen atom,
3) a trifluoromethyl group,
4) a C₁₋₆ alkyl group,
5) a C₁₋₆ alkoxy group, or
6) a C₅₋₆ cycloalkyl group,
provided that at least one of R⁶, R⁷, R⁸ and R⁹ is a C₁₋₆ alkyl group, a trifluoromethyl group, a C₁₋₆ alkoxy group, or a C₅₋₆ cycloalkyl group.

10. The compound of any one of claims 2 to 9, which is represented by the following formula [A-8], or a pharmaceutically acceptable salt thereof, or a solvate thereof: 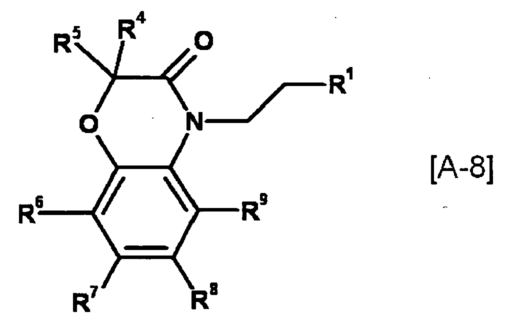 wherein
R¹ is
1) -COOH, or
2) a -COO-C₁₋₂ alkyl group:
R⁴ is a hydrogen atom;
R⁵ is a C₃₋₄ alkyl group;
R⁶ and R⁸ are the same or different and each is
1) a halogen atom,
2) a trifluoromethyl group,
3) a C₁₋₆ alkyl group, or
4) a C₅₋₆ cycloalkyl group:
R⁷ is
1) a hydrogen atom,
2) a halogen atom,
3) a trifluoromethyl group, or
4) a C₁₋₆ alkyl group:
R⁹ is a hydrogen atom,
provided that at least one of R⁶, R⁷ and R⁸ is a C₁₋₆ alkyl group, a trifluoromethyl group, or a C₅₋₆ cycloalkyl group.

11. The compound of any one of claims 2 to 10, which is represented by the following formula [A-9], or a pharmaceutically acceptable salt thereof, or a solvate thereof: 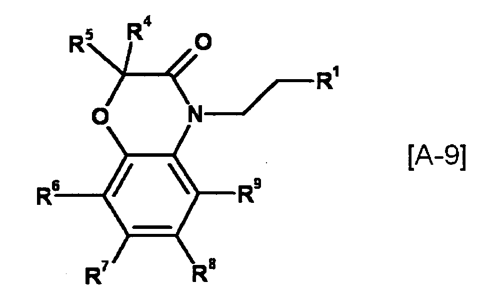 wherein
R¹ is -COOH;
R⁴ is a hydrogen atom;
R⁵ is a C₃₋₄ alkyl group;
R⁶ is
1) a chlorine atom, or
2) a C₁₋₆ alkyl group:
R⁷ is
1) a hydrogen atom, or
2) a C₁₋₆ alkyl group:
R⁸ is
1) a chlorine atom, or
2) a C₁₋₆ alkyl group:
R⁹ is a hydrogen atom,
provided that at least one of R⁶, R⁷ and R⁸ is a C₁₋₆ alkyl group.

12. The compound of claim 2, which is represented by the following formula [B-1], or a pharmaceutically acceptable salt thereof, or a solvate thereof: 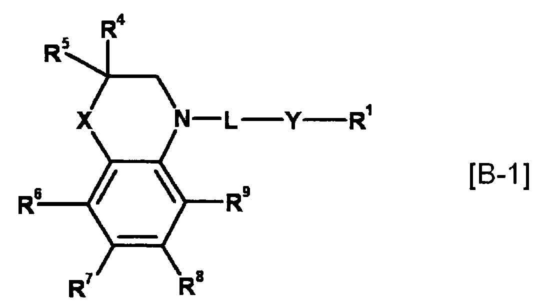 wherein
X is
1) an oxygen atom,
2) a sulfur atom,
3) -S(=O)- or
4) -S(=O)₂-_{:}
L is
1) a single bond, or
2) -(C=O)-:
Y is
1) a single bond,
2) a C₁₋₄ alkylene group optionally substituted by the same or different one or more substituents selected from the aforementioned group A,
3) a C₂₋₄ alkenylene group optionally substituted by the same or different one or more substituents selected from the aforementioned group A, or
4) a C₁₋₄ alkylene-C₆₋₁₄ arylene group optionally substituted by the same or different one or more substituents selected from the aforementioned group A:
R¹ is
1) -COOH, or
2) -COOR wherein R is
(a) a C₁₋₆ alkyl group optionally substituted by the same or different one or more substituents selected from the aforementioned group A,
(b) an allyl group optionally substituted by the same or different one or more substituents selected from the aforementioned group A,
(c) a phenyl group optionally substituted by the same or different one or more substituents selected from the aforementioned group A, or
(d) a benzyl group optionally substituted by the same or different one or more substituents selected from the aforementioned group A;
R⁴ and R⁵ are the same or different and each is
1) a hydrogen atom,
2) a C₁₋₆ alkyl group optionally substituted by the same or different one or more substituents selected from the aforementioned group A,
3) a C₂₋₆ alkenyl group optionally substituted by the same or different one or more substituents selected from the aforementioned group A,
4) a C₆₋₁₄ aryl group optionally substituted by the same or different one or more substituents selected from the aforementioned group A,
5) a C₇₋₁₂ aralkyl group optionally substituted by the same or different one or more substituents selected from the aforementioned group A, or
6) a halogen atom, or
7) R⁴ and R⁵ form, together with the carbon atom bonded thereto, a saturated carbocycle having 3 to 6 carbon atoms (said carbocycle is optionally substituted by the same or different one or more substituents selected from the aforementioned group A);
R⁶, R⁷, R⁸ and R⁹ are the same or different and each is
1) a hydrogen atom,
2) a halogen atom,
3) a C₁₋₆ alkyl group optionally substituted by the same or different one or more substituents selected from the aforementioned group A,
4) a C₁₋₆ alkoxy group optionally substituted by the same or different one or more substituents selected from the aforementioned group A,
5) a C₃₋₈ cycloalkyl group optionally substituted by the same or different one or more substituents selected from the aforementioned group A,
6) a C₆₋₁₄ aryl group optionally substituted by the same or different one or more substituents selected from the aforementioned group A,
7) a nitro group,
8) a cyano group,
9) -COOH, or
10) -COOR wherein R is as defined above, or
11) R⁶ and R⁷, R⁷ and R⁸ or R⁸ and R⁹ form, together with the carbon atom bonded thereto, a saturated or unsaturated carbocycle having 3 to 14 carbon atoms (said carbocycle is optionally substituted by the same or different one or more substituents selected from the aforementioned group A), provided that the following compounds are excluded,
(E)-4-(2,2-dimethyl-6-nitro-2,3-dihydrobenzo[1,4]oxazin-4-yl)-4-oxo-2-butenoic acid,
(6-methoxy-8-methyl-2,3-dihydrobenzo[1,4]oxazin-4-yl)acetic acid,
(2,2-dimethyl-6-nitro-2,3-dihydrobenzo[1,4]oxazin-4-yl)acetic acid, and
(2E,4E)-3-methyl-6-oxo-6-(6,6,9,9-tetramethyl-2,3,6,7,8,9-hexahydronaphtho[2,3-b][1,4]oxazin-4-yl)2,4-hexadienoic acid.

13. The compound of claim 2 or 12, wherein
X is
1) an oxygen atom, or
2) a sulfur atom:
L is
1) a single bond, or
2) -(C=O)-:
Y is
1) a single bond,
2) a C₁₋₄ alkylene group optionally substituted by the same or different one or more substituents selected from the aforementioned group A,
3) a C₂₋₄ alkenylene group optionally substituted by the same or different one or more substituents selected from the aforementioned group A, or
4) a C₁₋₄ alkylene-C₆₋₁₄ arylene group optionally substituted by the same or different one or more substituents selected from the aforementioned group A:
R¹ is
1) -COOH, or
2) -COOR wherein R is
(a) a C₁₋₆ alkyl group optionally substituted by the same or different one or more substituents selected from the aforementioned group A,
(b) an allyl group optionally substituted by the same or different one or more substituents selected from the aforementioned group A,
(c) a phenyl group optionally substituted by the same or different one or more substituents selected from the aforementioned group A, or
(d) a benzyl group optionally substituted by the same or different one or more substituents selected from the aforementioned group A;
R⁴ and R⁵ are the same or different and each is
1) a hydrogen atom,
2) a C₁₋₆ alkyl group,
3) a phenyl group, or
4) a benzyl group:
R⁶, R⁷, R⁸ and R⁹ are the same or different and each is
1) a hydrogen atom,
2) a halogen atom,
3) a trifluoromethyl group,
4) a C₁₋₆ alkyl group,
5) a C₁₋₆ alkoxy group,
6) a C₃₋₈ cycloalkyl group, or
7) a nitro group, or
8) R⁶ and R⁷, R⁷ and R⁸ or R⁸ and R⁹ form, together with the carbon atom bonded thereto, a saturated or unsaturated carbocycle having 3 to 14 carbon atoms (said carbocycle is optionally substituted by the same or different one or more substituents selected from the aforementioned group A), provided that the following compounds are excluded,
(E)-4-(2,2-dimethyl-6-nitro-2,3-dihydrobenzo[1,4]oxazin-4-yl)-4-oxo-2-butenoic acid,
(6-methoxy-8-methyl-2,3-dihydrobenzo[1,4]oxazin-4-yl)acetic acid,
(2,2-dimethyl-6-nitro-2,3-dihydrobenzo[1,4]oxazin-4-yl)acetic acid, and
(2E,4E)-3-methyl-6-oxo-6-(6,6,9,9-tetramethyl-2,3,6,7,8,9-hexahydronaphtho[2,3-b][1,4]oxazin-4-yl)2,4-hexadienoic acid;
or a pharmaceutically acceptable salt thereof, or a solvate thereof.

14. The compound of any one of claims 2, 12 and 13, wherein
X is an oxygen atom;
L is -(C=O)-;
Y is
1) a single bond,
2) a C₁₋₄ alkylene group, or
3) a C₂₋₄ alkenylene group:
R¹ is
1) -COOH, or
2) a -COO-C₁₋₆ alkyl group:
R⁴ and R⁵ are the same or different and each is
1) a hydrogen atom,
2) a C₂₋₆ alkyl group,
3) a phenyl group, or
4) a benzyl group:
R⁶ and R⁸ are the same or different and each is
1) a halogen atom,
2) a trifluoromethyl group,
3) a C₁₋₆ alkyl group, or
4) a C₃₋₈ cycloalkyl group: and
R⁷ and R⁹ are each a hydrogen atom;
or a pharmaceutically acceptable salt thereof, or a solvate thereof.

15. The compound of claim 2, which is selected from the following group, or a pharmaceutically acceptable salt thereof, or a solvate thereof:
(8-isopropyl-3-oxo-2,3-dihydrobenzo[1,4]oxazin-4-yl)acetic acid (Example 10),
3-(2,8-diisopropyl-3-oxo-2,3-dihydrobenzo[1,4]oxazin-4-yl)propionic acid (Example 14),
(8-isopropyl-3-thioxo-2,3-dihydrobenzo[1,4]oxazin-4-yl)acetic acid (Example 15),
3-(2,8-diisopropyl-3-thioxo-2,3-dihydrobenzo[1,4]oxazin-4-yl)propionic acid (Example 16),
4-(2,8-diisopropyl-3-oxo-2,3-dihydrobenzo[1,4]oxazin-4-yl)butyric acid (Example 18),
4-(2,8-diisopropyl-3-thioxo-2,3-dihydrobenzo[1,4]oxazin-4-yl)butyric acid (Example 20),
(8-isopropyl-3-oxo-2-phenyl-2,3-dihydrobenzo[1,4]oxazin-4-yl)acetic acid (Example 26),
3-(8-isopropyl-2,2-dimethyl-3-oxo-2,3-dihydrobenzo[1,4]oxazin-4-yl)propionic acid (Example 33),
(8-isopropyl-2,2-dimethyl-3-oxo-2,3-dihydrobenzo[1,4]oxazin-4-yl)acetic acid (Example 34),
3-(8-isopropyl-3-oxo-2,3-dihydrobenzo[1,4]oxazin-4-yl)propionic acid (Example 35),
3-(8-isopropyl-3-oxo-2-phenyl-2,3-dihydrobenzo[1,4]oxazin-4-yl)propionic acid (Example 38),
(2-ethyl-8-isopropyl-3-oxo-2,3-dihydrobenzo[1,4]oxazin-4-yl)acetic acid (Example 39),
3-(2-ethyl-8-isopropyl-3-oxo-2,3-dihydrobenzo[1,4]oxazin-4-yl)propionic acid (Example 40),
2-[(2,8-diisopropyl-3-oxo-2,3-dihydrobenzo[1,4]oxazin-4-yl)methyl]benzoic acid (Example 41),
3-(2,8-diisopropyl-3-oxo-2,3-dihydrobenzo[1,4]oxazin-4-yl)-2,2-dimethylpropionic acid (Example 42),
3-(2-isopropyl-7-methyl-3-oxo-2,3-dihydrobenzo[1,4]oxazin-4-yl)propionic acid (Example 43),
3-(2-isopropyl-8-methyl-3-oxo-2,3-dihydrobenzo[1,4]oxazin-4-yl)propionic acid (Example 44),
3-(8-tert-butyl-2-isopropyl-3-oxo-2,3-dihydrobenzo[1,4]oxazin-4-yl)propionic acid (Example 46),
3-(8-methyl-3-oxo-2-phenyl-2,3-dihydrobenzo[1,4]oxazin-4-yl)propionic acid (Example 47),
3-(7-methyl-3-oxo-2-phenyl-2,3-dihydrobenzo[1,4]oxazin-4-yl)propionic acid (Example 48),
3-(8-tert-butyl-3-oxo-2-phenyl-2,3-dihydrobenzo[1,4]oxazin-4-yl)propionic acid (Example 49),
3-(8-cyclohexyl-2-isopropyl-3-oxo-2,3-dihydrobenzo[1,4]oxazin-4-yl)propionic acid (Example 50),
3-(8-ethyl-2-isopropyl-3-oxo-2,3-dihydrobenzo[1,4]oxazin-4-yl)propionic acid (Example 53),
3-(2-isopropyl-6-methyl-3-oxo-2,3-dihydrobenzo[1,4]oxazin-4-yl)propionic acid (Example 58),
3-(8-ethyl-3-oxo-2-phenyl-2,3-dihydrobenzo[1,4]oxazin-4-yl)propionic acid (Example 59),
3-(2-isopropyl-6,8-dimethyl-3-oxo-2,3-dihydrobenzo[1,4]oxazin-4-yl)propionic acid (Example 61),
3-(6,8-dimethyl-3-oxo-2-phenyl-2,3-dihydrobenzo[1,4]oxazin-4-yl)propionic acid (Example 72),
3-((S)-2-isopropyl-6,8-dimethyl-3-oxo-2,3-dihydrobenzo[1,4]oxazin-4-yl)propionic acid (Example 73),
3-((R)-2-isopropyl-6,8-dimethyl-3-oxo-2,3-dihydrobenzo[1,4]oxazin-4-yl)propionic acid (Example 74),
3-(6-methyl-3-oxo-2-phenyl-2,3-dihydrobenzo[1,4]oxazin-4-yl)propionic acid (Example 75),
3-(6-chloro-2-isopropyl-8-methyl-3-oxo-2,3-dihydrobenzo[1,4]oxazin-4-yl)propionic acid (Example 81),
3-(6,8-dimethyl-3-oxo-2,3-dihydrobenzo[1,4]oxazin-4-yl)propionic acid (Example 82),
3-(2,6,8-trimethyl-3-oxo-2,3-dihydrobenzo[1,4]oxazin-4-yl)propionic acid (Example 86),
3-(2-ethyl-6,8-dimethyl-3-oxo-2,3-dihydrobenzo[1,4]oxazin-4-yl)propionic acid (Example 87),
3-(6-tert-butyl-2-isopropyl-3-oxo-2,3-dihydrobenzo[1,4]oxazin-4-yl)propionic acid (Example 88),
3-(2,2,6,8-tetramethyl-3-oxo-2,3-dihydrobenzo[1,4]oxazin-4-yl)propionic acid (Example 89),
3-(2-isobutyl-6,8-dimethyl-3-oxo-2,3-dihydrobenzo[1,4]oxazin-4-yl)propionic acid (Example 90),
3-((R)-2-isobutyl-6,8-dimethyl-3-oxo-2,3-dihydrobenzo[1,4]oxazin-4-yl)propionic acid (Example 91),
3-(2-benzyl-6,8-dimethyl-3-oxo-2,3-dihydrobenzo[1,4]oxazin-4-yl)propionic acid (Example 92),
3-(2-butyl-6,8-dimethyl-3-oxo-2,3-dihydrobenzo[1,4]oxazin-4-yl)propionic acid (Example 93),
3-(6,8-dichloro-2-isopropyl-7-methyl-3-oxo-2,3-dihydrobenzo[1,4]oxazin-4-yl)propionic acid (Example 94),
3-(2-isopropyl-3-oxo-6-trifluoromethyl-2,3-dihydrobenzo[1,4]oxazin-4-yl)propionic acid (Example 97),
3-(8-chloro-2-isopropyl-6-methyl-3-oxo-2,3-dihydrobenzo[1,4]oxazin-4-yl)propionic acid (Example 98),
3-(2-isopropyl-3-oxo-2,3-dihydronaphtho[1,2-b][1,4]oxazin-4-yl)propionic acid (Example 99),
3-(6-ethyl-2-isopropyl-8-methyl-3-oxo-2,3-dihydrobenzo[1,4]oxazin-4-yl)propionic acid (Example 101),
3-(6,8-dimethyl-3-oxo-2-propyl-2,3-dihydrobenzo[1,4]oxazin-4-yl)propionic acid (Example 102),
3-[2-isopropyl-3-oxo-6,8-bis(trifluoromethyl)-2,3-dihydrobenzo[1,4]oxazin-4-yl]propionic acid (Example 103), 3-(2-isopropyl-6-methoxy-8-methyl-3-oxo-2,3-dihydrobenzo[1,4]oxazin-4-yl)propionic acid (Example 104),
3-[(R)-2-((S)-sec-butyl)-6,8-dimethyl-3-oxo-2,3-dihydrobenzo[1,4]oxazin-4-yl]propionic acid (Example 105),
3-((R)-8-chloro-2-isopropyl-6-methyl-3-oxo-2,3-dihydrobenzo[1,4]oxazin-4-yl)propionic acid (Example 106),
3-((R)-6-chloro-2-isopropyl-8-methyl-3-oxo-2,3-dihydrobenzo[1,4]oxazin-4-yl)propionic acid (Example 107),
3-((S)-8-chloro-2-isopropyl-6-methyl-3-oxo-2,3-dihydrobenzo[1,4]oxazin-4-yl)propionic acid (Example 109), and
3-((S)-6-chloro-2-isopropyl-8-methyl-3-oxo-2,3-dihydrobenzo[1,4]oxazin-4-yl)propionic acid (Example 110).

16. The compound of claim 2, which is selected from the following group, or a pharmaceutically acceptable salt thereof, or a solvate thereof:
(3-oxo-2,3-dihydrobenzo[1,4]oxazin-4-yl)acetic acid methyl ester (Example 1),
(3-oxo-2,3-dihydrobenzo[1,4]thiazin-4-yl)acetic acid methyl ester (Example 2),
(2,2-dimethyl-3-oxo-2,3-dihydrobenzo[1,4]oxazin-4-yl)acetic acid (Example 5),
(2-isopropyl-3-thioxo-2,3-dihydrobenzo[1,4]oxazin-4-yl)acetic acid (Example 13),
(3-thioxo-2,3-dihydrobenzo[1,4]oxazin-4-yl)acetic acid (Example 17),
3-(2-isopropyl-3-oxo-2,3-dihydrobenzo[1,4]thiazin-4-yl)propionic acid (Example 22),
(2-isopropyl-3-oxo-2,3-dihydrobenzo[1,4]thiazin-4-yl)acetic acid (Example 30),
3-(2-isopropyl-3-oxo-2,3-dihydrobenzo[1,4]oxazin-4-yl)propionic acid (Example 32),
3-(8-bromo-2-isopropyl-3-oxo-2,3-dihydrobenzo[1,4]oxazin-4-yl)propionic acid (Example 51),
3-(7-chloro-2-isopropyl-3-oxo-2,3-dihydrobenzo[1,4]oxazin-4-yl)propionic acid (Example 62),
3-(6-chloro-2-isopropyl-3-oxo-2,3-dihydrobenzo[1,4]oxazin-4-yl)propionic acid (Example 63),
3-(8-bromo-3-oxo-2-phenyl-2,3-dihydrobenzo[1,4]oxazin-4-yl)propionic acid (Example 66),
3-(7-chloro-3-oxo-2-phenyl-2,3-dihydrobenzo[1,4]oxazin-4-yl)propionic acid (Example 76),
3-(6-chloro-3-oxo-2-phenyl-2,3-dihydrobenzo[1,4]oxazin-4-yl)propionic acid (Example 77),
3-(6,8-dichloro-2-isopropyl-3-oxo-2,3-dihydrobenzo[1,4]oxazin-4-yl)propionic acid (Example 80),
3-(2-isopropyl-7-nitro-3-oxo-2,3-dihydrobenzo[1,4]oxazin-4-yl)propionic acid (Example 95),
3-(2-isopropyl-6-nitro-3-oxo-2,3-dihydrobenzo[1,4]oxazin-4-yl)propionic acid (Example 96),
3-(8-chloro-2-isopropyl-3-oxo-2,3-dihydrobenzo[1,4]oxazin-4-yl)propionic acid (Example 100),
3-((R)-6,8-dichloro-2-isopropyl-3-oxo-2,3-dihydrobenzo[1,4]oxazin-4-yl)propionic acid (Example 108), and
3-((S)-6,8-dichloro-2-isopropyl-3-oxo-2,3-dihydrobenzo[1,4]oxazin-4-yl)propionic acid (Example 111).

17. The compound of claim 2, which is selected from the following group, or a pharmaceutically acceptable salt thereof, or a solvate thereof:
4-(2,8-diisopropyl-2,3-dihydrobenzo[1,4]oxazin-4-yl)-4-oxobutyric acid (Example 21),
4-(2,3-dihydrobenzo[1,4]oxazin-4-yl)-4-oxobutyric acid (Example 23),
5-(2,3-dihydrobenzo[1,4]oxazin-4-yl)-5-oxovaleric acid (Example 24),
(Z)-4-(2,3-dihydrobenzo[1,4]oxazin-4-yl)-4-oxo-2-butenoic acid (Example 25),
6-(2,3-dihydrobenzo[1,4]oxazin-4-yl)-6-oxohexanoic acid (Example 27),
(E)-4-(2,3-dihydrobenzo[1,4]oxazin-4-yl)-4-oxo-2-butenoic acid (Example 28),
(2,8-diisopropyl-2,3-dihydrobenzo[1,4]oxazin-4-yl)oxoacetic acid (Example 29),
3-(2,8-diisopropyl-2,3-dihydrobenzo[1,4]oxazin-4-yl)-3-oxopropionic acid (Example 31),
(E)-4-(2,8-diisopropyl-2,3-dihydrobenzo[1,4]oxazin-4-yl)-4-oxo-2-butenoic acid (Example 36),
(Z)-4-(2,8-diisopropyl-2,3-dihydrobenzo[1,4]oxazin-4-yl)-4-oxo-2-butenoic acid (Example 37),
(E)-4-(8-isopropyl-2-phenyl-2,3-dihydrobenzo[1,4]oxazin-4-yl)-4-oxo-2-butenoic acid (Example 45),
(E)-4-(8-tert-butyl-2-isopropyl-2,3-dihydrobenzo[1,4]oxazin-4-yl)-4-oxo-2-butenoic acid (Example 52),
(E)-4-(2-isopropyl-8-methyl-2,3-dihydrobenzo[1,4]oxazin-4-yl)-4-oxo-2-butenoic acid (Example 54),
(E)-4-(8-methyl-2-phenyl-2,3-dihydrobenzo[1,4]oxazin-4-yl)-4-oxo-2-butenoic acid (Example 55),
(E)-4-(2-isopropyl-7-methyl-2,3-dihydrobenzo[1,4]oxazin-4-yl)-4-oxo-2-butenoic acid (Example 56),
(E)-4-(7-methyl-2-phenyl-2,3-dihydrobenzo[1,4]oxazin-4-yl)-4-oxo-2-butenoic acid (Example 57),
(E)-4-(8-ethyl-2-isopropyl-2,3-dihydrobenzo[1,4]oxazin-4-yl)-4-oxo-2-butenoic acid (Example 60),
(E)-4-(2-isopropyl-6-methyl-2,3-dihydrobenzo[1,4]oxazin-4-yl)-4-oxo-2-butenoic acid (Example 64),
(E)-4-(6-methyl-2-phenyl-2,3-dihydrobenzo[1,4]oxazin-4-yl)-4-oxo-2-butenoic acid (Example 65),
(E)-4-(2-ethyl-8-isopropyl-2,3-dihydrobenzo[1,4]oxazin-4-yl)-4-oxo-2-butenoic acid (Example 67),
(E)-4-(7-chloro-2-isopropyl-2,3-dihydrobenzo[1,4]oxazin-4-yl)-4-oxo-2-butenoic acid (Example 68),
(E)-4-(6-chloro-2-isopropyl-2,3-dihydrobenzo[1,4]oxazin-4-yl)-4-oxo-2-butenoic acid (Example 69),
(E)-4-(7-chloro-2-phenyl-2,3-dihydrobenzo[1,4]oxazin-4-yl)-4-oxo-2-butenoic acid (Example 70),
(E)-4-(6-chloro-2-phenyl-2,3-dihydrobenzo[1,4]oxazin-4-yl)-4-oxo-2-butenoic acid (Example 71),
(E)-4-(8-bromo-2-isopropyl-2,3-dihydrobenzo[1,4]oxazin-4-yl)-4-oxo-2-butenoic acid (Example 78),
(E)-4-(8-cyclohexyl-2-isopropyl-2,3-dihydrobenzo[1,4]oxazin-4-yl)-4-oxo-2-butenoic acid (Example 79),
(E)-4-(6-chloro-2-isopropyl-8-methyl-2,3-dihydrobenzo[1,4]oxazin-4-yl)-4-oxo-2-butenoic acid (Example 83),
(E)-4-(6-chloro-8-methyl-2-phenyl-2,3-dihydrobenzo[1,4]oxazin-4-yl)-4-oxo-2-butenoic acid (Example 84), and
(E)-4-(6,8-dimethyl-2,3-dihydrobenzo[1,4]oxazin-4-yl)-4-oxo-2-butenoic acid (Example 85).

18. The compound of claim 2, which is selected from the following group, or a pharmaceutically acceptable salt thereof, or a solvate thereof:
3-(2-isopropyl-6,8-dimethyl-3-oxo-2,3-dihydrobenzo[1,4]oxazin-4-yl)propionic acid (Example 61),
3-((S)-2-isopropyl-6,8-dimethyl-3-oxo-2,3-dihydrobenzo[1,4]oxazin-4-yl)propionic acid (Example 73),
3-((R)-2-isopropyl-6,8-dimethyl-3-oxo-2,3-dihydrobenzo[1,4]oxazin-4-yl)propionic acid (Example 74),
3-(6-chloro-2-isopropyl-8-methyl-3-oxo-2,3-dihydrobenzo[1,4]oxazin-4-yl)propionic acid (Example 81),
3-(2-isobutyl-6,8-dimethyl-3-oxo-2,3-dihydrobenzo[1,4]oxazin-4-yl)propionic acid (Example 90),
3-((R)-2-isobutyl-6,8-dimethyl-3-oxo-2,3-dihydrobenzo[1,4]oxazin-4-yl)propionic acid (Example 91),
3-(8-chloro-2-isopropyl-6-methyl-3-oxo-2,3-dihydrobenzo[1,4]oxazin-4-yl)propionic acid (Example 98),
3-(6-ethyl-2-isopropyl-8-methyl-3-oxo-2,3-dihydrobenzo[1,4]oxazin-4-yl)propionic acid (Example 101),
3-(6,8-dimethyl-3-oxo-2-propyl-2,3-dihydrobenzo[1,4]oxazin-4-yl)propionic acid (Example 102),
3-[(R)-2-((S)-sec-butyl)-6,8-dimethyl-3-oxo-2,3-dihydrobenzo[1,4]oxazin-4-yl]propionic acid (Example 105),
3-((R)-8-chloro-2-isopropyl-6-methyl-3-oxo-2,3-dihydrobenzo[1,4]oxazin-4-yl)propionic acid (Example 106), and
3-((R)-6-chloro-2-isopropyl-8-methyl-3-oxo-2,3-dihydrobenzo[1,4]oxazin-4-yl)propionic acid (Example 107).

19. The compound of claim 2, which is selected from the following group, or a pharmaceutically acceptable salt thereof, or a solvate thereof:
3-(2-isopropyl-6,8-dimethyl-3-oxo-2,3-dihydrobenzo[1,4]oxazin-4-yl)propionic acid (Example 61),
3-(6,8-dimethyl-3-oxo-2-phenyl-2,3-dihydrobenzo[1,4]oxazin-4-yl)propionic acid (Example 72),
3-((S)-2-isopropyl-6,8-dimethyl-3-oxo-2,3-dihydrobenzo[1,4]oxazin-4-yl)propionic acid (Example 73),
3-((R)-2-isopropyl-6,8-dimethyl-3-oxo-2,3-dihydrobenzo[1,4]oxazin-4-yl)propionic acid (Example 74),
3-(6-chloro-2-isopropyl-8-methyl-3-oxo-2,3-dihydrobenzo[1,4]oxazin-4-yl)propionic acid (Example 81),
3-(2-ethyl-6,8-dimethyl-3-oxo-2,3-dihydrobenzo[1,4]oxazin-4-yl)propionic acid (Example 87),
3-(2-isobutyl-6,8-dimethyl-3-oxo-2,3-dihydrobenzo[1,4]oxazin-4-yl)propionic acid (Example 90),
3-((R)-2-isobutyl-6,8-dimethyl-3-oxo-2,3-dihydrobenzo[1,4]oxazin-4-yl)propionic acid (Example 91),
3-(2-butyl-6,8-dimethyl-3-oxo-2,3-dihydrobenzo[1,4]oxazin-4-yl)propionic acid (Example 93),
3-(6,8-dichloro-2-isopropyl-7-methyl-3-oxo-2,3-dihydrobenzo[1,4]oxazin-4-yl)propionic acid (Example 94),
3-(8-chloro-2-isopropyl-6-methyl-3-oxo-2,3-dihydrobenzo[1,4]oxazin-4-yl)propionic acid (Example 98),
3-(6-ethyl-2-isopropyl-8-methyl-3-oxo-2,3-dihydrobenzo[1,4]oxazin-4-yl)propionic acid (Example 101), 3-(6,8-dimethyl-3-oxo-2-propyl-2,3-dihydrobenzo[1,4]oxazin-4-yl)propionic acid (Example 102),
3-[2-isopropyl-3-oxo-6,8-bis(trifluoromethyl)-2,3-dihydrobenzo[1,4]oxazin-4-yl]propionic acid (Example 103),
3-(2-isopropyl-6-methoxy-8-methyl-3-oxo-2,3-dihydrobenzo[1,4]oxazin-4-yl)propionic acid (Example 104),
3-[(R)-2-((S)-sec-butyl)-6,8-dimethyl-3-oxo-2,3-dihydrobenzo[1,4]oxazin-4-yl]propionic acid (Example 105),
3-((R)-8-chloro-2-isopropyl-6-methyl-3-oxo-2,3-dihydrobenzo[1,4]oxazin-4-yl)propionic acid (Example 106),
3-((R)-6-chloro-2-isopropyl-8-methyl-3-oxo-2,3-dihydrobenzo[1,4]oxazin-4-yl)propionic acid (Example 107),
3-((S)-8-chloro-2-isopropyl-6-methyl-3-oxo-2,3-dihydrobenzo[1,4]oxazin-4-yl)propionic acid (Example 109), and
3-((S)-6-chloro-2-isopropyl-8-methyl-3-oxo-2,3-dihydrobenzo[1,4]oxazin-4-yl)propionic acid (Example 110).

20. A pharmaceutical composition comprising the compound of any one of claims 2 to 19, or a pharmaceutically acceptable salt thereof, or a solvate thereof.

21. A URAT1 activity inhibitor comprising the compound of any one of claims 2 to 19, or a pharmaceutically acceptable salt thereof, or a solvate thereof.

22. An agent for lowering the blood uric acid level, comprising the compound of the formula [1] of claim 1, or a pharmaceutically acceptable salt thereof, or a solvate thereof.

23. An agent for lowering the blood uric acid level, comprising the compound of any one of claims 2 to 19, or a pharmaceutically acceptable salt thereof, or a solvate thereof.

24. An agent for the treatment or prophylaxis of a pathology involving uric acid, which comprises the compound of the formula [1] of claim 1, or a pharmaceutically acceptable salt thereof, or a solvate thereof.

25. An agent for the treatment or prophylaxis of a pathology involving uric acid, which comprises the compound of any one of claims 2 to 19, or a pharmaceutically acceptable salt thereof, or a solvate thereof.

26. The agent of claim 24 or 25, wherein the pathology involving uric acid is hyperuricemia, gouty tophus, acute gouty arthritis, chronic gouty arthritis, gouty kidney, urinary lithiasis, renal dysfunction, a coronary heart disease or ischemic cardiac disease.

27. The agent of claim 24 or 25, wherein the pathology involving uric acid is hyperuricemia, gouty tophus, acute gouty arthritis, chronic gouty arthritis or gouty kidney.

28. The agent of claim 24 or 25, wherein the pathology involving uric acid is hyperuricemia.

29. A method for the treatment or prophylaxis of a pathology involving uric acid, which comprises administering a pharmaceutically effective amount of the compound of the formula [1] of claim 1, or a pharmaceutically acceptable salt thereof, or a solvate thereof.

30. A method for the treatment or prophylaxis of a pathology involving uric acid, which comprises administering a pharmaceutically effective amount of the compound of any one of claims 2 to 19, or a pharmaceutically acceptable salt thereof, or a solvate thereof.

31. The method of claim 29 or 30, wherein the pathology involving uric acid is hyperuricemia, gouty tophus, acute gouty arthritis, chronic gouty arthritis, gouty kidney, urinary lithiasis, renal dysfunction, a coronary heart disease or an ischemic cardiac disease.

32. The method of claim 29 or 30, wherein the pathology involving uric acid is hyperuricemia, gouty tophus, acute gouty arthritis, chronic gouty arthritis or gouty kidney.

33. The method of claim 29 or 30, wherein the pathology involving uric acid is hyperuricemia.

34. A method for inhibition of a URAT1 activity, which comprises administering a pharmaceutically effective amount of the compound of the formula [1] of claim 1, or a pharmaceutically acceptable salt thereof, or a solvate thereof.

35. A method for inhibition of a URAT1 activity, which comprises administering a pharmaceutically effective amount of the compound of any one of claim 2 to 19, or a pharmaceutically acceptable salt thereof, or a solvate thereof.

36. A method for lowering a blood uric acid level which comprises administering a pharmaceutically effective amount of the compound of the formula [1] of claim 1, or a pharmaceutically acceptable salt thereof, or a solvate thereof.

37. A method for lowering a blood uric acid level which comprises administering a pharmaceutically effective amount of the compound of any one of claims 2 to 19, or a pharmaceutically acceptable salt thereof, or a solvate thereof.

38. Use of the compound of the formula [1] of claim 1 or a pharmaceutically acceptable salt thereof, or a solvate thereof for the production of an agent for the treatment or prophylaxis of a pathology involving uric acid.

39. Use of the compound of any one of claims 2 to 19, or a pharmaceutically acceptable salt thereof, or a solvate thereof for the production of an agent for the treatment or prophylaxis of a pathology involving uric acid.

40. The use of claim 38 or 39, wherein the pathology involving uric acid is hyperuricemia, gouty tophus, acute gouty arthritis, chronic gouty arthritis, gouty kidney, urinary lithiasis, renal dysfunction, a coronary heart disease or an ischemic cardiac disease.

41. The use of claim 38 or 39, wherein the pathology involving uric acid is hyperuricemia, gouty tophus, acute gouty arthritis, chronic gouty arthritis or gouty kidney.

42. The use of claim 38 or 39, wherein the pathology involving uric acid is hyperuricemia.

43. Use of the compound of the formula [1] of claim 1 or a pharmaceutically acceptable salt thereof, or a solvate thereof for the production of a URAT1 activity inhibitor.

44. Use of the compound of any one of claims 2 to 19, or a pharmaceutically acceptable salt thereof, or a solvate thereof for the production of a URAT1 activity inhibitor.

45. Use of the compound of the formula [1] of claim 1 or a pharmaceutically acceptable salt thereof, or a solvate thereof for the production of a blood uric acid-lowering agent.

46. Use of the compound of any one of claims 2 to 19, or a pharmaceutically acceptable salt thereof, or a solvate thereof for the production of a blood uric acid-lowering agent.

47. A pharmaceutical composition for the treatment or prophylaxis of a pathology involving uric acid, which comprises the compound of the formula [1] of claim 1, or a pharmaceutically acceptable salt thereof, or a solvate thereof.

48. A pharmaceutical composition for the treatment or prophylaxis of a pathology involving uric acid, which comprises the compound of any one of claims 2 to 19, or a pharmaceutically acceptable salt thereof, or a solvate thereof.

49. The pharmaceutical composition of claim 47 or 48, wherein the pathology involving uric acid is hyperuricemia, gouty tophus, acute gouty arthritis, chronic gouty arthritis, gouty kidney, urinary lithiasis, renal dysfunction, a coronary heart disease or an ischemic cardiac disease.

50. The pharmaceutical composition of claim 47 or 48, wherein the pathology involving uric acid is hyperuricemia, gouty tophus, acute gouty arthritis, chronic gouty arthritis or gouty kidney.

51. The pharmaceutical composition of claim 47 or 48, wherein the pathology involving uric acid is hyperuricemia.

52. A commercial package comprising the pharmaceutical composition of any one of claims 47 to 49 and a written matter associated therewith, the written matter stating that the pharmaceutical composition can or should be used for the treatment or prophylaxis of a disease selected from hyperuricemia, gouty tophus, acute gouty arthritis, chronic gouty arthritis, gouty kidney, urinary lithiasis, renal dysfunction, a coronary heart disease and an ischemic cardiac disease.
